# EUROPEAN PATENT SPECIFICATION

(11) **EP 3 889 138 B1**
(45) Date of publication and mention of the grant of the patent: **20.11.2024**
(21) Application number: 19888999.0
(22) Date of filing: 27.11.2019
(51) Int. Cl.: C07D 235/02, C07D 263/52, C07D 269/02, C07D 277/60, C07D 283/02, A61P 35/00, A61K 31/381, A61K 31/4184, A61K 31/428

(54) **HISTONE ACETYLASE P300 INHIBITOR AND USE THEREOF**
HISTON-ACETYLASE-P300-INHIBITOR UND DESSEN VERWENDUNG
INHIBITEUR D'HISTONE ACÉTYLASE P300 ET UTILISATION ASSOCIÉE

(30) Priority: 27.11.2018 CN 201811427686
(43) Date of publication of application: 06.10.2021
(73) Proprietor: Hinova Pharmaceuticals Inc., Chengdu, Sichuan 610041 (CN)
(72) Inventor: FAN, Lei, Chengdu, Sichuan 610041 (CN); WANG, Fei, Chengdu, Sichuan 610041 (CN); WU, Xiaoquan, Chengdu, Sichuan 610041 (CN); XU, Kexin, Chengdu, Sichuan 610041 (CN); CHEN, Ke, Chengdu, Sichuan 610041 (CN); LUO, Tongchuan, Chengdu, Sichuan 610041 (CN); ZHANG, Shaohua, Chengdu, Sichuan 610041 (CN); DU, Wu, Chengdu, Sichuan 610041 (CN); ZHANG, Chengzhi, Chengdu, Sichuan 610041 (CN); HUO, Yongxu, Chengdu, Sichuan 610041 (CN); TU, Zhilin, Chengdu, Sichuan 610041 (CN); LI, Xinghai, Chengdu, Sichuan 610041 (CN); CHEN, Yuanwei, Chengdu, Sichuan 610041 (CN)
(74) Representative: Altmann Stößel Dick Patentanwälte PartG mbB
(86) International application number: PCT/CN2019/121086
(87) International publication number: WO 2020/108500

(56) References cited:
- WO-A1-2019/050924
- CN-A- 110 386 927
- US-A1- 2018 193 315
- SEGURA-CABRERA, ALDO: "Integrative Computational Protocol for the Di- scovery of Inhibitors of the Helicobacter Pylori Nickel Response Regulator (NikR", JOURNAL OF MOLECULAR MODELING, 1 March 2011 (2011-03-01), XP055540478, ISSN: 1610-2940
- LASKO, LOREN M.: "Discovery of a Selective Catalytic p300/CBP Inhibitor That Targets Lineage-Specific Tumours", NATURE, 5 October 2017 (2017-10-05), XP055575598, ISSN: 0028-0836, DOI: 10.1038/nature24028
- SHRIMP, JONATHAN: "Chemical Control of a CRISPR-Cas9 Acetyltransferase", ACS CHEMICAL BIOLOGY, 8 January 2018 (2018-01-08), XP055712820, ISSN: 1554-8937
- MICHAELIDES, MICHAEL R.: "Discovery of Spiro Oxazolidinediones as Sele- ctive, Orally Bioavailable Inhibitors of p300/CBP Histone Acetyltransfera- ses", ACS MEDICINAL CHEMISTRY LETTERS, 13 December 2017 (2017-12-13), XP055619926, ISSN: 1948-5875

## Description

### Technical field

The present invention belongs to the technical field of medicinal chemistry, and specifically relates to a class of histone acetylase p300 inhibitors, as well as its preparation and use.

### Background art

Post-translational modifications of histones, such as acetylation, phosphorylation, methylation, ADP-ribosylation, ubiquitination, etc., play an important role in the physiological and pathological processes of eukaryotic organisms. Among them, the acetylation modification of histones has received more and more attention in the research field of life science. The acetylation modification of histones has important biological functions in gene transcription regulation, cell differentiation, cell proliferation, cell cycle regulation, cell apoptosis, and the same. The acetylation modification of histones is accomplished by histone acetyltransferase (HAT, also known as histone acetylase). The histone acetyltransferase catalyzes the transfer of acetyl from Acetyl-CoA to the specific lysine epsilon-N. So far, the structures of many histone acetyltransferases have been determined and published. According to the sequence conservation and the distribution position, it can be divided into at least six large families: Gcn5/PCAF family, MYST family, p300/CBP family, nuclear receptor co-activator family, TAFII250 family and TFIIC family, etc.

The acetylation status of lysines at specific sites of histones is regulated and maintained by histone acetylase (HAT) and histone deacetylase (HDAC). The acetylation state of histones directly regulates the interaction between histone and DNA, thereby regulating the interaction between related enzymes in the process of DNA replication, transcription, repair and recombination and DNA. If the balance between both of them is disrupted, the acetylation state of histones regulated by the enzyme will be abnormal, which will seriously affect the life activities of the body and cause diseases such as malignant tumors, heart disease, diabetes and neurodegenerative disorders and so on.

Currently, in the histone acetyltransferase (HAT) family, p300 has become a research hotspot. Studies have shown that histone acetyltransferase p300 plays an important role in the occurrence of tumors. The expression of p300 gene is increased in some tumor cells, and the proliferation of tumor cells will be inhibited after the p300 gene is knocked out.

At present, there are more studies aiming at HDAC inhibitors, and even certain inhibitors are marketed as anti-cancer drugs. However, compared with HDAC, there are few studies on HAT inhibitors, especially drug-like inhibitors with high affinity and high selectivity have not yet been reported. As one of the two important antagonistic targets for balancing the *in vivo* acetylation level, the biological significance and research status of HAT provide us with a valuable opportunity to study new cancer treatment targets and develop novel drugs.

Segura et al, "Integrative computational protocol for the discovery of inhibitors of the Helicobacter pylori nickel response regulator (NikR)" J. Mol. Model (2011) pages 3075 to 384 discloses a discloses hydantoin spiro fused to an indene moiety, in which the hydantoin is N-substituted by CH₂CONH-phenyl, the phenyl being further substituted by triazole fused to cycloheptane that shows anti-bacterial activity. Lasko et al, "Discovery of a potent catalytic p300/CBP inhibitor that targets lineage-specific tumors" Nature Vol. 550, (2018) pages 128 to 132, discloses oxazolidindiones spiro fused to an indane ring which is substituted by urea, and in which the oxazolidindione nitrogen is substituted by amidomethyl, the amide nitrogen being substituted by benzyl and by CH(CH₃)CF₃ or CH(CH₃)-cyclopropyl that are P300 inhibitors useful for the treatment of cancer. Shrimp et al, "Chemical control of a CRISPR-Cas9 acetyltransferase" ACS Chem. Biol. Vol. 13 (2018) pages 455 to 460 discloses an imidazolidin-2,5-dione spiro fused to an indane ring having two fluorine substituents and a pyrazole substituent, and in which a ring nitrogen is substituted by amidomethyl, the amide being substituted by 4-fluorobenzyl and CH(CH₃)CF₃ that is a P300 inhibitor useful for the treatment of cancer. Michaelides et al, "Discovery of Spiro Oxazolidinediones as Selective, Orally Bioavailable Inhibitors of p300/CBP Histone Acetyltransferases" ACS Med. Chem. Lett. Vol.9 (2018) pages 28 to 33, discloses oxazolidindiones and imidazolidindiones spiro fused to an indane ring which may be substituted by groups including methylureido, and in which the ring nitrogen is substituted by amidomethyl, the amide being substituted by benzyl and CH(CH₃)CF₃ or CH(CH₃)cyclopropyl, that are P300 inhibitors useful for the treatment of cancer. WO2019050924A1 discloses imidazolidinedione spiro fused to an indane ring substituted by pyrazole and by fluorine, and in which the ring nitrogen is substituted by amidomethyl, the amide being substituted by benzyl and CH(CCH₃)CF₃ and having histone acetylase inhibiting activity for the treatment of cancer, said compound can be administered with another anticancer agent which can be a CDK4/6 inhibitor. WO2019201291A1 discloses oxazolidindiones and imidazolidiendiones spiro fused to an indane ring which is substituted by methylureido or pyrazolyl, and in which the ring nitrogen is substituted by amidomethyl, the amide nitrogen being part of a heterocyclic ring, said compounds having histone acetylase inhibiting activity for the treatment of cancer. US2018071262A1 discloses oxazolidindiones spiro fused to an indane ring and in which the oxazolidinedione nitrogen is substituted by amido methyl, the amide being substituted by alkyl groups said compounds having histone acetylase inhibiting activity for the treatment of cancer.

Due to the special biological function of p300/CBP, the research on HAT mainly focuses on the inhibitors of histone acetylase p300. Therefore, the development of a histone acetylase p300 inhibitor with high affinity and high selectivity is of great significance for the treatment of cancer.

### Content of the invention

The object of the present invention is to provide a class of histone acetylase p300 inhibitors, as well as its preparation and use.

The present invention provides a compound of formula (II) or a stereoisomer, a solvate or a pharmaceutically acceptable salt, or an isotope-substituted form thereof: 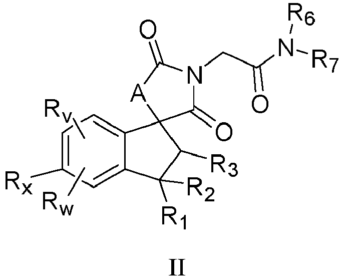
wherein, Rₓ is selected from 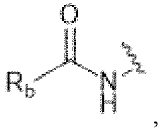 amino, 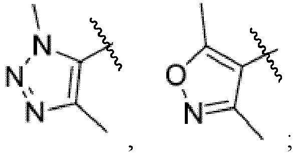 R_{b} is selected from methyl, halomethyl, -YRₐ; Rₐ is selected from methyl and cyclopropyl; Y is selected from NH or O;
Rv and Rw are independently selected from the group consisting of H, halogen, methyl;
R₁ is selected from H, F, and CH₃; R₂ is selected from H, F, and CH₃; R₃ is H; or, R₁ and R₂ are both CH₂ and linked to form three-membered ring; or, R₂ is CH₂, R₃ is H, and linked to form three-membered ring;
A is O or S;
R₆ and R₇ are linked to form heterocyclic ring or substituted heterocyclic ring; said heterocyclic ring and substituted heterocyclic ring are 4 to 6 membered ring
wherien
the heterocyclic ring or substituted heterocyclic ring formed by R₆ and R₇ is selected from 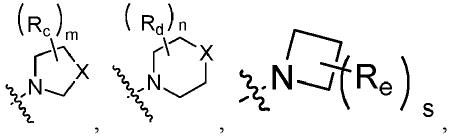 substituted or unsubstituted bridged ring, substituted or unsubstituted fused ring, and substituted or unsubstituted parallel ring, wherein X is CH₂, NH, O, S, or SO₂; each of m, n, and s is independently selected from an integer of 1 to 5; each of R_{c}, R_{d}, and Rₑ is independently selected from the group consisting of H, halogen, cyano, carboxyl, nitro, alkyl, substituted alkyl, alkoxyl, alkenyl, alkynyl, 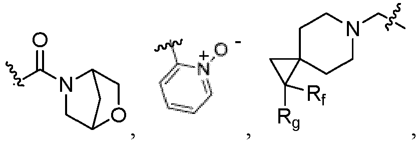 cycloalkyl, substituted cycloalkyl, heterocyclyl, substituted heterocyclyl, aryl, substituted aryl, heteroaryl, substituted heteroaryl, bridged ring, fused ring and parallel ring; R_{f} and R_{g} are halogen;
each of the substituents in said bridged ring, fused ring or parallel ring is independently selected from Boc group, fluorinated C₁₋₆ alkyl, substituted or unsubstituted heterocyclyl, and alkanoyl, preferably selected from Boc group, fluoromethyl, 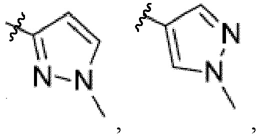 and -COCH₃;
each of the subsitutents in said R_{c}, R_{d}, and Rₑ is independently selected from the group consisting of halogen, C₁₋₆ alkyl, halogenated C₁₋₆ alkyl, C₁₋₆ alkoxyl, halogenated C₁₋₆ alkoxyl, and hydroxyl.

Further,
the heterocyclic ring or substituted heterocyclic ring formed by linkage of said R₆ and R₇ is 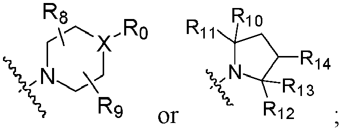
wherein, X is C, N, O, S, or SO₂; R₈ and R₉ are independently selected from the group consisting of H, alkyl, substituted alkyl, alkoxyl, alkenyl, alkynyl, cycloalkyl, substituted cycloalkyl, heterocyclyl, substituted heterocyclyl, aryl, substituted aryl, aromatic heterocyclyl, and substituted aromatic heterocyclyl;
R₀ is none, H or alkoxyl; or, R₈ and R₉ are linked to form a fused ring or bridged ring; R₁₀, R₁₁, R₁₂, R₁₃, and R₁₄ are independently selected from the group consisting of H, halogen, cyano, carboxyl, nitro, alkyl, substituted alkyl, cycloalkyl, substituted cycloalkyl, heterocyclyl, substituted heterocyclyl, aryl, substituted aryl, aromatic heterocyclyl, and substituted aromatic heterocyclyl; or, R₁₀ and R₁₁ are linked to form a ring;
each of the substituents in said R₈, R₉, R₁₀, R₁₁, R₁₂, R₁₃, and R₁₄ is independently selected from the group consisting of halogen, C₁₋₆ alkyl, halogenated C₁₋₆ alkyl, C₁₋₆ alkoxyl, halogenated C₁₋₆ alkoxyl, and hydroxyl.

Further,
X is C or O; R₈ and R₉ are independently selected from the group consisting of H, alkyl, substituted alkyl, aryl, substituted aryl, aromatic heterocyclyl, and substituted aromatic heterocyclyl;
R₁₀, R₁₁, R₁₂, R₁₃, and R₁₄ are independently selected from the group consisting of H, cyano, carboxyl, alkyl, alkenyl, substituted alkyl, cycloalkyl, substituted cycloalkyl, heterocyclyl, substituted heterocyclyl, aryl, substituted aryl, aromatic heterocyclyl, andsubstituted aromatic heterocyclyl; or, R₁₀ and R₁₁ are linked to form a ring when both of them are alkyl;
the substituents in said R₈, R₉, R₁₀, R₁₁, R₁₂, R₁₃, and R₁₄ are as described above.

Further,
R₈ and N are linked to the same carbon atom and is selected from phenyl and substituted phenyl; R₉ is selected from H, alkyl, and substituted alkyl;
R₁₀ and R₁₁ are independently selected from the group consisting of H, C₁₋₆ alkyl, cyano, carboxyl, substituted alkyl, C₃₋₆ cycloalkyl, and C₂₋₆ alkenyl; or, R₁₀ and R₁₁ are both CH₂, and linked to form three-membered ring;
R₁₂ and R₁₃ are independently selected from the group consisting of H, methyl, phenyl, substituted phenyl, heteroaryl, substituted heteroaryl, cycloalkyl and substituted cycloalkyl; R₁₄ is selected from H and phenyl;
the substituents in said R₈, R₉, R₁₀, R₁₁, R₁₂, R₁₃, and R₁₄ are as described above.

Further,
A is O.

Further,
said compound has a structure of formula III: 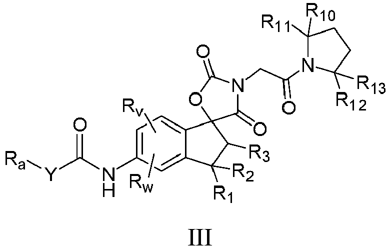
ewherein, Ra is methyl or cyclopropyl;
Y is NH or O;
Rᵥ and R_{w} are independently selected from the group consisting of H, halogen, and methyl;
R₁ is selected from H, F, and CH₃; R₂ is selected from H, F, and CH₃; R₃ is H; or, R₁ and R₂ are both CH₂ and linked to form three-membered ring; or, R₂ is CH₂, R₃ is H, and linked to form three-membered ring;
R₁₀ and R₁₁ are independently selected from the group consisting of H, C₁₋₆ alkyl, cyano, carboxyl, substituted C₁₋₆ alkyl, C₃₋₆ cycloalkyl, and C₂₋₆ alkenyl; the substituent in said C₁₋₆ alkyl is selected from halogen, hydroxyl, and C₁₋₆ alkyl; or, R₁₀ and R₁₁ are both CH₂, and linked to form three-membered ring; preferably, R₁₀ and R₁₁ are independently selected from the group consisting of H, methyl, ethyl, isopropyl, cyano, carboxyl, halogenated methyl, cyclopropyl, vinyl, methoxy-substituted methyl, and hydroxy-substituted methyl; or, R₁₀ and R₁₁ are both CH₂, and linked to form three-membered ring;
R₁₂ and R₁₃ are independently selected from the group consisting of H, methyl, phenyl, substituted phenyl, heteroaryl, substituted heteroaryl, cycloalkyl and substituted cycloalkyl; said cycloalkyl being a 5 to 6 membered cycloalkyl;
each of the substituents in said substituted phenyl, substituted heteroaryl, substituted cycloalkyl is independently selected from halogen, C₁₋₃ alkyl, halogenated C₁₋₃ alkyl, C₁₋₃ alkoxyl, halogenated C₁₋₃ alkoxyl, and hydroxy;
the isotopic substitution form is deuterated.

Further,
the structure of said compound is one of the following:

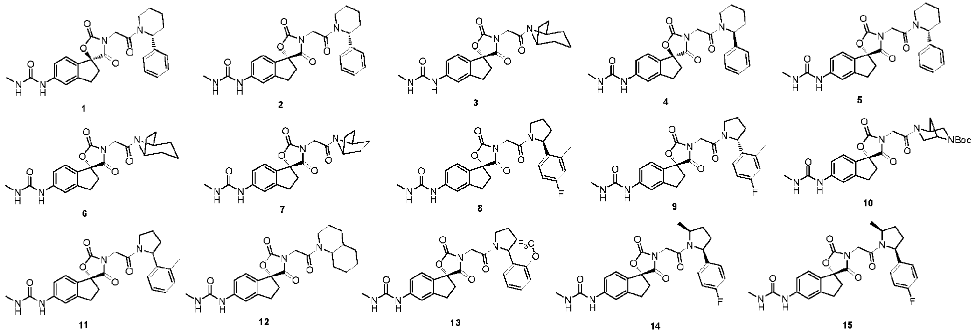

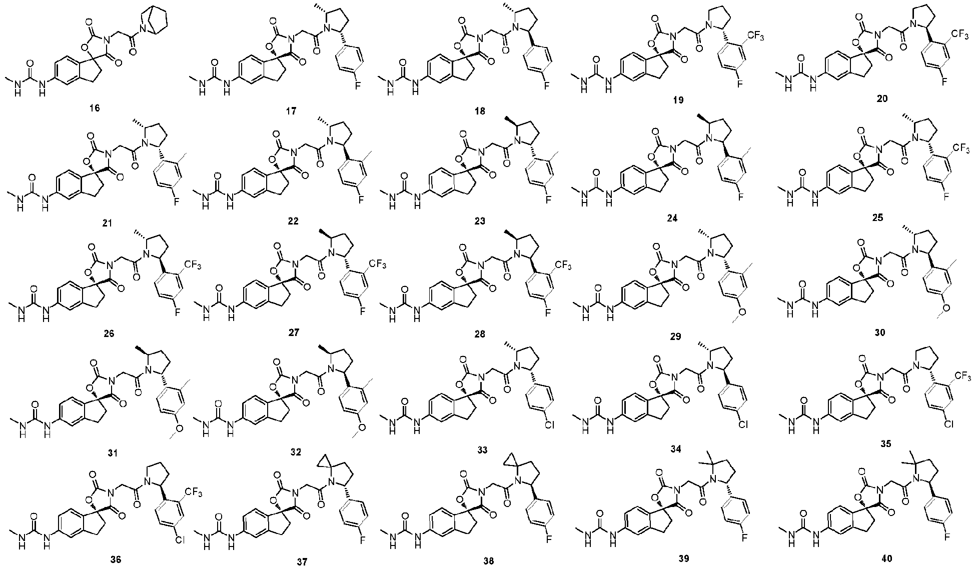

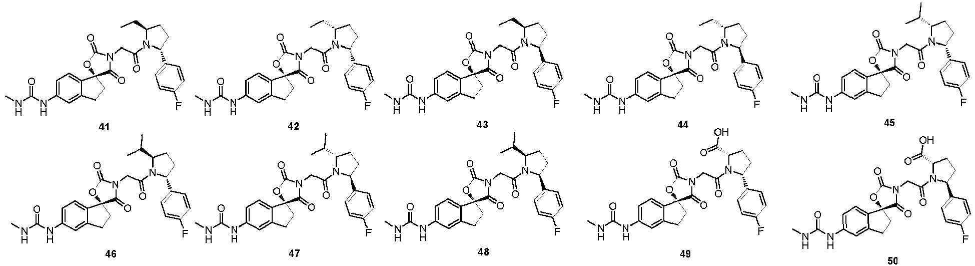

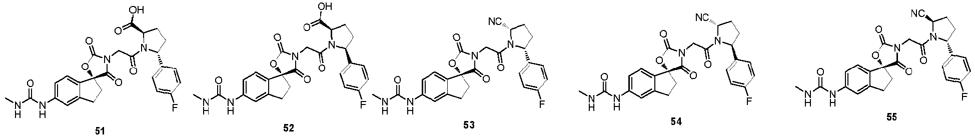

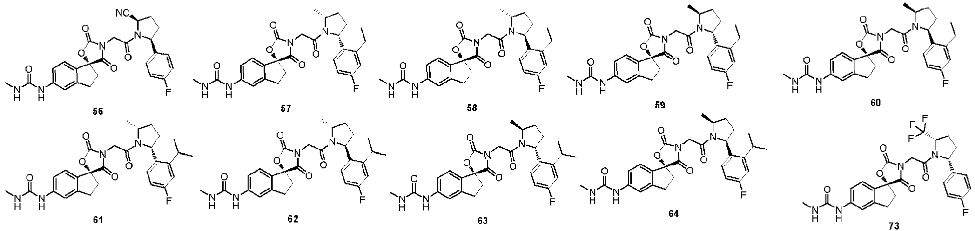

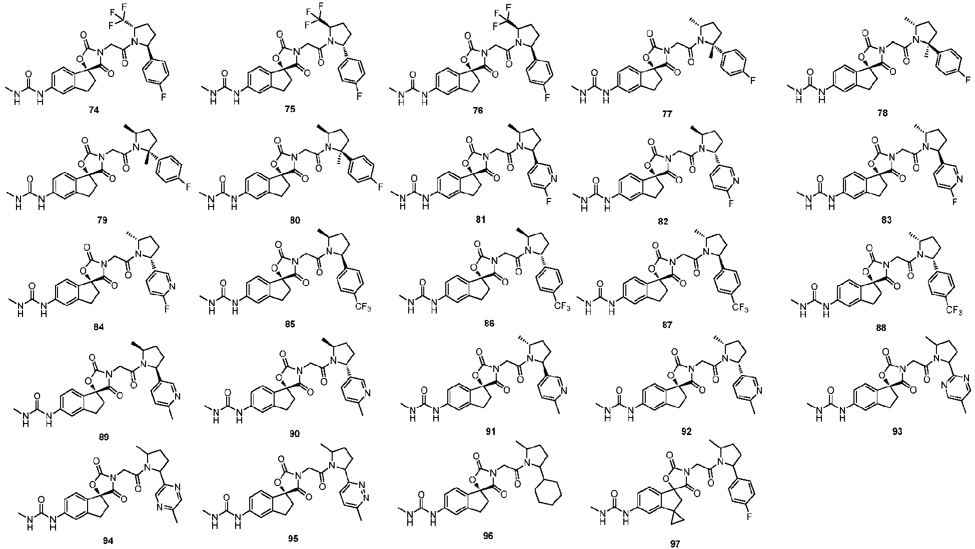

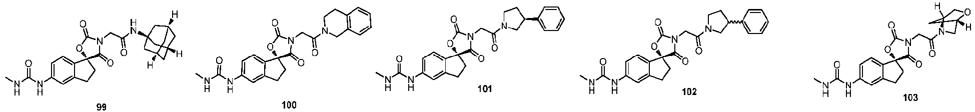

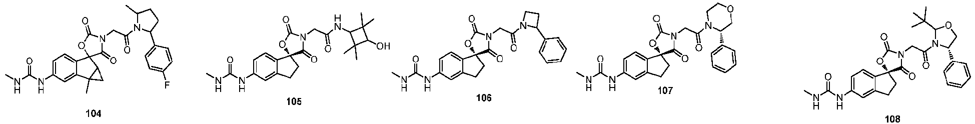

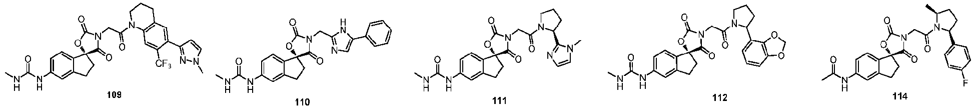

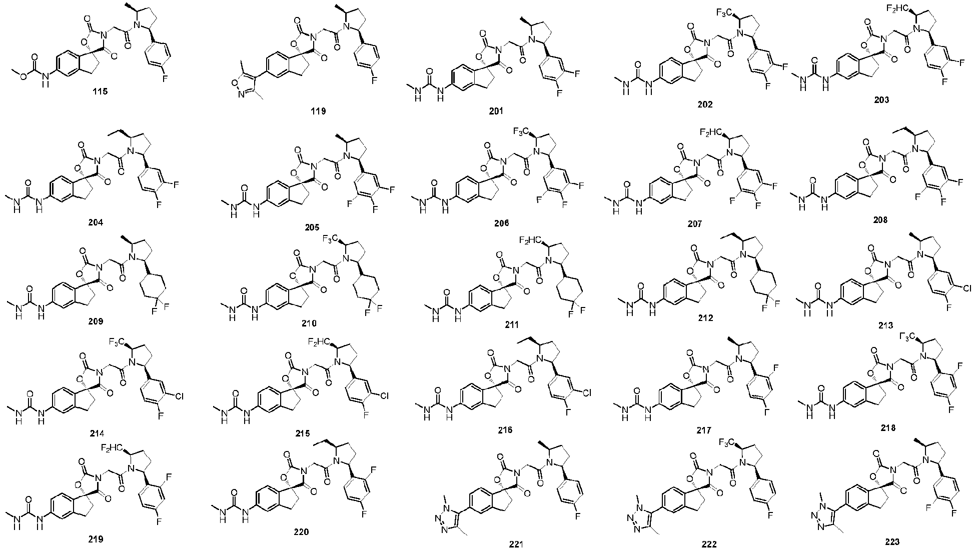

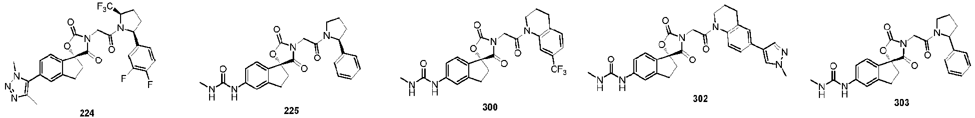

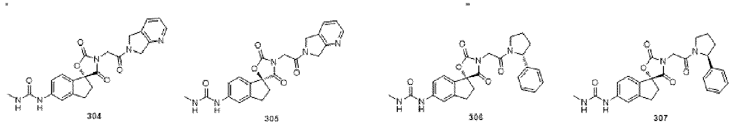

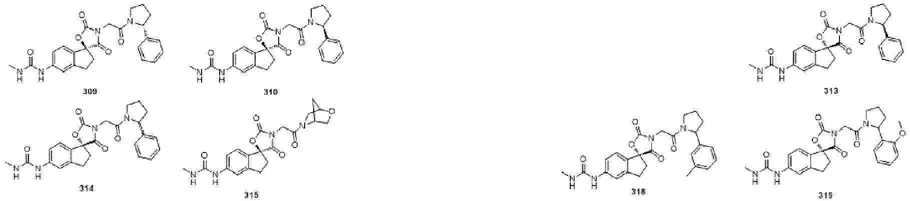

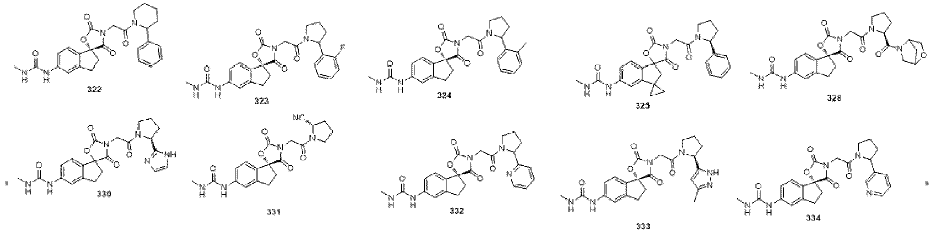

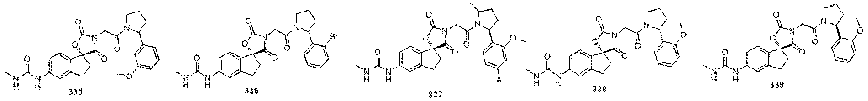

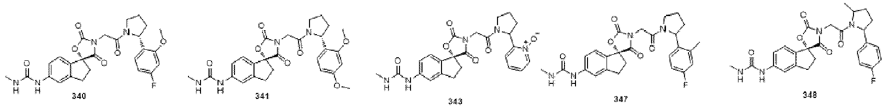

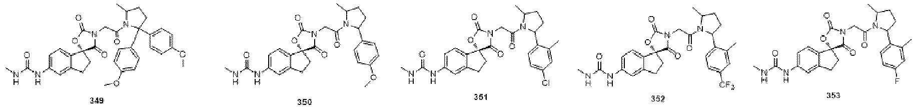

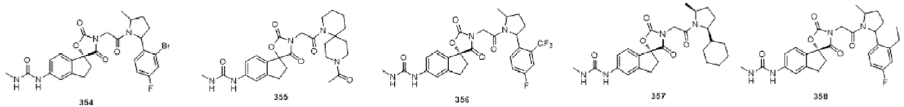

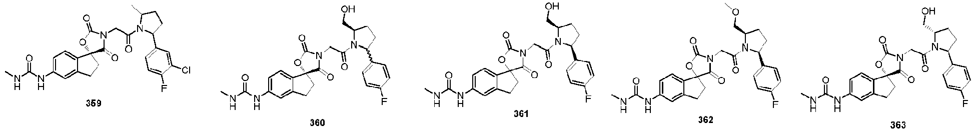

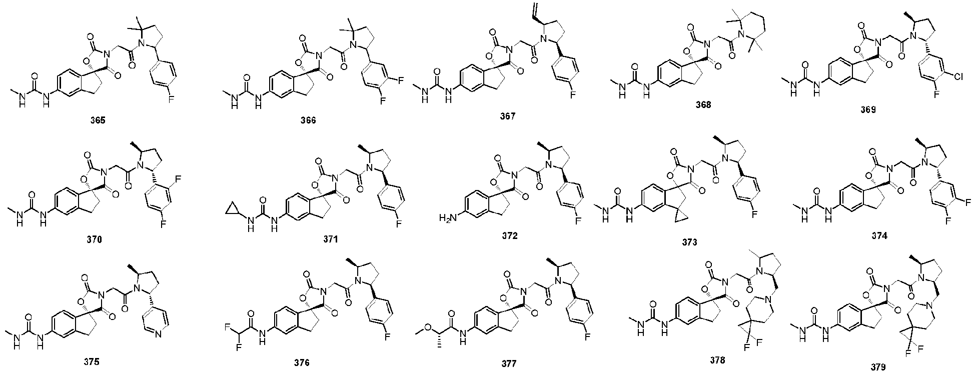

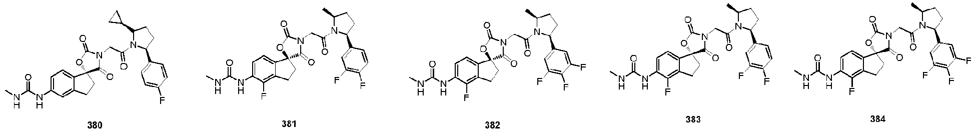

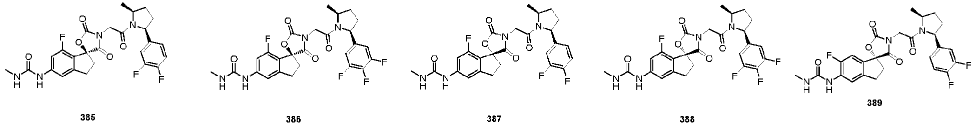

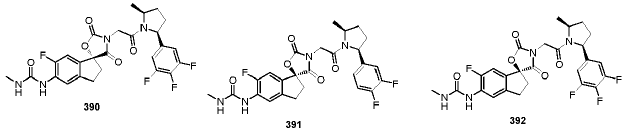

The present invention further provides a method for preparing the above compounds, and said method is: 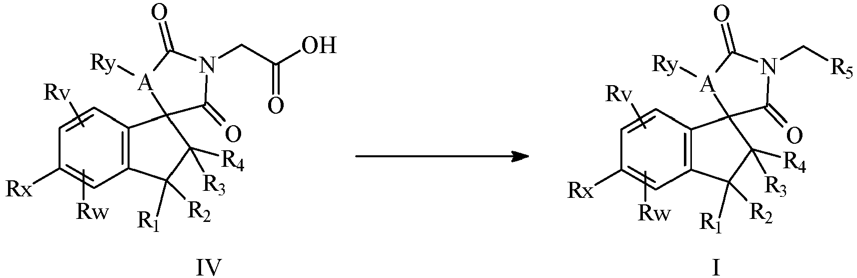
using compound of formula IV and 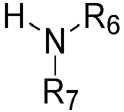 as starting materials to react, to obtain the product;
wherein, A, Ry, Rv, Rw, Rx, R₁, R₂, R₃, R₄, R₅ are as described above, while R₆ and R₇ are as described above.

Further,
the reaction temperature is in the range of 15 to 30 °C, and the reaction time is in the range of 0.5 to 2 hours; preferably, the reaction temperature is 20 °C, and the reaction time is 1 h;
the reaction is carried out under the action of DIEA and HATU, and the molar ratio of compound of formula IV, 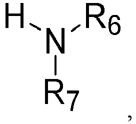 DIEA, and HATU is 1:1:3:1.

The present invention also provides a use of the compound mentioned above or a stereoisomer, a solvate or a pharmaceutically acceptable salt, or an isotope-substituted form thereof in the preparation of histone acetylase inhibitors.

Further,
said histone acetylase is p300.

Further, the histone acetylase inhibitor is a drug for the treatment of cancer, metabolic diseases, neurological diseases and/or inflammation; preferably, the cancer is prostate cancer, leukemia, lymphoma, breast cancer or multiple myeloma.

The present invention also provides a pharmaceutical composition, that is a preparation prepared by using the compound mentioned above or a stereoisomer, a solvate or a pharmaceutically acceptable salt, or an isotope-substituted form thereof as active ingredient, with the addition of pharmaceutically acceptable excipients.

The present invention also provides a combination drug, that contains the same or different specification of unit preparations of the compound mentioned above and anticancer drug for simultaneous or separated administration, as well as pharmaceutically acceptable carriers.

Further, the anticancer drug is a CDK4/6 inhibitor; preferably, the CDK4/6 inhibitor is palbociclib.

In the present invention, "isotope-substituted form" denotes the compound obtained by replacing one or more atoms in a compound with its corresponding isotope, such as the hydrogen in a compound is replaced with protium, deuterium or tritium.

Experiments have shown that the compound prepared in the present invention can effectively inhibit histone acetylase p300, and thus inhibit the proliferation of cancer cells (including prostate cancer cells, leukemia cells, lymphoma cells, breast cancer cells, multiple myeloma cells, etc.). Said compound has very good application prospects in the preparation of histone acetylase p300 inhibitors and drugs for the treatment of cancer. Meanwhile, the combination of the compound according to the present invention and CDK4/6 inhibitor creates a synergistic effect on inhibiting the proliferation of cancer cells, and has a very important value in the preparation of a drug combination.

Based on the above content of the present invention, according to the common technical knowledge and the conventional means in the field, without department from the above basic technical spirits, other various modifications, alternations or changes can further be made.

### Examples

By the following specific examples and experimental examples of said embodiments, the above content of the present invention is further illustrated. But it should not be construed that the scope of the above subject matter of the present invention is limited to the following examples. The techniques realized based on the above content of the present invention are all within the scope of the present invention.

Unless otherwise indicated, the reagents and test equipment used in the present invention are all conventional and commercially available reagents and equipment.

### Synthesis of intermediate compounds (R)-5-bromo-2,3-dihydrospiro[indene-1,5'-oxazolidine]-2',4'-dione (Int 1-4) and (S)-5-bromo-2,3-dihydrospiro[indene-1,5'-oxazolidine]-2',4'-dione (Int 1-5)

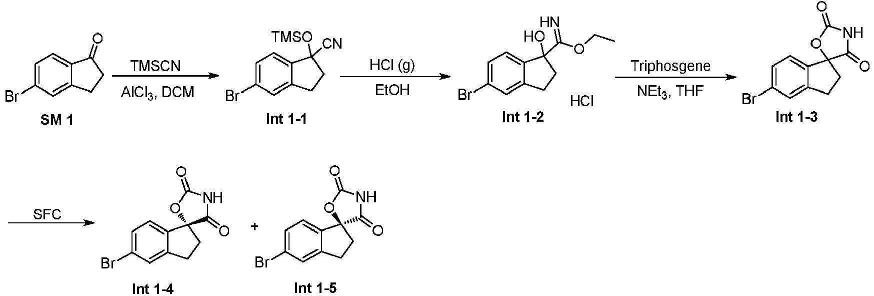

### Synthesis of 5-bromo-1-((trimethylsilyl)oxy)-2,3-dihydro-1H-indene-1-carbonitrile (Int 1-1)

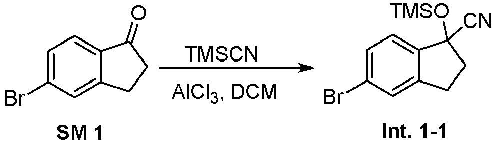

To a 100 mL reaction flask, were added AlCl₃ (1.26 g, 9.4 mmol), 5-bromoindanone (10 g, 47 mmol), and 100 mL of dry dichloromethane, to which was slowly added trimethylsilyl cyanide (9.4 g, 94 mmol) in an ice bath. The mixture was then stirred at room temperature for 5 h. The reaction solution was poured into a 200 mL saturated KHCO₃ aqueous solution, and extracted with dichloromethane three times. The organic phases were combined, dried over anhydrous sodium sulfate, and concentrated. After column chromatography, white intermediate **Int 1-1** (10 g) was obtained, with a yield of 68%. MS: *m*/*z* 310, 312 [M+H]⁺. ¹H NMR (400 MHz, DMSO-d6) δ 7.60 (dq, J = 1.6, 0.8 Hz, 1H), 7.54 (ddt, J = 8.1, 1.6, 0.8 Hz, 1H), 7.44 (d, J = 8.1 Hz, 1H), 3.11 - 3.02 (m, 1H), 3.01 - 2.91 (m, 1H), 2.72 (ddd, J = 13.5, 7.8, 5.8 Hz, 1H), 2.40 (ddd, J= 13.4, 7.8, 5.6 Hz, 1H), 0.16 (s, 9H).

### Synthesis of 5-bromo-1-hydroxyl-2,3-dihydro-1H-indene-1-carboximidic acid ethyl ester (Int 1-2)

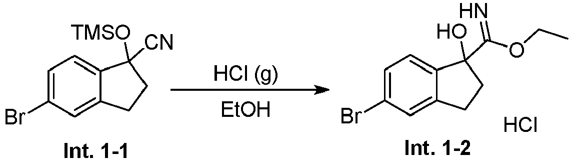

Intermediate **Int. 1-1** (10 g, 32.3 mmol) was dissolved in 60 mL of dry ethanol, and then HCl gas (home-made, dry) was led in. The reaction was detected by TLC, and after completion of the reaction, the solution was directly concentrated to obtain 9.6 g crude product as light yellow solid, that was directly used in the next step.

### Synthesis of 5-bromo-2,3-dihydrospiro[indene-1,5'-oxazolidine]-2',4'-dione (Int. 1-3):

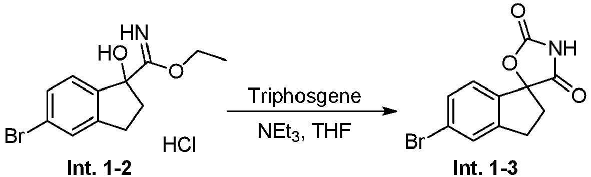

Intermediate **Int. 1-2** (9.6 g) was suspended in dry THF (120 mL), to which was added triethylamine (20 mL, 5 e.q.) in an ice bath, and then added triphosgene (3.8 g, 0.4 e.q.) in batches. The mixture was stirred in the ice bath for additional 2 h, and then slowly adjusted to pH<5 with HCl (2 N, a.q.). After stirring for 1 h, the reaction solution was extracted with EA, dried over anhydrous sodium sulfate, and concentrated, followed by pulping in EA/PE=1/1, to obtain white intermediate **Int. 1-3** (5.3 g), with a yield of 58.3% for two steps. MS: *m*/*z* 282, 284 [M+H]⁺.

### Synthesis of (R)-5-bromo-2,3-dihydrospiro[indene-1,5'-oxazolidine]-2',4'-dione (Int. 1-4) and (S)-5-bromo-2,3-dihydrospiro[indene-1,5'-oxazolidine]-2',4'-dione (Int. 1-5):

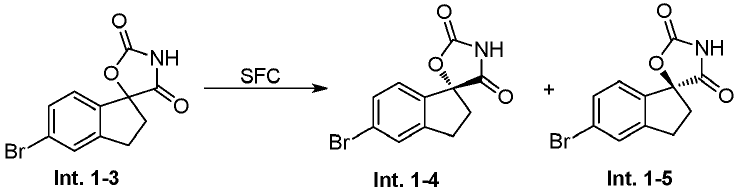

Chiral separation conditions: apparatus: SFC-80 (Thar, Waters); chiral separation column: CHIRALCEL AD (30*250mm, 5µm) (Daicel); column temperature: 35°C; mobile phase: A=CO₂, B= MeOH; peak time: t₁ = 12.1 min, t₂ = 15 min.

After chiral separation of intermediate Int. **1-3** (5 g), intermediate **Int. 1-4** was obtained (t, = 12.1min, 2.3 g), e.e = 99 %, [α]₂₀^{D} = + 28° (c 1.0, MeOH), ¹H NMR (400 MHz, DMSO-d₆): δ 12.18 (s, 1H), 7.71 (d, J = 1.9 Hz, 1H), 7.61 (m, 1H), 7.44-7.28 (m, 1H), 3.14-3.02 (m, 1H), 3.02-2.90 (m, 1H), 2.66 (m, 1H), 2.59-2.39 (m, 1H); Intermediate Int. 1-5 (t₂ = 15 min, 2.3g), e.e = 99%, [α]₂₀^{D} = -30° (c 1.0, MeOH), ¹H NMR (400 MHz, DMSO-d₆): δ 12.18 (s, 1H), 7.71 (d, J = 1.9 Hz, 1H), 7.61 (m, 1H), 7.44-.28 (m, 1H), 3.14-3.02 (m, 1H), 3.02-2.90 (m, 1H), 2.66 (m, 1H), 2.59-2.39 (m, 1H).

### Synthesis of general intermediate compound 2-(5-(3-methylureido)-2',4'-dioxo-2,3-dihydrospiro[indene-1,5'-oxazolidine]-3'-yl)acetic acid (Int. 1-10)

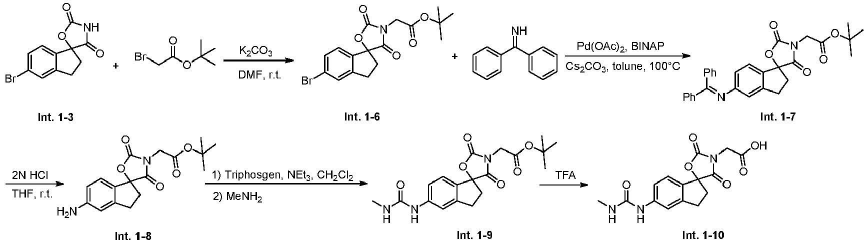

### Synthesis of 2-(5-bromo-2',4'-dioxo-2,3-dihydrospiro[indene-1,5'-oxazolidine]-3'-yl) acetic acid t-butyl ester (Int. 1-6):

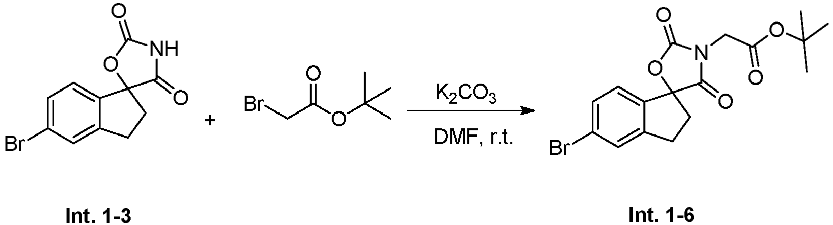

Intermediate **Int. 1-3** (10 g, 35.4 mmol), tert-butyl bromoacetate (7.5 g, 38.4 mmol), and potassium carbonate (7.3 g, 52.9 mmol) were added in 100 mL of DMF, and then stirred at room temperature for 4 h, to which was added 200 mL water, followed by extracting with ethyl acetate three times. The organic phase was combined and dried over anhydrous Na₂SO₄, and concentrated. The residue was swirled in petroleum ether and filtered, to obtain white solid (12 g), with a yield of 86%. MS: *m*/*z* 396, 398 [M+H]⁺.

### Synthesis of 2-(5-((diphenylmethylene)amino)-2',4'-dioxo-2,3-dihydrospiro[indene- 1,5'-oxazolidine]-3'-yl)acetic acid t-butyl ester (Int. 1-7):

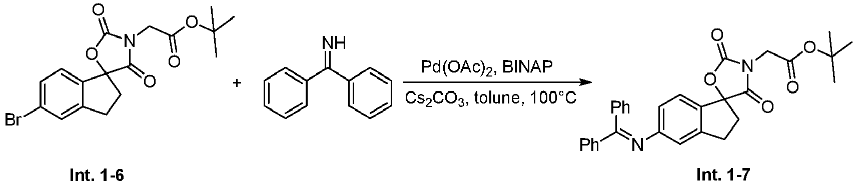

Intermediate **Int. 1-3** (11.5 g, 29 mmol), benzophenonimine (7 g, 38 mmol), palladium acetate (260 mg, 1.16 mmol), 2,2'-bis(diphenylphosphino)-1,1'-binaphthyl (1.61 g, 2.58 mmol), and cesium carbonate (13.2 g, 40 mmol) were added in 50 mL of toluene, and heated to 100 °C under N₂ atmosphere. The reaction was stirred for 4 h, concentrated, and subjected to column chromatography to obtain grey solid (12 g), with a yield of 83.1%. MS: *m*/*z* 497 [M+H]⁺.

### Sythesis of 2-(5-amino-2',4'-dioxo-2,3-dihydrospiro[indene-1,5'-oxazolidine]-3'-yl) acetic acid t-butyl ester (Int. 1-8):

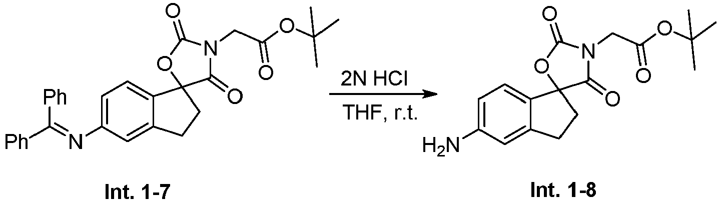

Intermediate **Int. 1-7** (12 g, 24 mmol) was dissolved in 20 mL tetrahydrofuran, to which was added 10 mL HCl (2 N) at room temperature, and the mixture was stirred for 30 min, followed by extracting with ethyl acetate three times. The organic phase was combined, dried over anhydrous Na₂SO₄, concentrated, and purified by column chromatography to obtain yellow solid (6.9 g), with a yield of 86%. MS: *m*/*z* 289 (M-43).

### Synthesis of 2-(5-(3-methylureido)-2',4'-dioxo-2,3-dihydrospiro[indene-1,5'- oxazolidine]-3'-yl)acetic acid t-butyl ester (Int. 1-9):

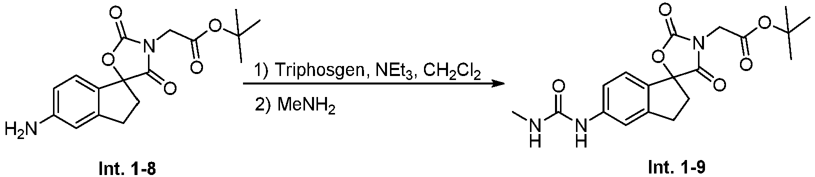

Triphosgene (3.5 g, 12 mmol) was placed in dichloromethane (25 mL). The solution of intermediate **Int. 1-8** (6.9 g, 20 mmol) and triethylamine (20 g, 200 mmol) in dichloromethane (25 mL) was slowly dropped into the reaction flask in an ice bath. Then, the mixture was stirred for 1 h, to which was added methylamine hydrochloride (7 g, 104 mmol), and the reaction was further stirred for 4 h. 100 mL of water was added, the organic layer was separated, and then extracted with dichloromethane. The organic phases were combined, dried over anhydrous sodium sulfate, concentrated, and purified by column chromatography, to obtain yellow solid (5 g), with a yield of 62%. MS: *m*/*z* 390 [M+H]⁺.

### Synthesis of 2-(5-(3-methylureido)-2',4'-dioxo-2,3-dihydrospiro[indene-1,5'- oxazolidine]-3'-yl)acetic acid (Int. 1-10):

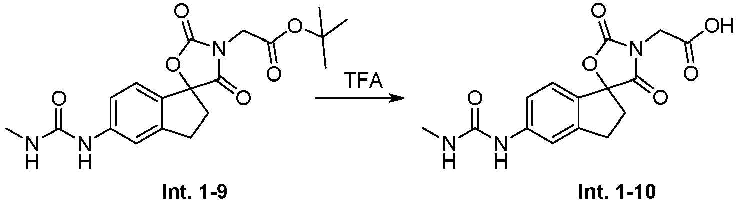

Intermediate **Int. 1-9** (5 g, 13 mmol) was dissolved in 5 mL of trifluoroacetic acid, stirred at room temperature overnight, and concentrated to obtain light yellow solid (4.1 g), with a yield of 96%. ¹H NMR (400 MHz, DMSO-d₆): δ 13.52 (s, 1H), 8.78 (s, 1H), 7.53 (s, 1H), 7.31-7.25 (m, 1H), 7.19-7.11(m, 1H), 6.12 (s, 1H), 4.38-4.18 (m, 2H), 3.18-2.98 (m, 2H), 2.71-2.57 (m, 4H), 2.55-2.41 (m, 1H).

### Synthesis of general intermediate compound (R)-2-(5-(3-methylureido)-2',4'-dioxo- 2,3-dihydrospiro[indene-1,5'-oxazolidine]-3'-yl)acetic acid (Int 1-15)

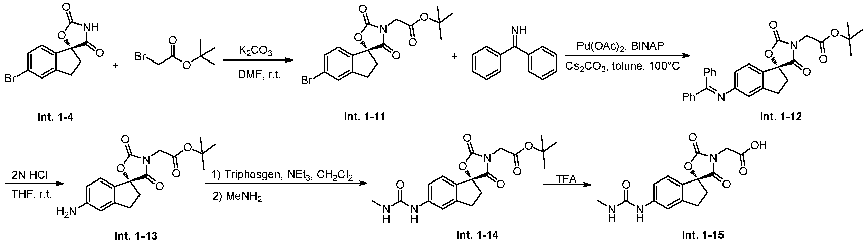

### Synthesis of (R)-2-(5-bromo-2',4'-dioxo-2,3-dihydrospiro[indene-1,5'-oxazolidine]- 3'-yl)acetic acid t-butyl ester (Int. 1-11):

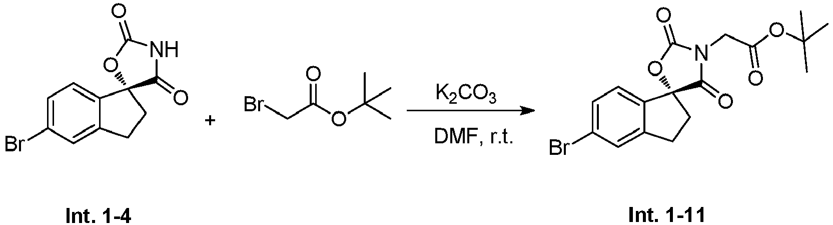

Intermediate **Int.1-4** (10 g, 35.4 mmol), tert-butyl bromoacetate (7.5 g, 38.4 mmol), and potassium carbonate (7.3 g, 52.9 mmol) were dissolved in 100 mL of DMF, and stirred at room temperature for 4 h. 200 mL water was added, and then extracted with ethyl acetate three times. The organic phase was combined, dried over anhydrous Na₂SO₄, and concentrated. The residue was swirled in petroleum ether and filtered, to obtain white solid (12.2 g), with a yield of 88%. MS: *m*/*z* 396, 398 [M+H]⁺.

### Synthesis of (R)-2-(5-((diphenylmethylene)amino)-2',4'-dioxo-2,3-dihydrospiro [indene-1,5'-oxazolidine]-3'-yl)acetic acid t-butyl ester (Int. 1-12):

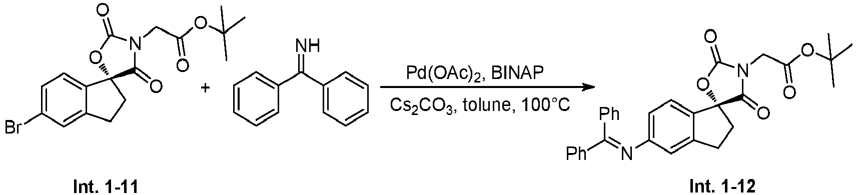

Intermediate **Int. 1-11** (11.5 g, 29 mmol), benzophenonimine (7 g, 38 mmol), palladium acetate (260 mg, 1.16 mmol), 2,2'-bis(diphenylphosphino)-1,1'-binaphthyl (1.61 g, 2.58 mmol), and cesium carbonate (13.2 g, 40 mmol) were dissolved in 50 mL toluene, and then N₂ was purged. The reaction was heated to 100 °C and stirred for 4 h. Then, the reaction was concentrated and purified by column chromatography to obtain 12.2 g grey solid, with a yield of 85%. MS: *m*/*z* 497 [M+H]⁺.

### Synthesis of (R)-2-(5-amino-2',4'-dioxo-2,3-dihydrospiro[indene-1,5'-oxazolidine]-3'-yl)acetic acid t-butyl ester (Int. 1-13):

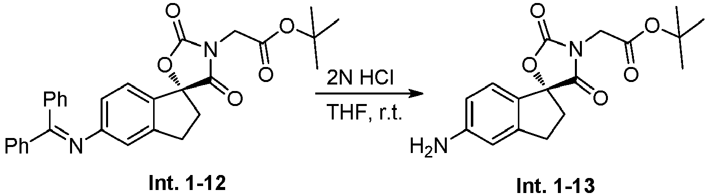

Intermediate **Int. 1-12** (12 g, 24 mmol) was dissolved in 20 mL of tetrahydrofuran, to which was added 10 mL of HCl (2 N) at room temperature, and then stirred for 30 min, followed by extracting with ethyl acetate three times. The organic phase was combined, dried over anhydrous Na₂SO₄, concentrated, and purified by column chromatography to obtain yellow solid (7.1 g), with a yield of 88%. MS: *m*/*z* 289 [M-43].

### Synthesis of (R)-2-(5-(3-Methylureido)-2',4'-dioxo-2,3-dihydrospiro[indene-1,5'-oxazolidine]-3'-yl)acetic acid t-butyl ester (Int. 1-14):

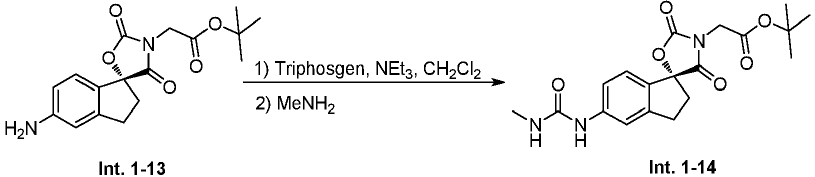

Triphosgene (3.5 g, 12 mmol) was placed in dichloromethane (25 mL). The solution of intermediate **Int. 1-13** (6.9 g, 20 mmol) and triethylamine (20 g, 200 mmol) in dichloromethane (25 mL) was slowly dropped into the reaction flask in an ice bath. Then, the mixture was stirred for 1 h, to which was added methylamine hydrochloride (7 g, 104 mmol), and the reaction was further stirred for 4 h. 100 mL of water was added, the organic layer was separated, and then extracted with dichloromethane. The organic phases were combined, dried over anhydrous sodium sulfate, concentrated, and purified by column chromatography, to obtain yellow solid (5.2 g), with a yield of 64%. MS: *m*/*z* 390 [M+H]⁺.

### Synthesis of (R)-2-(5-(3-methylureido)-2',4'-dioxo-2,3-dihydrospiro[indene-1,5'-oxazolidine]-3'-yl)acetic acid (Int. 1-15):

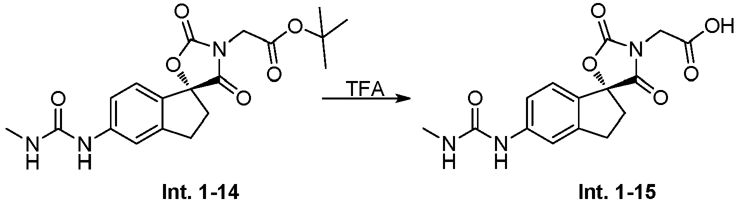

Intermediate **Int. 1-14** (5 g, 13 mmol) was dissolved in 5 mL of trifluoroacetic acid, stirred at room temperature overnight, and concentrated to obtain light yellow solid (4.2 g), with a yield of 98%. MS: *m*/*z* 334 [M+H]⁺; ¹H NMR (400 MHz, DMSO): *δ* 13.52 (s, 1H), 8.78 (s, 1H), 7.53 (s, 1H), 7.31-7.25 (m, 1H), 7.19-7.11(m, 1H), 6.12 (s, 1H), 4.38-4.18 (m, 2H), 3.18-2.98 (m, 2H), 2.71-2.57 (m, 4H), 2.55-2.41 (m, 1H). [α]₂₀^{D}= + 53.3° (c 1.0, MeOH)

### Synthsis of general intermediate compound (S)-2-(5-(3-methylureido)-2',4'-dioxo- 2,3-dihydrospiro[indene-1,5'-oxazolidine]-3'-yl)acetic acid (Int 1-20)

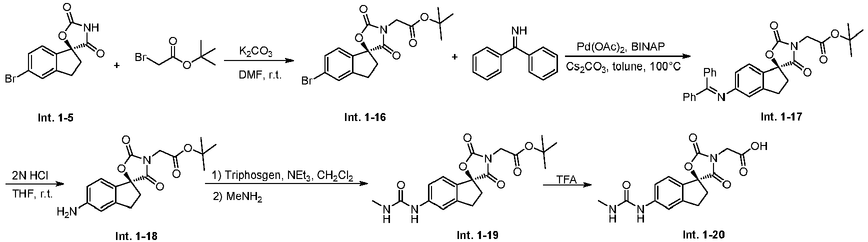

### Synthesis of (S)-2-(5-bromo-2',4'-dioxo-2,3-dihydrospiro[indene-1,5'-oxazolidine]- 3'-yl)acetic acid t-butyl ester (Int. 1-16):

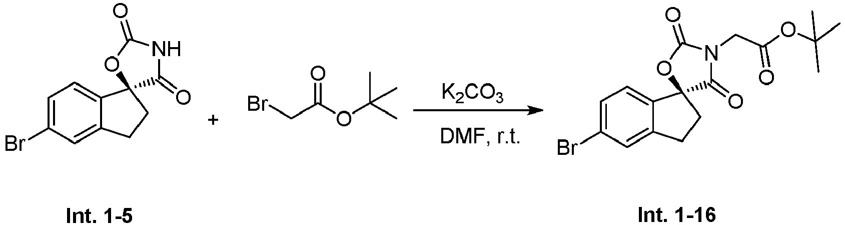

Intermediate **Int.1-5** (10 g, 35.4 mmol), tert-butyl bromoacetate (7.5 g, 38.4 mmol), and potassium carbonate (7.3 g, 52.9 mmol) were dissolved in DMF (100 mL), and stirred at room temperature for 4 h. 200 mL of water was added, and then extracted with ethyl acetate three times. The organic phase was combined, dried over anhydrous Na₂SO₄, and concentrated. The residue was swirled in petroleum ether and filtered, to obtain white solid (12.1 g), with a yield of 86%. MS: *m*/*z* 396, 398 [M+H]⁺.

### Synthesis of (S)-2-(5-((diphenylmethylene)amino)-2',4'-dioxo-2,3-dihydrospiro [indene-1,5'-oxazolidine]-3'-yl)acetic acid t-butyl ester (Int. 1-17):

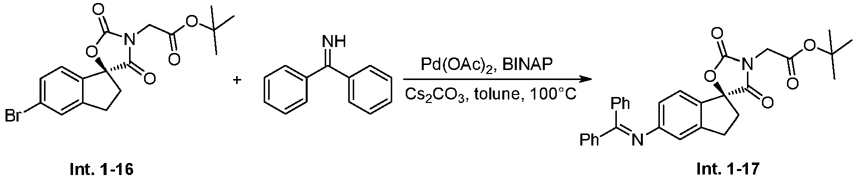

Intermediate **Int. 1-16** (11.5 g, 29 mmol), benzophenonimine (7 g, 38 mmol), palladium acetate (260 mg, 1.16 mmol), 2,2'-bis(diphenylphosphino)-1,1'-binaphthyl (1.61 g, 2.58 mmol), and cesium carbonate (13.2 g, 40 mmol) were dissolved in 50 mL toluene, and then N₂ was purged. The reaction was heated to 100 °C and stirred for 4 h. Then, the reaction was concentrated and purified by column chromatography to obtain 12.1 g grey solid, with a yield of 84%. MS: *m*/*z* 497 [M+H]⁺.

### Synthesis of (S)-2-(5-amino-2',4'-dioxo-2,3-dihydrospiro[indene-1,5'-oxazolidine]-3'-yl)acetic acid t-butyl ester (Int. 1-18):

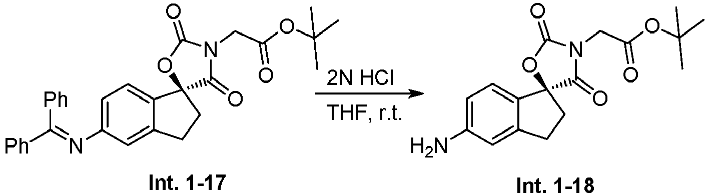

Intermediate **Int. 1-17** (12 g, 24 mmol) was dissolved in 20 mL of tetrahydrofuran, to which was added 10 mL of HCl (2 N) at room temperature, and then stirred for 30 min, followed by extracting with ethyl acetate three times. The organic phase was combined, dried over anhydrous Na₂SO₄, concentrated, and purified by column chromatography to obtain yellow solid (7 g), with a yield of 88%. MS: *m*/*z* 289 [M-43].

(S)-2-(5-(3-Methylureido) -2',4'-dioxo-2,3-dihydrospiro[ 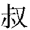 indene-1,5'-oxazolidine]-3'-yl)acetic acid t-butyl ester (Int. 1-19) 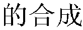 :

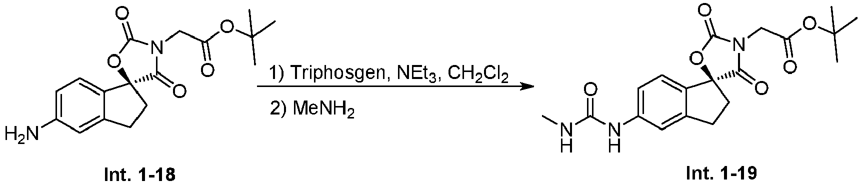

Triphosgene (3.5 g, 12 mmol) was placed in dichloromethane (25 mL). The solution of intermediate **Int. 1-18** (6.9 g, 20 mmol) and triethylamine (20 g, 200 mmol) in dichloromethane (25 mL) was slowly dropped into the reaction flask in an ice bath. Then, the mixture was stirred for 1 h, to which was added methylamine hydrochloride (7 g, 104 mmol), and the reaction was further stirred for 4 h. 100 mL water was added, the organic layer was separated, and then extracted with dichloromethane. The organic phases were combined, dried over anhydrous sodium sulfate, concentrated, and purified by column chromatography, to obtain yellow solid (5.1 g), with a yield of 63%. MS: *m*/*z* 390 [M+H]⁺.

### Synthesis of (S)-2-(5-(3-methylureido)-2',4'-dioxo-2,3-dihydrospiro[indene-1,5'-oxazolidine]-3'- yl)acetic acid (Int. 1-20):

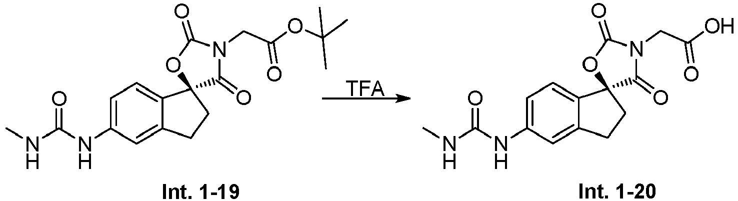

Intermediate **Int. 1-19** (5 g, 13 mmol) was dissolved in 5 mL of trifluoroacetic acid, stirred at room temperature overnight, and concentrated to obtain light yellow solid (4.09 g), with a yield of 96%. MS: *m*/*z* 334 [M+H]⁺; ¹H NMR (400 MHz, DMSO-d₆): *δ* 13.52 (s, 1H), 8.78 (s, 1H), 7.53 (s, 1H), 7.31-7.25 (m, 1H), 7.19-7.11(m, 1H), 6.12 (s, 1H), 4.38-4.18 (m, 2H), 3.18-2.98 (m, 2H), 2.71-2.57 (m, 4H), 2.55-2.41 (m, 1H).

### Example 1 Synthesis of compound 1-((S)-2',4'-dioxo-3'-(2-oxo-2-((R)-2-phenylpiperazin-1-yl)ethyl)-2,3-dihydrospiro[[indene-1,5'-oxazolidin]-5-yl)-3-methylurea (1)

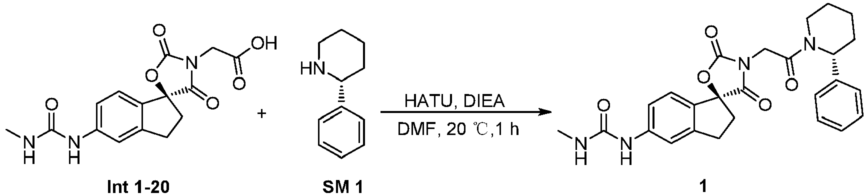

DMF (3 mL) was added to a 25 mL reaction flask, to which were then added **Int 1-20** (333 mg, 1 mmol), SM1 (97 mg, 1 mmol), and DIEA (N,N-diisopropylethylamine, 387 mg, 3 mmol), and HATU (O-(7-nitrobenzotriazole)-N,N,N',N'- tetramethyluronium hexafluorophosphate, 381 mg, 1 mmol) was finally added. The system was reacted at 20°C for 1 h. After completion of the reaction, the reaction solution was poured into 20 mL of water, and extracted with 30 mL (10 mL×3) of ethyl acetate. The organic phases were combined, dried over anhydrous sodium sulfate, and purified by column chromatography to obtain compound **1** (248 mg), with a yield of 52%. MS: *m*/*z* 477 [M+H]⁺.

### Example 2 Synthesis of compound 1-((R)-2',4'-dioxo-3'-(2-oxo-2-((R)-2-phenylpiperazine-1-yl)ethyl)-2,3-dihydrospiro[[indene-1,5'-oxazolidin]-5-yl)-3-methylurea (2)

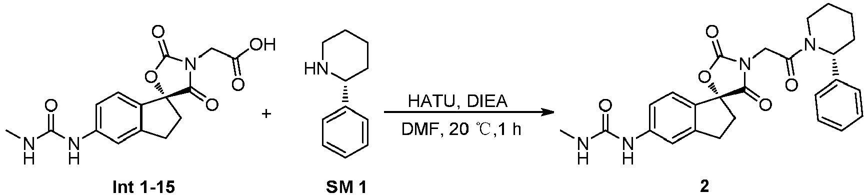

DMF (3 mL) was added to a 25 mL reaction flask, to which were then added **Int 1-15** (333 mg, 1 mmol), SM1 (97 mg, 1 mmol), and DIEA (387 mg, 3 mmol), and HATU (381 mg, 1 mmol) was finally added. The system was reacted at 20°C for 1 h. After completion of the reaction, the reaction solution was poured into 20 mL of water, and extracted with 30 mL (10 mL×3) of ethyl acetate. The organic phases were combined, dried over anhydrous sodium sulfate, and purified by column chromatography to obtain compound **2** (252 mg), with a yield of 53%. MS: *m*/*z* 477 [M+H]⁺.

### Example 3 Synthesis of compound 1-((R)-3'-(2-((1R,5S)-8-azabicyclo[3.2.1] octan-8-yl)-2-oxoethyl)-2',4'-dioxo-2,3-dihydrospiro[indene-1,5'-oxazolidine]-5-yl)-3-methylurea (3)

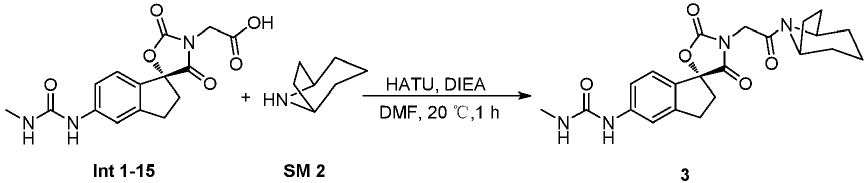

DMF (3 mL) was added to a 25 mL reaction flask, to which were then added **Int 1-15** (333 mg, 1 mmol), SM3 (111 mg, 1 mmol), and DIEA (387 mg, 3 mmol), and HATU (381 mg, 1 mmol) was finally added. The system was reacted at 20°C for 1 h. After completion of the reaction, the reaction solution was poured into 20 mL of water, and extracted with 30 mL (10 mL×3) of ethyl acetate. The organic phases were combined, dried over anhydrous sodium sulfate, and purified by column chromatography to obtain compound **3** (226 mg), with a yield of 53%. MS: *m*/*z* 427 [M+H]⁺.

### Example 4 Synthesis of compound 1-((S)-2',4'-dioxo-3'-(2-oxo-2-((S)-2- phenylpiperidin-1-yl)ethyl)-2,3-dihydrospiro[indene-1,5'-oxazolidine]-5-yl)-3-methylurea (4)

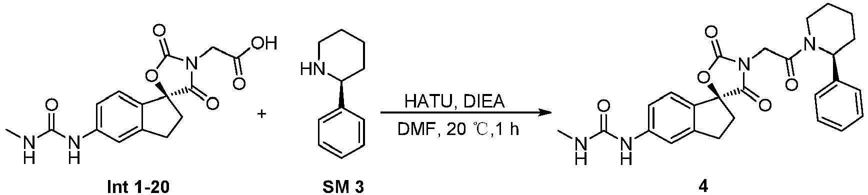

DMF (3 mL) was added to a 25 mL reaction flask, to which were then added **Int 1-20** (333 mg, 1 mmol), SM4 (161 mg, 1 mmol), and DIEA (387 mg, 3 mmol), and finally HATU (381 mg, 1 mmol) was added. The system was reacted at 20°C for 1 h. After completion of the reaction, the reaction solution was poured into water (20 mL), and extracted with 30 mL (10 mL×3) of ethyl acetate. The organic phases were combined, dried over anhydrous Na₂SO₄, and purified by column chromatography to obtain compound **4** (300 mg), with a yield of 63%. MS: *m*/*z* 477 [M+H]⁺.

### Example 5 Synthesis of compound 1-((R)-2',4'-dioxo-3'-(2-oxo-2-((S)-2- phenylpiperidin-1-yl)ethyl)-2,3-dihydrospirofindene-1,5'-oxazolidine]-5-yl)-3-methylurea (5)

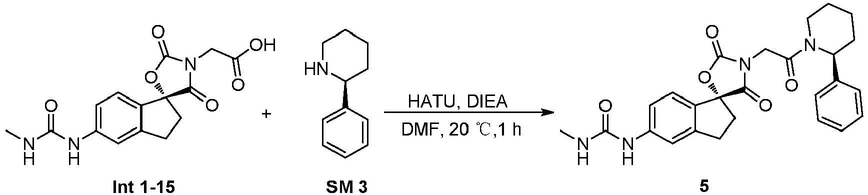

DMF (3 mL) was added to a 25 mL reaction flask, to which were then added **Int 1-15** (333 mg, 1 mmol), SM1 (161 mg, 1 mmol), and DIEA (387 mg, 3 mmol), and finally HATU (381 mg, 1 mmol) was added. The system was reacted at 20°C for 1 h. After completion of the reaction, the reaction solution was poured into water (20 mL), and extracted with 30 mL (10 mL×3) of ethyl acetate. The organic phases were combined, dried over anhydrous Na₂SO₄, and purified by column chromatography to obtain compound **5** (300 mg), with a yield of 63%. MS: *m*/*z* 477 [M+H]⁺.

### Example 7 Synthesis of compound 1-((R)-3'-(2-((1S,4S)-7-azabicyclo [2.2.1]heptan-7-yl)-2-oxoethyl)-2',4'-dioxo-2,3-dihydrospiro[indene-1,5'-oxazolidine]-5-yl)-3-methylurea (7)

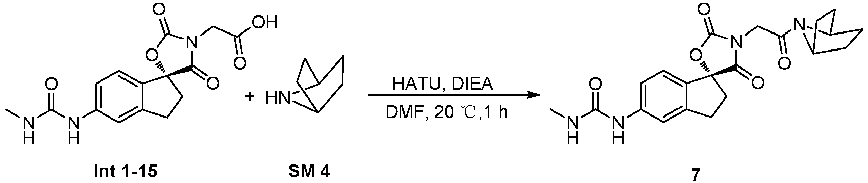

DMF (3 mL) was added to a 25 mL reaction flask, to which were then added **Int 1-15** (333 mg, 1 mmol), **SM4** (97 mg, 1 mmol), and DIEA (387 mg, 3 mmol), and finally HATU (381 mg, 1 mmol) was added. The system was reacted at 20 °C for 1 h. After completion of the reaction, the reaction solution was poured into water (20 mL), and extracted with 30 mL (10 mL×3) of ethyl acetate. The organic phases were combined, dried over anhydrous Na₂SO₄, and purified by column chromatography to obtain compound 7 (260 mg), with a yield of 63%. MS: *m*/*z* 413 [M+H]⁺.

### Example 8 Synthesis of compound 1-((R)-3'-(2-((S)-2-(4-fluoro-2-methylphenyl) pyrrolidin-1-yl)-2-oxoethyl)-2',4'-dioxo-2,3-dihydrospiro[indene-1, 5'-oxazolidine]-5-yl)-3-methylurea (8)

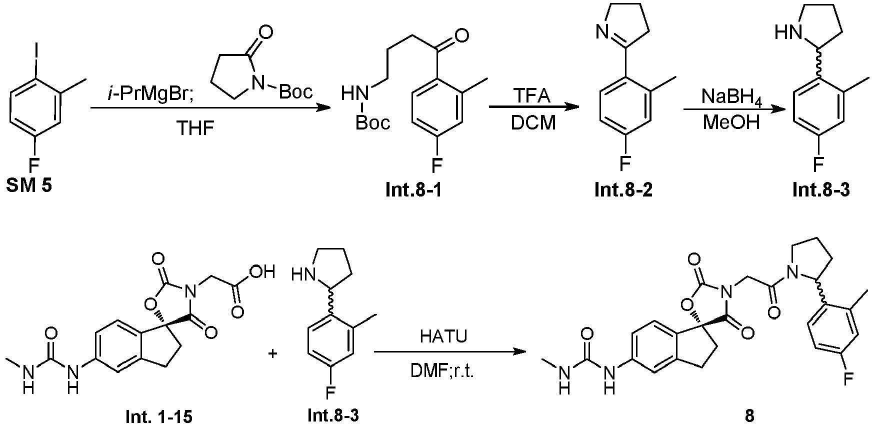

### Synthesis of intermediate (4-(4-fluoro-2-methylphenyl)-4-oxobutyl)carbamic acid t-butyl ester (Int.8-1)

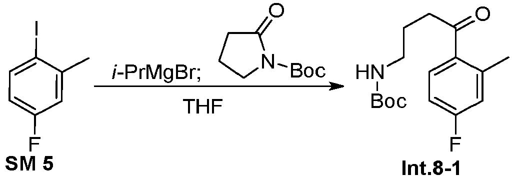

To a reaction flask containing SM5 (2.36 g, 10 mmol), was added dry THF (23 mL), and then under N₂ protection, the solution of isopropylmagnesium bromide in tetrahydrofuran (10 mL, 1M in THF) was added to the reaction flask in an ice-water bath. After that, the ice-water bath was removed, and the reaction was stirred at room temperature for 7 h. Then, N-Boc-2-pyrrolidone (1.85 g, 10 mmol) was finally added, and the mixture was allowed to react at room temperature for 12 h. The reaction solution was poured into the saturated aqueous solution of ammonium chloride, extracted with 30 mL (10 mL×3) of ethyl acetate. The organic phases were combined, dried over anhydrous Na₂SO₄, and purified by column chromatography to obtain **Int. 8-1** (1.47 g, 5 mmol), with a yield of 50%. MS: *m*/*z* 296 [M+H]⁺.

### Synthesis of intermediate 5-(4-fluoro-2-methylphenyl)-3,4-dihydro-2H-pyrrole (Int.8-2)

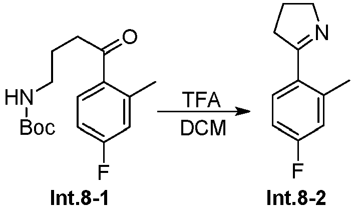

**Int 8-1** (1.47 g, 5 mmol) was dissolved in 15 mL of DCM, to which was added 3.7 mL of TFA, and the mixture was stirred at room temperature until the reaction was completed by TLC detection. After pH value of the solution was adjusted to be neutral with the saturated aqueous solution of sodium bicarbonate, the solution was extracted with DCM (10 mL × 3), dried with anhydrous Na₂SO₄, and rotatory evaporated to dry, to obtain **Int.8-2** (0.79 g; 90%), MS: *m*/*z* 178 [M+H]⁺.

### Synthesis of intermediate 2-(4-fluoro-2-methylphenyl)pyrrolidine (Int.8-3)

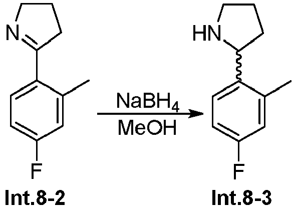

**Int.8-2** (0.79 g; 4.5 mmol) was added in a reaction flask containing 8 mL of methanol, to which was added sodium borohydride (0.68 g; 18 mmol) in batches under stirring at room temperature. After the addition, the reaction solution was continued stirring at room temperature until the reaction was completed by TLC detection. Then, the reaction solution was poured into water, and extracted with 30 mL (10 mL×3) of EA. The organic phases were combined, dried over anhydrous Na₂SO₄, and separated by column chromatography to obtain **Int.8-3** (0.79 g; 97%), MS: *m*/*z* 180 [M+H]⁺.

### Synthesis of compound 1-((R)-3'-(2-((S)-2-(4-fluoro-2-tolyl)pyrrolidin-1-yl)-2- oxoethyl)-2',4'-dioxo-2,3-dihydrospiro[indene-1,5'-oxazolidine]-5-yl)-3-methylurea (8)

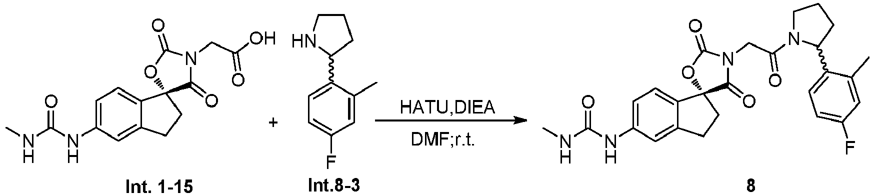

DMF (3 mL) was added to a 25 mL reaction flask, to which were then added **Int 1-15** (333 mg, 1 mmol), **Int.8-3** (179 mg, 1 mmol), and DIEA (387 mg, 3 mmol), and finally HATU (381 mg, 1 mmol) was added. The system was reacted at 20 °C for 1 h. After completion of the reaction, the reaction solution was poured into water (20 mL), and extracted with 30 mL (10 mL×3) of ethyl acetate. The organic phases were combined, dried over anhydrous Na₂SO₄, and purified by column chromatography to obtain compound **8** (260 mg), with a yield of 56%. MS: *m*/*z* 495 [M+H]⁺; ¹H NMR (400 MHz, CDCl₃) δ 7.33 (d, J = 38.5 Hz, 2H), 7.06 (s, 1H), 6.94 (d, J = 9.8 Hz, 2H), 6.83-6.74 (m, 1H), 5.13 (s, 1H), 3.93-3.68 (m, 3H), 3.12 (s, 1H), 3.01 (s, 1H), 2.82 (s, 2H), 2.72 (s, 4H), 2.49 (s, 2H), 2.39 (s, 2H), 2.24 (d, J = 3.9 Hz, 1H), 1.77 (s, 1H), 1.25 (s, 1H).

### Example 10 Synthesis of compound (1S,4S)-5-(2-((R)-5-(3-methylureido) -2',4'-dioxo-2,3-dihydrospiro[indene-1,5'-oxazolidine]t-butyl-3'-yl)acetyl)-2,5-diazabicyclo[2.2.1]heptane-2-carboxylic acid t-butyl ester (10)

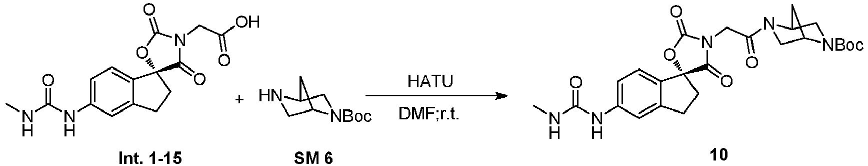

DMF (3 mL) was added to a 25 mL reaction flask, to which were then added **Int 1-15** (333 mg, 1 mmol), **SM 6** (198 mg, 1 mmol), and DIEA (387 mg, 3 mmol), and finally HATU (381 mg, 1 mmol) was added. The system was reacted at 20 °C for 1 h. After completion of the reaction, the reaction solution was poured into water (20 mL), and extracted with 30 mL (10 mL×3) of ethyl acetate. The organic phases were combined, dried over anhydrous Na₂SO₄, and purified by column chromatography to obtain compound **10** (370 mg), with a yield of 72%. MS: *m*/*z* 514 (M+H⁺); ¹H NMR (400 MHz, CDCl₃) δ 7.52 (s, 1H), 7.32 (d, J = 7.8 Hz, 1H), 6.94 (d, J = 8.3 Hz, 1H), 4.88 (s, 1H), 4.44 (m, 4H), 3.62-3.33 (m, 4H), 3.14 (s, 1H), 3.03 (s, 1H), 2.76 (s, 3H), 2.54 (s, 1H), 1.99-1.78 (m, 2H), 1.47 (s, 9H), 1.26 (d, J = 9.1 Hz, 1H).

### Example 11 Synthesis of compound 1-((1R)-2',4'-dioxo-3'-(2-oxo-2-(2-(o-tolyl) pyrrolidin-1-yl)ethyl)-2,3-dihydrospiro[indene-1,5'-oxazolidine]-5-yl)-3-methylurea (11)

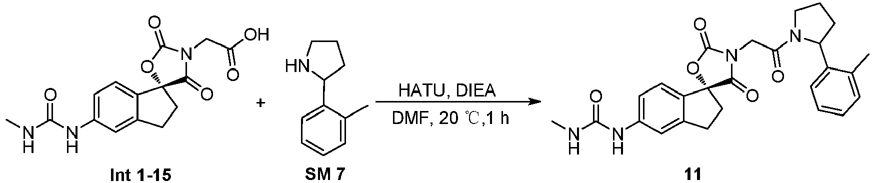

DMF (3 mL) was added to a 25 mL reaction flask, to which were then added **Int 1-15** (333 mg, 1 mmol), **SM 7** (198 mg, 1 mmol), and DIEA (387 mg, 3 mmol), and finally HATU (381 mg, 1 mmol) was added. The system was reacted at 20 °C for 1 h. After completion of the reaction, the reaction solution was poured into water (20 mL), and extracted with 30 mL (10 mL×3) of ethyl acetate. The organic phases were combined, dried over anhydrous Na₂SO₄, and purified by column chromatography to obtain compound **11** (233 mg, yield 50%). MS: *m*/*z* 477 [M+H]⁺; ¹H NMR (400 MHz, DMSO): δ 8.94-8.45 (m, 1H), 7.54 (d, J = 9.5 Hz, 1H), 7.36-7.26 (m, 1H), 7.26-7.10 (m, 2H), 7.10-6.77 (m, 3H), 6.24-5.88 (m, 1H), 5.43-5.11 (m, 1H), 4.71-4.00 (m, 2H), 3.84 (d, J = 11.5 Hz, 3H), 3.78-3.44 (m, 2H), 3.09 (m, 1H), 3.03-2.89 (m, 1H), 2.67-2.55 (m, 4H), 2.55-2.50 (m,1H), 2.50-2.25 (m 1H), 2.02-1.59 (m, 3H).

### Example 12 Synthesis of compound 1-methyl-3-((1R)-3'-(2-(octahydroquinolin-1- (2H)-yl)-2-oxoethyl)-2',4'-dioxo-2,3-dihydrospiro[indene-1,5'-oxazolidine]-5-yl)urea (12)

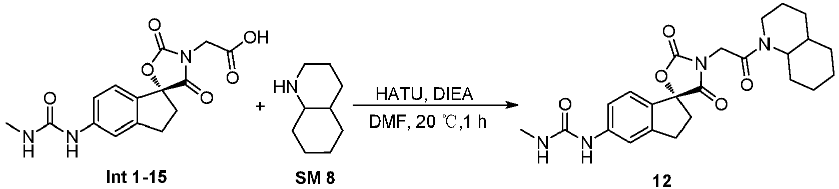

DMF (3 mL) was added to a 25 mL reaction flask, to which were then added **Int 1-15** (333 mg, 1 mmol), **SM 8** (139 mg, 1 mmol), and DIEA (387 mg, 3 mmol), and finally HATU (381 mg, 1 mmol) was added. The system was reacted at 20 °C for 1 h. After completion of the reaction, the reaction solution was poured into water (20 mL), and extracted with 30 mL (10 mL×3) of ethyl acetate. The organic phases were combined, dried over anhydrous Na₂SO₄, and purified by column chromatography to obtain compound **12** (227 mg, yield 50%). MS: *m*/*z* 455 [M+H]⁺; ¹H NMR (400 MHz, DMSO): *δ* 8.72 (s, 1H), 7.55 (s, 1H),7.35-7.33 (m, 1H), 7.25-7.17(m,1H), 6.12-6.06 (m 1H), 4.66-4.21 (m, 2H), 4.20-3.47 (m, 2H), 3.33-2.78 (m, 4H), 2.74-2.55 (m, 4H), 2.55-2.50(m,1H)1.91-1.20 (m, 12H).

### Example 13 Synthesis of compound 1-((1R)-2',4'-dioxo-3'-(2-oxo-2-(2-(2-(trifluoromethoxyl)phenyl)pyrrolidin-1-yl)ethyl)-2,3-dihydrospiro[indene-1,5'-oxazolidine]-5-yl)-3-methylurea (13)

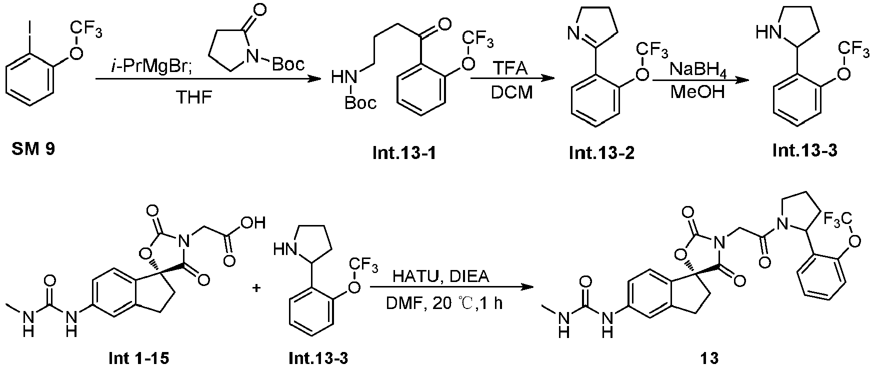

### Synthesis of intermediate 4-oxo-4-(2-(trifluoromethoxyl)phenyl)butyl)carbamic acid t-butyl ester (Int.13-1)

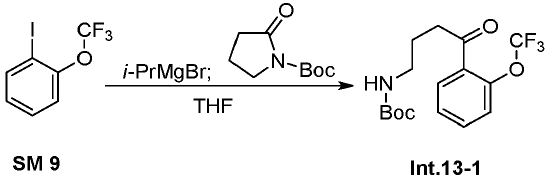

To a reaction flask, was added dry THF (30 mL), followed by addition of **SM 9** (2.88 g, 10 mmol), and then under N₂ protection, isopropylmagnesium bromide (10 mL, 1M in THF) was added in an ice-water bath. After that, the mixture was reacted at room temperature for 7 h, and t-butyl-2-oxopyrrolidin-1-carboxylate (1.85 g, 10 mmol) was finally added, and the mixture was further allowed to react at room temperature for 12 h. The reaction solution was poured into the saturated aqueous solution of ammonium chloride, extracted with 30 mL (10 mL×3) of ethyl acetate. The organic phases were combined, dried over anhydrous Na₂SO₄, and purified by column chromatography to obtain **Int. 13-2** (1.74 g, 5 mmol), with a yield of 50%. MS: *m*/*z* 348 [M+H]⁺.

### Synthesis of intermediate 5-(2-(trifluoromethoxyl)phenyl)-3,4-dihydro-2H-pyrrole (Int. 13-2)

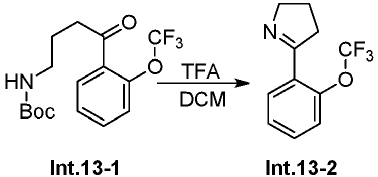

To a reaction solution was added DCM (20 mL), to which was then added **Int 13-2** (1.74 g, 5 mmol), followed by addition of TFA (5 mL). After that, the mixture was reacted at room temperature for 7 h, and the reaction solution was poured into the saturated aqueous solution of sodium bicarbonate. The solution was extracted with 30 mL (10 mL × 3) of DCM. The organic phases were combined, dried with anhydrous Na₂SO₄, and purified by column chromatography, to obtain **Int 13-3** (1.04 g, 4.5 mmol), with a yield of 90%. MS: *m*/*z* 230 [M+H]⁺.

### Synthesis of intermediate 2-(2-(trifluoromethoxyl)phenyl)pyrrolidine (Int. 13-4)

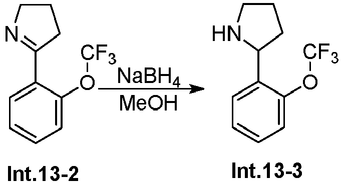

Methanol (15 mL) was added to a reaction flask, to which was then added **Int 13-3** (1.04 g, 4.5 mmol), followed by addition of sodium borohydride (0.76 g, 20 mmol) in batches. After that, the mixture was reacted at room temperature for 1 h. The reaction solution was poured into water, and extracted with 30 mL (10 mL×3) of ethyl acetate. The organic phases were combined, dried over anhydrous Na₂SO₄, and concentrated to obtain **Int 13-4** (1.04 g, 4.5 mmol), with a yield of 98%. MS: *m*/*z* 232 [M+H]⁺.

### Synthesis of compound 1-((1R)-2',4'-dioxo-3'-(2-oxo-2-(2-(2-(trifluoromethoxyl) phenyl)pyrrolidin-1-yl)ethyl)-2,3-dihydrospiro[indene-1,5'-oxazolidine]-5-yl)-3-methylurea (13)

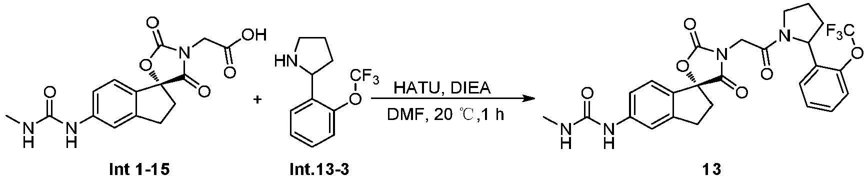

DMF (3 mL) was added to a 25 mL reaction flask, to which were then added **Int 1-15** (333 mg, 1 mmol), **Int13-3** (231 mg, 1 mmol), and DIEA (387 mg, 3 mmol), and finally HATU (381 mg, 1 mmol) was added. The system was reacted at 20 °C for 1 h. After completion of the reaction, the reaction solution was poured into water (20 mL), and extracted with 30 mL (10 mL×3) of ethyl acetate. The organic phases were combined, dried over anhydrous Na₂SO₄, and purified by column chromatography to obtain compound **13** (227 mg), with a yield of 50%. MS: *m*/*z* 547 [M+H]⁺. ¹H NMR (400 MHz, CDCl₃): δ 7.65-7.29 (m, 5H), 7.19-6.98 (m, 2H), 5.49-5.27 (m, 2H), 4.54-4.41 (m, 1H), 3.80 (m, 2H), 3.10-3.02 (m, 1H), 3.01-2.88 (m, 1H), 2.66 (s, 3H), 2.59-2.41 (m, 4H), 2.06-1.83 (m, 4H).

### Example 14 Synthesis of compound 1-((R)-3'-(2-((2S,5S)-2-(4-fluorophenyl)-5-methylpyrrolidin-1-yl)-2-oxoethyl)-2',4'-dioxo2,3-dihydrospiro[indene-1,5'-oxazolidine]-5-yl)-3-methylurea (14)

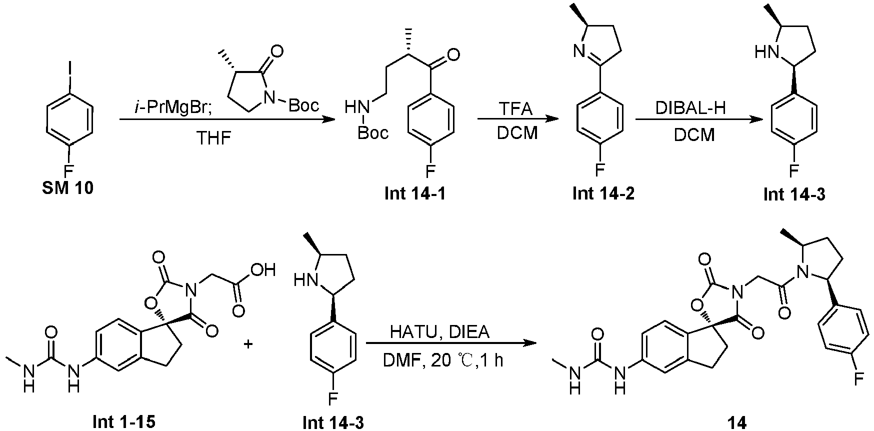

### Synthesis of intermediate (S)-t-butyl-(4-(4-fluorophenyl)-3-methyl-4-oxobutyl) carbamate (Int. 14-1)

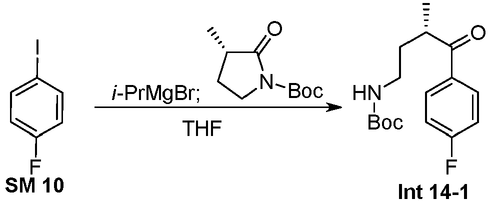

To a 100 mL reaction flask, was added dry THF (25 mL), followed by addition of 1-fluoro-4-iodobenzene (5 g, 22.5 mmol), and then isopropylmagnesium chloride (10 mL, 2.0 mol/L in THF) was slowly added in an ice-water bath. After that, the ice-water bath was removed, and the reaction was warmed to room temperature and stirred for 3 h. The starting material (S)-2-methyl-5-oxopyrrolidin-1-carboxylic acid t-butyl ester (4.9 g, 24.8 mmol) was dissolved in THF (10 mL), and quickly added to the above reaction solution. The mixture was allowed to react at room temperature for 3 h. After completion of the reaction, the reaction was quenched by addition of the saturated aqueous solution of ammonium chloride (30 mL), and extracted with 90 mL (30 mL×3) of ethyl acetate. The organic phases were combined, dried over anhydrous Na₂SO₄, and concentrated to obtain crude product **Int. 14-1** (8 g). MS: *m*/*z* 296 [M+H]⁺.

### Synthesis of intermediate (S)-5-(4-fluorophenyl)-2-methyl-3,4-dihydro-2H-pyrrole (Int. 14-2)

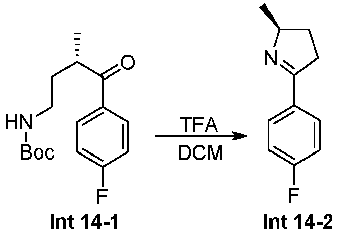

The crude product obtained in the previous step was dissolved in DCM (50 mL), to which was added TFA (5 mL), and the mixture was stirred and reacted at room temperature for 16 h. After completion of the reaction, the solution was concentrated, followed by addition of water (50 mL), and then pH was adjusted to 8 to 9 with sodium bicarbonate. The solution was extracted with 90 mL (30 mL×3) of ethyl acetate, and the organic phases were combined, dried over anhydrous Na₂SO₄, concentrated, and purified by column chromatography to obtain compound **Int. 14-2** (2.4 g, mmol), with a two-step yield of 60%. MS: *m*/*z* 178 [M+H]⁺.

### Synthesis of intermediate (2S,SS)-2-(4-fluorophenyl)-5-methylpyrrolidine Int. 14-3

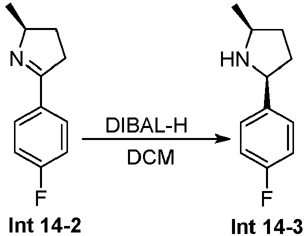

To a reaction flask was added DCM (1 mL), to which was then added **Int 14-2** (2 g, 11.3 mmol), followed by addition of DIBAL-H (12 mL, 1M in hexane) in ice-water bath. After that, the mixture was reacted at room temperature for 1 h, and quenched by adding a small amount of water. The solution was filtered, washed with DCM (1 mL×3), and concentrated, to obtain **Int 14-3** (1.82 g, 10.17 mmol), with a yield of 90%. MS: *m*/*z* 180 [M+H]⁺. ¹H NMR (400 MHz, CDCl₃) δ 7.41-7.29 (m, 2H), 7.05-6.91 (m, 2H), 4.14 (t, J = 8.0 Hz, 1H), 3.32 (dd, J = 13.9, 7.2 Hz, 1H), 2.24-2.08 (m, 1H), 2.04-1.93 (m, 1H), 1.72 (dddd, J = 12.5, 10.1, 8.4, 5.9 Hz, 1H), 1.57 (brs, 1H), 1.53-1.43 (m, 1H), 1.24 (d, J = 6.2 Hz, 3H).

### Synthesis of compound 1-((R)-3'-(2-((2S,5S)-2-(4-fluorophenyl)-5-methylpyrrolidin- 1-yl)-2-oxoethyl)-2',4'-dioxo2,3-dihydrospiro[indene-1,5'-oxazolidine]-5-yl)-3-methylurea (14)

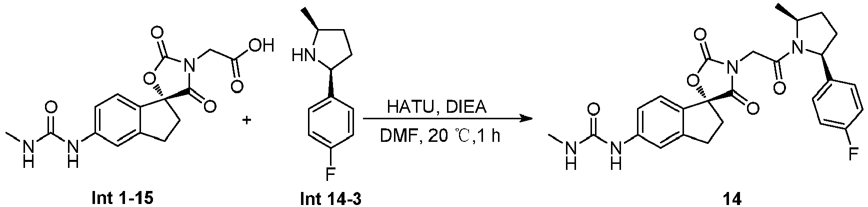

DMF (3 mL) was added to a 25 mL reaction flask, to which were then added **Int 1-15** (333 mg, 1 mmol), **Int 14-3** (179 mg, 1 mmol), and DIEA (387 mg, 3 mmol), and finally HATU (381 mg, 1 mmol) was added. The system was reacted at 20 °C for 1 h. After completion of the reaction, the reaction solution was poured into water (20 mL), and extracted with 30 mL (10 mL×3) of ethyl acetate. The organic phases were combined, dried over anhydrous Na₂SO₄, and purified by column chromatography to obtain compound **14** (247 mg), with a yield of 50%. MS: *m*/*z* 495 [M+H]⁺. ¹H NMR (400 MHz, CDCl₃) δ 7.44-7.35 (m, 2H), 7.33-7.27 (m, 2H), 7.12 (td, J = 8.6, 2.4 Hz, 2H), 7.04 (d, J = 8.2 Hz, 1H), 4.98-4.89 (m, 1H), 4.41 (d, J = 6.7 Hz, 1H), 4.21-4.09 (m, 2H), 3.63 (s, 1H), 3.17 (dd, J = 15.8, 7.4 Hz, 1H), 3.07 (s, 1H), 2.89 (s, 3H), 2.79 (d, J = 1.4 Hz, 3H), 2.72 (d, J = 6.7 Hz, 1H), 2.56-2.41 (m, 1H), 2.04 (dd, J = 21.7, 13.1 Hz, 2H), 1.65 (s, 1H).

### Example 15 Synthesis of compound 1-((R)-3'-(2-((5S)-2-(4-fluorophenyl)-5-methylpyrrolidin-1-yl)-2-oxoethyl)-2',4'-dioxo2,3-dihydrospiro[indene-1,5'-oxazolidine]-5-yl)-3-methylurea (15)

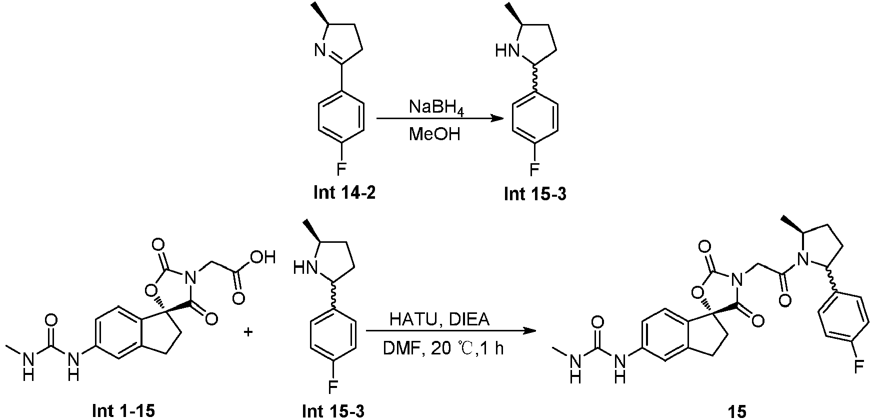

### Synthesis of intermediate (5S)-2-(4-fluorophenyl)-5-methylpyrrolidine (Int 15-3)

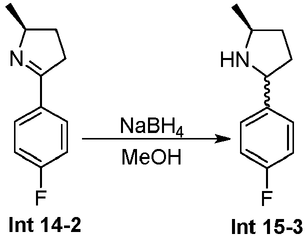

**Int 14-2** (2 g, 11.3 mmol) was dissolved in methanol (50 mL), to which was added sodium borohydride (0.86 g, 22.6 mmol) in portions, and the solution was stirred at room temperature for 2 h and concentrated. Water (50 mL) was added, and then the resultant solution was extracted with ethyl acetate (10 mL × 3). The organic phases were combined, dried over anhydrous Na₂SO₄, and purified to obtain **Int 15-3** (1.72 g, 9.6 mmol), with a yield of 85%. MS: *m*/*z* 180 [M+H]⁺.

### Synthesis of compound 1-((R)-3'-((SS)-2-(2-(4-fluorophenyl)-5-methylpyrrolidin- 1-yl)-2-oxoethyl)-2',4'-dioxo2,3-dihydrospiro[indene-1,5'-oxazolidine]-5-yl)-3-methylurea (15)

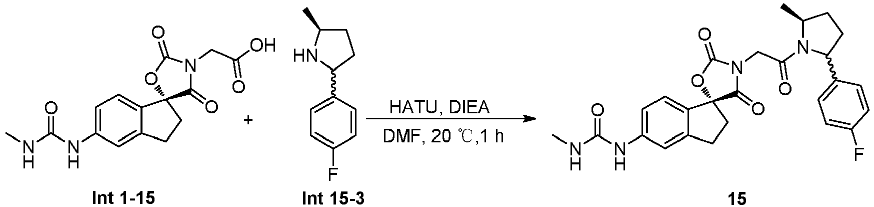

DMF (3 mL) was added to a 25 mL reaction flask, to which were then added **Int 1-15** (333 mg, 1 mmol), **Int 15-3** (179 mg, 1 mmol), and DIEA (387 mg, 3 mmol), and finally HATU (381 mg, 1 mmol) was added. The system was reacted at 20 °C for 1 h. After completion of the reaction, the reaction solution was poured into water (20 mL), and extracted with 30 mL (10 mL×3) of ethyl acetate. The organic phases were combined, dried over anhydrous Na₂SO₄, and purified by column chromatography to obtain compound **15** (260 mg), with a yield of 52%. MS: *m*/*z* 495 [M+H]⁺.

### Example 16 Synthesis of compound 1-(3'-(2-(2-azabicyclo[2.2.1]heptan- 2-yl)-2-oxoethyl)-2',4'-dioxo-2,3-dihydrospiro[indene-1,5'-oxazolidine]-5-yl)-3-methylurea (16)

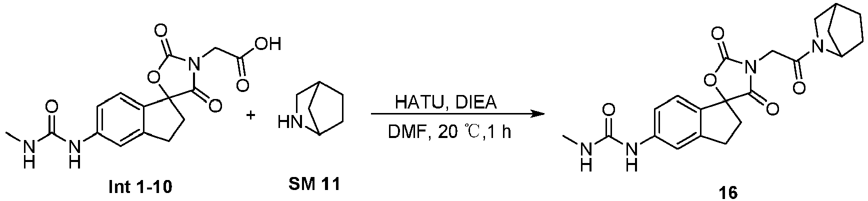

DMF (3 mL) was added to a 25 mL reaction flask, to which were then added **Int 1-10** (333 mg, 1 mmol), **SM 11** (97 mg, 1 mmol), and DIEA (387 mg, 3 mmol), and finally HATU (381 mg, 1 mmol) was added. The system was reacted at 20 °C for 1 h. After completion of the reaction, the reaction solution was poured into water (20 mL), and extracted with 30 mL (10 mL×3) of ethyl acetate. The organic phases were combined, dried over anhydrous Na₂SO₄, and purified by column chromatography to obtain compound **16** (206 mg), with a yield of 50%. MS: *m*/*z* 413 [M+H]⁺. ¹H NMR (400 MHz, CDCl₃): δ 7.48 (m, 2H), 7.32 (m, 1H), 6.89 (m, 1H), 5.39-5.22 (m, 1H), 4.52 (m, 1H), 4.36-4.12 (m, 2H), 3.43 (m, 1H), 3.24-3.08 (m, 2H), 3.01 (m, 1H), 2.74 (m, 3H), 2.58-2.46 (m, 1H), 1.77 (m, 3H), 1.31-1.22 (m, 4H).

### Example 17 Synthesis of compound 1-((R)-3'-(2-((2R,5R)-2-(4-fluorophenyl)-5-methylpyrrolidin-1-yl)-2-oxoethyl)-2',4'-dioxo-2,3-dihydrospiro[indene-1, 5'-oxazolidine]-5-yl)-3-methylurea (17)

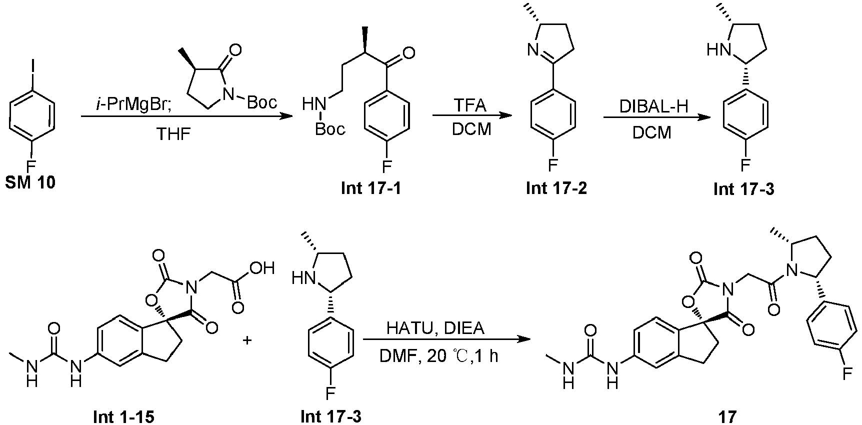

### Synthesis of intermediate (R)-t-butyl-(4-(4-fluorophenyl)-3-methyl-4-oxobutyl) carbamate (Int 17-1)

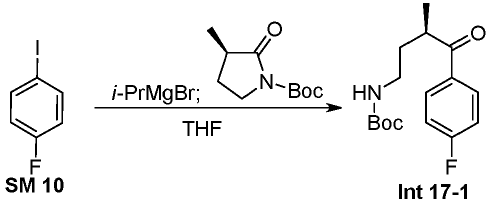

To a 100 mL reaction flask, was added dry THF (25 mL), followed by addition of 1-fluoro-4-iodobenzene (5 g, 22.5 mmol), and then isopropylmagnesium chloride (10 mL, 2.0 mol/L in THF) was slowly added in an ice-water bath. After that, the ice-water bath was removed, and the reaction was warmed to room temperature and stirred for 3 h. The starting material (R)-2-methyl-5-oxopyrrolidin-1-carboxylic acid t-butyl ester (4.9 g, 24.8 mmol) was dissolved in THF (10 mL), and quickly added to the above reaction solution. The mixture was allowed to react at room temperature for 3 h. After completion of the reaction, the reaction was quenched by addition of the saturated aqueous solution of ammonium chloride (30 mL), and extracted with 90 mL (30 mL×3) of ethyl acetate. The organic phases were combined, dried over anhydrous Na₂SO₄, and concentrated to obtain crude product **Int. 17-1** (7.5 g). MS: *m*/*z* 296 [M+H]⁺.

### Synthesis of intermediate (R)-5-(4-fluorophenyl)-2-methyl-3,4-dihydro-2H-pyrrole (Int 17-2)

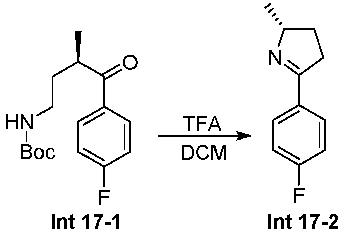

The crude product obtained in the previous step was dissolved in DCM (50 mL), to which was added TFA (5 mL), and the mixture was stirred and reacted at room temperature for 16 h. After completion of the reaction, the solution was concentrated, followed by addition of water (50 mL), and then pH was adjusted to 8 to 9 with sodium bicarbonate. The solution was extracted with 90 mL (30 mL×3) of ethyl acetate, and the organic phases were combined, dried over anhydrous Na₂SO₄, concentrated, and purified by column chromatography to obtain compound **Int. 17-2** (2.2 g), with a two-step yield of 59%. MS: *m*/*z* 178 [M+H]⁺.

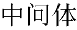 (2R,5R)-2-(4-fluorophenyl)-5-methylpyrrolidine **Int 17-3**

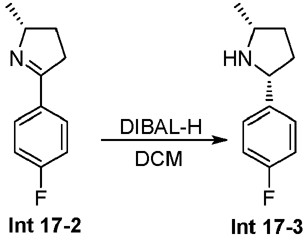 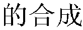

To a reaction flask was added DCM (1 mL), to which was then added **Int 17-2** (2 g, 11.3 mmol), followed by addition of DIBAL-H (12 mL, 1M in hexane) in ice-water bath. After that, the mixture was reacted at room temperature for 1 h, and quenched by adding a small amount of water. The solution was filtered, washed with DCM (1 mL×3), and concentrated, to obtain **Int 17-3** (1.82 g, 10.17 mmol), with a yield of 90%. MS: *m*/*z* 180 [M+H]⁺. ¹H NMR (400 MHz, CDCl₃) δ 7.41-7.29 (m, 2H), 7.05-6.91 (m, 2H), 4.14 (t, J = 8.0 Hz, 1H), 3.32 (dd, J = 13.9, 7.2 Hz, 1H), 2.24-2.08 (m, 1H), 2.04-1.93 (m, 1H), 1.72 (dddd, J = 12.5, 10.1, 8.4, 5.9 Hz, 1H), 1.57 (brs, 1H), 1.53-1.43 (m, 1H), 1.24 (d, J = 6.2 Hz, 3H).

### Synthesis of compound 1-((R)-3'-(2-((2R, 5R)-2-(4-fluorophenyl)-5-methylpyrrolidin- 1-yl)-2-oxoethyl)-2',4'-dioxo-2,3-dihydrospiro[indene-1,5'-oxazolidine]-5-yl)-3-methylurea (17)

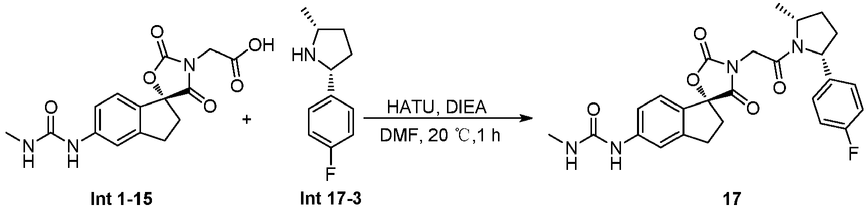

DMF (3 mL) was added to a 25 mL reaction flask, to which were then added **Int 1-15** (333 mg, 1 mmol), **Int 17-3** (179 mg, 1 mmol), and DIEA (387 mg, 3 mmol), and finally HATU (381 mg, 1 mmol) was added. The system was reacted at 20 °C for 1 h. After completion of the reaction, the reaction solution was poured into water (20 mL), and extracted with 30 mL (10 mL×3) of ethyl acetate. The organic phases were combined, dried over anhydrous Na₂SO₄, and purified by column chromatography to obtain compound **17** (233 mg), with a yield of 48%. MS: *m*/*z* 495 [M+H]⁺. ¹H NMR (400 MHz, CDCl₃) δ 7.44-7.35 (m, 2H), 7.33-7.27 (m, 2H), 7.12 (td, J = 8.6, 2.4 Hz, 2H), 7.04 (d, J = 8.2 Hz, 1H), 4.98-4.89 (m, 1H), 4.41 (d, J = 6.7 Hz, 1H), 4.21-4.09 (m, 2H), 3.63 (s, 1H), 3.17 (dd, J = 15.8, 7.4 Hz, 1H), 3.07 (s, 1H), 2.89 (s, 3H), 2.79 (d, J = 1.4 Hz, 3H), 2.72 (d, J = 6.7 Hz, 1H), 2.56-2.41 (m, 1H), 2.04 (dd, J = 21.7, 13.1 Hz, 2H), 1.65 (s, 1H).

### Example 18 Synthesis of compound 1-((R)-3'-(2-((5R)-2-(4-fluorophenyl)-5-methylpyrrolidin-1-yl)-2-oxoethyl)-2',4'-dioxo-2,3-dihydrospiro[indene-1, 5'-oxazolidine]-5-yl)-3-methylurea (18)

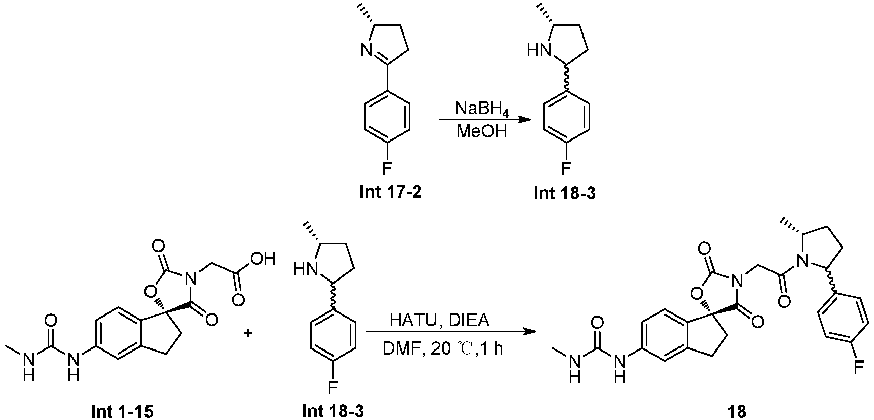

### Synthesis of intermediate (5R)-2-(4-fluorophenyl)-5-methylpyrrolidine Int 18-3

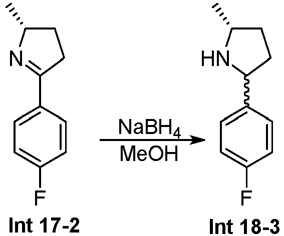

**Int 17-2** (2 g, 11.3 mmol) was dissolved in methanol (50 mL), to which was added sodium borohydride (0.86 g, 22.6 mmol) in portions, and the solution was stirred at room temperature for 2 h. After completion of the reaction, the solution was concentrated. Water (50 mL) was added, and then the resultant solution was extracted with 90 mL (30 mL × 3) of ethyl acetate. The organic phases were combined, dried over anhydrous Na₂SO₄, concentrated, and then purified by column chromatography, to obtain **Int 18-3** (1.72 g, 9.6 mmol), with a yield of 85%. MS: *m*/*z* 180 [M+H]⁺.

### Synthesis of compound 1-((R)-3'-(2-((SR)-2-(4-fluorophenyl)-5-methylpyrrolidin- 1-yl)-2-oxoethyl)-2',4'-dioxo-2,3-dihydrospiro[indene-1,5'-oxazolidine]-5-yl)-3-methylurea (18)

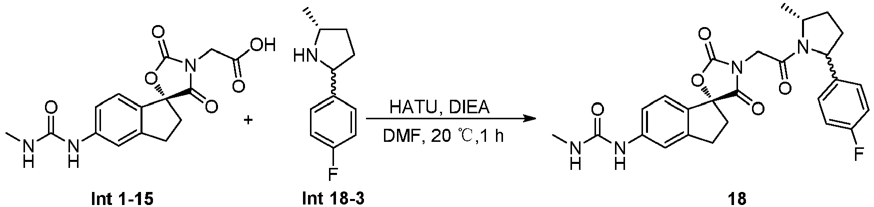

DMF (3 mL) was added to a 25 mL reaction flask, to which were then added **Int 1-15** (333 mg, 1 mmol), **Int 18-3** (179 mg, 1 mmol), and DIEA (387 mg, 3 mmol), and finally HATU (381 mg, 1 mmol) was added. The system was reacted at 20 °C for 1 h. After completion of the reaction, the reaction solution was poured into water (20 mL), and extracted with 30 mL (10 mL×3) of ethyl acetate. The organic phases were combined, dried over anhydrous Na₂SO₄, and purified by column chromatography to obtain compound **18** (223 mg), with a yield of 48%. MS: *m*/*z* 495 [M+H]⁺.

### Example 21 Synthesis of compound 1-(R)-3'-(2-((SR)-2-(4-fluoro-2-methylphenyl)- 5-methylpyrrolidin-1-yl)-2-oxoethyl)-2',4'-dioxo-2,3-dihydrospiro[indene-1, 5'-oxazolidine]-5-yl)-3-methylurea (21)

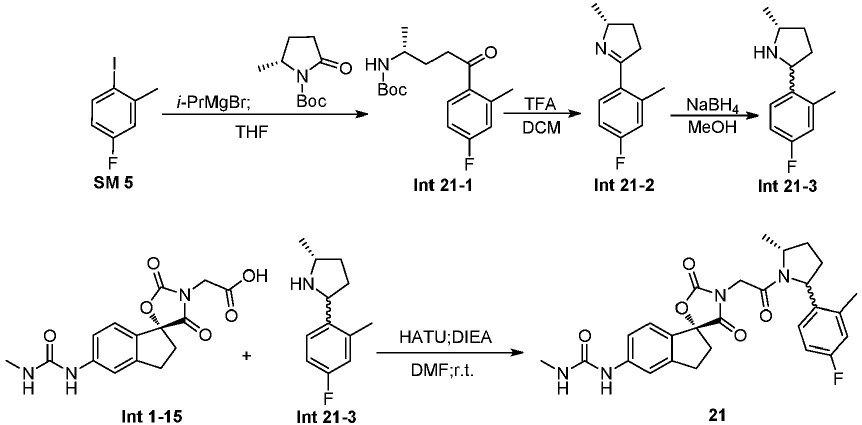

### Synthesis of intermediate (R)-(4-(4-fluoro-2-methylphenyl)-3-methyl-4-oxobutyl) carbamic acid t-butyl ester (Int 21-1)

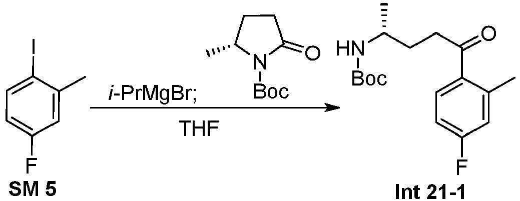

To a reaction flask containing SM5 (2.36 g, 10 mmol), was added dry THF (23 mL), and then under N₂ protection, isopropylmagnesium bromide (10 mL, 1M in THF) was added to the reaction flask in an ice-water bath. After that, the ice-water bath was removed, and the reaction was stirred at room temperature for 7 h. Then, (R)-2-methyl-5-oxopyrrolidin-1-carboxylic acid t-butyl ester (1.99 g, 10 mmol) was finally added, and the mixture was allowed to react at room temperature for 12 h. The reaction solution was poured into the saturated aqueous solution of ammonium chloride, extracted with 30 mL (10 mL×3) of ethyl acetate. The organic phases were combined, dried over anhydrous Na₂SO₄, and purified by column chromatography to obtain **Int. 21-1** (2.59 g, 8.5 mmol), with a yield of 85%. MS: *m*/*z* 306 [M+H]⁺.

### Synthesis of intermediate (R)-5-(4-fluoro-2-methylphenyl)-2-methyl-3,4-dihydro-2H-pyrrole (Int 21-2)

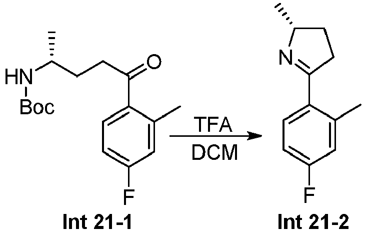

**Int 21-1** (2.59 g, 8.5 mmol) was dissolved in 26 mL DCM, to which was added 2.6 mL TFA, and the mixture was stirred at room temperature until the reaction was completed by TLC detection. After pH value of the solution was adjusted to be neutral with the saturated aqueous solution of sodium bicarbonate, the solution was extracted with DCM (10 mL × 3), dried with anhydrous Na₂SO₄, and rotatory evaporated to dry, to obtain **Int 21-2** (1.46 g; 90%), MS: *m*/*z* 192 [M+H]⁺.

### Synthesis of intermediate (2R,5R)-2-(4-fluoro-2-methylphenyl)-5-methylpyrrolidine Int 21-3

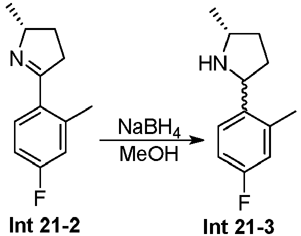

**Int 21-2** (1.46 g; 7.65 mmol) was added in a reaction flask containing 8 mL methanol, to which was added sodium borohydride (1.16 g; 30.6 mmol) in batches under stirring at room temperature. After the addition, the reaction solution was continued stirring at room temperature until the reaction was completed by TLC detection. Then, the reaction solution was poured into water, and extracted with 30 mL (10 mL×3) of EA. The organic phases were combined, dried over anhydrous Na₂SO₄, and separated by column chromatography to obtain **Int 21-3** (1.35 g; 91%), MS: *m*/*z* 194 [M+H]⁺.

### Synthesis of compound 1-(R)-3'-(2-((5R)-2-(4-fluoro-2-methylphenyl)-5- methylpyrrolidin-1-yl)-2-oxoethyl)-2',4'-dioxo-2,3-dihydrospiro[indene-1,5'-oxazolidine]-5-yl)-3-methylurea (21)

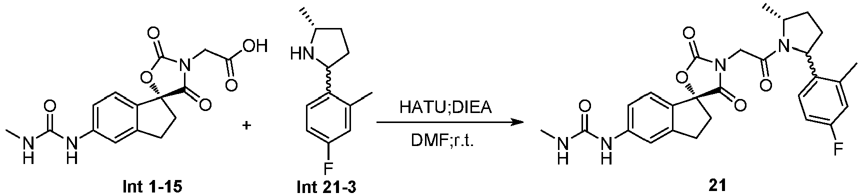

To a 25 mL round-bottomed flask containing 1 mL DMF, were respectively added **Int 1-15** (30 mg; 0.09 mmol), **Int 21-3** (17 mg; 0.09 mmol), DIEA (18 mg; 0.14 mmol), and HATU(34 mg; 0.09 mmol), and then the reaction solution was stirred at room temperature until the reaction was completed by TLC detection. H₂O (5 mL) was added, and the resultant solution was extracted with EA (5 mL × 3), and purified by scratching TLC plate, to obtain white solid powder **21** (30 mg), with a yield of 65%. MS: *m*/*z* 509 [M+H]⁺.

### Example 25 Synthesis of compound 1-((R)-3'-(2-2-(4-fluoro-2-(trifluoromethyl) phenyl)-5-methylpyrrolidin-1-yl)-2-oxoethyl)-2',4'-dioxo-2,3-dihydrospiro[indene-1,5'-oxazolidine]-5-yl)-3-methylurea (25)

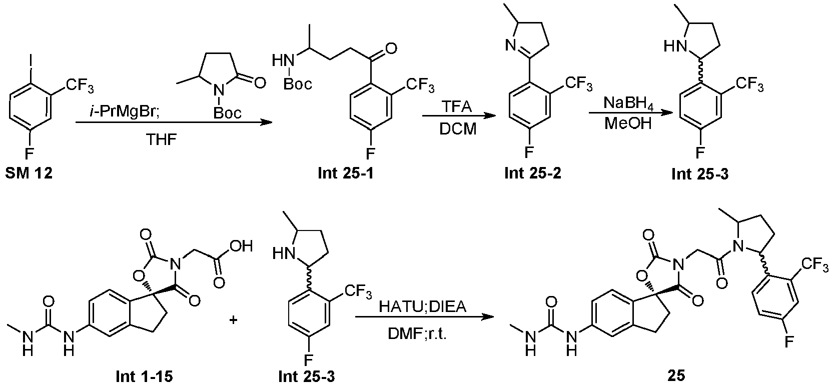

### Synthesis of intermediate (R)-t-butyl-(5-(4-fluoro-2-(trifluoromethyl)phenyl)-5- oxopentan-2-yl)carbamate (Int 25-1)

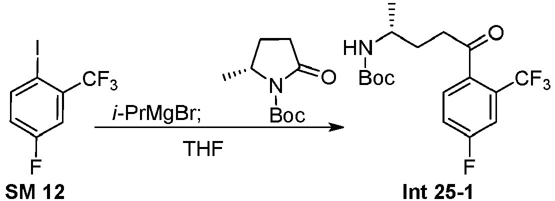

To a reaction flask, was added dry THF (30 mL), followed by addition of **SM 12** (2.9 g, 10 mmol), and then under N₂ protection, isopropylmagnesium bromide (10 mL, 1M in THF) was added in an ice-water bath. After that, the mixture was reacted at room temperature for 7 h, and (R)-t-butyl-2-methyl-5-oxopyrrolidin-1-carboxylate (1.99 g, 10 mmol) was finally added, and the mixture was further allowed to react at room temperature for 12 h. The reaction solution was poured into the saturated aqueous solution of ammonium chloride, extracted with 30 mL (10 mL×3) of ethyl acetate. The organic phases were combined, dried over anhydrous Na₂SO₄, and purified by column chromatography to obtain **Int 25-1** (1.82 g, 5 mmol), with a yield of 50%. MS: *m*/*z* 364 [M+H]⁺.

### Synthesis of intermediate (R)-5-(4-fluoro-2-(trifluoromethyl)phenyl)-2-methyl-3,4-dihydro-2H-pyrrole (Int 25-2)

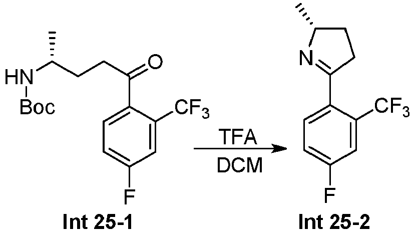

To a reaction flask was added DCM (20 mL), to which was then added **Int 25-1** (1.82 g, 5 mmol), followed by addition of TFA (2 mL). The mixture was reacted at room temperature for 7 h. The reaction solution was poured into the saturated aqueous solution of sodium bicarbonate, and extracted with 30 mL (10 mL × 3) of DCM. The organic phases were combined, dried over anhydrous Na₂SO₄, and purified by column chromatography, to obtain **Int 25-2** (1.1 g, 4.5 mmol), with a yield of 90%. MS: *m*/*z* 246 [M+H]⁺.

### Synthesis of intermediate 2-(4-fluoro-2-(trifluoromethyl)phenyl)-5-methylpyrrolidine (Int 25-3)

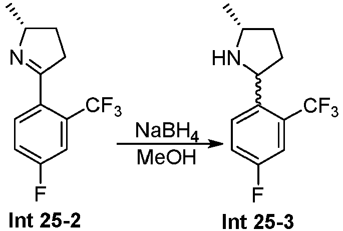

Methanol (15 mL) was added to a reaction flask, to which was then added **Int 25-2** (1.1 g, 4.5 mmol), followed by addition of sodium borohydride (0.76 g, 20 mmol) in batches. After that, the mixture was reacted at room temperature for 1 h. The reaction solution was poured into water, and extracted with 30 mL (10 mL×3) of ethyl acetate. The organic phases were combined, dried over anhydrous Na₂SO₄, and concentrated to obtain the isomer mixture, which was separated to provide **Int 25-3** (0.96 g, 3.9 mmol), with a yield of 86%. MS: *m*/*z* 248 [M+H]⁺.

### Synthsis of compound 1-((R)-3'-(2-2-(4-fluoro-2-(trifluoromethyl)phenyl)-5-methylpyrrolidin-1-yl)-2-oxoethyl)-2',4'-dioxo-2,3-dihydrospiro[indene-1, 5'-oxazolidine]-5-yl)-3-methylurea (25)

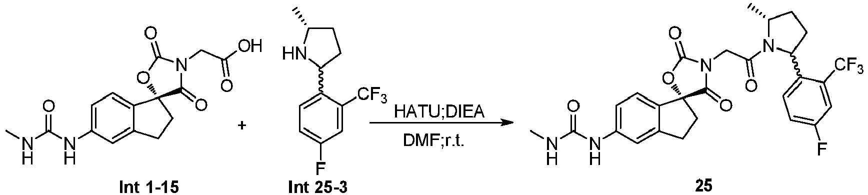

To a 25 mL round-bottomed flask containing 1 mL DMF, were respectively added **Int 1-15** (30 mg; 0.09 mmol), **Int 25-3** (22 mg; 0.09 mmol), and HATU(34 mg; 0.09 mmol), and then the reaction solution was stirred at room temperature until the reaction was completed by TLC detection. H₂O (5 mL) was added, and the resultant solution was extracted with EA (5 mL × 3), and purified by scratching TLC plate, to obtain white solid powder **25** (27 mg), with a yield of 50%. MS: *m*/*z* 563 [M+H]⁺.

### Example 39 Synthesis of compound 1-((R)-3'-(2-(5-(4-fluorophenyl)-2,2-dimethylpyrrolidin-1-yl)-2-oxoethyl)-2',4'-dioxo-2,3-dihydrospirofindene-1,5'-oxazolidine]-5-yl)-3-methylurea (39)

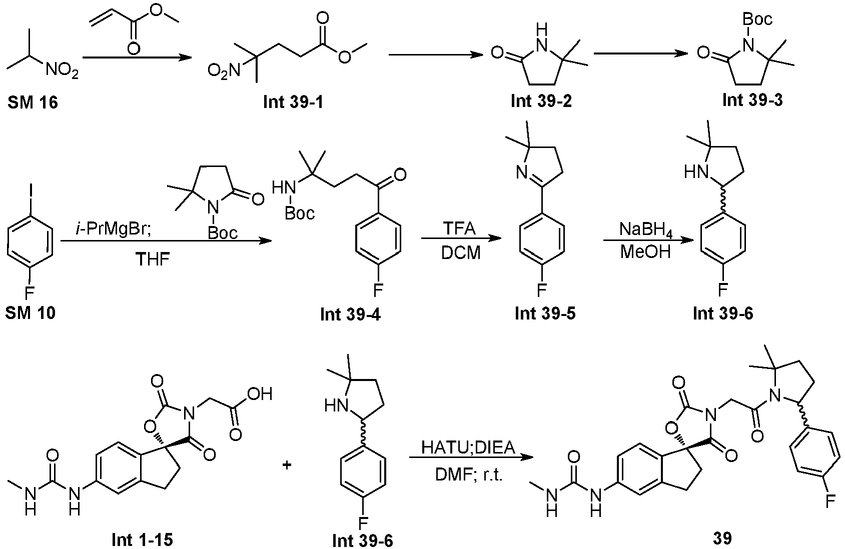

### Synthesis of intermediate compound 4-methyl-4-nitrovaleric acid methyl ester (Int 39-1)

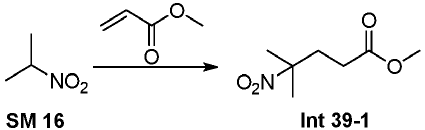

To a reaction flask was introduced DCM (40 mL), to which were then added **SM 16** (4.4 g, 0.05 mol) and methyl acrylate (4.3 g, 0.05 mol), followed by addition of DBU (3 mL). After that, the mixture was reacted at room temperature for 24 h. The reaction solution was poured into water, and extracted with 30 mL (10 mL×3) of DCM. The organic phases were combined, dried over anhydrous Na₂SO₄, and purified by column chromatography, to obtain **Int 39-1** (5.25 g, 0.03 mmol), with a yield of 60%.

### Synthesis of intermediate compound 5,5-dimethylpyrrolidin-2-one (Int 39-2)

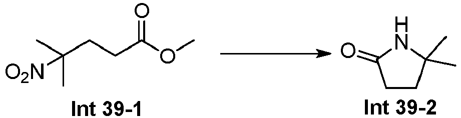

To a reaction flask was introduced MeOH (50 mL), to which were then added **Int 39-1** (5.2 g, 0.03 mol) and Pd/C (400 mg), and then the hydrogenation reaction was carried out at room temperature for 24 h. The reaction solution was poured into water, and extracted with 30 mL (10 mL×3) of DCM. The organic phases were combined, dried over anhydrous Na₂SO₄, and purified by column chromatography, to obtain **Int 39-2** (2.6 g, 0.023 mmol), with a yield of 77%.

### Synthesis of intermediate 2,2-dimethyl-5-oxopyrrolidin-1-carboxylic acid t-butyl ester (Int 39-3)

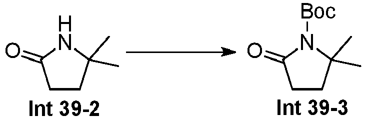

To a reaction flask was introduced DCM (20 mL), to which were then added **Int 39-2** (2.6 g, 0.023 mol) and DMAP (800 mg), and the mixture was reacted at room temperature for 24 h. The reaction solution was poured into water, and extracted with 30 mL (10 mL×3) of DCM. The organic phases were combined, dried over anhydrous Na₂SO₄, and purified by column chromatography, to obtain **Int 39-3** (3.9 g, 0.018 mmol), with a yield of 79%. MS: *m*/*z* 214 [M+H]⁺.

### Synthesis of intermediate (5-(4-fluorophenyl)-2-methyl-5-oxopentan-2-yl)carbamic acid t-butyl ester (Int 39-4)

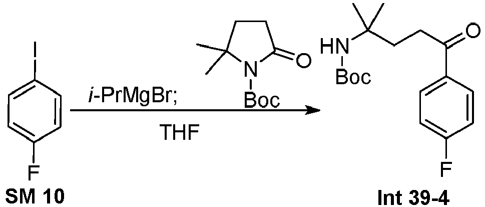

To a reaction flask, was added dry THF (30 mL), followed by addition of **Int 39-3** (2.1 g, 10 mmol), and then under N₂ protection, isopropylmagnesium bromide (10 mL, 1M in THF) was added in an ice-water bath. After that, the mixture was reacted at room temperature for 7 h, and **SM 10** (2.13 g, 10 mmol) was finally added, and the mixture was further allowed to react at room temperature for 12 h. The reaction solution was poured into the saturated aqueous solution of ammonium chloride, extracted with 30 mL (10 mL×3) of ethyl acetate.

The organic phases were combined, dried over anhydrous Na₂SO₄, and purified by column chromatography to obtain **Int 39-4** (1.7 g, 5.5 mmol), with a yield of 55%. MS: *m*/*z* 310 [M+H]⁺.

### Synthesis of intermediate 5-(4-fluorophenyl)-2,2-dimethyl-3,4-dihydro-2H-pyrrole (Int 39-5)

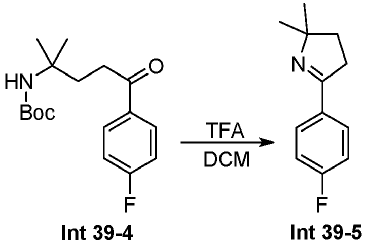

To a reaction flask was introduced DCM (20 mL), to which were then added **Int 39-4** (1.54 g, 5 mmol), followed by addition of TFA (6 mL). The mixture was reacted at room temperature for 7 h. The reaction solution was poured into the saturated aqueous solution of sodium bicarbonate, and extracted with 30 mL (10 mL × 3) of DCM. The organic phases were combined, dried over anhydrous Na₂SO₄, and purified by column chromatography, to obtain **Int 39-5** (864 mg, 4.5 mmol), with a yield of 90%. MS: *m*/*z* 192 [M+H]⁺.

### Synthesis of intermediate 5-(4-fluorophenyl)-2,2-dimethylpyrrolidine (Int 39-6)

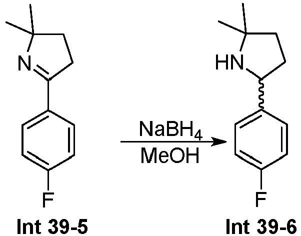

Methanol (15 mL) was added to a reaction flask, to which was then added **Int 39-5** (768 g, 4 mmol), followed by addition of sodium borohydride (0.76 g, 20 mmol) in batches. After that, the mixture was reacted at room temperature for 1 h. The reaction solution was poured into water, and extracted with 30 mL (10 mL×3) of EA. The organic phases were combined, dried over anhydrous Na₂SO₄, and concentrated to obtain **Int 39-6** (718 mg, 3.9 mmol), with a yield of 94%. MS: *m*/*z* 194 [M+H]⁺.

### Synthesis of compound 1-((R)-3'-(2-(5-(4-fluorophenyl)-2,2-dimethylpyrrolidin- 1-yl)-2-oxoethyl)-2',4'-dioxo-2,3-dihydrospiro[indene-1,5'-oxazolidine]-5-yl)-3-methylurea (39)

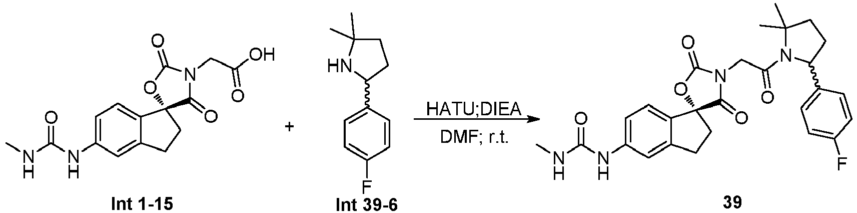

DMF (3 mL) was added to a 25 mL reaction flask, to which were then added **Int 1-15** (333 mg, 1 mmol), **Int 39-6** (194 mg, 1 mmol), and DIEA (387 mg, 3 mmol), and finally HATU (381 mg, 1 mmol) was added. The system was reacted at 20 °C for 1 h. After completion of the reaction, the reaction solution was poured into water (20 mL), and extracted with 30 mL (10 mL×3) of ethyl acetate. The organic phases were combined, dried over anhydrous Na₂SO₄, and purified by column chromatography to obtain compound **39** (280 mg), with a yield of 55%. MS: *m*/*z* 509 [M+H]⁺.

### Example 43 Synthesis of compound 1-((R)-3'-(2-((2S,5S)-2-ethyl-5-(4-fluorophenyl) pyrrolidin-1-yl)-2-oxoethyl)-2',4'-dioxo-2,3-dihydrospiro[indene-1, 5'-oxazolidine]-5-yl)-3-methylurea (43)

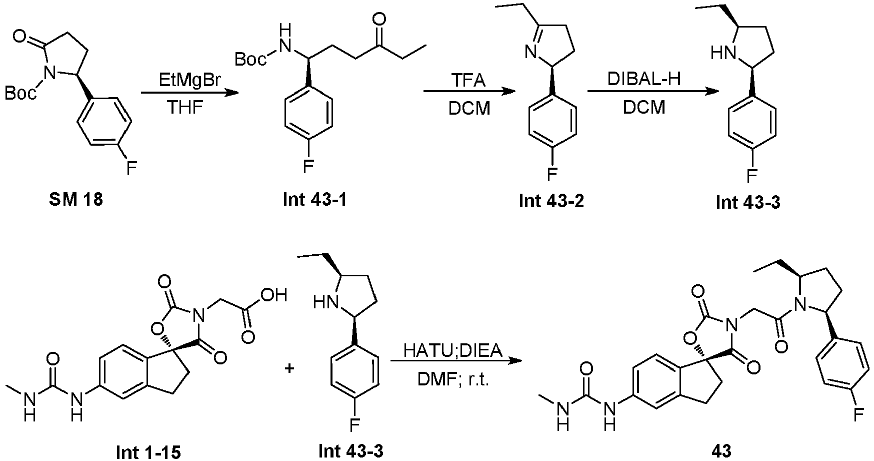

### Synthesis of intermediate (R)-(1-(4-fluorophenyl)-4-oxohexyl)carbamic acid t-butyl ester (Int 43-1)

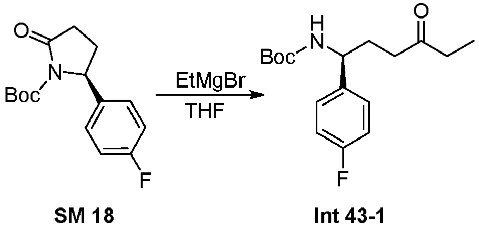

To a reaction flask, was added dry THF (30 mL), followed by addition of **SM 18** (2.8 g, 10 mmol), and then under N₂ protection, ethylmagnesium bromide (10 mL, 1M in THF) was added in an ice-water bath. After that, the mixture was reacted at room temperature for 12 h. The reaction solution was poured into the saturated aqueous solution of ammonium chloride, extracted with 30 mL (10 mL×3) of ethyl acetate. The organic phases were combined, dried over anhydrous Na₂SO₄, and purified by column chromatography to obtain **Int. 43-1** (2.17 g, 7 mmol), with a yield of 70%. MS: *m*/*z* 310 [M+H]⁺.

### Synthesis of intermediate (S)-5-ethyl-2-(4-fluorophenyl)-3,4-dihydro-2H-pyrrole (Int 43-2)

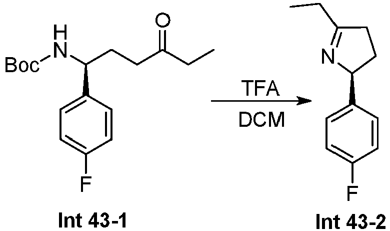

To a reaction solution was added DCM (20 mL), to which was then added **Int 43-1** (1.54 g, 5 mmol), followed by addition of TFA (6 mL). After that, the mixture was reacted at room temperature for 7 h, and the reaction solution was poured into the saturated aqueous solution of sodium bicarbonate. The resultant solution was extracted with 30 mL (10 mL × 3) of DCM. The organic phases were combined, dried over anhydrous Na₂SO₄, and purified by column chromatography, to obtain **Int 43-2** (764 mg, 4 mmol), with a yield of 80%. MS: *m*/*z* 192 [M+H]⁺.

### Synthesis of intermediate (2S,SS)-2-ethyl-5-(4-fluorophenyl)pyrrole (Int 43-3)

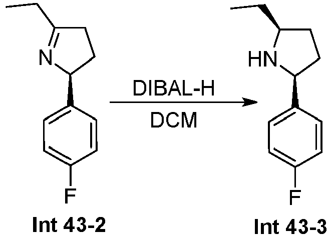

To a reaction flask was added DCM (15 mL), to which was then added **Int 43-2** (764 mg, 4 mmol), followed by addition of DIBAL-H (5 mL, 1M in hexane) in ice-water bath. After that, the mixture was reacted at room temperature for 1 h, and quenched by adding a small amount of water. The solution was filtered, washed with DCM (1 mL×3), and concentrated, to obtain **Int 43-3** (694 mg, 3.6 mmol), with a yield of 90%. MS: *m*/*z* 194 [M+H]⁺. ¹H NMR (400 MHz, CDCl₃) δ 7.37 (dt, J = 9.1, 4.7 Hz, 2H), 6.99 (t, J = 8.7 Hz, 2H), 4.15 (dd, J = 14.6, 7.0 Hz, 1H), 3.13 (dd, J = 13.9, 7.0 Hz, 1H), 2.23-2.08 (m, 1H), 2.07-1.94 (m, 1H), 1.80-1.65 (m, 1H), 1.63-1.46 (m, 3H), 1.25 (m, 1H), 0.95 (ddd, J = 9.6, 5.5, 2.5 Hz, 3H).

### Synthesis of compound 1-((R)-3'-(2-((2S,5S)-2-ethyl-5-(4-fluorophenyl)pyrrolidin-1-yl)-2-oxoethyl)-2',4'-dioxo-2,3-dihydrospiro[indene-1,5'-oxazolidine]-5-yl)-3-methylurea (43)

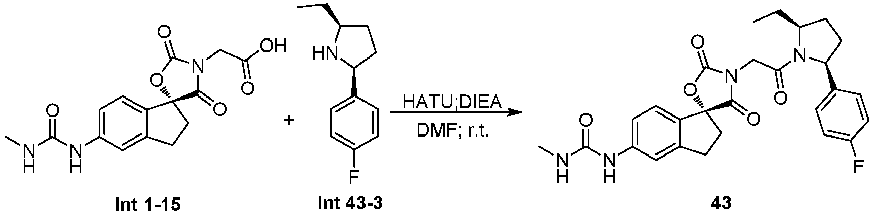

DMF (3 mL) was added to a 25 mL reaction flask, to which were then added **Int 1-15** (333 mg, 1 mmol), **Int 43-3** (193 mg, 1 mmol), and DIEA (387 mg, 3 mmol), and finally HATU (381 mg, 1 mmol) was added. The system was reacted at 20 °C for 1 h. After completion of the reaction, the reaction solution was poured into water (20 mL), and extracted with 30 mL (10 mL×3) of ethyl acetate. The organic phases were combined, dried over anhydrous Na₂SO₄, and purified by column chromatography to obtain compound **43** (279 mg), with a yield of 55%. MS: *m*/*z* 509 [M+H]⁺.

### Example 47 Synthesis of compound 1-((R)-3'-(2-((2S,5R)-2-(4-fluorophenyl)-5-isopropylpyrrol-1-yl)-2-oxoethyl)-2',4'-dioxo-2,3-dihydrospiro[indene-1, 5'-oxazolidine]-5-yl)-3-methylurea (47)

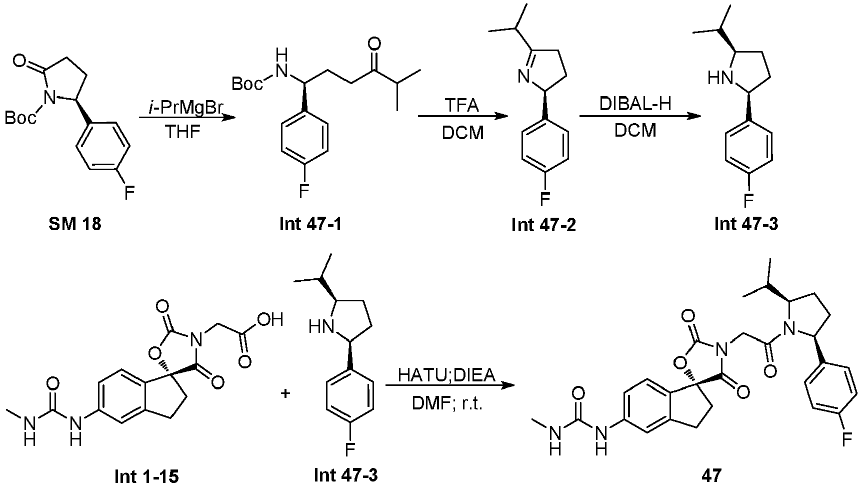

### Synthesis of intermediate (S)-(1-(4-fluorophenyl)-5-methyl-4-oxohexyl)carbamic acid t-butyl ester(Int 47-1)

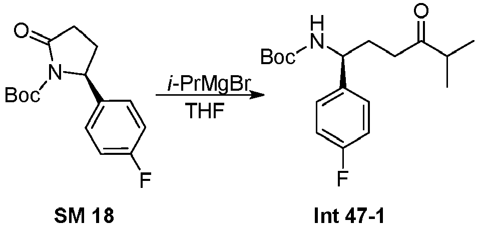

To a reaction flask, was added dry THF (30 mL), followed by addition of **SM 18** (2.8 g, 10 mmol), and then under N₂ protection, isopropylmagnesium bromide (10 mL, 1M in THF) was added in an ice-water bath. After that, the mixture was reacted at room temperature for 12 h. The reaction solution was poured into the saturated aqueous solution of ammonium chloride, extracted with 30 mL (10 mL×3) of ethyl acetate. The organic phases were combined, dried over anhydrous Na₂SO₄, and purified by column chromatography to obtain **Int. 47-1** (2.2 g, 7 mmol), with a yield of 70%. MS: *m*/*z* 324 [M+H]⁺.

### Synthesis of intermediate (S)-2-(4-fluorophenyl)-5-isopropyl-3,4-dihydro-2H-pyrrole (Int 47-2)

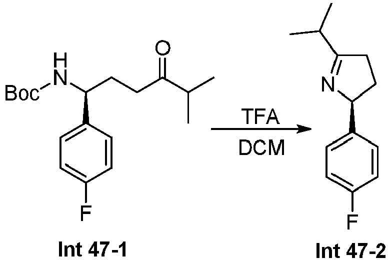

To a reaction solution was added DCM (20 mL), to which was then added **Int 47-1** (1.62 g, 5 mmol), followed by addition of TFA (2 mL). After that, the mixture was reacted at room temperature for 7 h, and the reaction solution was poured into the saturated aqueous solution of sodium bicarbonate. The resultant solution was extracted with 30 mL (10 mL × 3) of DCM. The organic phases were combined, dried over anhydrous Na₂SO₄, and purified by column chromatography, to obtain **Int 47-2** (820 mg, 4 mmol), with a yield of 80%. MS: *m*/*z* 206 [M+H]⁺.

### Synthesis of intermediate (2S,5R)-2-(4-fluorophenyl)-5-isopropylpyrrolidine (Int 47-3)

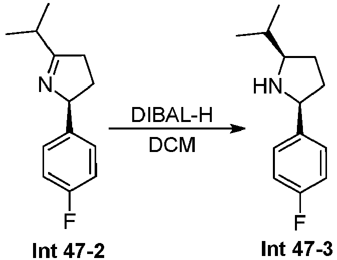

To a reaction flask was added DCM (15 mL), to which was then added **Int 47-2** (820 mg, 4 mmol), followed by addition of DIBAL-H (5 mL, 1M in hexane) in ice-water bath. After that, the mixture was reacted at room temperature for 1 h, and quenched by adding a small amount of water. The solution was filtered, washed with DCM (15 mL×3), and concentrated, to obtain **Int 43-7** (749 mg, 3.6 mmol), with a yield of 90%. MS: *m*/*z* 208 [M+H]⁺. ¹H NMR (400 MHz, CDCl₃) δ 7.42-7.34 (m, 2H), 7.03-6.94 (m, 2H), 4.14 (t, J = 7.8 Hz, 1H), 2.90 (q, J = 7.7 Hz, 1H), 2.12 (dddd, J = 11.9, 9.4, 6.8, 5.2 Hz, 1H), 1.98-1.88 (m, 1H), 1.72-1.64 (m, 1H), 1.61-1.53 (m, 1H),1.25 (m, 1H), 0.97 (d, J = 6.6 Hz, 3H), 0.93-0.85 (m, 4H).

### Synthesis of compound 1-((R)-3'-(2-((2S,5R)-2-(4-fluorophenyl)-5-isopropylpyrrol- 1-yl)-2-oxoethyl)-2',4'-dioxo-2,3-dihydrospiro[indene-1,5'-oxazolidine]-5-yl)-3-methylurea (47)

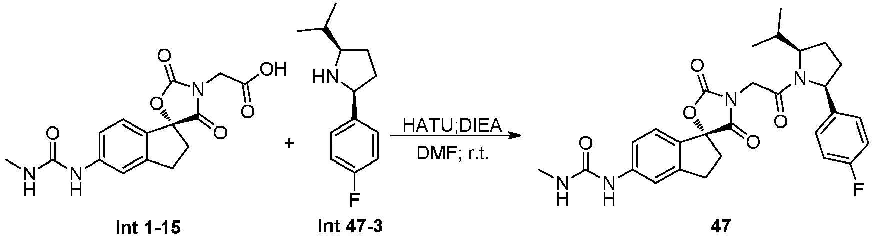

DMF (3 mL) was added to a 25 mL reaction flask, to which were then added **Int 1-15** (333 mg, 1 mmol), **Int 47-3** (208 mg, 1 mmol), and DIEA (387 mg, 3 mmol), and finally HATU (381 mg, 1 mmol) was added. The system was reacted at 20 °C for 1 h. After completion of the reaction, the reaction solution was poured into water (20 mL), and extracted with 30 mL (10 mL×3) of ethyl acetate. The organic phases were combined, dried over anhydrous Na₂SO₄, and purified by column chromatography to obtain compound **47** (266 mg), with a yield of 51%. MS: *m*/*z* 523 [M+H]⁺.

### Example 57 Synthesis of compound 1-((R)-3'-(2-(2-(2-ethyl-4-fluorophenyl)-5-methylpyrrolidin-1-yl)-2-oxoethyl)-2',4'-dioxo-2,3-dihydrospiro[indene-1, 5'-oxazolidine]-5-yl)-3-methylurea (57)

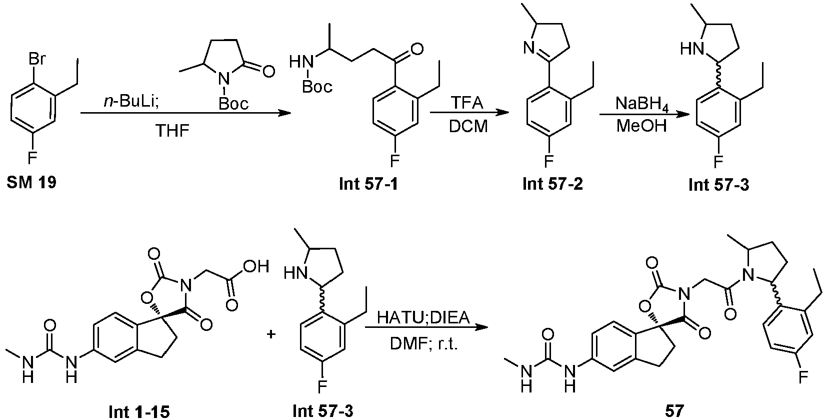

### Synthesis of intermediate (5-(2-ethyl-4-fluorophenyl)-5-oxopentan-2-yl)carbamic acid t-butyl ester (Int 57-1)

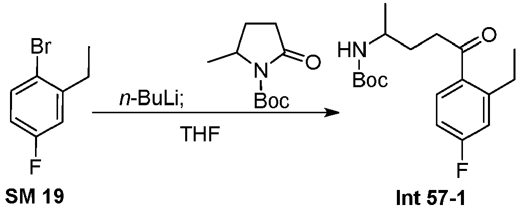

To a 50 mL reaction flask, were added compound **SM 19** (400 mg, 2 mmol) and tetrahydrofuran (4 mL), and the mixture was cooled to -70 °C under nitrogen protection, then butyl lithium (2.5 M in hexane, 0.8 mL, 2 mmol) was added to the reaction solution. After reacting at this temperature for 2 to 3 h, a solution of 2-methyl-5-oxopiperidone-N-formic acid t-butyl ester (400 mg, 2 mmol) in tetrahydrofuran (3 mL) was added. After adding, the reaction was naturally warmed. After completion of the reaction, the saturated aqueous solution of ammonium chloride (5 mL) was added, and the resultant solution was extracted with ethyl acetate (5 mL×3). The organic phases were combined, dried over anhydrous Na₂SO₄, and purified by column chromatography to obtain compound **Int 57-1** (440 mg, yield 68%). MS: *m*/*z* 324.2 [M+H]⁺.

### Synthesis of intermediate 5-(2-ethyl-4-fluorophenyl)-2-methyl-3,4-dihydro-2H- pyrrole (Int 57-2)

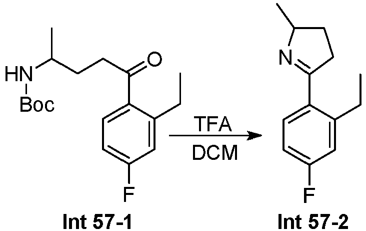

Crude Int 57-1 (440 mg, 1.4 mmol) and dichloromethane (4 mL) were added to a 25 mL reaction flask, to which was then added trifluoroacetic acid (0.4 mL), and the mixture was reacted at room temperature overnight. After the reaction was completed, the solvent was rotatory evaporated *in vacuo.* The aqueous solution of sodium bicarbonate was added to adjust pH to about 7, and then extracted with ethyl acetate (5 mL×3). The organic phases were combined, dried over anhydrous sodium sulfate, and the solvent was removed by rotatory evaporation *in vacuo,* to obtain the crude compound **Int 57-2** (260 mg, yield 93%). MS: *m*/*z* 206.1 [M+H]⁺.

### Synthesis of intermediate 2-(2-ethyl-4-fluorophenyl)-5-methylpyrrolidine (Int 57-3)

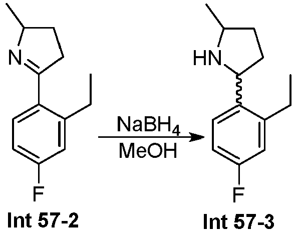

To a 50 mL reaction flask were added **Int 57-2** (260 mg, 1.3 mmol) and methanol (3 mL), to which was added sodium borohydride (48 mg, 1.3 mmol) in batches in an ice bath. After the reaction was completed, water (5 mL) was added, and then extracted with ethyl acetate (5 mL×3). The organic phases were combined, dried with anhydrous Na₂SO₄, and the solvent was removed by rotatory evaporation *in vacuo,* to obtain the crude compound, that was separated to obtain **Int 57-3** (251 mg). MS: *m*/*z* 208.1 [M+H]⁺.

### Synthesis of compound 1-((R)-3'-(2-(2-(2-ethyl-4-fluorophenyl)-5-methylpyrrolidin- 1-yl)-2-oxoethyl)-2',4'-dioxo-2,3-dihydrospiro[indene-1,5'-oxazolidine]-5-yl)-3-methylurea (57)

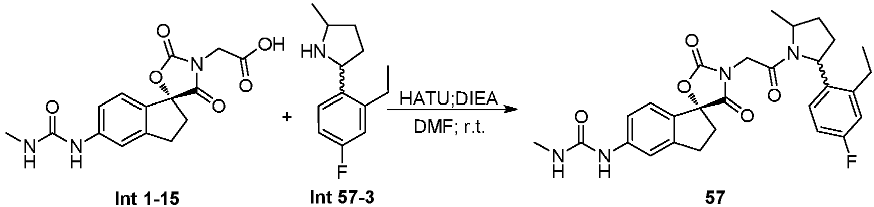

To a 25 mL reaction flask, were added compound **Int 1-15** (33 mg, 0.1 mmol), **Int 57-3** (31 mg, 0.15 mmol), DIEA(39 mg, 0.3 mmol), and DMF (1 mL), to which was added HATU (57 mg, 0.15 mmol) under stirring. The mixture was stirred overnight at room temperature. After the reaction was completed, water (5 mL) was added to the reaction solution, and then extracted with ethyl acetate (5 mL×3). The organic phases were combined, dried with anhydrous Na₂SO₄, and purified by column chromatography to obtain compound **57** (20 mg, yield 38%). MS: *m*/*z* 523.2 [M+H]⁺. ¹H NMR (400 MHz, DMSO) δ 8.72 (s, 1H), 7.53 (d, J = 7.1 Hz, 1H), 7.30-6.87 (m, 5H), 6.09 (d, J = 4.7 Hz, 1H), 5.22 (dt, J = 15.9, 7.1 Hz, 1H), 4.68 (dd, J = 24.4, 16.7 Hz, 1H), 4.47-4.16 (m, 2H), 3.44 (t, J = 17.1 Hz, 1H), 3.08 (dt, J = 15.6, 7.6 Hz, 1H), 3.01-2.91 (m, 1H), 2.89 (s, 1H), 2.82- 2.72 (m, 1H), 2.63 (t, J = 3.9 Hz, 3H), 2.42 (ddd, J = 10.8, 9.9, 5.5 Hz, 2H), 2.05 (dt, J = 14.0, 7.3 Hz, 1H), 1.73 (ddd, J = 20.2, 13.2, 7.1 Hz, 1H), 1.53 (td, J = 12.1, 6.2 Hz, 1H), 1.42 (dd, J = 6.2, 4.3 Hz, 3H), 1.26-1.20 (m, 3H).

### Example 74 Synthesis of compound 1-((R)-3'-(2-((2S,5R)-2-(4-fluorophenyl)-5-(trifluoromethyl)pyrrolidin-1-yl)-2-oxoethyl)-2',4'-dioxo-2,3-dihydrospirofindene-1,5'-oxazolidine]-5-yl)-3-methylurea (74)

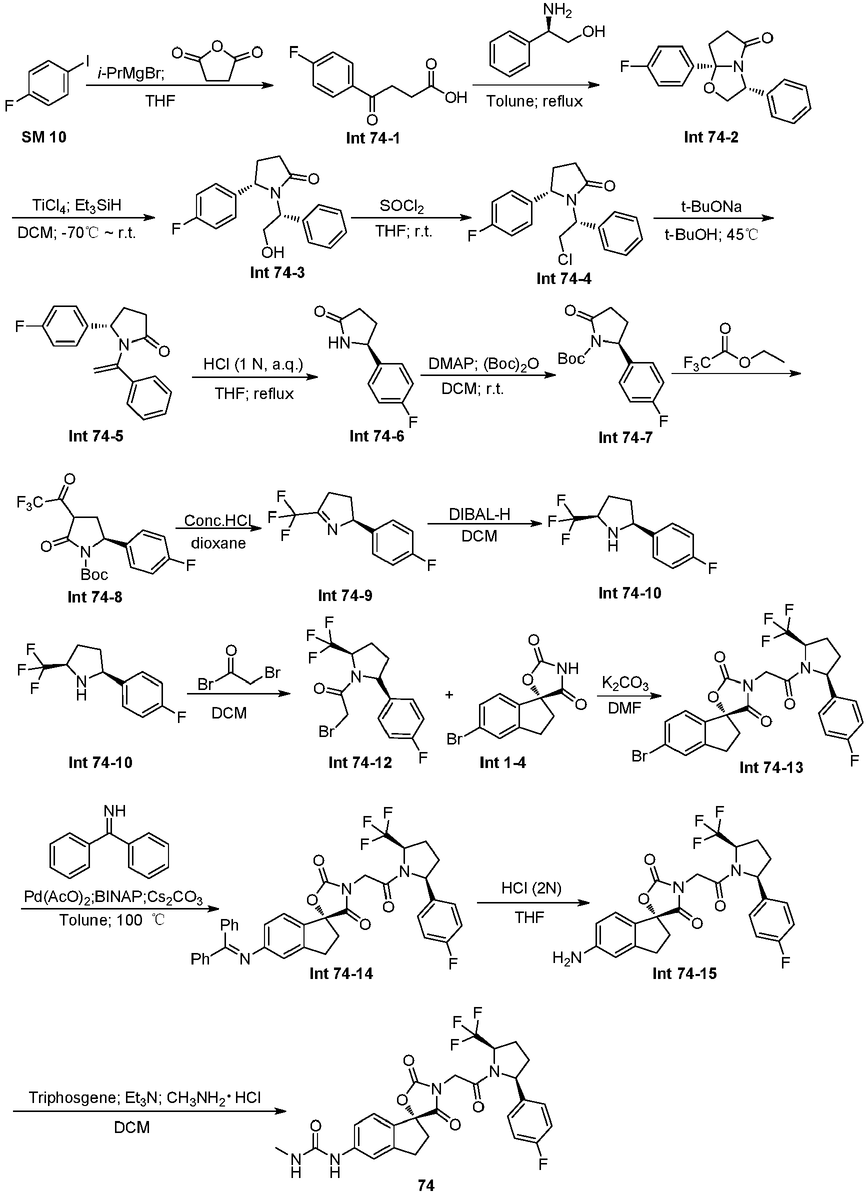

### Synthesis of intermediate (3R,7aR)-7a-(4-fluorophenyl)-3- phenyltetrahydropyrrolidin[2,1-b]oxazol-5(6H)-one (Int 74-2)

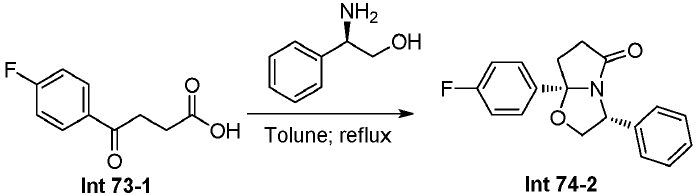

Intermediate **Int 73-1**(6.1 g, 31 mmol) and (R)-2-amino-1-phenylethan-1-ol (4.3 g, 31 mmol) were added into 50 mL toluene, and a water separator was used to divide water, and then the mixture was reacted at 148 °C for 20 h. After concentration and column chromatography, 6 g Int 74-2 was obtained as white solid, with a yield of 66%. MS: *m*/*z* 298 [M+H]⁺.

### Synthesis of intermediate (S)-5-(4-fluorophenyl)-1-((R)-2-hydroxyl-1-phenylethyl) pyrrolidin-2-one (Int 74-3)

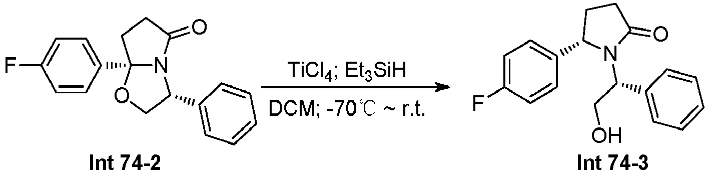

Intermediate **Int 74-2** (6 g, 20 mmol) and triethylsilicon hydride (7.5 g, 65 mmol) were added to 100 mL dichloromethane, to which was drop added titanium tetrachloride solution (20 mL, 1M in hexane) at -78°C. After addition, the reaction was slowly returned to room temperature and stirred for 4 h. The saturated aqueous solution of ammonium chloride was added, and the resultant solution was extracted with ethyl acetate, dried over anhydrous Na₂SO₄, and concentrated, to obtain the crude product, that was directly used in the next step.

### Synthesis of intermediate (S)-5-(4-fluorophenyl)-1-((R)-2-chloro-1-phenylethyl) pyrrolidin-2-one (Int 74-4)

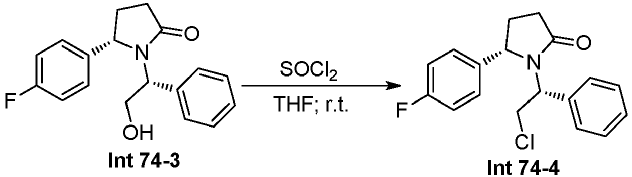

To a 250 mL reaction flask, were added **Int 74-3** (10 g, 33 mmol) and tetrahydrofuran (100 mL), to which thionyl chloride (8 g, 67 mmol) was slowly added in an ice bath. The reaction was naturally warmed to room temperature. The reaction was carried out at room temperature for another 2 h. After completion of the reaction, the solvent was rotatory evaporated under vacuum to obtain the crude compound **Int 74-4** (13 g, yield 122%). MS: *m*/*z* 318.1 [M+H]⁺.

### Synthesis of ((S)-5-(4-fluorophenyl)-1-(1-phenylvinyl)pyrrolidin-2-one (Int 74-5)

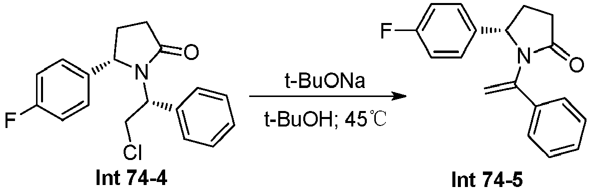

To a 250 mL reaction flask, were added crude **Int 74-4** (13 g, 41 mmol) and tert-butanol (130 mL), to which was then added sodium tert-butoxide (7.9 g, 82 mmol). The reaction was heated to 40 °C and kept for 2 to 3 h. After completion of the reaction, the solvent was rotatory evaporated, and then water (50 mL) was added. The solution was extracted with ethyl acetate (5 mL×3). The organic phases were combined, dried over anhydrous Na₂SO₄, and the solvent was rotatory evaporated, to obtain the crude compound **Int 74-5** (11 g, yield 96%). MS: *m*/*z* 282.1 [M+H]⁺.

### Synthesis of intermediate (R)-5-(4-fluorophenyl)pyrrolidin-2-one (Int 74-6)

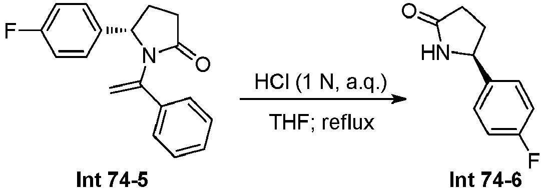

Crude **Int 74-5** (11 g, 39 mmol), 2N aqueous HCl solution (50 mL) and tetrahydrofuran (60 mL) were added to a 250 mL reaction flask, and the reaction was heated to 80 °C and kept for 2 to 3 h. After the reaction was completed, pH value was adjusted to about 7 with the saturated aqueous solution of sodium bicarbonate, and the reaction solution was extracted with ethyl acetate (50 mL×3). The organic phases were combined, dried over anhydrous Na₂SO₄, and the solvent was rotatory evaporated in vacuum. The residue was whirled in ethyl acetate (20 mL) for 1 h, and then filtered to obtain compound **Int 74-6** (5 g, yield 74%). MS: *m*/*z* 180.1 [M+H]⁺.

### Synthesis of intermediate (S)-2-(4-fluorophenyl)-5-oxopyrrolidin-1-carboxylic acid t-butyl ester (Int 74-7)

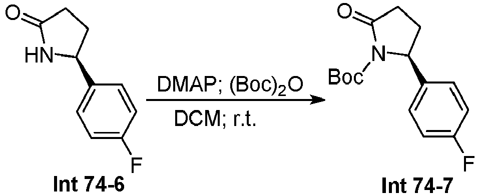

To a 100 mL reaction flask, were added crude **Int 74-6** (5 g, 28 mmol), di-tert-butyl dicarbonate (7.4 g, 34 mmol), and dichloromethane (50 mL), to which was finally added 4-dimethylaminopyridine (3.4 g, 28 mmol) in batches. The reaction was stirred overnight at room temperature. After the reaction was completed, 0.5 N aqueous hydrochloric acid (20 mL) was added, and the layers were separated. The organic phase was dried with anhydrous sodium sulfate, and the solvent was rotatory evaporated in vacuum, followed by purification via column chromatography, to obtain compound **Int 74-7** (7.5 g, yield 96%). MS: *m*/*z* 280.1 [M+H]⁺.

### Synthesis of intermediate (5S)-5-(4-fluorophenyl)-2-oxo-3-(2,2,2-trifluoroacetyl) pyrrolidin-1-carboxylic acid t-butyl ester (Int 74-8):

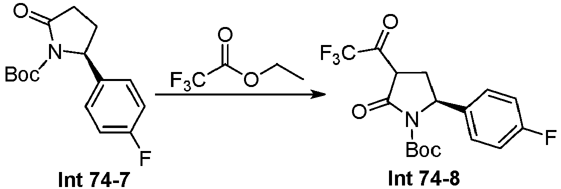

Intermediate **Int 74-7** (2.8 g, 10 mmol) and ethyl trifluoroacetate (2.8 g, 20 mmol) were dissolved in 50 mL of tetrahydrofuran, to which was added sodium hydride (0.8 g, 20 mmol), and the mixture was heated to 50 °C, and reacted for 4 h. The reaction solution was poured into cold water, and its pH was adjusted to 5 to 6 with 2 N hydrochloric acid. The reaction solution was extracted with ethyl acetate, dried over anhydrous Na₂SO₄, and concentrated, to obtain the crude product that was directly used in the next step.

### Synthesis of intermediate (S)-2-(4-fluorophenyl)-5-(trifluoromethyl)-3,4-dihydro-2H-pyrrole (Int 74-9)

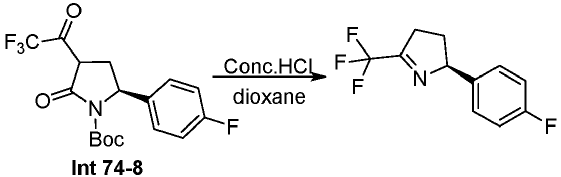

The crude product obtained in the previous step was dissolved in 10 mL dioxane, to which was added 30 mL concentrated hydrochloric acid. The mixture was heated to 100 °C, and stirred overnight. The pH was adjusted to 7 to 8 with sodium bicarbonate, and the reaction solution was extracted with ethyl acetate, dried over anhydrous sodium sulfate, and concentrated. The residue was dissolved in 20 mL of dichloromethane, to which was added 1 mL of trifluoroacetic acid. The reaction was stirred overnight at room temperature and concentrated, to which was added water. Its pH was adjusted to 7 to 8, and then the solution was extracted with ethyl acetate, dried over anhydrous sodium sulfate, concentrated, and purified by column chromatography to obtain 1.5 g **Int 74-9** as colorless liquid, with a two-step yield of 65%. MS: *m*/*z* 232 [M+H]⁺.

### Synthesis of intermediate (2S,5R)-2-(4-fluorophenyl)-5-(trifluoromethyl)pyrrolidine (Int 74-10)

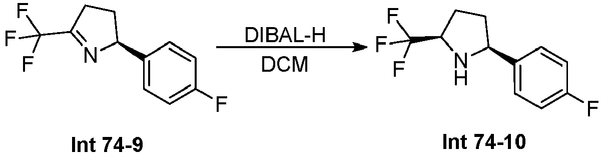

Intermediate **Int 74-9** (1.65 g, 5 mmol) was dissolved in 50 mL dichloromethane, to which was added DIBAL-H (5.5 mL, 1 M in hexane) in an ice bath, and the mixture was stirred for 4 h, then diatomite was added, followed by addition of 10 mL of water. The solution was filtered, extracted with ethyl acetate, dried with anhydrous sodium sulfate, concentrated, and purified by column chromatography, to obtain a pale yellow liquid **Int 74-10** (740 mg), with a yield of 65%. ¹H NMR (400 MHz, CDCl₃) δ 7.42-7.36 (m, 2H), 7.03-6.96 (m, 2H), 4.32 (dd, J = 9.7, 5.2 Hz, 1H), 3.81 (ddd, J = 14.1, 12.5, 6.9 Hz, 1H), 2.16-2.03 (m, 3H), 1.70 (dq, J = 9.6, 8.5 Hz, 1H).

### Synthesis of intermediate 2-bromo-1-((2S,SR)-2-(4-fluorophenyl)-5-(trifluoromethyl) pyrrolidin-1-yl)ethan-1-one (Int 74-12)

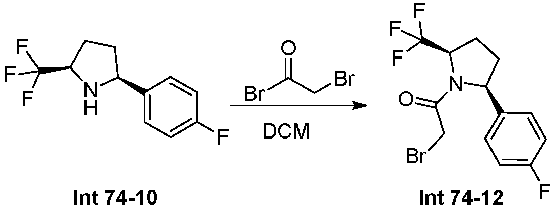

Intermediate **Int 74-10** (233 mg, 10 mmol) was dissolved in 10 mL dichloromethane, to which bromoacetyl bromide (200 mg, 10 mmol) was added dropwise in an ice bath. The mixture was stirred for 1 h, extracted with dichloromethane, and purified by column chromatography to obtain 320 mg **Int 74-12** as white solid, with a yield of 90%. MS: *m*/*z* 354, 356 [M+H]⁺.

### Synthesis of intermediate (R)-5-bromo-3'-(2-((2S,5R)-2-(4-fluorophenyl)-5-(trifluoromethyl)pyrrolidin-1-yl)-2-oxoethyl)-2,3-dihydrospiro[indene-1,5'-oxazolidine]-2',4'-dione (Int 74-13)

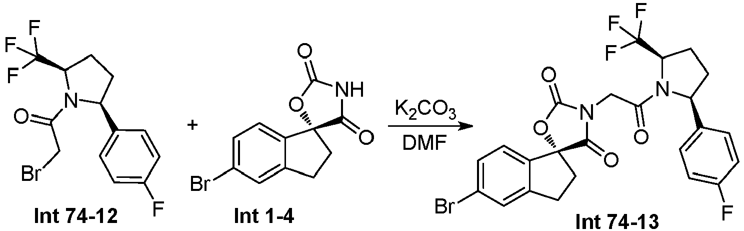

**Int 1-4** (140 mg, 5 mmol), intermediate **Int 74-12** (176 mg, 5 mmol) and potassium carbonate (140 mg, 10 mmol) were dissolved in 10 mL N,N-dimethylformamide, and the mixture was stirred at room temperature for 4 h, to which was added 50 mL of water. The solution was filtere and dried, to obtain 500 mg of **Int 74-13** as white solid, with a yield of 90%. MS: *m*/*z* 555, 557 [M+H]⁺.

### Synthesis of intermediate (R)-5-(diphenylmethylene)amino)-3'-(2-((2S,5R)-2- (4-fluorophenyl)-5-(trifluoromethyl)pyrrolidin-1-yl)-2-oxoethyl)-2,3-dihydrospiro[indene-1,5'-oxazolidine]-2',4'-dione (Int 74-14)

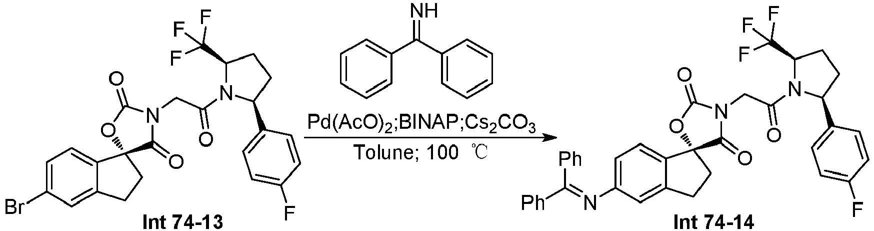

Intermediate **Int 74-13** (100 mg, 0.28 mmol), benzophenonimine (62 mg, 0.34 mmol), palladium acetate (13 mg, 0.06 mmol), 2,2'-bis(diphenylphosphino)-1,1'-binaphthyl (72 mg, 0.12 mmol), and cesium carbonate (136 mg, 0.42 mmol) were dissolved in 5 mL toluene, and the system was purged with N₂. The reaction was heated to 100 °C and stirred for 4 h, followed by concentration and column chromatography, to obtain 110 mg of **Int 74-14** as grey solid, with a yield of 84%. MS: *m*/*z* 656 [M+H]⁺.

### Synthesis of intermediate (R)-5-amino-3'-(2-((2S,5R)-2-(4-fluorophenyl)-5-(trifluoromethyl)pyrrolidin-1-yl)-2-oxoethyl)-2,3-dihydrospiro[indene-1,5'-oxazolidine]-2',4'-dione (Int 74-15)

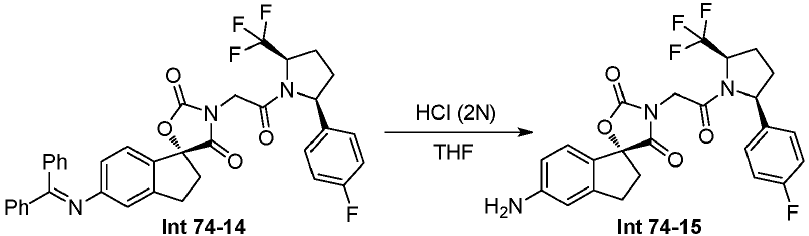

Intermediate **Int 74-14** (110 mg, 0.24 mmol) was dissolved in 5 mL tetrahydrofuran, to which was added 2N hydrochloric acid (5 mL), and the mixture was stirred for 30 min, followed by extracting with ethyl acetate three times. The organic phase was combined, and dried over anhydrous Na₂SO₄, concentrated, and purified by column chromatography to obtain **Int 74-15** (62 mg) as yellow solid, with a yield of 88%. MS: *m*/*z* 448 [M-43].

### Synthesis of compound 1-((R)-3'-(2-((2S,5R)-2-(4-fluorophenyl)-5-(trifluoromethyl) pyrrolidin-1-yl)-2-oxoethyl)-2',4'-dioxo-2,3-dihydrospiro[indene-1, 5'-oxazolidine]-5-yl)-3-methylurea (74)

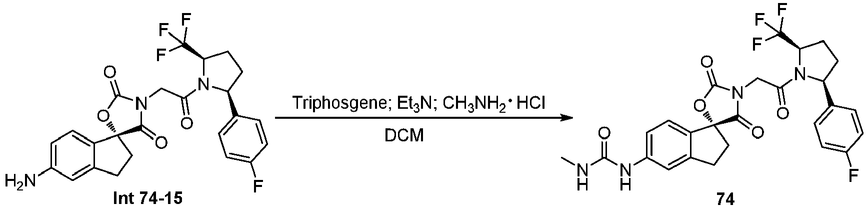

Triphosgene (35 mg, 12 mmol) was placed in dichloromethane (5 mL). The mixed solution of intermediate **Int. 74-15** (6.9 mg, 0.2 mmol), triethylamine (200 mg, 2 mmol), and dichloromethane (5 mL) was slowly dropped into the reaction flask in an ice bath. Then, the mixture was stirred for 1 h, to which was added methylamine hydrochloride (33 mg, 0.5 mmol), and the reaction was further stirred for 4 h. 100 mL of water was added, the organic layer was separated, and then extracted with dichloromethane. The organic phases were combined, dried over anhydrous sodium sulfate, concentrated, and purified by column chromatography, to obtain yellow solid (30 mg), with a yield of 45%. MS: *m*/*z* 549 [M+H]⁺.

### Example 96 Synthesis of compound 1-((1R)-3'-(2-(2-cyclohexyl-5-methylpyrrolidin- 1-yl)-2-oxoethyl)-2',4'-dioxo-2,3-dihydrospiro[indene-1,5'-oxazolidine]-5-yl)-3-methylurea (96)

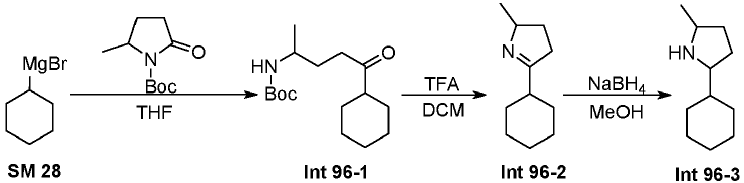

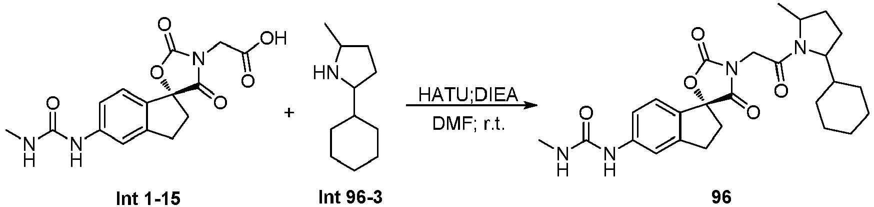

### Synthesis of intermediate (5-cyclohexyl-5-oxopentan-2-yl)carbamic acid t-butyl ester (Int 96-1)

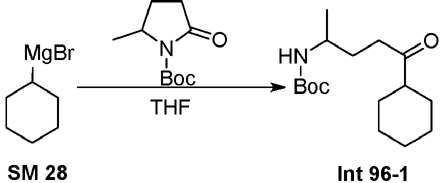

To a reaction flask, was added dry THF (30 mL), followed by addition of **SM 28** (10 mL, 10 mmol), and then under N₂ protection, 2-methyl-5-oxopyrrolidin-1-carboxylic acid t-butyl ester (2 g, 10 mmol) was added in an ice-water bath. After that, the mixture was reacted at room temperature for 12 h. The reaction solution was poured into the saturated aqueous solution of ammonium chloride, extracted with 30 mL (10 mL×3) of ethyl acetate. The organic phases were combined, dried over anhydrous Na₂SO₄, and purified by column chromatography to obtain **Int 96-1** (1.6 g, 5.5 mmol), with a yield of 55%. MS: *m*/*z* 284 [M+H]⁺.

### Synthesis of intermediate 5-cyclohexyl-2-methyl-3,4-dihydro-2H-pyrrole (Int 96-2)

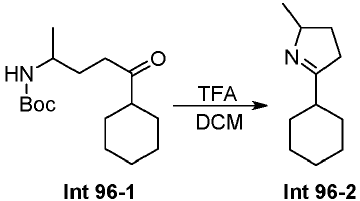

To a reaction solution was added DCM (20 mL), to which was then added **Int 96-1** (1.42 g, 5 mmol), followed by addition of TFA (2 mL). After that, the mixture was reacted at room temperature for 7 h, and the reaction solution was poured into the saturated aqueous solution of sodium bicarbonate. The resultant solution was extracted with 30 mL (10 mL × 3) of DCM. The organic phases were combined, dried over anhydrous Na₂SO₄, and purified by column chromatography, to obtain **Int 96-2** (660 mg, 4 mmol), with a yield of 80%. MS: *m*/*z* 166 [M+H]⁺.

### Synthesis of intermediate 2-cyclohexyl-5-methylpyrrolidine (Int 96-3)

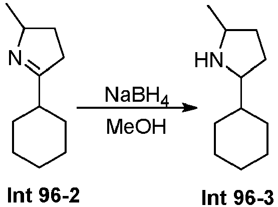

Methanol (15 mL) was added to a reaction flask, to which was then added **Int 96-2** (660 g, 4 mmol), followed by addition of sodium borohydride (0.76 g, 20 mmol) in batches. After that, the mixture was reacted at room temperature for 1 h. The reaction solution was poured into water, and extracted with 30 mL (10 mL×3) of EA. The organic phases were combined, dried over anhydrous Na₂SO₄, and concentrated to obtain **Int 96-3** (618 mg, 3.7 mmol), with a yield of 94%. MS: *m*/*z* 168 [M+H]⁺.

### Synthesis of compound 1-((1R)-3'-(2-(2-cyclohexyl-5-methylpyrrolidin- 1-yl)-2-oxoethyl)-2',4'-dioxo-2,3-dihydrospiro[indene-1,5'-oxazolidine]-5-yl)-3-methylurea (96)

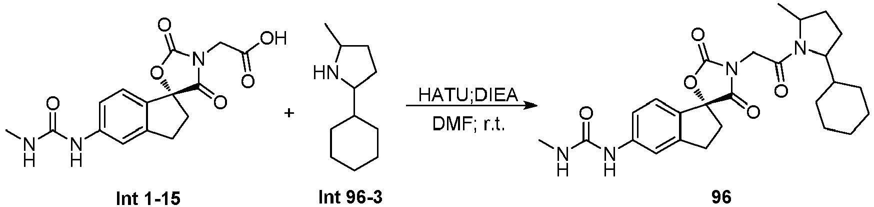

DMF (3 mL) was added to a 25 mL reaction flask, to which were then **added Int 1-15** (333 mg, 1 mmol), **Int 96-3** (167 mg, 1 mmol), and DIEA (387 mg, 3 mmol), and finally HATU (381 mg, 1 mmol) was added. The system was reacted at 20 °C for 1 h. After completion of the reaction, the reaction solution was poured into water (20 mL), and extracted with 30 mL (10 mL×3) of ethyl acetate. The organic phases were combined, dried over anhydrous Na₂SO₄, and purified by column chromatography to obtain compound **96** (266 mg), with a yield of 55%. MS: *m*/*z* 483 [M+H]⁺.

### Example 97 Synthesis of compound 1-(3'-(2-(2-(4-fluorophenyl)-5- methylpyrrolidin-1-yl)-2-oxoethyl)-2',4'-dioxo-2,3-dihydrospiro[cyclopropane-1,1'-indene-1,5'-oxazolidine]-5-yl)-3-methylurea (97)

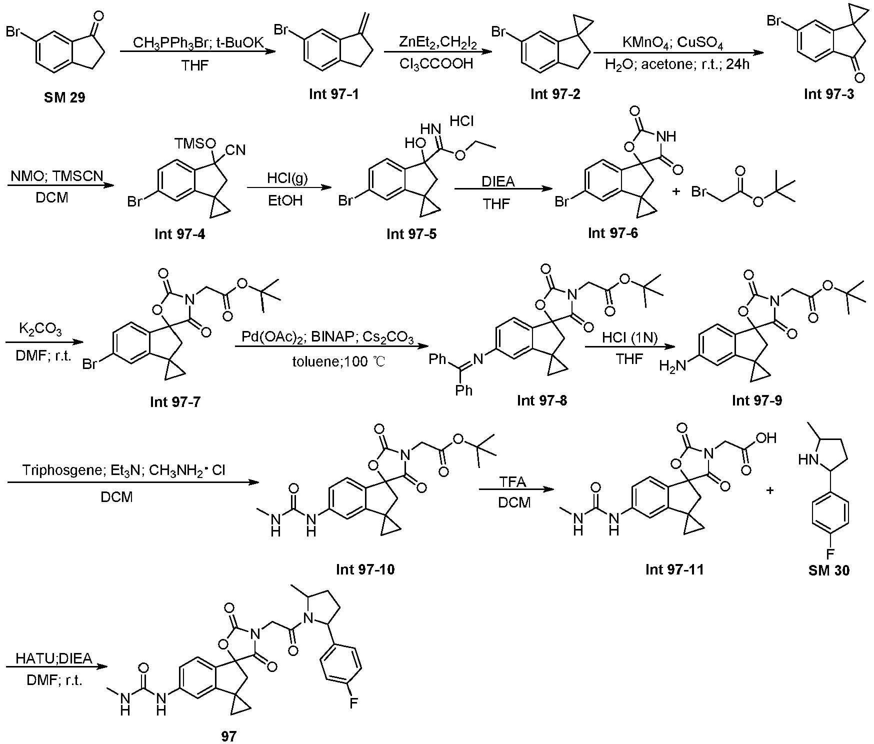

### Synthesis of intermediate 6-bromo-1-methylene-2,3-dihydro-1H-indene (Int 97-1)

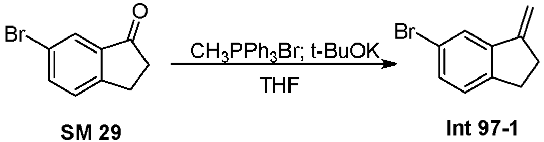

(Bromomethyl)triphenylphosphine (35.6 g, 100 mmol) was dissolved in THF (300 mL), and under nitrogen protection, t-BuOK (11.2 g, 100 mmol) was added in batches in an ice-water bath. The mixture was reacted at this temperature for 1 h, to obtain the solution for use. **SM 29** (21 g, 100 mmol) was dissolved in THF (300 mL), and under the conditions of cooling in an ice-water bath, the solution for use was added into the solution of **SM 29** in THF dropwise. After that, the reaction was continually carried out for half an hour in the ice-water bath. The reaction solution was poured into water (1 L), and extracted with ethyl acetate three times. The organic phase was washed with saturated brine, and concentrated to obtain a crude product, that was purified by column chromatography to obtain **Int 97-1** (12 g), with a yield of 60%. ¹H NMR (400 MHz, CDCl3) δ 7.59 (m, 1H), 7.32 - 7.28 (m, 1H), 7.11 (m, 1H), 5.43 (m, 1H), 5.06 (m, 1H), 2.97 - 2.84 (m, 2H), 2.84 - 2.71 (m, 2H).

### Synthesis of intermediate 6'-bromo-2',3'-dihydrospiro[cyclopropane-1,1'-indene] (Int 97-2)

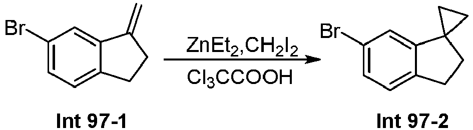

Diiodomethane (26.8 g, 100 mmol) was dissolved in DCM (50 mL), and under nitrogen protection and at the temperature of -70 °C, the organic solution of diethyl zinc (100 mL, 1N in ?) was drop added, and then the mixture was reacted at -45°C for 2 h, to which was then added the solution of trichloroacetic acid (16.3 g, 100 mmol) in dichloromethane dropwise, followed by reaction at -15 °C for 2 h. Finally, the solution **of int 97-1** (10.4 g, 50 mmol) in dichloromethane was drop added and then the reaction was carried out at room temperature for 12 h. The reaction solution was poured into water, and extracted with dichloromethane three times. The organic phase was washed with saturated brine, and concentrated to obtain the crude product, that was purified by column chromatography to obtain **Int 97-2** (8.8 g), with a yield of 80%. ¹H NMR (400 MHz, CDCl3) δ 7.20-7.18 (m, 1H), 7.05 - 7.03 (m, 1H), 6.76 (s, 1H), 2.99-2.95 (m, 2H), 2.14-2.08 (m, 2H), 0.96-0.87 (m, 4H).

### Synthesis of intermediate 6'-bromo-spiro[cyclopropane-1,1'-indene]-3'(2'H)-one (Int 97-3)

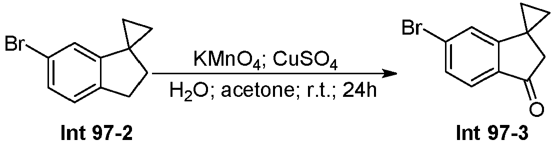

**Int 97-2** (8.8 g, 40 mmol) was dissolved in acetone (100 mL), to which were added water (100 mL), potassium permanganate (15.8 g, 100 mmol), copper sulfate pentahydrate (25 g, 100 mmol), and then the mixture was reacted at room temperature for 24 h. The reaction solution was poured into water, and extracted with ethyl acetate 10 times. The organic phase was washed with saturated brine, and concentrated to obtain the crude product, that was purified by column chromatography to obtain **Int 97-3** (3.8 g), with a yield of 40%. MS: *m*/*z* 237, 239 [M+H]⁺.

### Synthesis of intermediate 6'-bromo-3'-((trimethylsilyl)oxy)-2',3'-dihydrospiro [cyclopropane-1,1'-indene]-3'-nitrile (Int 97-4)

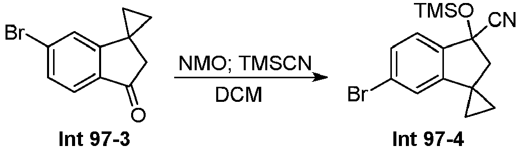

**Int 97-3** (3.8 g, 16 mmol) was dissolved in DCM (100 mL), to which were added TMSCN (2 g, 20 mmol) and NMO (580 mg, 5 mmol), and then the mixture was reacted at room temperature for 16 h. The reaction solution was poured into saturated aqueous solution of NaHCO₃, and extracted three times. The organic phase was washed with saturated brine, and concentrated to obtain the crude product, that was purified by column chromatography to obtain **Int 97-4** (2.7 g, 8 mmol)), with a yield of 50%. MS: *m*/*z* 336, 338 [M+H]⁺.

### Synthesis of intermediate 6'-bromo-3'-hydroxyl-2',3'-dihydrospiro[cyclopropane- 1,1'-indene]-3'-carbonimide hydrochloride (Int 97-5)

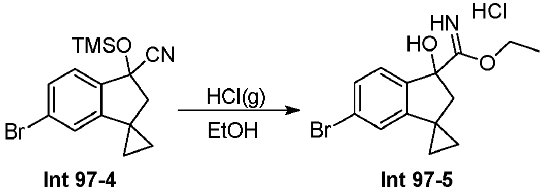

**Int 97-4** (2.7 g, 8 mmol) was dissolved in EtOH (30 mL), to which was continuously filled with dry HCl gas for 5 h. The reaction solution was concentrated and dried, to obtain **Int 97-5** (2.8 g, 8 mmol), with a yield of 100%.

### Synthesis of intermediate 5-bromo-2,3-dihydrospiro[cyclopropane-indene-1,5'-oxazolidine]-2',4'-dione (Int 97-6)

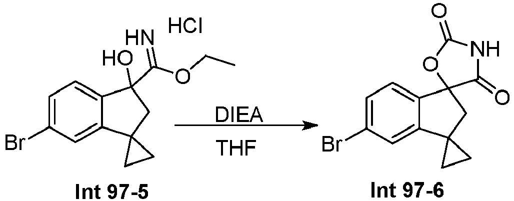

**Int. 97-5** (2.8 g, 8 mmol) was dissolved in dry THF (120 mL), to which was added DIEA (5.2 g, 40 mmol) in an ice bath, and the mixture was stirred for 5 min, then triphosgene (2.4 g, 8 mmol) was added in batches. After that, the mixture was reacted 1h. The reaction solution was poured into water, and extracted with ethyl acetate three times. The organic phase was washed with saturated brine, and concentrated to obtain the crude product, that was purified by column chromatography to obtain **Int 97-6** (1.96 g, 6.4 mmol), with a yield of 80%. MS: *m*/*z* 308, 310 [M+H]⁺.

### Synthesis of intermediate 2-(5-bromo-2',4'-dioxo-2,3-dihydrospiro[cyclopropane- indene-1,5'-oxazolidine]-3'-yl)acetic acid t-butyl ester (Int 97-7)

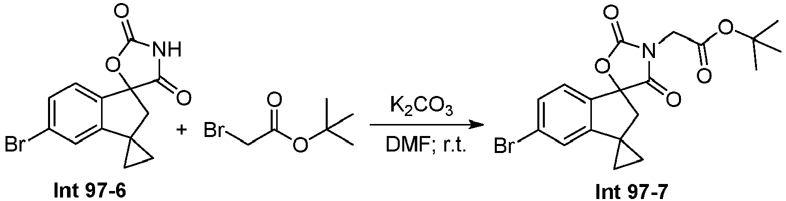

**Int 97-6** (1.96 g, 6.4 mmol) was dissolved in DMF(20 mL), to which were added t-butyl bromoacetate (1.36 g, 7 mmol) and potassium carbonate (1.38 g, 10 mmol), and the mixture was reacted 3 h. The reaction solution was poured into water, and extracted three times. The organic phase was washed with saturated brine, and concentrated to obtain the crude product, that was purified by column chromatography to obtain **Int 97-7** (2.42 g, 5.76 mmol), with a yield of 90%. MS: *m*/*z* 422, 424 [M+H]⁺.

### Synthesis of intermediate 2-(5-((diphenylmethylene)amino)-2',4'-dioxo-2,3-dihydrospiro[cyclopropane-indene-1,5'-oxazolidine]-3'-yl)acetic acid t-butyl ester (Int 97-8)

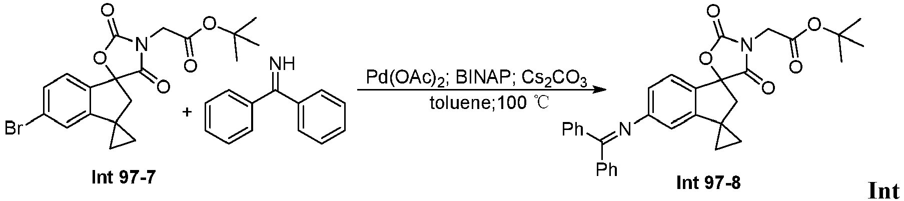

**97-7**(2.42 g, 5.76 mmol), benzophenonimine (1.05 g, 5.76 mmol), cesium carbonate (1.88 g, 5.76 mmol), palladium acetate (224 mg, 1 mmol), and BINAP(311 mg, 0.5 mmol) were dissolved in toluene (20 mL), and the system was filled with N₂ three times. The mixture was heated to 100 °C and reacted for 5 h. The reaction solution was poured into water, and extracted with ethyl acetate three times. The organic phase was washed with saturated brine, and concentrated to obtain the crude product, that was purified by column chromatography to obtain **Int 97-8** (2.4 g, 4.6 mmol), with a yield of 80%. MS: *m*/*z* 523 [M+H]⁺.

### Synthesis of intermediate 2-(5-amino-2',4'-dioxo-2,3-dihydrospiro[cyclopropane- indene-1,5'-oxazolidine]-3'-yl)acetic acid t-butyl ester (Int 97-9)

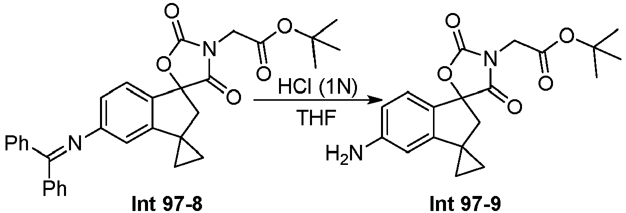

**Int. 97-5** (2.4 g, 4.6 mmol) was dissolved in THF (20 mL), to which was added hydrochloric acid solution (10 mL, 1 N), and the mixture was reacted at room temperature for 2 h. The reaction solution was treated with NaHCO₃ solution, and extracted with ethyl acetate three times. The organic phase was washed with saturated brine, and concentrated to obtain the crude product, that was purified by column chromatography to obtain **Int 97-9** (1.64 g, 4.6 mmol), with a yield of 100%. MS: *m*/*z* 315 [M-43].

### Synthesis of intermediate 2-(5-(3-methylureido)-2',4'-dioxo-2,3-dihydrospiro [cyclopropane-indene-1,5'-oxazolidine]-3'-yl)acetic acid t-butyl ester (Int 97-10)

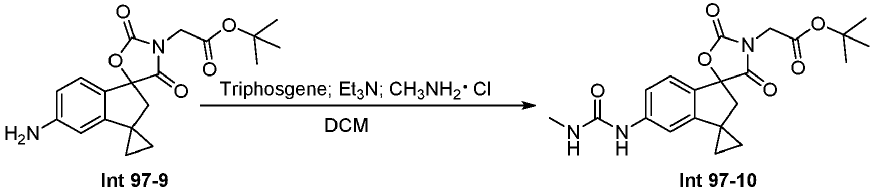

**Int 97-9** (1.64 g, 4.6 mmol) was dissolved in THF (15 mL), to which was added triphosgene (1.4 g, 4.6 mmol), and the mixture was reacted at room temperature for 10 min, then DIEA (3.9 g, 30 mmol) was added, and finally methylamine hydrochloride (310 mg, 4.6 mmol) was added. The mixture was reacted at room temperature for 1 h. The reaction solution was poured into water, and extracted with ethyl acetate three times. The organic phase was washed with saturated brine, and concentrated to obtain the crude product, that was purified by column chromatography to obtain **Int 97-10** (1.7 g, 4.1 mmol), with a yield of 90%. MS: *m*/*z* 416 [M+H]⁺.

### Synthesis of intermediate 2-(5-(3-methylureido) -2',4'-dioxo-2,3-dihydrospiro [cyclopropane-indene-1,5'-oxazolidine]-3'-yl)acetic acid (Int 97-11)

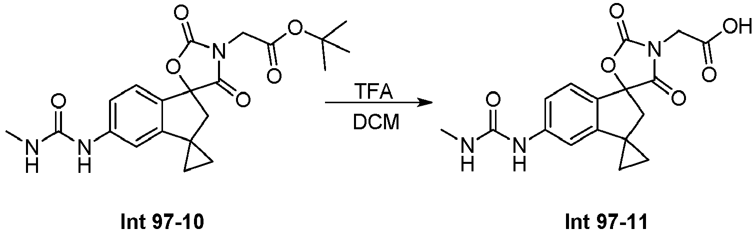

**Int 97-10** (1.7 g, 4.1 mmol) was dissolved in DCM (15 mL), to which was added TFA (1.5 mL), and the mixture was reacted at room temperature for 1 h, and concentrated to dry to obtain **Int 97-11** (1.47 g, 4.1 mmol), with a yield of 100%. MS: *m*/*z* 360 [M+H]⁺.

### Synthesis of compound 1-(3'-(2-(2-(4-fluorophenyl)-5-methylpyrrolidin-1-yl)-2- oxoethyl)-2',4'-dioxo-2,3-dihydrospiro[cyclopropane-1,1'-indene-1,5'-oxazolidine]-5-yl)-3-methylurea (97)

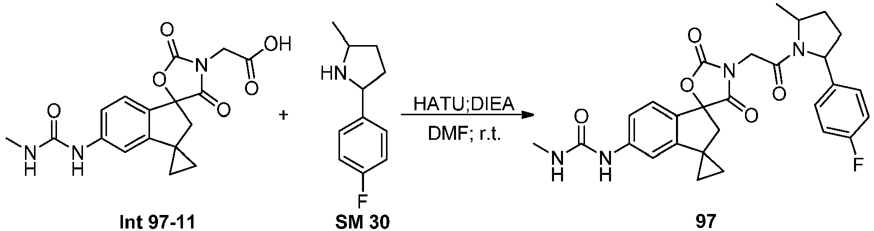

Intermediate **Int 97-11** (30 mg, 89 µmol) was dissolved in DMF (1 mL), to which were added DIEA (35 mg, 270 µmol) and HATU (41 mg, 108 µmol), followed by addition **of SM 30** (20 mg, 0.1 mmol), and the mixture was stirred at room temperature for 16 h. To the reaction solution was added water (10 mL), and extracted with ethyl acetate two times. The organic phase was washed with saturated brine, and concentrated to obtain the crude product, that was purified by column chromatography to obtain product **97** (26 mg) as off-white solid, with a yield of 50%. MS: *m*/*z* 521.2 [M+H]⁺. ¹H NMR (400 MHz, DMSO): δ 8.71 (s, 1H), 7.44-7.35 (m, 1H), 7.32-7.19 (m, 3H), 7.17-7.03 (m, 3H), 6.13-6.00 (m, 1H), 5.26- 4.92 (m, 1H), 4.52 (m, 2H), 4.27-3.41 (m, 1H), 2.77 (m, 1H), 2.62 (t, J = 3.8 Hz, 3H), 2.39 (dd, J = 13.6, 7.7 Hz, 2H), 2.01 (ddd, J = 11.8, 10.4, 6.5 Hz, 1H), 1.95-1.84 (m, 1H), 1.74 (dd, J = 49.7, 7.9 Hz, 1H), 1.52 (dd, J = 11.0, 4.1 Hz, 1H), 1.38 (dd, J = 6.1, 3.8 Hz, 3H), 1.10-1.05 (m, 1H), 1.03-0.95 (m, 1H), 0.93-0.83 (m, 1H).

### Reference Example 98 Synthesis of compound 1-(3'-(2-(N-( 4-fluorobenzyl)-N-((S)-1,1,1-trifluoropropan-2-yl))-2-oxoethyl)-2',4'-dioxo-2,3-dihydrospiro[cyclopropane-1,1'-indene-1,5'-oxazolidine-5-yl)-3-methylurea (98)

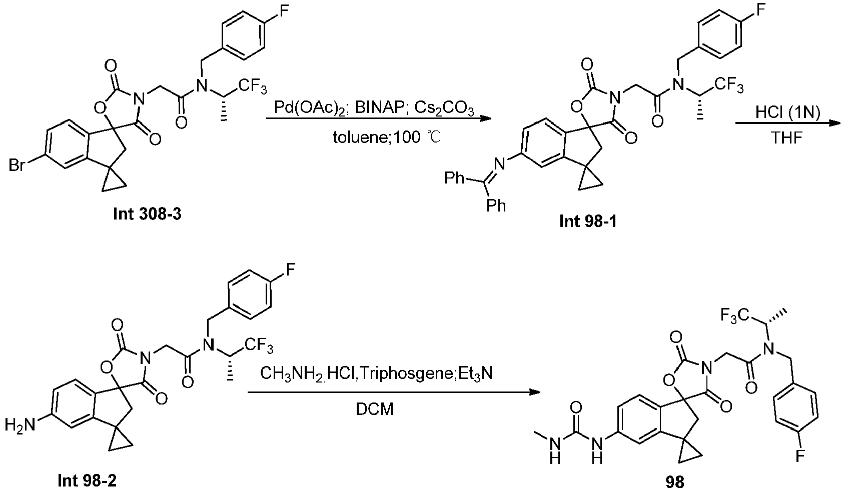

### Synthesis of intermediate 2-(5-((diphenylmethylene)amino)-2',4'-dioxo-2,3-dihydrospiro[cyclopropane-indene-1,5'-oxazole]-3'-yl)-N-(4-fluorobenzyl)-N-((S)-1,1,1-trifluoropropan-2-yl)acetamide (Int 98-1)

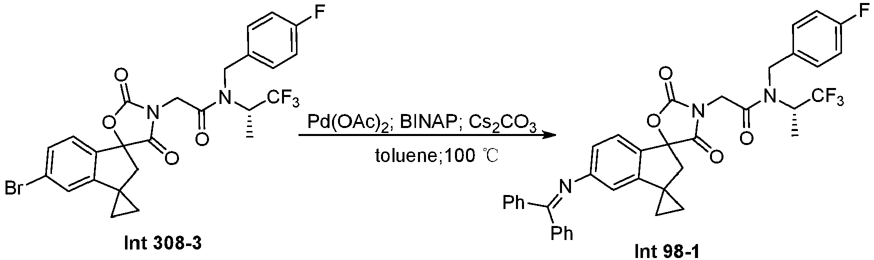

**Int 308-3** (3.27 g, 5.76 mmol), benzophenonimine (1.05 g, 5.76 mmol), cesium carbonate (1.88 g, 5.76 mmol), palladium acetate (224 mg, 1 mmol), and BINAP(311 mg, 0.5 mmol) were dissolved in toluene (20 mL), and the system was charged with N₂ three times. The mixture was heated to 100 °C and reacted for 5 h. The reaction solution was poured into water, and extracted with ethyl acetate three times. The organic phase was washed with saturated brine, and concentrated to obtain the crude product, that was purified by column chromatography to obtain **Int 98-1** (3 g, 4.6 mmol), with a yield of 80%. MS: *m*/*z* 670 [M+H]⁺.

### Synthesis of intermediate 2-(5-amino-2',4'-dioxo-2,3-dihydrospiro[cyclopropane- indene-1,5' -oxazole]-3'-yl)-N-(4-fluorobenzyl)-N-((S)-1,1,1-trifluoropropan-2-yl)acetamide (Int 98-2)

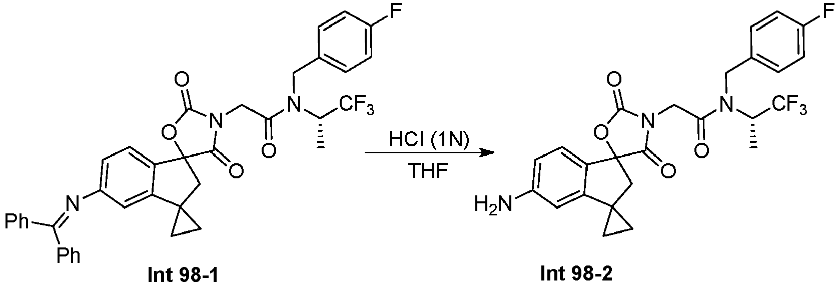

**Int. 98-1** (3 g, 4.6 mmol) was dissolved in THF (20 mL), to which was added hydrochloric acid solution (10 mL, 1 N), and the mixture was reacted at room temperature for 2 h. The reaction solution was treated with NaHCO₃ solution, and extracted with ethyl acetate three times. The organic phase was washed with saturated brine, and concentrated to obtain the crude product, that was purified by column chromatography to obtain **Int 98-2** (2.3 g, 4.6 mmol), with a yield of 100%. MS: *m*/*z* 462 [M-43].

### Synthesis of compound 2-(5-(3-methylureido)-2',4'-dioxo-2,3-dihydrospiro [cyclopropane-indene-1,5'-oxazole]-3'-yl)-N-(4-fluorobenzyl)-N-((S)-1, 1, 1-trifluoropropan-2-yl)acetamide (98)

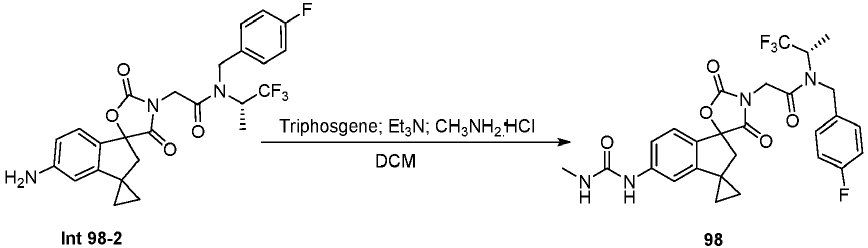

**Int 98-2** (2.3 g, 4.6 mmol) was dissolved in THF(20 mL), to which was added triphosgene (1.4 g, 4.6 mmol), and the mixture was reacted at room temperature for 10 min, followed by addition of DIEA (3.9 g, 30 mmol), and finally methylamine hydrochloride (310 mg, 4.6 mmol) was added. The mixture was reacted at room temperature for 1 h. The reaction solution was poured into water, and extracted with ethyl acetate three times. The organic phase was washed with saturated brine, and concentrated to obtain the crude product, that was purified by column chromatography to obtain compound **98** (1.15 g, 2.05 mmol), with a yield of 45%. MS: *m*/*z* 563 [M+H]⁺. ¹H NMR (400 MHz, CDCl₃): δ 7.32 (m, 2H), 7.13 (m, 2H), 6.94 (m, 3H), 5.55-5.39 (m, 1H), 4.69 (m, 2H), 4.43 (m, 1H), 4.24 (m, 1H), 2.90 (m, 1H), 2.79 (s, 3H), 2.44 (m, 1H), 1.33 (m, 3H), 1.16-1.00 (m, 4H).

### Example 99 Synthesis of compound N-((3S,5S,7S)-adamantan-1-yl)-2-((R)-(5- (3-methylureido)-2',4'-dioxo-2,3-dihydrospiro[indene-1,5'-oxazole]-3'-yl)acetamide (99)

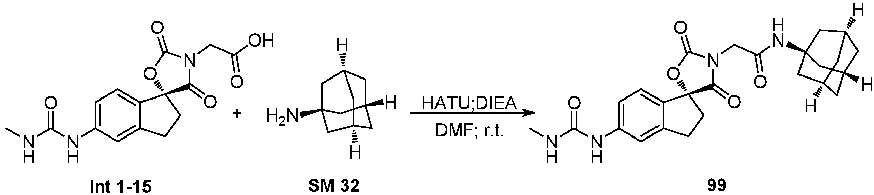

To a

30 mL reaction flask, were added compound **Int 1-15** (30 mg, 0.09 mmol), DMF (1 mL), DIEA (35 mg, 0.27 mmol), and HATU(41 mg, 0.108 mmol), followed by addition of **SM 32** (21 mg, 0.14 mmol). The mixture was stirred at room temperature for 16 h. To the reaction solution, was added water (5 mL), and then extracted with ethyl acetate (5 mL×3). The organic phase was washed with saturated brine, and concentrated to obtain the crude product, that was purified by column chromatography to obtain the product **99** (18 mg) as off-white solid, with a yield of 43%. MS: *m*/*z* 467.2 [M+H]⁺. ¹H NMR (400 MHz, DMSO) δ: 8.72 (s, 1H), 7.82 (s, 1H), 7.55 (s, 1H), 7.33-7.06 (m, 2H), 6.10 (q, J = 4.4 Hz, 1H), 4.09 (q, J = 16.6 Hz, 2H), 3.66 -3.55(m, 1H), 3.18-3.05 (m, 1H), 3.05-2.92 (m, 1H), 2.68-2.56 (m, 4H), 2.49-2.40 (m, 1H), 2.02 (s, 3H), 1.93 (d, J = 2.0 Hz, 6H), 1.82-1.68 (m, 1H), 1.63 (s, 6H).

### Example 100 Synthesis of compound (R)-1-(3'-(2-(3,4-dihydroisoquinolin- 2(1H)-yl)-2-oxoethyl)-2',4'-dioxo-2,3-dihydrospiro[indene 1,5'-oxazolidine]-5-yl) -3-methylurea (100)

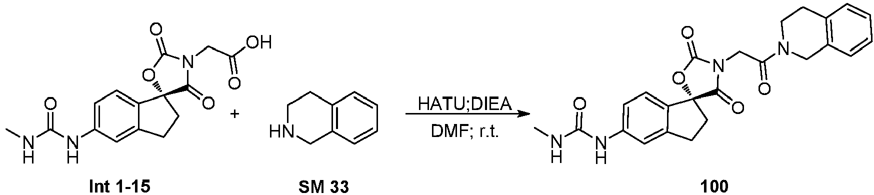

To a 30 mL reaction flask, were added compound **Int 1-15** (30 mg, 90 µmol), DMF (1 mL), DIEA (35 mg, 0.27 mmol), and HATU (41 mg, 0.108 mmol), followed by addition of **SM 33** (18 mg, 135 µmol). The mixture was stirred at room temperature for 16 h. To the reaction solution, was added water (5 mL), and then extracted with ethyl acetate (5 mL×3). The organic layers were combined, dried over anhydrous Na₂SO₄, and concentrated to obtain the crude product, that was purified by column chromatography to provide the product **100** (20 mg, yield 50%). MS: *m*/*z* 449.2 [M+H]⁺. ¹H NMR (400 MHz, DMSO): δ 8.74 (s, 1H), 7.56 (s, 1H), 7.37 (d, J = 8.4 Hz, 1H), 7.30-7.00 (m, 5H), 6.10 (q, J = 4.4 Hz, 1H), 4.78 (s, 1H), 4.73-4.44 (m, 3H), 3.78-3.71 (m, 2H), 3.17-3.09 (m, 1H), 3.05-2.91 (m, 2H), 2.82 (t, J = 6.0 Hz, 1H), 2.70-2.57 (m, 4H), 2.55-2.51(m, 1H).

### Example 101 Synthesis of compound 1-((R)-(2',4'-dioxo-3'-(2-oxo-2-((S)-3-phenylpyrrolidin-1-yl)ethyl)-2,3-dihydrospiro[indene-1, 5'-oxazolidine]-5-yl)-3-methylurea (101)

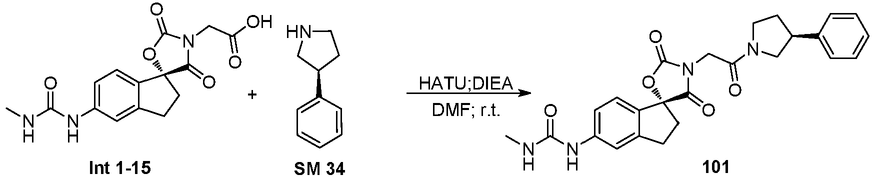

To a 30 mL reaction flask, were added compound **Int 1-15** (30 mg, 90 µmol), DMF (1 mL), DIEA (35 mg, 0.27 mmol), and HATU (41 mg, 0.108 mmol), followed by addition of **SM 34** (20 mg, 135 µmol). The mixture was stirred at room temperature for 16 h. To the reaction solution, was added water (5 mL), and then extracted with ethyl acetate (5 mL×3). The organic layers were combined, dried over anhydrous Na₂SO₄, and concentrated to obtain the crude product, that was purified by column chromatography to provide the product **101** (16 mg, yield 38%). MS: *m*/*z* 463 [M+H]⁺.

### Example 102 Synthesis of compound (R)-1-(2',4'-dioxo-3'-(2-oxo-2-(3- phenylpyrrolidin-1-yl)ethyl)-2,3-dihydrospiro[indene-1,5'-oxazolidine]-5-yl)-3-methylurea (102)

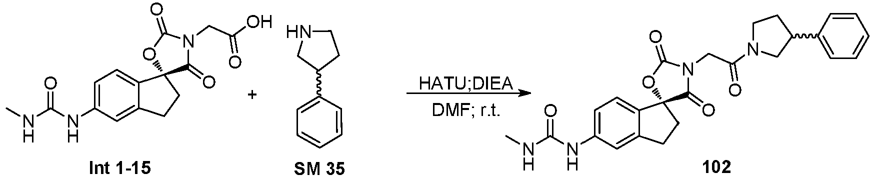

To a 30 mL reaction flask, were added compound **Int 1-15** (30 mg, 90 µmol), DMF (1 mL), DIEA (35 mg, 270 µmol), and HATU (41 mg, 108 µmol), followed by addition of **SM 35** (20 mg, 135 µmol). The mixture was stirred at room temperature for 16 h. To the reaction solution, was added water (5 mL), and then extracted with ethyl acetate (5 mL×3). The organic layers were combined, dried over anhydrous Na₂SO₄, and concentrated to obtain the crude product, that was purified by column chromatography to provide the product **102** (18 mg, yield 43%). MS: *m*/*z* 463 [M+H]⁺. ¹H NMR (400 MHz, DMSO): δ 8.73 (s, 1H), 7.57 (s, 1H), 7.44-7.30 (m, 5H), 7.30-7.04 (m, 2H), 6.09 (d, J = 4.5 Hz, 1H), 4.58-4.34 (m, 2H), 3.95-3.82 (m, 1H), 3.71-3.61 (m, 1H), 3.56-3.49 (m, 1H), 3.43-3.38 (m, 1H), 3.35-3.21 (m, 1H), 3.15-3.09 (m, 1H), 3.03-3.00 (m, 1H), 2.71-2.56 (m, 4H), 2.57-2.51 (m, 1H), 2.42-2.16 (m, 1H), 2.15-1.83 (m, 1H).

### Example 103 Synthesis of compound 1-((R)-(3'-(2-((1S,4R)-2-oxa-5-azabicyclo [2.2.1]heptan-5-yl)-2-oxoethyl)-2',4'-dioxo-2,3-dihydrospiro[indene-1,5'-oxazolidine] -5-yl)-3-methylurea (103)

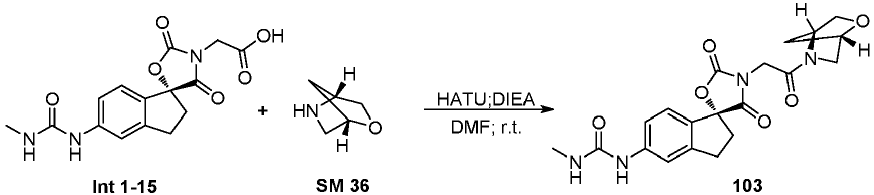

Intermediate **Int 1-15** (30 mg, 90 µmol) was dissolved in DMF (1 mL), to which were added DIEA (35 mg, 270 µmol) and HATU (41 mg, 108 µmol), followed by addition of **SM 36** (15 mg, 0.1 mmol), and the mixture was stirred at room temperature for 16 h. To the reaction solution was added water (10 mL), and extracted with ethyl acetate two times. The organic phase was washed with saturated brine, and concentrated to obtain the crude product, that was purified by column chromatography to obtain product **103** (20 mg) as off-white solid, with a yield of 50%. MS: *m*/*z* 415 [M+H]⁺.

### Example 105 Synthesis of compound (R)-N-(3-hydroxyl-2,2,4,4- tetramethylcyclobutyl)-2-(5-(3-Methylureido)-2',4'-dioxo-2,3-dihydrospiro[indene-1,5'-oxazole]-3'-yl)acetamide (105)

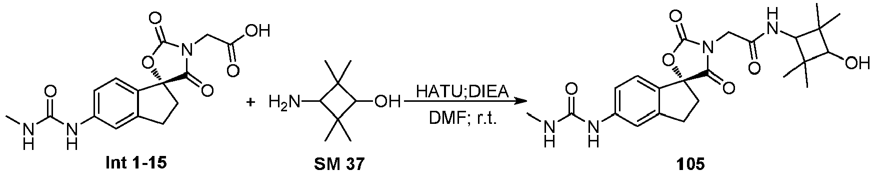

Intermediate **Int 1-15** (30 mg, 90 µmol) was dissolved in DMF (1 mL), to which were added DIEA (35 mg, 270 µmol) and HATU (41 mg, 108 µmol), followed by addition of **SM 37** (19 mg, 135 µmol), and the mixture was stirred at room temperature for 16 h. To the reaction solution was added water (10 mL), and extracted with ethyl acetate two times. The organic phase was washed with saturated brine, and concentrated to obtain the crude product, that was purified by column chromatography to obtain product **105** (17 mg) as off-white solid, with a yield of 65%. MS: *m*/*z* 459 [M+H]⁺.

### Example 106 Synthesis of compound 1-((1R)-(2',4'-dioxo-3'-(2-oxo-2-(2- phenylazetidin-1-yl)ethyl)-2,3-dihydrospiro[indene-1,5'-oxazolidine]-5-yl)-3-methylurea (106):

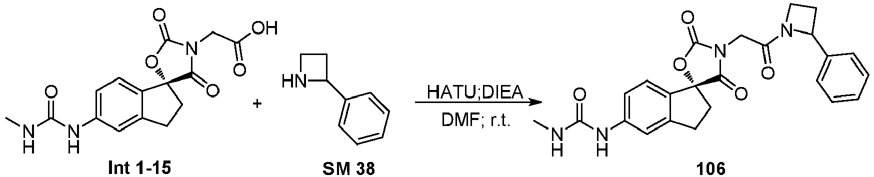

To a 30 mL reaction flask, were added compound **Int 1-15** (30 mg, 90 µmol), DMF (1 mL), DIEA (35 mg, 270 µmol), and HATU (41 mg, 108 µmol), followed by addition of **SM 38** (18 mg, 135 µmol). The mixture was stirred at room temperature for 16 h. To the reaction solution, was added water (5 mL), and then extracted with ethyl acetate (5 mL×3). The organic layers were combined, dried over anhydrous Na₂SO₄, and concentrated to obtain the crude product, that was purified by column chromatography to provide the product 106 (16 mg, yield 40%). MS: *m*/*z* 449 [M+H]⁺. ¹H NMR (400 MHz, DMSO): δ 8.72 (s, 1H), 8.72 (s, 1H), 7.63-7.50 (m, 2H), 7.47 (t, J = 6.9 Hz, 1H), 7.42-7.08 (m, 5H), 6.07 (d, J = 2.9 Hz, 1H), 5.76-5.14 (m, 1H), 4.51-3.87 (m, 4H), 3.19-3.03 (m, 1H), 3.03-2.75 (m, 1H), 2.86-2.74 (m, 1H), 2.69-2.56 (m, 4H), 2.48-2.35 (m, 1H), 2.25-1.94 (m, 1H).

### Example 107 Synthsis of compound 1-((R)-(2',4'-dioxo-3'-(2-oxo-2-((S)-3-phenylmorpholinyl)ethyl)-2,3-dihydrospiro[indene-1,5'-oxazolidine]-5-yl)-3-methylurea (107)

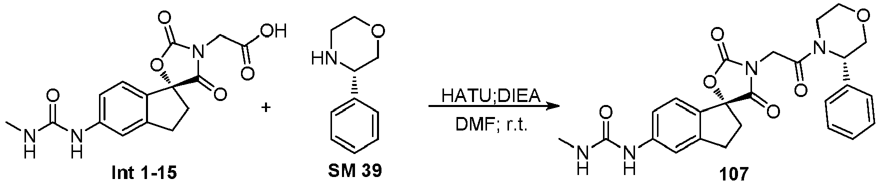

To a 30 mL reaction flask, were added compound **Int 1-15** (30 mg, 90 µmol), DMF (1 mL), DIEA (35 mg, 270 µmol), and HATU (41 mg, 108 µmol), followed by addition of **SM 39** (22 mg, 135 µmol). The mixture was stirred overnight at room temperature. To the reaction solution, was added water (5 mL), and then extracted with ethyl acetate (5 mL×3). The organic layers were combined, dried over anhydrous Na₂SO₄, and concentrated to obtain the crude product, that was purified by column chromatography to provide the product **107** (19 mg, yield 44%). MS: *m*/*z* 479 [M+H]⁺. ¹H NMR (400 MHz, DMSO) δ 8.72 (s, 1H), 7.56 (s, 1H), 7.44 (d, J = 7.2 Hz, 1H), 7.40-7.15 (m, 6H), 6.09 (d, J = 4.4 Hz, 1H), 5.31 (d, J = 113.0 Hz, 1H), 4.92-4.51 (m, 2H), 4.43 (d, J = 12.2 Hz, 1H), 4.24-3.90 (m, 1H), 3.89 -3.72 (m, 2H), 3.56 (dt, J = 23.1, 10.7 Hz, 1H), 3.28 (d, J = 11.3 Hz, 1H), 3.13 (dt, J = 15.5, 7.5 Hz, 1H), 3.06-2.95 (m, 1H), 2.65 (t, J = 11.3 Hz, 4H), 2.52 (s, 1H).

### Example 108 Synthesis of compound 1-((1R)-(3'-(2-((4S)-2-(t-butyl)-4- phenyloxazolidin-3-yl)-2-oxoethyl)-2',4'-dioxo-2,3-dihydrospiro[indene-1,5'-oxazolidine]-5-yl)-3-methylurea (108)

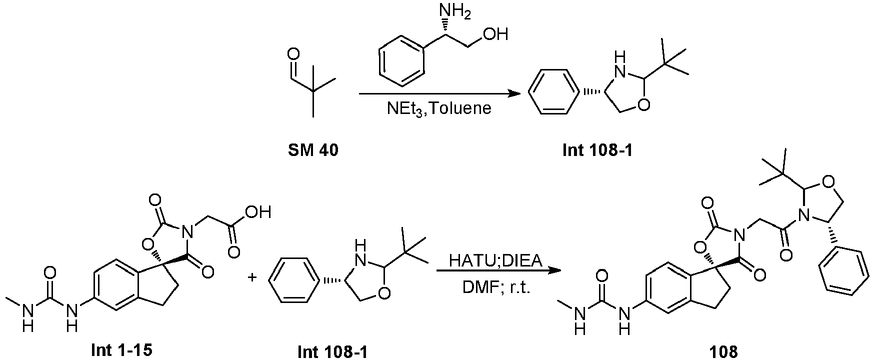

### Synthesis of intermediate (4S)-2-t-butyl-4-phenyloxazolidine (Int 108-1)

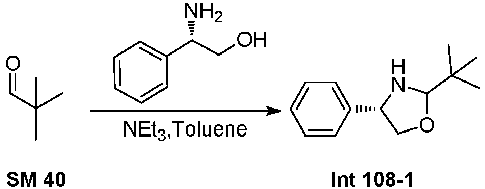

Compound (S)-2-amino-2-phenylethanol (10 g, 73 mmol), **SM 40** (45 g, 80 mmol), triethylamine (707 mg, 7.3 mmol), and toluene (100 mL) were added to a 250 mL reaction flask, and the reaction solution was refluxed to remove water for about 7 to 8 h. After the reaction was completed, the solvent was rotatory evaporated under reduced pressure to obtain the crude product, which was purified by column chromatography to provide **Int 108-1** (2.3 g, yield 15%). MS: *m*/*z* 206 [M+H]⁺.

### Synthesis of compound 1-((1R)-(3'-(2-((4S)-2-(t-butyl)-4-phenyloxazolidin-3-yl)-2-oxoethyl)-2',4'-dioxo-2,3-dihydrospiro[indene-1,5'-oxazolidine]-5-yl)-3-methylurea (108)

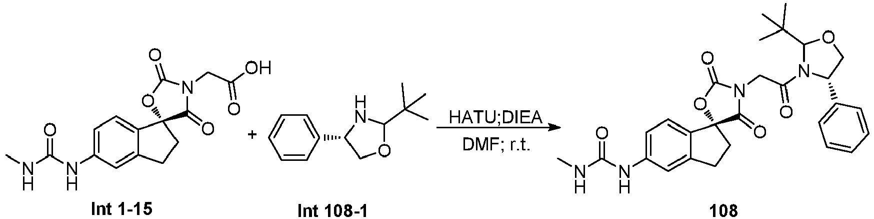

To a 30 mL reaction flask, were added compound **Int 1-15** (30 mg, 90 µmol), DMF (1 mL), DIEA (35 mg, 270 µmol), and HATU (41 mg, 108 µmol), followed by addition of **Int 108-1** (28 mg, 135 µmol). The mixture was stirred overnight at room temperature. To the reaction solution, was added water (5 mL), and then extracted with ethyl acetate (5 mL×3). The organic layers were combined, dried over anhydrous Na₂SO₄, and concentrated to obtain the crude product, that was purified by column chromatography to provide the product **108** (13 mg, yield 28%). MS: *m*/*z* 521.2 [M+H]⁺. ¹H NMR (400 MHz, DMSO) δ 8.73 (d, J = 4.4 Hz, 1H), 7.49 (dt, J = 14.7, 10.3 Hz, 5H), 7.34 (t, J = 6.8 Hz, 1H), 7.30-7.18 (m, 2H), 6.10 (dd, J = 4.4, 2.0 Hz, 1H), 5.55 (t, J = 6.2 Hz, 1H), 5.33 (s, 1H), 4.72 (t, J = 15.9 Hz, 1H), 4.52 (t, J = 7.0 Hz, 1H), 4.17-4.12 (m, 1H), 4.08-3.93 (m, 1H), 3.18-3.05 (m, 1H), 2.99 (dd, J = 12.6, 9.6 Hz, 1H), 2.71-2.56 (m, 4H), 2.44 (s, 1H), 0.87 (d, J = 6.5 Hz, 9H).

### Example 109 Synthesis of compound (R)-1-methyl-3-(3'-(2-(6-(1-methyl-1H- pyrazol-3-yl)-7-(trifluoromethyl)-3,4-dihydroquinolin-1-(2H)-yl)-2-oxoethyl)-2',4'-dioxo-2,3-dihydrospirofindene-1,5'-oxazolidine-5-yl)urea (109)

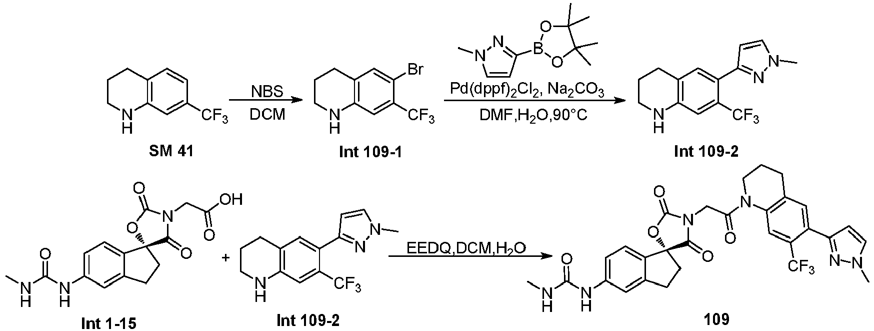

### Synthesis of intermediate 6-bromo-7-(trifluoromethyl)-1,2,3,4-tetrahydroquinoline (Int 109-1)

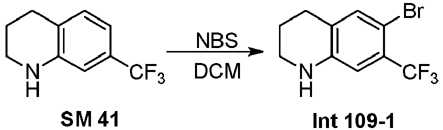

Compound **SM 41** (200 mg, 1 mmol) and dichloromethane (3 mL) were added to a 30 mL reaction flask, to which was then added N-bromosuccinimide (178 mg, 1 mmol), and the mixture was stirred overnight at room temperature. Water (5 mL) was added to the reaction solution, and then the solution was extracted with dichloromethane (5 mL×3). The organic layers were combined, dried with anhydrous sodium sulfate, and concentrated to obtain the crude product, that was purified by column chromatography to provide the product **Int 109-1** (94 mg, yield 34%). MS: *m*/*z* 280 [M+H]⁺.

### Synthesis of intermediate 6-(1-methyl-1H-pyrazol-3-yl)-7-(trifluoromethyl)-1,2,3,4-tetrahydroquinoline (Int 109-2)

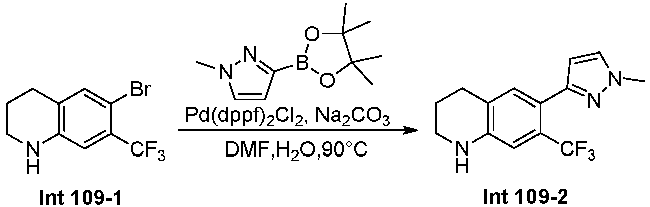

To a 50 mL reaction flask were added compound **Int 109-1** (94 mg, 0.34 mmol), 1-methyl-1H-pyrazol-5-boronic acid pinacol ester (84 mg, 0.4 mmol), sodium carbonate (139 mg, 1 mmol), DMF (3 mL) and water (0.1 mL), and after charging nitrogen, [1,1'-bis(diphenylphosphino)ferrocene]dichloropalladium (30 mg, 0.04 mmol) was added. The reaction solution was protected by nitrogen, and stirred overnight at 90 °C. After completion of the reaction, water (5 mL) was added to the reaction solution, and the solution was extracted with ethyl acetate (5 mL×3). The organic layers were combined, dried over anhydrous Na₂SO₄, and concentrated to obtain the crude product, that was purified by column chromatography to provide the product **Int 109-2** (35 mg, yield 36%). MS: *m*/*z* 282 [M+H]⁺.

### Synthesis of compound (R)-1-methyl-3-(3'-(2-(6-(1-methyl-1H-pyrazol-3-yl)- 7-(trifluoromethyl)-3,4-dihydroquinolin-1-(2H)-yl)-2-oxoethyl)-2',4'-dioxo-2,3-dihydrospirofindene-1,5' -oxazolidine -5 -yl)urea (109)

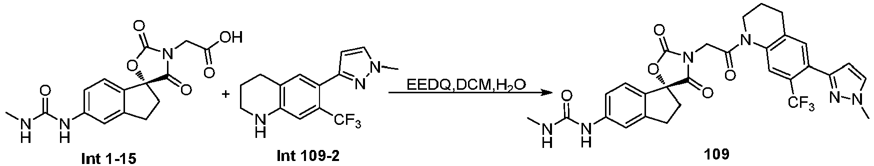

To a 30 mL reaction flask, were added compound **Int 1-15** (30 mg, 90 µmol), **Int 109-2** (25 mg, 90 µmol), dichloromethane (1 mL) and water (1 mL), followed by addition of 2-ethoxy-1-ethoxycarbonyl-1,2-dihydroquinoline (34 mg, 135 µmol), and the mixture was stirred overnight at room temperature. To the reaction solution, was added water (5 mL), and then extracted with dichloromethane (5 mL×3). The organic layers were combined, dried over anhydrous Na₂SO₄, and concentrated to obtain the crude product, that was purified by column chromatography to provide the product **109** (15 mg, yield 28%). MS: *m*/*z* 597.2 [M+H]⁺. ¹H NMR (400 MHz, DMSO) δ 8.75 (s, 1H), 8.26 (s, 1H), 7.89 (s, 1H), 7.56 (d, J = 5.3 Hz, 2H), 7.39 (s, 1H), 7.26 (dt, J = 8.5, 5.1 Hz, 2H), 6.14-6.05 (m, 1H), 4.74 (s, 2H), 3.89 (s, 5H), 3.14 (dt, J = 15.6, 7.6 Hz, 1H), 3.01 (ddd, J = 16.3, 8.6, 3.3 Hz, 1H), 2.85 (t, J = 6.3 Hz, 2H), 2.66 (dd, J = 18.8, 6.3 Hz, 4H), 2.54 (s, 1H), 1.98 (dd, J = 12.4, 6.5 Hz, 2H).

### Example 110 Synthesis of compound (R)-1-(2',4'-dioxo-3'-((5-phenyl-1H-imidazol- 2-yl)methyl)-2,3-dihydrospiro[indene-1,5'-oxazolidine]-5-yl)-3-methylurea (110)

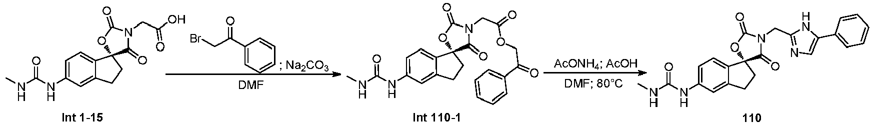

### Synthesis of intermediate (R)-2-oxo-2-phenylethyl-2-(5-(3-methylureido)-2',4'-dioxo- 2,3-dihydrospiro[indene-1,5'-oxazolidine]-3'-yl)acetic acid ethyl ester (Int 110-1)

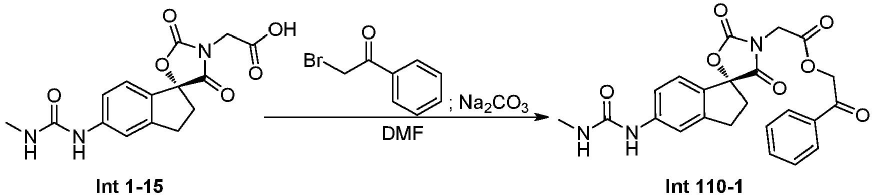

To a 30 mL reaction flask, were added compound **Int 1-15** (20 mg, 60 µmol), 2-bromo-1-acetophenone (12 mg, 60 µmol), DMF (1 mL), and DIEA (9 mg, 72 µmol). The mixture was stirred overnight at room temperature. After completion of the reaction, to the reaction solution, was added water (5 mL), and then extracted with ethyl acetate (5 mL×3). The organic layers were combined, dried over anhydrous Na₂SO₄, and concentrated to obtain the crude product **Int 110-1** (27 mg, yield 100%). MS: *m*/*z* 452.1 [M+H]⁺.

### Synthesis of compound (R)-1-(2',4'-dioxo-3'-((5-phenyl-1H-imidazol-2-yl)methyl)- 2,3-dihydrospiro[indene-1,5'-oxazolidine]-5-yl)-3-methylurea (110)

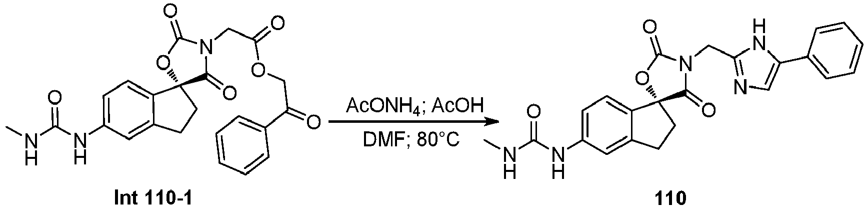

To a 30 mL reaction flask, were added compound **Int 110-1** (27 mg, 60 µmol), ammonium acetate (14 mg, 180 µmol), acetic acid (2 mL) and DMF (1 mL). The mixture was heated to 80 °C and stirred for 5 to 6 h. To the reaction solution, was added water (5 mL), and then extracted with ethyl acetate (5 mL×3). The organic layers were combined, dried over anhydrous Na₂SO₄, and concentrated to obtain the crude product **110** (12 mg, yield 46%). MS: *m*/*z* 432.2 [M+H]⁺. ¹H NMR (400 MHz, DMSO) δ 12.28 (s, 1H), 8.75 (d, J = 9.2 Hz, 1H), 7.78 (d, J = 7.2 Hz, 1H), 7.66-7.58 (m, 2H), 7.53 (d, J = 8.2 Hz, 1H), 7.44-7.16 (m, 5H), 6.13- 6.05 (m, 1H), 4.89-4.71 (m, 2H), 3.14 (dd, J = 15.8, 7.9 Hz, 1H), 3.01 (ddd, J = 16.3, 8.7, 3.3 Hz, 1H), 2.78-2.61 (m, 4H), 2.59-2.52 (m, 1H).

### Example 111 Synthesis of 1-methyl-3-((R)-(3'-(2-(2-(1-methyl-1H-imidazol-2-yl) pyrrolidin-1-yl)-2-oxoethyl)-2',4'-dioxo-2,3-dihydrospiro[indene-1,5'-oxazolidine]-5-yl)urea (111)

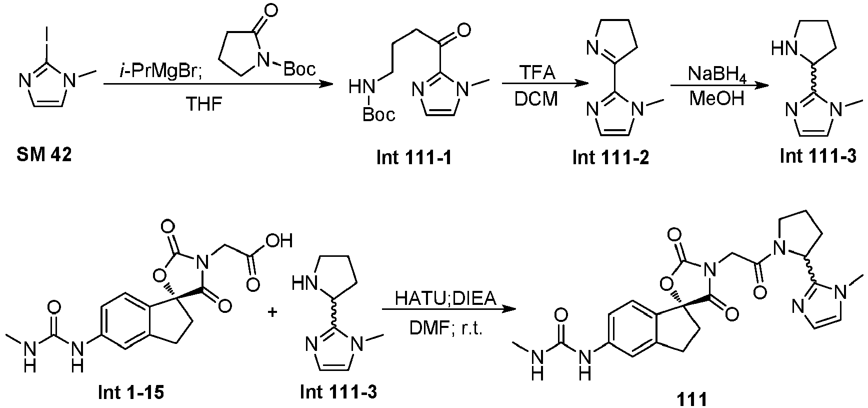

### Synthesis of intermediate (4-(1-methyl-1H-imidazol-2-yl)-4-oxobutyl)carbamic acid t-butyl ester (Int 111-1)

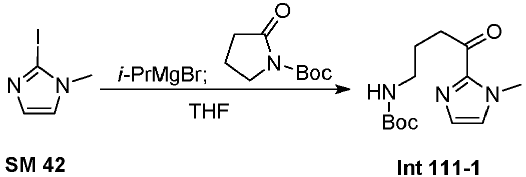

To a reaction flask containing **SM 42** (2.08 g, 10 mmol), was added dry THF (30 mL), and then under N₂ protection, the solution of isopropylmagnesium bromide in tetrahydrofuran (10 mL, 1M in THF) was added in an ice-water bath. After that, the ice-water bath was removed, and the mixture was stirred at room temperature for 7 h, and N-Boc-2-pyrrolidone (1.85 g, 10 mmol) was finally added. The mixture was further allowed to react at room temperature for 12 h. The reaction solution was poured into the saturated aqueous solution of ammonium chloride, and extracted with 30 mL (10 mL×3) of ethyl acetate. The organic phases were combined, dried over anhydrous Na₂SO₄, and purified by column chromatography to obtain **Int 111-1** (1.34 g, 5 mmol), with a yield of 50%. MS: *m*/*z* 268 [M+H]⁺.

### Synthesis of 2-(3,4-dihydro-2H-pyrrol-5-yl)-1-methyl-1H-imidazole (Int 111-2)

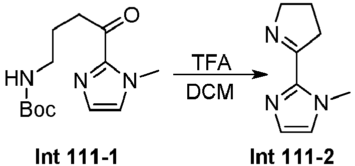

**Int 111-1** (1.34 g, 5 mmol) was dissolved in 13 mL DCM, to which was added 1.3 mL TFA, and the mixture was stirred at room temperature until the reaction was completed by TLC detection. pH was adjusted to be neutral with the saturated aqueous solution of sodium bicarbonate, and then the reaction solution was extracted with DCM (10 mL × 3), dried with anhydrous Na₂SO₄, and rotatory evaporated to dry, to obtain **Int 111-2** (0.67 g; 90%), MS: *m*/*z* 150 [M+H]⁺.

### Synthesis of 1-methyl-2-(pyrrolidin-2-yl)-1H-imidazole (Int 111-3)

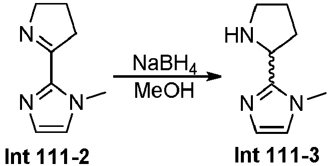

**Int 111-2** (0.67 g; 4.5 mmol) was added into a reaction flask containing 6 mL methanol, to which was added sodium borohydride (0.68 g; 18 mmol) in batches under stirring at room temperature. After addition, the mixture was continually stirred at room temperature until the reaction was completed by TLC detection. The reaction solution was poured into water, and extracted with 30 mL (10 mL×3) of EA. The organic phases were combined, dried over anhydrous Na₂SO₄, and separated by column chromatography to obtain **Int 111-3** (0.61 g; 90%). MS: *m*/*z* 152 [M+H]⁺.

### Synthesis of compound 1-methyl-3-((R)-(3'-(2-(2-(1-methyl-1H-imidazol-2-yl) pyrrolidin-1-yl)-2-oxoethyl)-2',4'-dioxo-2,3-dihydrospiro[indene-1,5'-oxazolidine]-5-yl)urea (111)

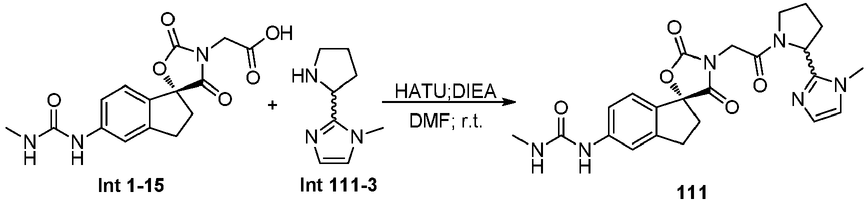

Intermediate **Int 1-15** (30 mg, 90 µmol) was dissolved in DMF (1 mL), to which were added DIEA (35 mg, 270 µmol) and HATU (41 mg, 108 µmol), followed by addition of **Int 111-3** (20.3 mg, 135 µmol), and the mixture was stirred at room temperature for 16 h. To the reaction solution was added water (10 mL), and extracted with ethyl acetate two times. The organic phase was washed with saturated brine, and concentrated to obtain the crude product, that was purified by column chromatography to obtain product **111** (17 mg, yield 63%) as off-white solid. MS: *m*/*z* 467 [M+H]⁺.

### Example 112 Synthesis 1-((1R)-(3'-(2-(2-(benzo[d][1,3]dioxolen-4-yl)pyrrolidin-1-yl)-2-oxoethyl)-2',4'-dioxo-2,3-dihydrospiro[indene-1,5'-oxazolidine]-5-yl)-3-methylurea (112)

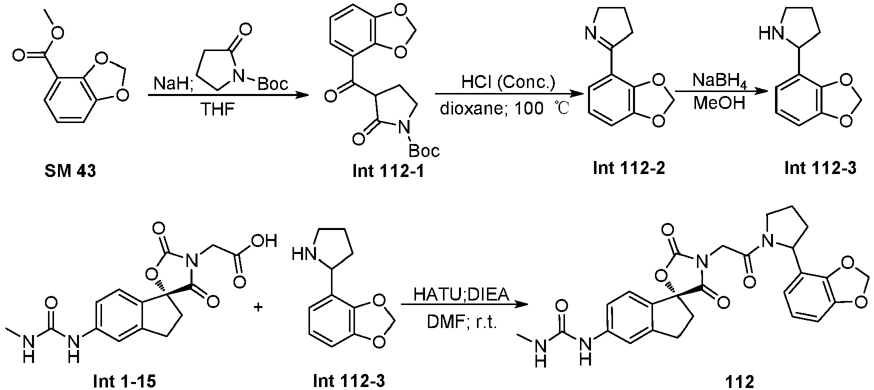

### Synthesis of t-butyl-3-(benzo[d][1,3]dioxin-4-carbonyl)-2-oxopyrrolidin- 1-carboxylate (Int 112-1)

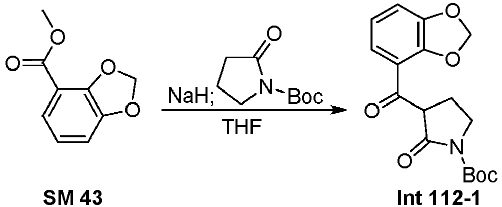

t-Butyl-2-oxopyrrolidin-1-carboxylate (1.8 g, 10 mmol) and benzo[d][1,3]dioxin-4-carboxylic acid methyl ester (1.8 g, 10 mmol) were dissolved in tetrahydrofuran (50 mL), to which was added sodium hydride (0.8 g, 20 mmol), and the reaction was heated to 50°C, and carried out for 4 h. The reaction solution was poured into cold water, and its pH was adjusted to be 5 to 6 with 2N hydrochloric acid. The reaction solution was extracted with ethyl acetate, dried over anhydrous Na₂SO₄, and concentrated, to obtain the crude product, that was directly used in the next step.

### Synthesis of intermediate 5-(benzo[d][1,3]dioxolen-4-yl)-3,4-dihydro-2H-pyrrole (Int 112-2)

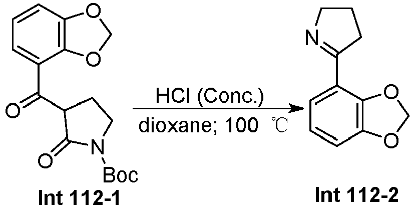

The crude product obtained in the previous step was dissolved in dioxane (10 mL), to which was added concentrated hydrochloric acid (30 mL), and the mixture was heated to 100°C, and stirred overnight. The pH of reaction solution was adjusted to be 7 to 8 with sodium bicarbonate, and then the solution was extracted with ethyl acetate, dried with anhydrous Na₂SO₄, and concentrated. The residue was dissolved in dichloromethane (20 mL), to which was added trifluoroacetic acid (2 mL). The mixture was stirred overnight at room temperature and concentrated, and then water was added. Its pH was adjusted to be 7 to 8, and then the reaction solution was extracted with ethyl acetate, dried over anhydrous Na₂SO₄, concentrated, and purified by column chromatography to provide **Int 112-2** (0.7 g) as colorless liquid, with a two-step yield of 37%. MS: *m*/*z* 190 [M+H]⁺.

### Synthesis of 2-(benzo[ d] [1,3]dioxolen-4-yl)pyrrolidine (Int 112-3)

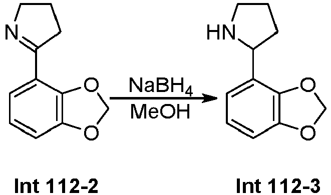

Intermediate **Int 112-2** (0.7 g, 3.7 mmol) was dissolved in methanol (10 mL), to which was added NaBH₄ (0.38 g, 10 mmol) under ice bath, and the mixture was stirred for 4 h, followed by addition of celite and water (10 mL). The solution was filtered, extracted with ethyl acetate, dried over anhydrous Na₂SO₄, concentrated, and purified by column chromatography to obtain **Int 112-3** as pale yellow liquid (650 mg), with a yield of 93%. MS: *m*/*z* 192 [M+H]⁺.

### Synthesis of compound 1-((1R)-(3'-(2-(2-(benzo[d][1,3]dioxolen-4-yl)pyrrolidin- 1-yl)-2-oxoethyl)-2',4'-dioxo-2,3-dihydrospiro[indene-1,5'-oxazolidine]-5-yl)-3-methylurea (112)

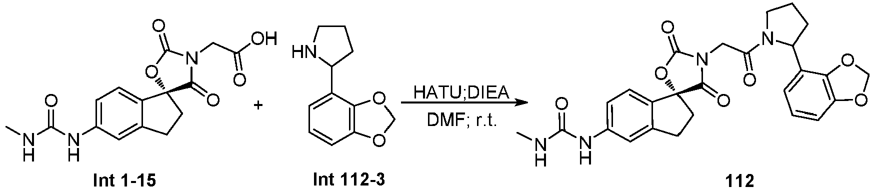

Intermediate **Int 1-15** (30 mg, 90 µmol) was dissolved in DMF (1 mL), to which were added DIEA (35 mg, 270 µmol) and HATU (41 mg, 108 µmol), followed by addition of **Int 112-3** (25.8 mg, 135 µmol), and the mixture was stirred at room temperature for 16 h. To the reaction solution was added water (10 mL), and extracted with ethyl acetate two times. The organic phase was washed with saturated brine, and concentrated to obtain the crude product, that was purified by column chromatography to obtain product **112** (17 mg, yield 61%) as off-white solid. MS: *m*/*z* 507 [M+H]⁺.

### Example 114 Synthesis of compound N-((R)-3'-(2-((2S,5S)-2-(4-fluorophenyl)-5-methylpyrrolidin-1-yl)-2-oxoethyl)-2',4'-dioxo-2,3-dihydrospiro[indene-1,5' -oxazolidine]-5-yl)acetamide (114)

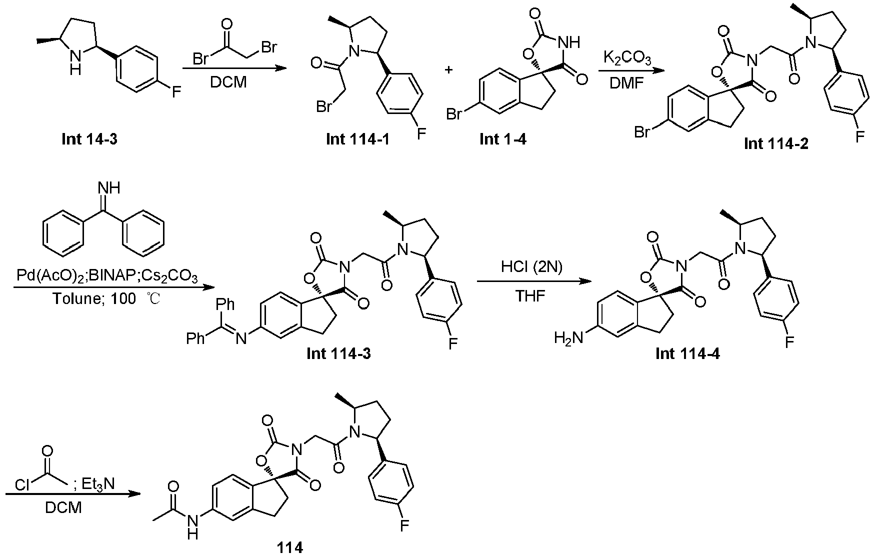

### Synthesis of intermediate 2-bromo-1-((2S,5S)-2-(4-fluorophenyl)-5-(methyl) pyrrolidin-1-yl)ethan-1-one (Int 114-1)

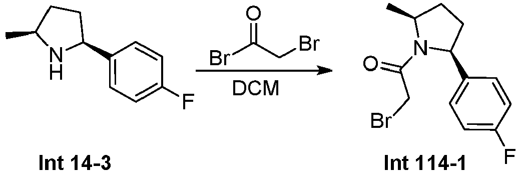

Intermediate **Int 14-3** (900 mg, 5 mmol) was dissolved in dichloromethane (10 mL), to which was added bromoacetyl bromide (200 mg, 10 mmol) dropwise under ice bath. The mixture was stirred for 1 h, extracted with dichloromethane, and purified by column chromatography to give **Int 114-1** (1.35 g) as white solid, with a yield of 90%. MS: *m*/*z* 300, 302 [M+H]⁺.

### Synthesis of intermediate (R)-5-bromo-3'-(2-((2S,5S)-2-(4-fluorophenyl)-5-(methyl) pyrrolidin-1-yl)-2-oxoethyl)-2,3-dihydrospiro[indene-1,5'-oxazolidine]-2',4'-dione (Int 114-2

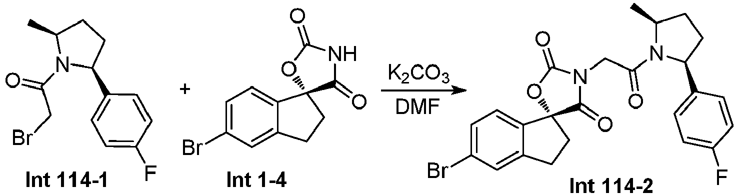

Intermediate **Int 1-4** (500 mg, 1.8 mmol), intermediate **Int 114-1** (550 mg, 1.8 mmol), and potassium carbonate (500 mg, 3.6 mmol) were dissolved in N,N-dimethylformamide (10 mL), and the mixture was stirred 4 h, then water (50 mL) was added. The solution was filtered and dried, to obtain **Int 114-2** (800 mg) as white solid, with a yield of 89%. MS: *m*/*z* 501, 503 [M+H]⁺.

### Synthesis of intermediate (R)-5-(diphenylmethylene )amino)-3'-(2-((2S,5S)-2- (4-fluorophenyl)-5-(methyl)pyrrolidin-1-yl)-2-oxoethyl)-2,3-dihydrospiro[indene-1,5'-oxazolidine]-2',4'-dione (Int 114-3)

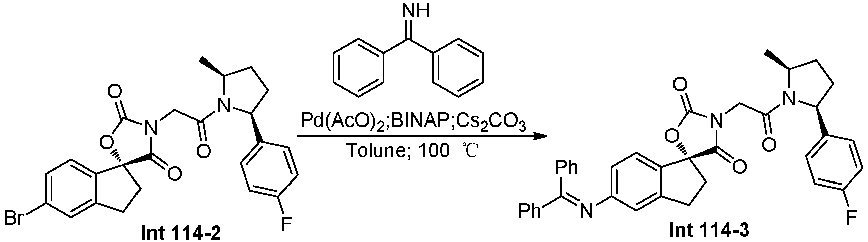

Intermediate **Int 114-2** (800 mg, 1.6 mmol), benzophenonimine (350 mg, 1.9 mmol), palladium acetate (13 mg, 0.06 mmol), 2,2'-bis(diphenylphosphino)-1,1'-binaphthyl (72 mg, 0.12 mmol) and cesium carbonate (780 mg, 2.4 mmol) were dissolved in toluene (5 mL), and the system was purged with N₂. The reaction solution was heated to 100 °C, stirred for 4 h, concentrated, and purified by column chromatography to obtain **Int 114-3** (770 mg) as grey solid, with a yield of 80%. MS: *m*/*z* 602 [M+H]⁺.

### Synthesis of intermediate (R)-5-amino-3'-(2-((2S,5S)-2-(4-fluorophenyl)-5-methylpyrrolidin-1-yl)-2-oxoethyl)-2,3-dihydrospiro[indene-1,5'-oxazolidine]-2',4'-dione (Int 114-4)

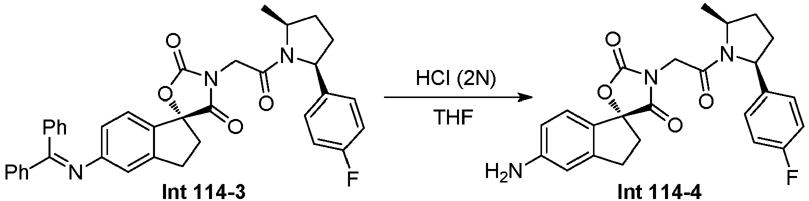

Intermediate **Int 114-3** (770 mg, 1.3 mmol) was dissolved in tetrahydrofuran (10 mL), to which was added hydrochloric acid (10 mL, 2N), and the mixture was stirred 30 min, followed by extracting with ethyl acetate three times. The organic phase was combined, and dried over anhydrous Na₂SO₄, concentrated, and purified by column chromatography to obtain **Int 114-4** as grey solid (400 mg), with a yield of 70%. MS: *m*/*z* 394(M-43).

### Synthesis of compound N-((R)-3'-(2-((2S,5S)-2-(4-fluorophenyl)-5-methylpyrrolidin- 1-yl)-2-oxoethyl)-2',4'-dioxo-2,3-dihydrospiro[indene-1,5'-oxazolidine]-5-yl)acetamide (114)

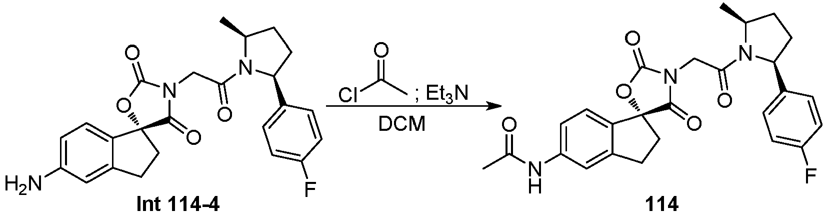

Intermediate **Int 114-4(30** mg, 0.2 mmol) and triethylamine (60 mg, 0.6 mmol) were dissolved in dichloromethane (5 mL), and then acetyl chloride (24 mg, 0.3 mmol) was slowly drop added into the reaction flask in ice bath. After addition, the mixture was stirred for 1 h, to which was added water (10 mL). The organic layer was separated, and the solution was extracted with dichloromethane. The organic phases were combined, dried over anhydrous Na₂SO₄, concentrated, and purified by column chromatography, to obtain **114** as yellow solid (20 mg), with a yield of 58%. MS: *m*/*z* 480.2 [M+H]⁺.

### Example 115 Synthesis of compound ((R)-3'-(2-((2S,5S)-2-(4-fluorophenyl)-5-methylpyrrolidin-1-yl)-2-oxoethyl)-2',4'-dioxo-2,3-dihydrospiro[indene-1,5'-oxazolidine]-5-yl)carbamic acid methyl ester (115)

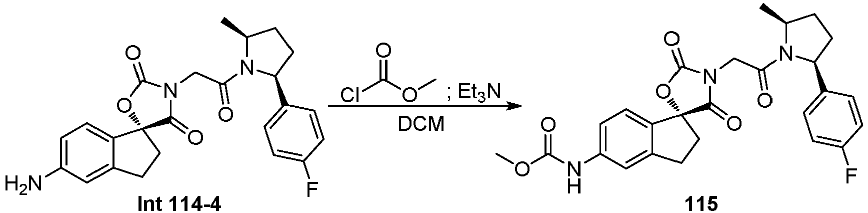

Intermediate **Int 114-4** (30 mg, 0.2 mmol) and triethylamine (60 mg, 0.6 mmol) were dissolved in dichloromethane (5 mL), and then methoxycarbonyl chloride (34 mg, 0.3 mmol) was slowly drop added into the reaction flask in ice bath. After addition, the mixture was stirred for 1 h, to which was added water (10 mL). The organic layer was separated, and the solution was extracted with dichloromethane. The organic phases were combined, dried over anhydrous Na₂SO₄, concentrated, and purified by column chromatography, to obtain 115 as yellow solid (23 mg), with a yield of 60%. MS: *m*/*z* 496.2 [M+H]⁺.

### Example 201 Synthesis of compound 1-(R)-3'-(2-((2S,5S)-2-(3,4-difluorophenyl)- 5-methylpyrrolidin-1-yl)-2-oxoethyl)-2',4'-dioxo-2,3-dihydrospiro[indene-1,5'-oxazolidine]-5-yl)-3-methylurea (201)

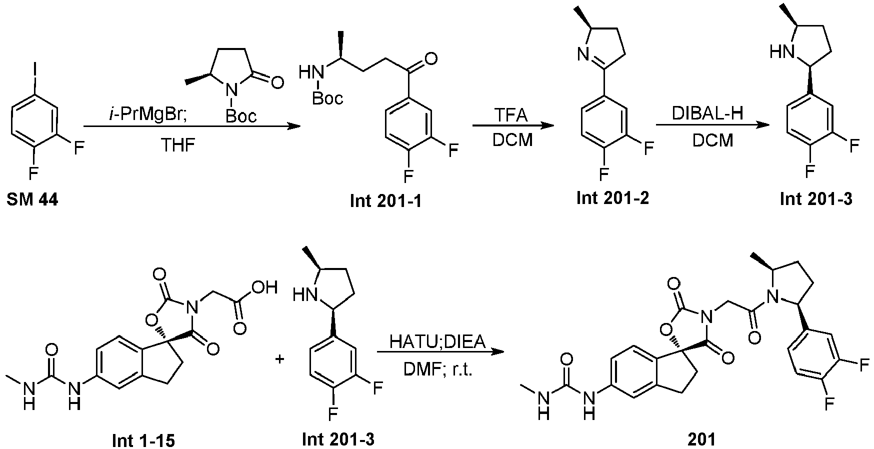

### Synthesis of intermediate (S)-(5-(3,4-difluorophenyl)-5-oxopentan-2-yl)carbamic acid t-butyl ester(Int 201-1)

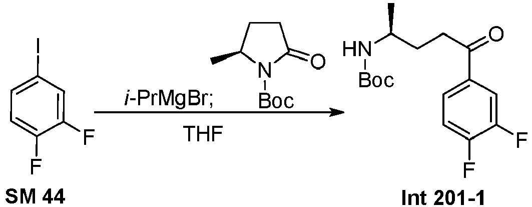

To a reaction flask, was added dry THF (30 mL), followed by addition of **SM 44** (2.4 g, 10 mmol), and then under N₂ protection, isopropylmagnesium bromide (10 mL, 1M in THF) was added in an ice-water bath. After that, the mixture was reacted at room temperature for 7 h, and (S)-2-methyl-5-oxopyrrolidin-1-carboxylic acid t-butyl ester (2 g, 10 mmol) was finally added, and the mixture was further allowed to react at room temperature for 12 h. The reaction solution was poured into the saturated aqueous solution of ammonium chloride, extracted with 30 mL (10 mL×3) of ethyl acetate. The organic phases were combined, dried over anhydrous Na₂SO₄, and purified by column chromatography to obtain **Int 201-1** (1.72 g, 5.5 mmol), with a yield of 55%. MS: *m*/*z* 314 [M+H]⁺.

### Synthesis of intermediate (S)-5-(3,4-difluorophenyl)-2-methyl-3,4-dihydro-2H- pyrrole (Int 201-2)

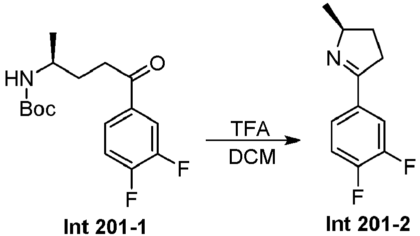

To a reaction solution was added DCM (20 mL), to which was then added **Int 201-1** (1.56 g, 5 mmol), followed by addition of TFA (2 mL). After that, the mixture was reacted at room temperature for 7 h, and the reaction solution was poured into the saturated aqueous solution of sodium bicarbonate. The resultant solution was extracted with 30 mL (10 mL × 3) of DCM. The organic phases were combined, dried over anhydrous Na₂SO₄, and purified by column chromatography, to obtain **Int 201-2** (780 mg, 4 mmol), with a yield of 80%. MS: m/z 196 [M+H]⁺. ¹H NMR (400 MHz, CDCl₃): δ 7.71 (ddd, J= 11.3, 7.8, 2.1 Hz, 1H), 7.63-7.50 (m, 1H), 7.18 (dt, J= 10.0, 8.3 Hz, 1H), 4.39-4.14 (m, 1H), 3.01 (m, 1H), 2.93-2.71 (m, 1H), 2.28 (m, 1H), 1.68-1.51 (m, 1H), 1.36 (d, J = 6.8 Hz, 3H).

### Synthesis of Int 201-3(Int 201-3)

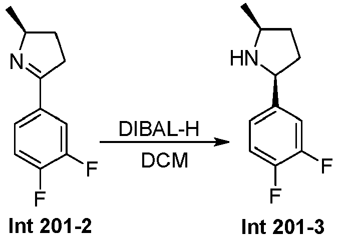

To a reaction flask was added DCM (15 mL), to which was then added **Int 201-2** (780 mg, 4 mmol), followed by addition of DIBAL-H (16 mL, 1M in hexane) in ice-water bath. After that, the mixture was reacted at room temperature for 1 h, and quenched by adding a small amount of water. The solution was filtered, washed with DCM (15 mL×3), and concentrated, to obtain **Int 201-3** (752 mg, 3.8 mmol), with a yield of 95%. MS: *m*/*z* 198 [M+H]⁺. ¹H NMR (400 MHz, CDCl₃): δ 7.25-7.16 (m, 1H), 7.09-7.04 (m, 2H), 4.22-3.95 (m, 1H), 3.38-3.27 (m, 1H), 2.20-2.10 (m, 1H), 1.96 (m, 1H), 1.73-1.58 (m, 1H), 1.51-1.38 (m, 1H), 1.24 (d, J = 6.2 Hz, 3H).

### Synthesis of compound 1-(R)-3'-(2-((2S,SS)-2-(3,4-difluorophenyl)-5- methylpyrrolidin-1-yl)-2-oxoethyl)-2',4'-dioxo-2,3-dihydrospiro[indene-1,5'-oxazolidine]-5-yl)-3-methylurea (201)

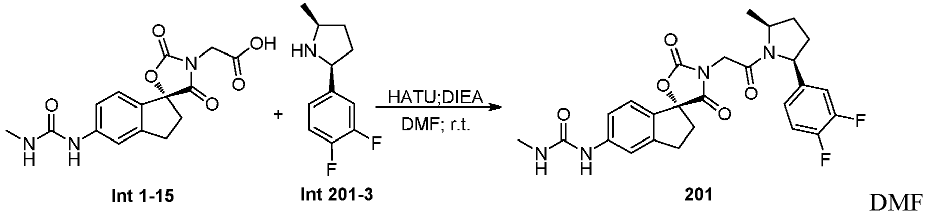

(3 mL) was added to a 25 mL reaction flask, to which were then added **Int 1-15** (333 mg, 1 mmol), **Int 201-3** (197 mg, 1 mmol), and DIEA (387 mg, 3 mmol), and finally HATU (381 mg, 1 mmol) was added. The system was reacted at 20 °C for 1 h. After completion of the reaction, the reaction solution was poured into water (20 mL), and extracted with 30 mL (10 mL×3) of ethyl acetate. The organic phases were combined, dried over anhydrous Na₂SO₄, and purified by column chromatography to obtain compound **201** (282 mg), with a yield of 55%. MS: *m*/*z* 513 [M+H]⁺. ¹H NMR (400 MHz, DMSO): δ 8.71 (s, 1H), 7.54 (d, J = 5.7 Hz, 2H), 7.44-7.33 (m, 1H), 7.29-7.15 (m, 3H), 6.08 (s, 1H), 5.29-4.91 (m, 1H), 4.41 (m, 2H), 3.52 (m, 1H), 3.18-2.93 (m, 2H), 2.63 (t, J = 3.8 Hz, 3H), 2.60-2.53 (m, 1H), 2.45-2.31 (m, 2H), 1.99 (s, 2H), 1.85-1.62 (m, 1H), 1.36 (d, J = 6.4 Hz, 3H).

### Example 205 Synthesis of compound 1-((R)-3'-(2-((2S,5R)-2-(3,4,5- trifluorophenyl)-5-methylpyrrolidin-1-yl)-2-oxoethyl)-2',4'-dioxo-2,3-dihydrospiro[indene-1, 5'-oxazolidine]-5-yl)-3-methylurea (205)

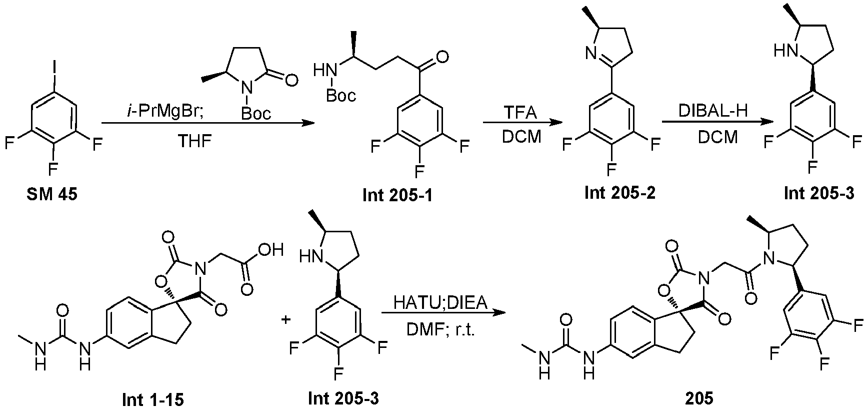

### Synthesis of intermediate (S)-(5-oxo-5-(3,4,5-trifluorophenyl)pentan-2-yl)carbamic acid t-butyl ester(Int 205-1)

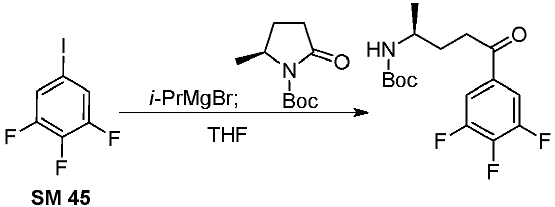

To a reaction flask, was added dry THF (30 mL), followed by addition of **SM 45** (2.57 g, 10 mmol), and then under N₂ protection, isopropylmagnesium bromide (10 mL, 1M in THF) was added in an ice-water bath. After that, the mixture was reacted at room temperature for 7 h, and (S)-2-methyl-5-oxopyrrolidin-1-carboxylic acid t-butyl ester (2 g, 10 mmol) was finally added, and the mixture was further allowed to react at room temperature for 12 h. The reaction solution was poured into the saturated aqueous solution of ammonium chloride, extracted with 30 mL (10 mL×3) of ethyl acetate. The organic phases were combined, dried over anhydrous Na₂SO₄, and purified by column chromatography to obtain **Int 205-1** (2 g, 6 mmol), with a yield of 60%. MS: *m*/*z* 332 [M+H]⁺.

### Synthesis of intermediate (S)-2-methyl-5-(3,4,5-trifluorophenyl)-3,4-dihydro-2H- pyrrole (Int 205-2)

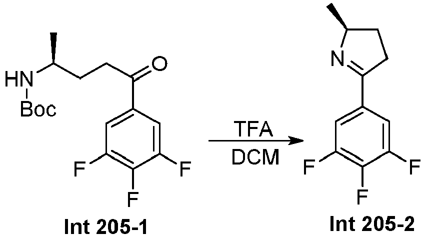

To a reaction flask was added DCM (20 mL), to which was then added **Int 205-1** (1.66 g, 5 mmol), followed by addition of TFA (2 mL), and then the mixture was reacted at room temperature for 7 h. The reaction solution was poured into the saturated aqueous solution of sodium bicarbonate, extracted with 30 mL (10 mL×3) of DCM. The organic phases were combined, dried over anhydrous Na₂SO₄, and purified by column chromatography to obtain **Int 205-2** (852 mg, 4 mmol), with a yield of 80%. MS: *m*/*z* 214 [M+H]⁺.

### Synthesis of intermediate (2S,SS)-2-methyl-5-(3,4,5-trifluorophenyl)-3,4-dihydro-2H-pyrrole (Int 205-3)

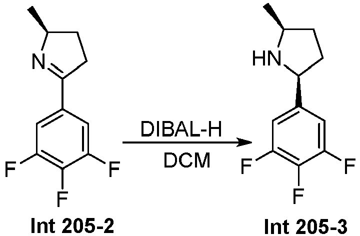

To a reaction flask was added DCM (15 mL), to which was then added **Int 205-2** (852 mg, 4 mmol), followed by addition of DIBAL-H (16 mL, 1M in hexane) in ice-water bath. After that, the mixture was reacted at room temperature for 1 h, and quenched by adding a small amount of water. The solution was filtered, washed with DCM (15 mL×3), and concentrated, to obtain **Int 205-3** (774 mg, 3.6 mmol), with a yield of 90%. MS: *m*/*z* 216 [M+H]⁺. ¹H NMR (400 MHz, CDCl₃): δ 7.09-6.99 (m, 2H), 4.12 (t, J = 7.7 Hz, 1H), 3.34 (m, 1H), 2.33 (m, 1H), 2.16 (m, 1H), 1.94 (m, 1H), 1.69-1.54 (m, 1H), 1.52-1.34 (m, 1H), 1.23 (d, J = 6.2 Hz, 3H).

### Synthesis of compound 1-((R)-3'-(2-((2S,SR)-2-(3,4,5-trifluorophenyl)-5- methylpyrrolidin-1-yl)-2-oxoethyl)-2',4'-dioxo-2,3-dihydrospiro[indene-1,5'-oxazolidine]-5-yl)-3-methylurea (205)

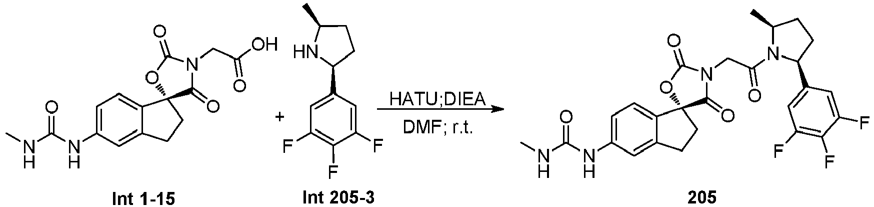

DMF (3 mL) was added to a 25 mL reaction flask, to which were then added **Int 1-15** (333 mg, 1 mmol), **Int 205-3** (215 mg, 1 mmol), and DIEA (387 mg, 3 mmol), and finally HATU (381 mg, 1 mmol) was added. The system was reacted at 20 °C for 1 h. After completion of the reaction, the reaction solution was poured into water (20 mL), and extracted with 30 mL (10 mL×3) of ethyl acetate. The organic phases were combined, dried over anhydrous Na₂SO₄, and purified by column chromatography to obtain compound **205** (265 mg), with a yield of 50%. MS: *m*/*z* 531 [M+H]⁺. ¹H NMR (400 MHz, DMSO): δ 8.72 (s, 1H), 7.54 (d, J = 4.3 Hz, 1H), 7.33-7.21 (m, 2H), 7.17 (s, 1H), 7.10 (dd, J = 8.9, 6.8 Hz, 1H), 6.16-6.00 (m, 1H), 5.08 (m, 1H), 4.77-4.34 (m, 2H), 3.60 (dd, J = 7.5, 5.4 Hz, 1H), 3.11 (dd, J = 16.0, 7.7 Hz, 1H), 2.98 (dd, J = 10.9, 5.6 Hz, 1H), 2.70-2.57 (m, 4H), 2.43-2.31 (m, 1H), 2.16-1.79 (m, 2H), 1.79-1.71 (m, 1H), 1.48 (d, J = 6.3 Hz, 1H), 1.35 (dd, J = 6.3, 2.4 Hz, 3H).

### Example 209 Synthesis of compound 1-((R)-3'-(2-((2S,5S)-2-(4,4-difluorocyclohexyl )-5-methylpyrrolidin-1-yl)-2-oxoethyl)-2',4'-dioxo-2,3-dihydrospiro[indene-1, 5'-oxazolidine]-5-yl)-3-methylurea (209)

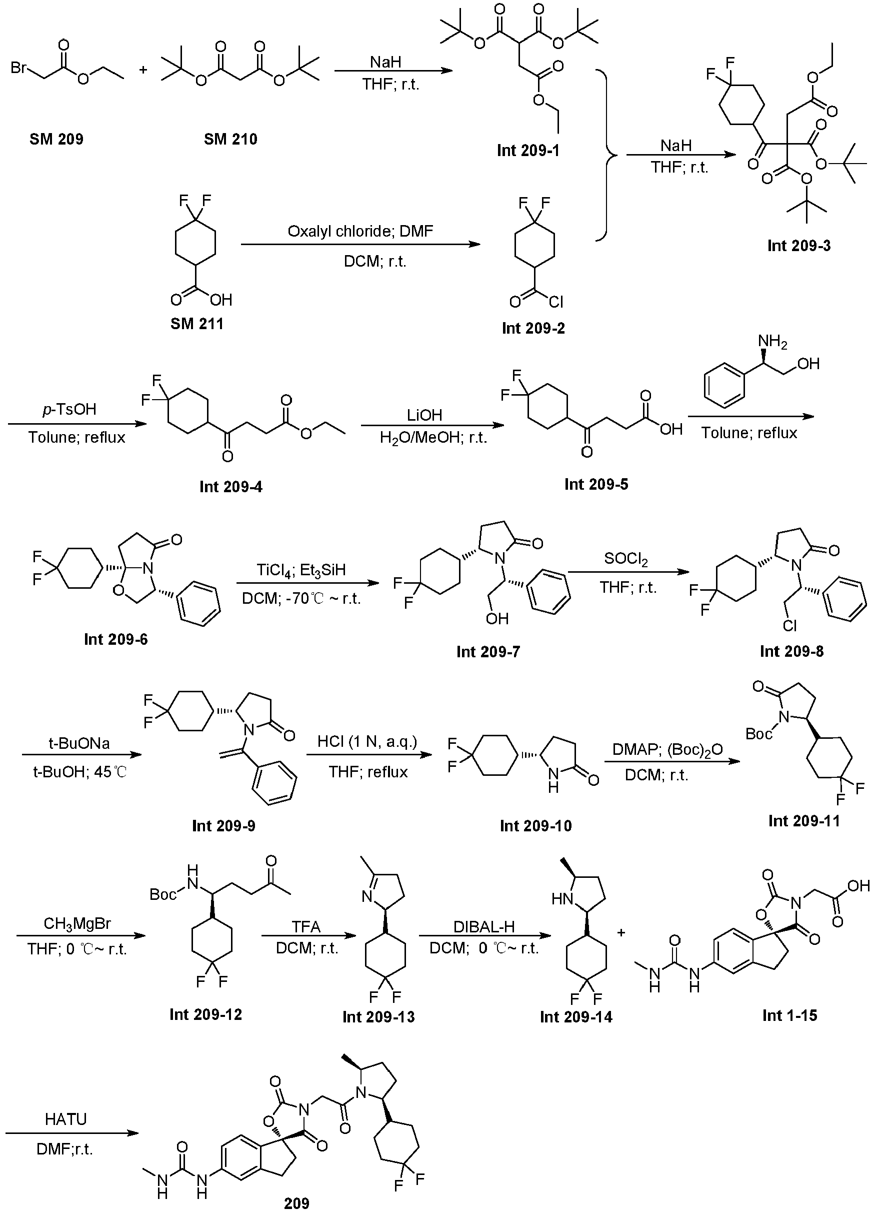

### Synthesis of 1,1-di-t-butyl 2-ethylethane-1, 1,2-tricarboxylate (Int 209-1)

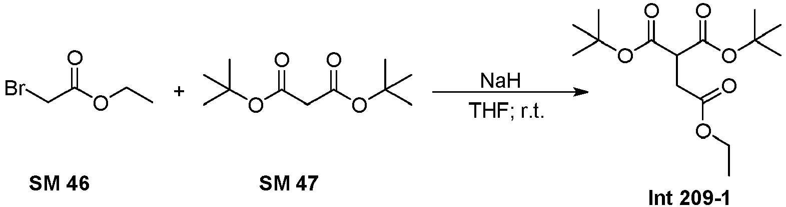

**SM 46** (21.6 g; 0.1 mol) was dissolved in THF (220 mL), to which was added NaH (6.67 g; 60%; 0.1 mol), and the mixture was stirred at room temperature for 30 min. Then, **SM 47** (16.7 g; 0.1 mol) was added to the reaction flask, and the mixture was still stirred at room temperature until the reaction was completed by TLC detection. The reaction solution was poured into ice water (220 mL), and extracted twice with ethyl acetate. The organic phase was washed with saturated brine, and concentrated to obtain the crude product, that was purified by column chromatography to obtain the oily product **Int 209-1** (25.6 g; 85 %). MS: *m*/*z* 303 [M+H]⁺.

### Synthesis of intermediate 4,4-difluorocyclohexanecarbonyl chloride (Int 209-2)

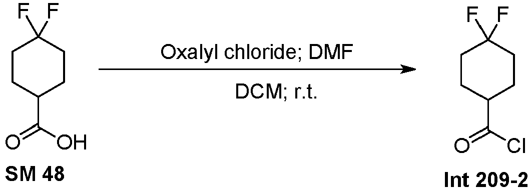

**SM 48** (16.4 g, 0.1 mol) was dissolved in DCM (17 mL), to which was added one drop of DMF, and then oxalyl chloride (14 g, 0.11 mol) was added dropwise. After addition, the reaction was stirred at room temperature for 4 h, and then concentrated, to obtain the crude product **Int 209-2** (18.2 g, 100%), that was directly used in the next step.

### Synthesis of intermediate 2,2-di-t-butyl-1-ethyl-3-(4,4-difluorocyclohexyl)- 3-oxopropane-1,2,2-tricarboxylate (Int 209-3)

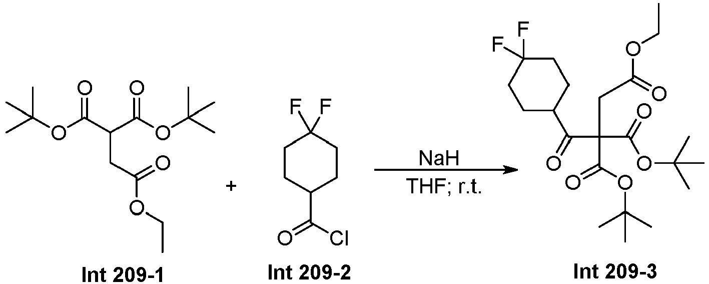

**Int 209-1** (25.6 g; 85 mmol) was dissolved in THF (250 mL), to which was added NaH (5.6 g; 60%; 85 mmol), and the mixture was stirred at room temperature for 30 min, followed by adding the solution of **Int 209-2** (18.2 g, 100 mmol) in THF (182 mL) dropwise. Then, the mixture was reacted at room temperature for 4 h. The reaction solution was poured into ice water (250 mL), and extracted twice with ethyl acetate. The organic phases were combined, washed with saturated brine, dried over anhydrous sodium sulfate, and concentrated to obtain the crude product, that was purified by column chromatography to provide the oily product **Int 209-3** (34.3 g, 90%). MS: *m*/*z* 449 [M+H]⁺.

### Synthesis of intermediate 4-(4,4-difluorocyclohexyl)-4-oxobutyric acid ethyl ester (Int 209-4)

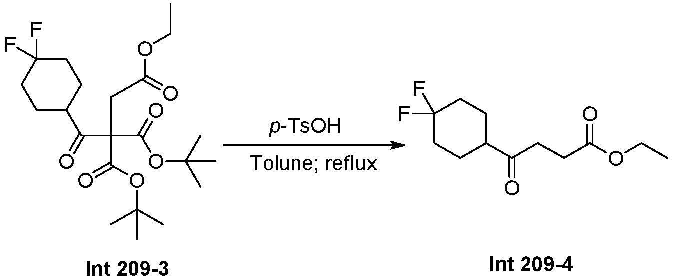

**Int 209-3** (34.3 g, 76 mmol) was added in toluene (340 mL), to which was added p-toluenesulfonic acid (1.3 g; 7.6 mmol), and the mixture was refluxed and monitored by TLC. After the reaction was completed, the solvent was evaporated under reduced pressure, and the residue was purified by column chromatography, to obtain the oily **Int 209-4** (15.4 g, 82%). MS: *m*/*z* 249 [M+H]⁺.

### Synthesis of intermediate 4-(4,4-difluorocyclohexyl)-4-oxobutyric acid (Int 209-5)

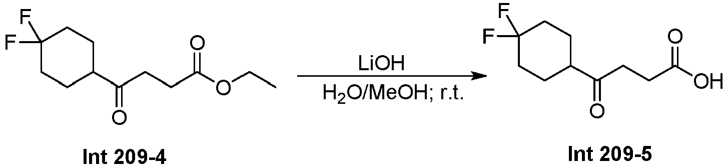

**Int 209-4** (15.4 g, 62 mmol) was added to a mixed solvent of water/methanol (1/10, 15 mL), to which was added lithium hydroxide monohydrate (13.0 g; 310 mmol), and the mixture was stirred at room temperature and monitored by TLC. After the reaction was completed, most of the solvent was removed by distillation under reduced pressure, and the pH was adjusted to be 2 to 3 with HCl (2 N) solution. The resultant solution was extracted with DCM (100 mL × 3), and the organic phases were combined, washed with saturated brine, and dried with anhydrous Na₂SO₄. The solvent was removed by distillation under reduced pressure, to obtain the product Int. **209-5** (12.5 g, 92%). MS: *m*/*z* 221 [M+H]⁺.

### Synthesis of intermediate (3R,7aR)-7a-(4,4-difluorocyclohexyl)-3-phenyltetrahydropyrrolo[2,1-b]oxazol-5(6H)-one (Int 209-6)

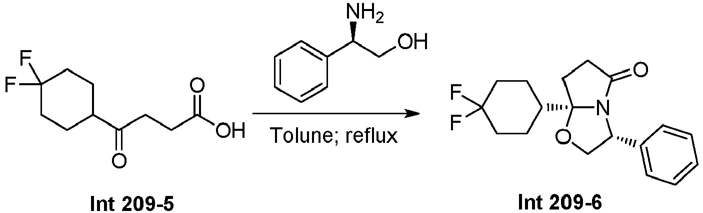

**Int 209-5** (12.5 g, 57 mmol) and D-phenylglycinol (7.8 g, 57 mmol) were added in toluene (130 mL), and then refluxed to separate water. The reaction was monitored by TLC. After completion of the reaction, the product **Int 209-6** (14.4 g, 79%) was obtained by column chromatography. MS: *m*/*z* 322 [M+H]⁺.

### Synthesis of intermediate (S)-5-(4,4-difluorocyclohexyl)-1-((R)-2-hydroxyl-1-phenylethyl)pyrrolidin-2-one (Int 209-7)

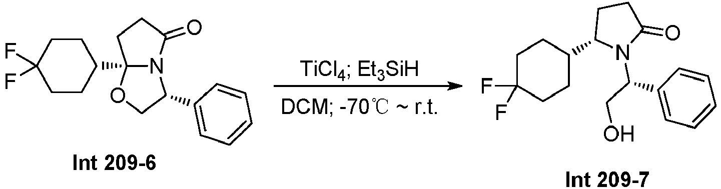

**Int 209-6** (14.4 g, 45 mmol) and Et₃SiH (17.21 g, 148.5 mmol) were added in DCM (150 mL), and the mixture was cooled to -70°C under nitrogen protection, to which was drop added the solution of TiCl₄ in THF (99 ml, 1M, 99 mmol), and the reaction temperature was controlled to be lower than -65 °C. After addition, the reaction was naturally warmed to room temperature, stirred, and monitored by TLC. After the completion of the reaction, the reaction solution was washed with saturated NaHCO₃ solution, then with saturated brine, and dried over anhydrous sodium sulfate. The product **Int 209-7** (13.8 g, 95%) was obtained by distillation under reduced pressure. MS: *m*/*z* 324 [M+H]⁺.

### Synthesis of intermediate (S)-1-((R)-2-chloro-1-phenylethyl)-5-(4,4-difluorocyclohexyl)pyrrolidin-2-one (Int 209-8)

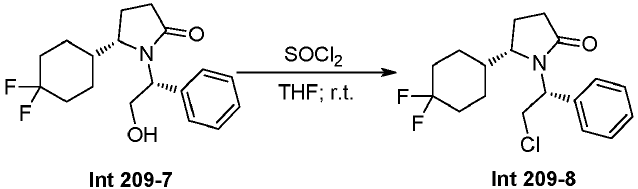

**Int 209-7** (13.8 g, 43 mmol) was added in THF (140 mL), to which was added thionyl chloride (10.2 g, 86 mmol) dropwise, and the reaction was stirred at room temperature, and detected by TLC. After completion of the reaction, the reaction solution was washed with saturated NaHCO₃ solution, and then with saturated brine, followed by drying over anhydrous Na₂SO₄ and distillation under reduced pressure, to obtain the product **Int 209-8** (13.9 g, 95%). MS: *m*/*z* 342 [M+H]⁺.

### Synthesis of intermediate (S)-5-(4,4-difluorocyclohexyl)-1-(1-phenylvinyl)pyrrolidin- 2-one (Int 209-9)

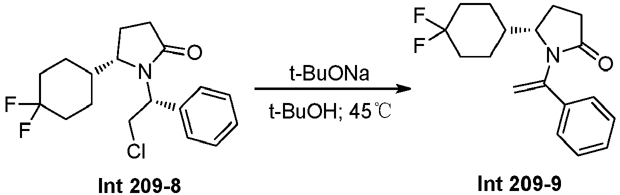

**Int 209-8** (13.9 g, 40 mmol) was dissolved in t-BuOH (70 mL), to which was added sodium t-butoxide (7.68 g, 80 mmol), and the reaction was stirred at 45 °C and detected by TLC. After completion of the reaction, part of solvent was rotatory evaporated, to which was added water (70 mL). The resultant solution was extracted with DCM (50 mL × 3), washed with saturated brine, dried over anhydrous Na₂SO₄, and evaporated under reduced pressure to obtain **Int 209-9** (11.6 g, 95%). MS: *m*/*z* 306 [M+H]⁺.

### Synthesis of intermediate (S)-5-(4,4-difluorocyclohexyl)pyrrolidin-2-one (Int 209-10)

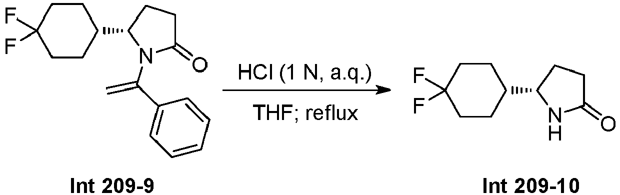

**Int 209-9** (11.6 g, 38 mmol) was dissolved in THF(120 mL), to which was added HCl (1N; 12 mL), and the reaction was refluxed and detected by TLC. After completion of the reaction, pH was adjusted to be 7 to 8 with saturated NaHCO₃ solution, and the solution was extracted with DCM (50 mL×3). The organic phases were combined, washed with saturated brine, dried over anhydrous Na₂SO₄, and purified by column chromatography to obtain the product **Int 209-10** (7.37 g, 95%). MS: *m*/*z* 204 (M+H⁺).

### Synthesis of intermediate (S)-2-(4,4-difluorocyclohexyl)-5-oxopyrrolidin-1- carboxylic acid t-butyl ester (Int 209-11)

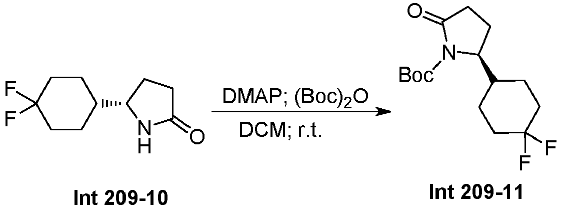

**Int 209-10** (7.37 g, 36 mmol) was dissolved in DCM (70 mL), to which were added DMAP (0.44 g, 3.6 mmol) and (Boc)₂O (7.84 g, 36 mmol), and the reaction was stirred at room temperature and detected by TLC. After completion of the reaction, the solution was rinsed with HCl (0.5 N; 70 mL×2), then with saturated brine, dried over anhydrous Na₂SO₄, and evaporated under reduced pressure to obtain the product **Int 209-11** (10.36 g, 95%). MS: *m*/*z* 304 [M+H]⁺.

### Synthesis of intermediate (S)-t-butyl-(1-(4,4-difluorocyclohexyl)-4-oxopentyl) carbamate (Int 209-12)

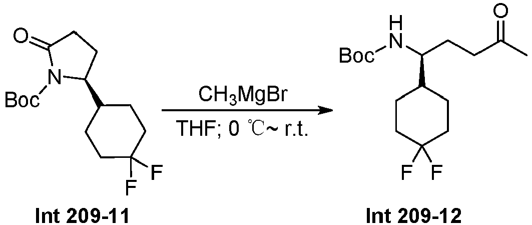

**Int 209-11** (303 mg, 1 mmol) was dissolved in tetrahydrofuran (3 mL), and after cooling to 0 °C in an ice-water bath, the solution of methylmagnesium bromide in tetrahydrofuran (1M in THF; 1 mL) was added dropwise. The reaction was naturally warmed to room temperature, then stirred, and detected by TLC. After completion of the reaction, the reaction solution was poured into the saturated aqueous solution of ammonium chloride (6 mL), and extracted with ethyl acetate (5 mL×3). The organic phases were combined, washed with saturated brine, dried over anhydrous sodium sulfate, and evaporated under reduced pressure to obtain the product **Int 209-12** (223 mg, 70%). MS: *m*/*z* 320 [M+H]⁺.

### Synthesis of intermediate (S)-2-(4,4-difluorocyclohexyl)-5-methyl-3,4-dihydro-2H- pyrrole (Int 209-13)

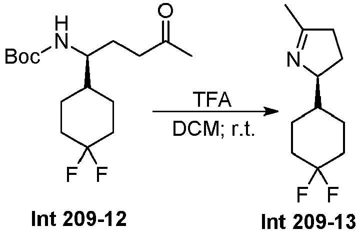

**Int 209-12** (223 mg, 0.7 mmol) was dissolved in dichloromethane (2 mL), to which was added trifluoroacetic acid (0.5 mL), and the reaction was stirred at room temperature, and detected by TLC. After completion of the reaction, the solvent was directly evaporated under reduced pressure, to obtain **Int 209-13** (131 mg, 93 %). MS: *m*/*z* 202 [M+H]⁺.

### Synthesis of intermediate (2S,SS)-2-(4,4-difluorocyclohexyl)-5-methylpyrrolidine (Int 209-14)

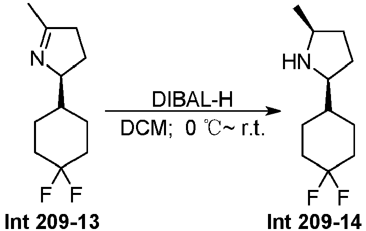

**Int 209-13** (131 mg, 0.65mmol) was dissolved in dichloromethane (1.5 mL), and after cooling to 0 °C in an ice-water bath, the solution of diisobutylaluminum hydride in n-hexane (1 M in hexane; 2.28 mL) was added dropwise. The reaction was naturally warmed to room temperature and stirred under TLC monitoring. After the reaction was completed, dichloromethane (3 mL) was added, and then the solution was cooled to 0 °C in an ice-water bath, to which were successively added water (0.09 mL), 15% NaOH aqueous solution (0.09 mL), water (0.23 mL), and anhydrous calcium sulfate. The solution was stirred for 10 min, filtered, and rinsed with dichloromethane, followed by drying over anhydrous sodium sulfate and evaporating under reduced pressure to obtain the product **Int 209-14** (112 mg, 85%). MS: *m*/*z* 204 [M+H]⁺.

### Synthesis of compound 1-((R)-3'-(2-((2S,5S)-2-(4,4-difluorocyclohexyl)-5-methylpyrrolidin-1-yl)-2-oxoethyl)-2',4'-dioxo-2,3-dihydrospiro[indene-1, 5'-oxazolidine]-5-yl)-3-methylurea (209)

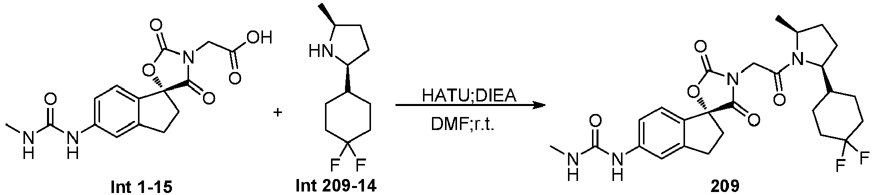

To a 30 mL reaction flask, were added **Int 1-15** (30 mg, 0.06 mmol), DMF (1 mL), **Int 209-14** (12 mg, 0.06 mmol), and HATU (34 mg, 0.09 mmol), and the system was reacted at room temperature for 3 h. After completion of the reaction, the reaction solution was poured into water (30 mL), and extracted with 15 mL (5 mL×3) ethyl acetate. The organic phases were combined, dried over anhydrous Na₂SO₄, and purified by column chromatography to obtain compound **209** (17 mg), with a yield of 57%. MS: *m*/*z* 519 [M+H]⁺.

### Example 212 Synthesis of compound 1-((R)-3'-(2-((2S,5R)-2-(4,4- difluorocyclohexyl)-5-ethyl-pyrrolidin-1-yl)-2-oxoethyl)-2',4'-dioxo-2,3-dihydrospiro[indene-1,5'-oxazolidine]-5-yl)-3-methylurea (212)

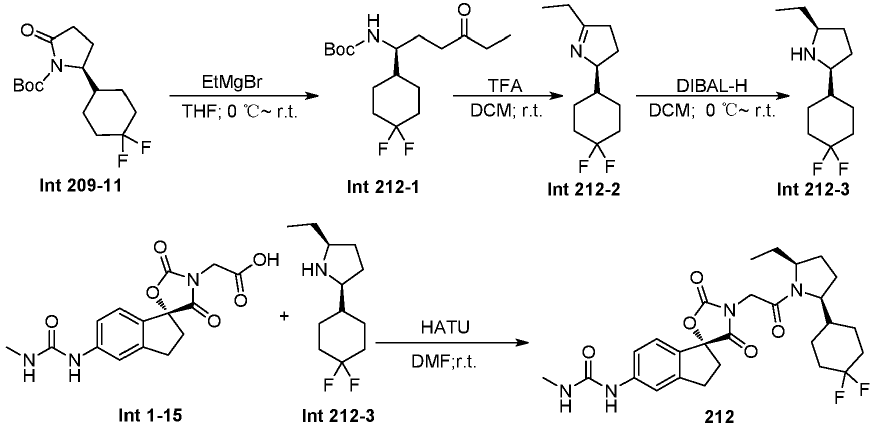

### Synthesis of intermediate (S)-(1-(4,4-difluorocyclohexyl)-4-oxohexyl)carbamic acid t-butyl ester (Int 212-1)

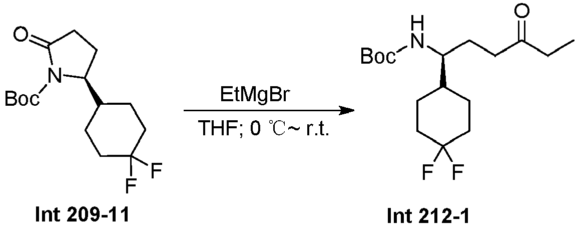

**Int 209-11** (303 mg, 1 mmol) was dissolved in tetrahydrofuran (3 mL), and after cooling to 0 °C in an ice-water bath, the solution of methylmagnesium bromide in tetrahydrofuran (1M in THF; 1 mL) was added dropwise. The reaction was naturally warmed to room temperature, then stirred, and detected by TLC. After completion of the reaction, the reaction solution was poured into the saturated aqueous solution of ammonium chloride (6 mL), and extracted with ethyl acetate (5 mL×3). The organic phases were combined, washed with saturated brine, dried over anhydrous sodium sulfate, and evaporated under reduced pressure to obtain the product **Int 212-1** (243 mg, 73%). MS: *m*/*z* 334 [M+H]⁺.

### Synthesis of intermediate (S)-2-(4,4-difluorocyclohexyl)-5-ethyl-3,4-dihydro-2H- pyrrole (Int 212-2)

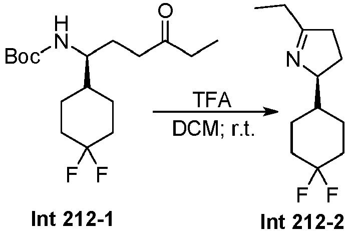

**Int 212-1** (243 mg, 0.7 mmol) was dissolved in dichloromethane (2 mL), to which was added trifluoroacetic acid (0.2 mL), and the reaction was stirred at room temperature, and detected by TLC. After completion of the reaction, the solvent was directly evaporated under reduced pressure, to obtain **Int 212-2** (138 mg, 88%). MS: *m*/*z* 216 [M+H]⁺.

### Synthesis of intermediate (2S,SS)-2-(4,4-difluorocyclohexyl)-5-ethylpyrrolidine (Int 212-3)

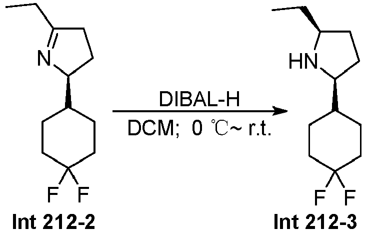

**Int 212-2** (138 mg, 0.64 mmol) was dissolved in dichloromethane (1.5 mL), and after cooling to 0 °C in an ice-water bath, the solution of diisobutylaluminum hydride in n-hexane (1 M in hexane; 2.28 mL) was added dropwise. The reaction was naturally warmed to room temperature and stirred under TLC monitoring. After the reaction was completed, dichloromethane (3 mL) was added, and then the solution was cooled to 0 °C in an ice-water bath, to which were successively added water (0.09 mL), 15% NaOH aqueous solution (0.09 mL), water (0.23 mL), and anhydrous calcium sulfate. The solution was stirred for 10 min, filtered, and rinsed with dichloromethane, followed by drying over anhydrous sodium sulfate and evaporating under reduced pressure to obtain the product **Int 212-3** (111 mg, 80%). MS: *m*/*z* 218 [M+H]⁺.

### Synthesis of compound 1-((R)-3'-(2-((2S,5R)-2-(4,4-difluorocyclohexyl)-5-ethyl-pyrrolidin-1-yl)-2-oxoethyl)-2',4'-dioxo-2,3-dihydrospiro[indene-1, 5'-oxazolidine]-5-yl)-3-methylurea (212)

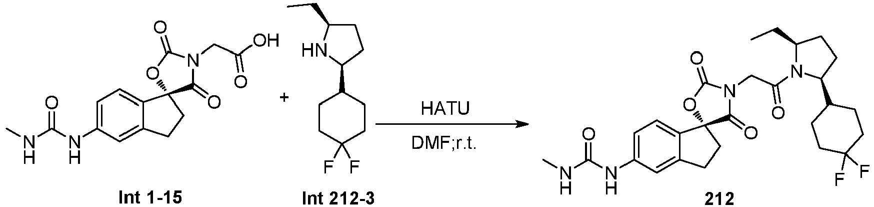

To a 30 mL reaction flask, were added **Int 1-15** (30 mg, 0.06 mmol), DMF (1 mL), **Int 212-3** (13 mg, 0.06 mmol), and HATU (34 mg, 0.09 mmol), and the system was reacted at room temperature for 3 h. After completion of the reaction, the reaction solution was poured into water (30 mL), and extracted with 15 mL (5 mL×3) ethyl acetate. The organic phases were combined, dried over anhydrous Na₂SO₄, and purified by column chromatography to obtain compound **212** (25 mg), with a yield of 78%. MS: *m*/*z* 533 [M+H]⁺.

### Example 213 Synthesis of 1-((R)-3'-(2-((2S,5S)-2-(3-chloro-4-fluorophenyl)-5-methylpyrrolidin-1-yl)-2-oxoethyl)-2',4'-dioxo-2,3-dihydrospiro[indene-1, 5'-oxazolidine]-5-yl)-3-methylurea (213)

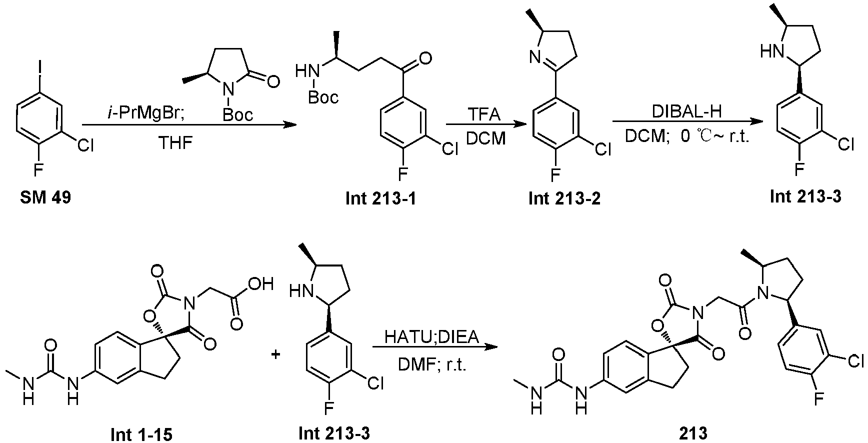

### Synthesis of intermediate (S)-(5-(3-chloro-4-fluorophenyl)-5-oxopentan-2-yl) carbamic acid t-butyl ester (Int 213-1)

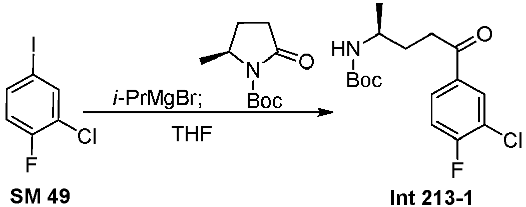

To a 100 mL reaction flask, was added dry THF (25 mL), followed by addition of 1-fluoro-2-chloro-4-iodobenzene (5 g, 19.5 mmol), and then after cooling to 0 °C in an ice-water bath, isopropylmagnesium chloride (10 mL, 2.0 mol/L in THF) was slowly added. After that, the ice-water bath was removed, and the reaction was warmed to room temperature and stirred for 3 h. The starting material (S)-2-methyl-5-oxopyrrolidin-1-carboxylic acid t-butyl ester (4.9 g, 24.8 mmol) was dissolved in THF (10 mL), and quickly added to the above reaction solution. The mixture was allowed to react at room temperature for 3 h. After completion of the reaction, the reaction was quenched by addition of the saturated aqueous solution of ammonium chloride (30 mL), and extracted with ethyl acetate (30 mL×3). The organic phases were combined, dried over anhydrous Na₂SO₄, and concentrated to obtain crude product **Int. 213-1** (8 g). MS: *m*/*z* 330 [M+H]⁺.

### Synthesis of intermediate (S)-5-(3-chloro-4-fluorophenyl)-2-methyl-3,4-dihydro-2H-pyrrolidine (Int 213-2)

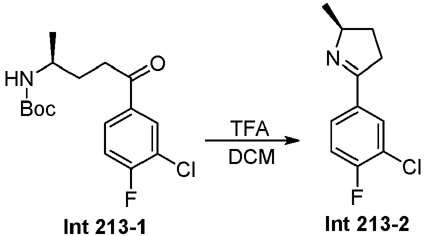

Crude product **Int 213-1** was dissolved in DCM (50 mL), to which was added TFA (5 mL), and the reaction was stirred at room temperature for 16 h. After completion of the reaction, the reaction solution was concentrated, and water (50 mL) was added to the residue. Sodium bicarbonate was used to adjust the pH to 7 to 8, and the solution was extracted with ethyl acetate (30 mL×3). The organic phases were combined, dried with anhydrous sodium sulfate, concentrated, and purified by column chromatography to obtain compound **Int 213-2** (2.4 g), with a two-step yield of 60%. MS: *m*/*z* 212 [M+H]⁺.

### Synthesis of intermediate (2S,SS)-2-(3-chloro-4-fluorophenyl)-5-methylpyrrolidine (Int 213-3)

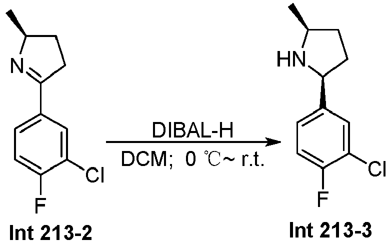

**Int 213-2** (2 g, 9.4mmol) was dissolved in DCM (50 mL), to which was then added DIBAL-H (22 mmol, 1M in hexane) in ice-water bath, and the mixture was reacted at room temperature for 2 h. After completion of the reaction, the solution was concentrated, and then water (50 mL) was added. The solution was extracted with DCM (30 mL×3). The organic phases were combined, dried over anhydrous Na₂SO₄, concentrated, and purified by column chromatography to obtain **Int 213-3** (650 mg), with a yield of 33%. MS: *m*/*z* 180 [M+H]⁺. ¹H NMR (400 MHz, CDCl3) δ 7.45 (dd, J = 7.2, 2.1 Hz, 1H), 7.23 (dddd, J = 8.4, 4.6, 2.2, 0.4 Hz, 1H), 7.06 (t, J = 8.7 Hz, 1H), 4.11 (t, J = 7.9 Hz, 1H), 3.31 (dd, J = 14.1, 7.0 Hz, 1H), 2.18-2.09 (m, 1H), 1.95 (dddd, J= 12.6, 8.7, 7.1, 5.8 Hz, 1H), 1.69-1.59 (m, 1H), 1.44 (dddd, J = 12.4, 10.2, 8.1, 6.2 Hz, 1H), 1.24 (t, J = 6.1 Hz, 3H).

### Synthesis of compound 1-((R)-3'-(2-((2S,5S)-2-(3-chloro-4-fluorophenyl)-5-methylpyrrolidin-1-yl)-2-oxoethyl)-2',4'-dioxo-2,3-dihydrospiro[indene-1, 5'-oxazolidine]-5-yl)-3-methylurea (213)

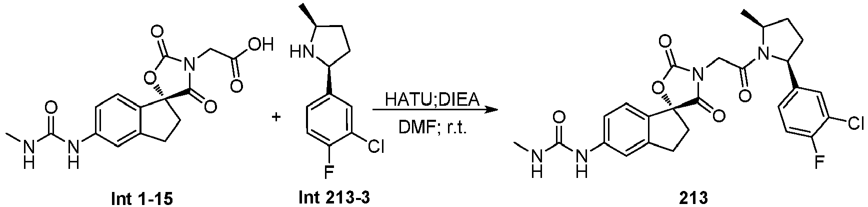

DMF (3 mL) was added to a 25 mL reaction flask, to which were then added **Int 1-15** (333 mg, 1 mmol), **Int 213-3** (213 mg, 1 mmol), and DIEA (387 mg, 3 mmol), and finally HATU (381 mg, 1 mmol) was added. The system was reacted at 20 °C for 1 h. After completion of the reaction, the reaction solution was poured into water (20 mL), and extracted with 30 mL (10 mL×3) ethyl acetate. The organic phases were combined, dried over anhydrous Na₂SO₄, and purified by column chromatography to obtain compound **213** (280 mg), with a yield of 60%. MS: *m*/*z* 529 [M+H]⁺.

### Example 217 Synthesis of compound 1-((R)-3'-(2-((2S,5S)-2-(2,4-difluorophenyl)- 5-methylpyrrolidin-1-yl)-2-oxoethyl)-2',4'-dioxo-2,3-dihydrospiro[indene-1, 5'-oxazolidine]-5-yl)-3-methylurea (217)

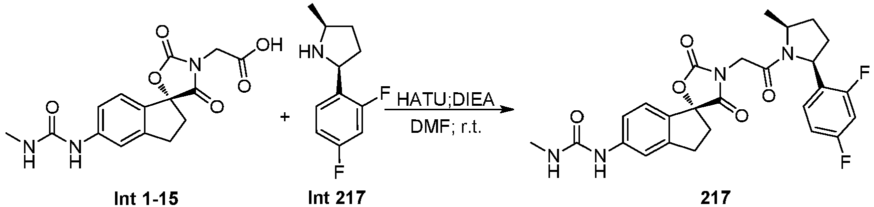

Intermediate **Int 1-15** (33 mg, 0.1 mmol) was dissolved in DMF (1 mL), to which were added DIEA (39 mg, 0.3 mmol) and HATU (38 mg, 0.1 mmol), followed by addition of **Int 217** (20 mg, 0.1 mmol), and the mixture was stirred at room temperature for 16 h. To the reaction solution was added water (10 mL), and extracted with ethyl acetate two times. The organic phase was washed with saturated brine, and concentrated to obtain the crude product, that was purified by column chromatography to obtain product **217** (17 mg) as off-white solid, with a yield of 55%. MS: *m*/*z* 513 [M+H]⁺. ¹H NMR (400 MHz, CDCl3) δ7.59-7.40 (m, 1H), 7.33 (m, 1H), 7.22-6.68 (m, 4H), 5.29-5.00 (m, 2H), 4.43-4.26 (m, 1H), 4.21-3.81 (m, 1H), 3.19-2.92 (m, 2H), 2.81-2.60 (m, 4H), 2.50 (m, 2H), 2.29-1.75 (m, 3H), 1.40 (m, 3H).

### Example 221 Synthesis of compound (R)-5-(1,4-dimethyl-1H-1,2,3-triazole -5-yl)-3'-(2-((2S,5S)-2-(4-fluorophenyl)-5-methylpyrrolidin-1-yl)-2-oxoethyl)-2,3-dihydrospiro[indene-1,5'-oxazolidine]-2',4'-dione (221)

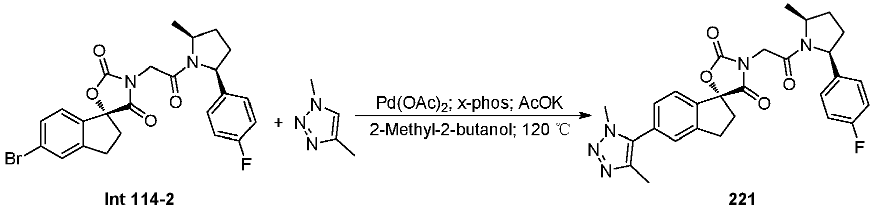

**Int 114-2** (50 mg, 0.1 mmol), 1,4-dimethyl-1H-1,2,3-triazole (11 mg, 0.11 mmol), palladium acetate (6 mg, 0.02 mmol), x-phos (8 mg, 0.025 mmol), and potassium acetate (25 mg, 0.2 mmol) were dissolved in t-pentyl alcohol (5 mL), and the system was purged with nitrogen, heated to 120 °C, stirred for 4 h, and concentrated. Water (5 mL) was added to the residue. The solution was extracted with ethyl acetate, dried over anhydrous Na₂SO₄, concentrated, and purified by column chromatography to give **221** as pale yellow solid (33 mg), with a yield of 64%. MS: *m*/*z* 518 [M+H]⁺. ¹H NMR (400 MHz, DMSO): δ 7.70-7.48 (m, 3H), 7.45-7.10 (m, 4H), 5.11 (dt, J = 16.3, 7.6 Hz, 1H), 4.60 (dd, J = 148.1, 16.6 Hz, 2H), 4.24 (dd, J = 11.9, 6.2 Hz, 1H), 3.93 (d, J = 7.7 Hz, 3H), 3.22 (m, 1H), 3.13 (m, 1H), 2.72-2.60 (m, 1H), 2.40 (m, 1H), 2.22 (d, J = 6.8 Hz, 3H), 2.10-1.96 (m, 1H), 1.90 (m, 1H), 1.74 (m, 1H), 1.54 (m, 1H), 1.37 (d, J = 6.4 Hz, 3H).

### Example 225 Synthesis of compound 1-((R)-2',4'-dioxo-3'-(2-oxo-2-((S)-2-phenylpyrrolidin-1-yl)ethyl)-2,3-dihydrospiro[indene-1, 5'-oxazolidine]-5-yl)-3-methylurea (225)

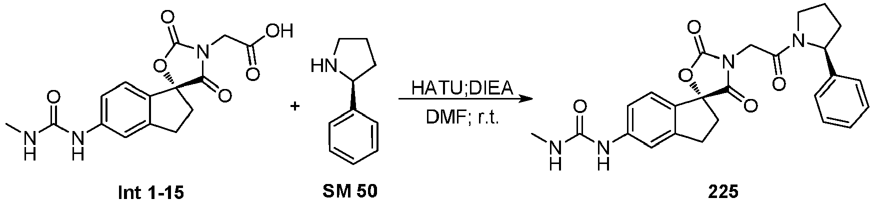

Intermediate **Int 1-15** (33 mg, 0.1 mmol) was dissolved in DMF (1 mL), to which were added DIEA (39 mg, 0.3 mmol) and HATU (38 mg, 0.1 mmol), followed by addition of **SM 50** (20 mg, 0.1 mmol), and the mixture was stirred at room temperature for 16 h. To the reaction solution was added water (10 mL), and extracted with ethyl acetate two times. The organic phase was washed with saturated brine, and concentrated to obtain the crude product, that was purified by column chromatography to obtain product **225** (23 mg) as off-white solid, with a yield of 50%. MS: *m*/*z* 463 [M+H]⁺. ¹H NMR (400 MHz, DMSO) δ 8.73 (d, J = 5.4 Hz, 1H), 7.54 (d, J = 5.0 Hz, 1H), 7.40 (t, J = 7.4 Hz, 1H), 7.30 (dt, J = 8.4, 7.1 Hz, 3H), 7.26-7.13 (m, 3H), 6.13-6.04 (m, 1H), 5.21 (dd, J = 64.8, 7.2 Hz, 1H), 4.62-3.88 (m, 2H), 3.71 (q, J = 9.2 Hz, 1H), 3.66-3.50 (m, 1H), 3.09 (td, J = 16.8, 8.1 Hz, 1H), 2.96 (tdd, J = 15.0, 10.0, 5.3 Hz, 1H), 2.69-2.55 (m, 4H), 2.41 (ddd, J = 14.3, 8.0, 3.4 Hz, 1H), 2.25 (ddd, J = 18.9, 9.4, 5.8 Hz, 1H), 1.97-1.68 (m, 3H).

### Example 300 Synthesis of compound (R)-1-(2',4'-dioxo-3'-(2-oxo-2-(7- (trifluoromethyl)-3,4-dihydroquinolin-1(2H)-yl)ethyl)-2,3-dihydrospiro[indene-1,5'-oxazolidine]-5-yl)-3-methylurea (300)

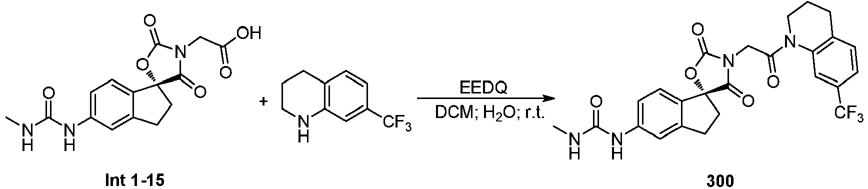

To a 30 mL reaction flask, were added **Int 1-15** (30 mg, 90 µmol), **SM 300** (18 mg, 90 µmol), dichloromethane (1 mL), and water (1 mL), and finally EEDQ (34 mg, 135 µmol) was added. The reaction was stirred overnight at room temperature. To the reaction solution, was added water (5 mL), and extracted with dichloromethane (5 mL×3). The organic layers were combined, dried over anhydrous Na₂SO₄, and concentrated to obtain the crude product, that was purified by column chromatography to provide the product **300** (20 mg), with a yield of 43%. MS: *m*/*z* 517.2 [M+H]⁺.

### Example 302 Synthesis of compound (R)-1-methyl-3-(3'-(2-(6-(1-methyl-1H- pyrazol-4-yl)-3,4-dihydroquinolin-1(2H)-yl)-2-oxoethyl)-2',4'-dioxo-2,3-dihydrospiro[indene-1,5'-oxazolidine]-5-yl)urea (302)

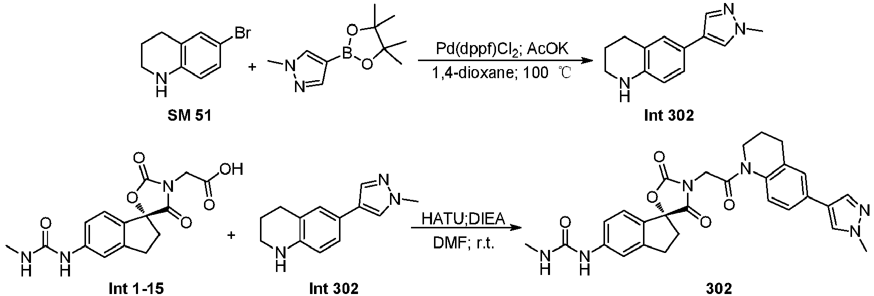

### Synthesis of intermediate 6-(1-methyl-1H-pyrazol-4-yl)-1,2,3,4-tetrahydroquinoline (Int 302-1):

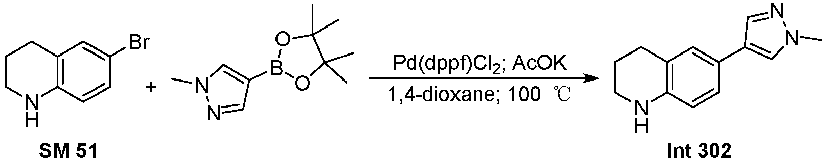

6-Bromo-1,2,3,4-tetrahydroquinoline (212 mg, 1 mmol), 1-methyl-4-(4,4,5,5- tetramethyl-1,3,2-dioxaborolan-2-yl)-1H-pyrazole (208 mg, 1 mmol), potassium acetate (200 mg, 2 mmol), and [1,1'-bis(diphenylphosphino)ferrocene] dichloropalladium(II) (73 mg, 0.1 mmol) were added to dioxane (20 mL), and the system was filled with nitrogen three times, heated to 100 °C, reacted for 4 h, and concentrated. Water was added to the residue, and the solution was extracted with ethyl acetate, dried over anhydrous Na₂SO₄, concentrated, and purified by column chromatography to obtain **Int 302-1** as yellow solid (98 mg), with a yield of 46%. MS: *m*/*z* 214 [M+H]⁺.

### Synthesis of compound (R)-1-methyl-3-(3'-(2-(6-(1-methyl-1H-pyrazol-4-yl)- 3,4-dihydroquinolin-1 (2H)-yl)-2-oxoethyl)-2',4'-dioxo-2,3 -dihydrospirofindene-1,5'-oxazolidine]-5-yl)urea (302)

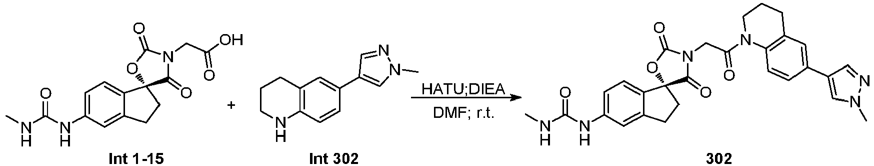

Intermediate **Int 1-15** (33 mg, 0.1 mmol), intermediate **Int 302** (22 mg, 0.11 mmol), HATU (50 mg, 0.13 mmol), and DIEA (20 mg, 0.15 mmol) were dissolved in DMF (5 mL). The reaction was stirred overnight, concentrated, and purified by column chromatography to obtain **302** (35 mg) as pale yellow solid, with a yield of 66%. MS: *m*/*z* 529 [M+H]⁺.

### Example 303 Synthesis of compound 1-((1R)-2',4'-dioxo-3'-(2-oxo-2-(2-phenylpyrrolidin-1-yl)ethyl)-2,3-dihydrospiro[indene-1, 5'-oxazolidine]-5-yl)-3-methylurea (303)

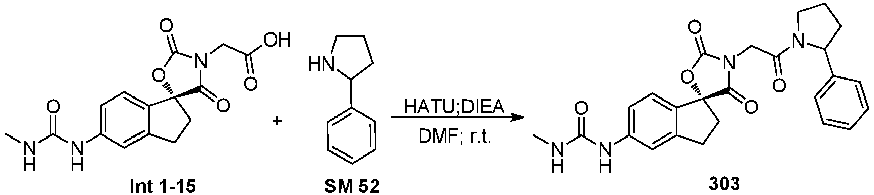

Intermediate **Int 1-15** (33 mg, 0.1 mmol) was dissolved in DMF (1 mL), to which were added DIEA (39 mg, 0.3 mmol) and HATU (38 mg, 0.1 mmol), followed by addition **of SM 52** (20 mg, 0.1 mmol), and the mixture was stirred at room temperature for 16 h. To the reaction solution was added water (10 mL), and extracted with ethyl acetate two times. The organic phase was washed with saturated brine, and concentrated to obtain the crude product, that was purified by column chromatography to obtain product **303** (23 mg) as off-white solid, with a yield of 50%. MS: *m*/*z* 463 [M+H]⁺.

### Example 304 Synthesis of compound (R)-1-(2',4'-dioxo-3'-(2-oxo-2-(5H-pyrrolo [3,4-b]pyridin-6(7H)-yl)ethyl)-2,3-dihydrospiro[indene-1,5'-oxazolidine]-5-yl)-3-methylurea (304)

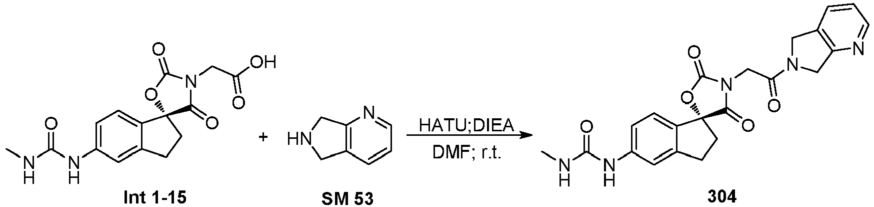

To a 25 mL reaction flask, was added DMF (1 mL), to which were then added **Int 1-15** (33 mg, 0.1 mmol), **SM 53** (12 mg, 0.1 mmol), DIEA (39 mg, 0.3 mmol), followed by addition of HATU (57 mg, 0.15 mmol). The system was reacted at room temperature overnight. After completion of the reation, water (5 mL) was added to the reaction solution, and the reasultant solution was extracted with ethyl acetate (5 mL×3). The organic phases were combined, dried over anhydrous Na₂SO₄, and purified by column chromatography to obtain compound **304** (19 mg), with a yield of 44%. MS: *m*/*z* 436.2 [M+H]⁺. ¹H NMR (400 MHz, DMSO) δ 8.75 (s, 1H), 8.50 (d, J = 4.3 Hz, 1H), 7.83 (d, J = 7.7 Hz, 1H), 7.60-7.54 (m, 1H), 7.42-7.32 (m, 2H), 7.25 (ddd, J = 8.4, 3.6, 1.9 Hz, 1H), 6.10 (q, J = 4.4 Hz, 1H), 5.06 (d, J = 18.9 Hz, 2H), 4.74 (d, J = 26.0 Hz, 2H), 4.67-4.51 (m, 2H), 3.18-3.09 (m, 1H), 3.01 (ddd, J = 16.4, 8.7, 3.3 Hz, 1H), 2.75-2.61 (m, 4H), 2.58-2.52 (m, 1H).

### Example 305 Synthesis of compound (S)-1-(3'-(2-(5,7-dihydro-6H-pyrrolo [3,4-b]pyridin-6-yl)-2-oxoethyl)-2',4'-dioxo-2,3-dihydrospiro[indene-1,5'-oxazolidine]-5-yl)-3-methylurea (305):

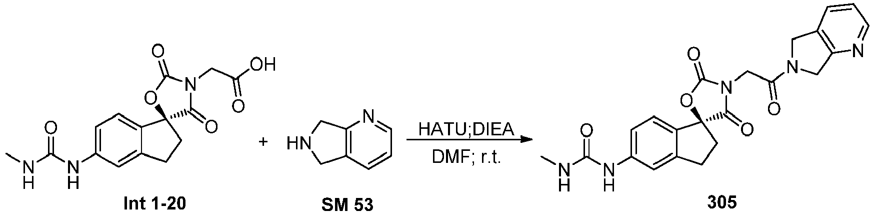

Intermediate **Int 1-15** (33 mg, 0.1 mmol), **SM 53(13** mg, 0.11 mmol), HATU (50 mg, 0.13 mmol), and DIEA (20 mg, 0.15 mmol) were dissolved in dichloromethane (5 mL). The reaction was stirred overnight, concentrated, and purified by column chromatography to obtain **305** (33 mg) as pale yellow solid, with a yield of 76%. MS: *m*/*z* 436 [M+H]⁺.

### Example 306 Synthesis of compound 1-((R)-2',4'-dioxo-3'-(2-oxo-2-((R)-2-phenylpyrrolidin-1-yl)ethyl)-2,3-dihydrospiro[indene-1,5'-oxazolidine]-5-yl)-3-methylurea (306)

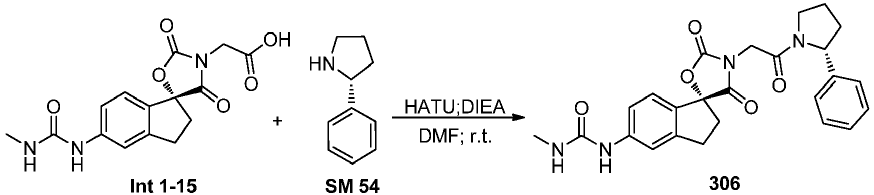

To a 25 mL reaction flask, was added DMF (1 mL), to which were then added **Int 1-15** (33 mg, 0.1 mmol), **SM 54** (15 mg, 0.1 mmol), DIEA (39 mg, 0.3 mmol), followed by addition of HATU (57 mg, 0.15 mmol). The system was reacted at room temperature overnight. After completion of the reation, water (5 mL) was added to the reaction solution, and the reasultant solution was extracted with ethyl acetate (5 mL×3). The organic phases were combined, dried over anhydrous Na₂SO₄, and purified by column chromatography to obtain compound **306** (17 mg), with a yield of 37%. MS: *m*/*z* 463.2 [M+H]⁺. ¹H NMR (400 MHz, DMSO) δ 8.70 (d, J = 8.4 Hz, 1H), 7.54 (d, J = 5.4 Hz, 1H), 7.41 (t, J = 7.5 Hz, 1H), 7.30 (dt, J = 10.4, 4.4 Hz, 3H), 7.23-7.10 (m, 3H), 6.10-6.03 (m, 1H), 5.20 (dd, J = 65.9, 7.6 Hz, 1H), 4.66-3.88 (m, 2H), 3.75-3.67 (m, 1H), 3.65-3.48 (m, 1H), 3.09 (dt, J = 13.8, 7.3 Hz, 1H), 2.96 (ddd, J = 16.1, 11.2, 4.7 Hz, 1H), 2.65-2.54 (m, 4H), 2.48-2.41 (m, 1H), 2.25 (ddd, J = 14.9, 11.8, 7.8 Hz, 1H), 1.98-1.71 (m, 3H).

### Reference Example 308 Synthesis of compound 2-(5-(3,5-dimethylisoxazol-4-yl)-2',4'-dioxo- 2,3-dihydrospiro[cyclopropane-indene-1,5'-oxazole]-3'-yl)-N-(4-fluorobenzyl)-N-((S)-1,1,1-trifluoropropan-2-yl)acetamide (308)

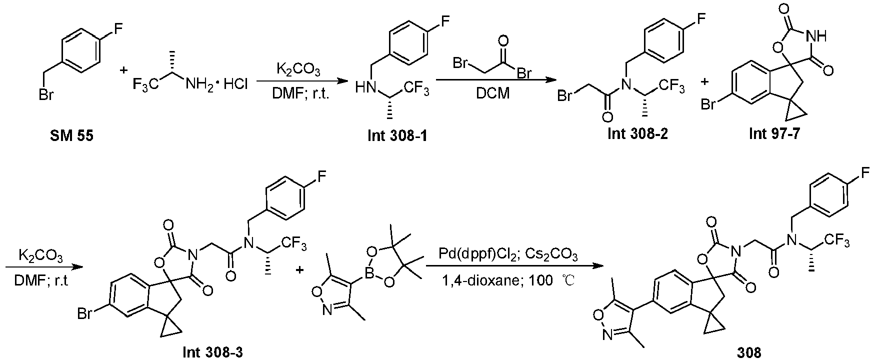

### Synthesis of intermediate (S)-1,1,1-trifluoro-N-(4-fluorobenzyl)propan-2-amine (Int 308-1)

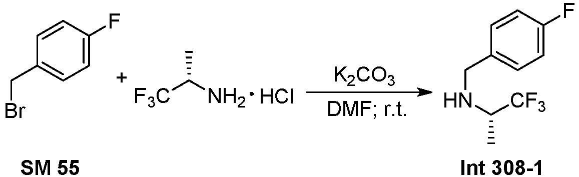

**SM 55** (1.88 g, 10 mmol) was dissolved in DMF (15 mL), to which were added potassium carbonate (2.76 g, 20 mmol) and (S)-1,1,1-trifluoroisopropylamine hydrochloride (1.5 g, 10 mmol), and the reaction was stirred at room temperature for 6 h. The reaction solution was poured into water (60 mL), and extracted with ethyl acetate three times. The organic phase was washed with saturated brine, and concentrated to obtain a crude product. The crude product was subjected to silica gel column chromatography to obtain intermediate **Int 308-1** (1.8 g, 8 mmol), with a yield of 80%. MS: *m*/*z* 222 [M+H]⁺.

### Synthesis of intermediate (S)-2-bromo-N-( 4-fluorobenzyl)-N-(1,1,1-trifluoropropan- 2-yl)acetamide (Int 308-2)

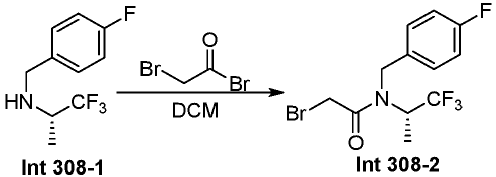

Intermediate **Int 308-1** (1.8 g, 8 mmol) was dissolved in DCM (15 mL) and cooled in an ice-water bath, to which was added bromoacetyl bromide (1.6 g, 8 mmol), then the mixture was stirred for 3 h. The reaction solution was poured into water (60 mL), and extracted with DCM three times. The organic phase was washed with saturated brine, and concentrated to obtain a crude product. The crude product was subjected to silica gel column chromatography to obtain intermediate **Int 308-2** (2.19 g, 6.4 mmol), with a yield of 80%. MS: *m*/*z* 342, 344 [M+H]⁺.

### Synthesis of intermediate 2-(5-bromo-2',4'-dioxo-2,3-dihydrospiro[cyclopropane- indene-1,5'-oxazole ]-3'-yl)-N-( 4-fluorobenzyl)-N-((S)-1,1,1-trifluoropropan-2-yl)acetamide ( Int 308-3)

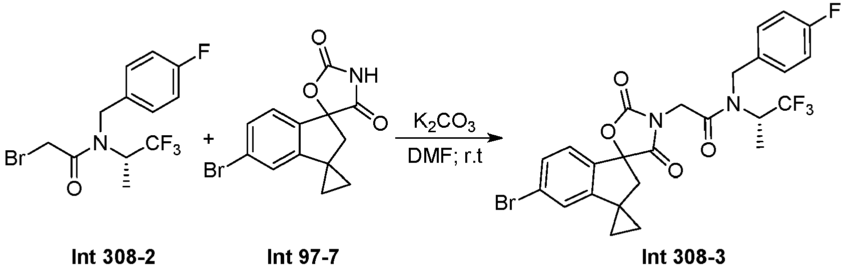

Intermediate **Int 308-2** (2.19 g, 6.4 mmol) and intermediate **Int 97-7** (1.97 g, 6.4 mmol) were dissolved in DMF (15 mL), to which was added potassium carbonate (1.38 g, 10 mmol), and the reaction was stirred at room temperature for 6 h. The reaction solution was poured into water (60 mL), and extracted with ethyl acetate three times. The organic phase was washed with saturated brine, and concentrated to obtain a crude product. The crude product was subjected to silica gel column chromatography to obtain intermediate **Int 308-3** (3.27 g, 5.76 mmol), with a yield of 90%. MS: *m*/*z* 569, 571 [M+H]⁺.

### Synthesis of compound 2-(5-(3,5-dimethylisoxazol-4-yl)-2',4'-dioxo-2,3-dihydrospiro [cyclopropane-indene-1,5'-oxazole]-3'-yl)-N-( 4-fluorobenzyl)-N-((S)-1,1,1-trifluoropropan-2-yl)acetamide (308)

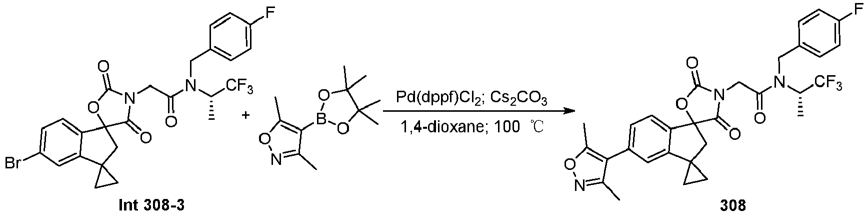

Intermediate **Int 308-3** (569 mg, 1 mmol) and 3,5-dimethylisoxazol-4-boric acid pinacol ester (223 mg, 1 mmol) were dissolved in dioxane (10 mL), to which were added cesium carbonate (652 mg, 2 mmol) and Pd(dppf)Cl₂ (73 mg, 0.1 mmol). The system was filled with N₂ three times. The reaction was heated to 100 °C and stirred for 6 h. Then, the reaction solution was poured to water (60 mL), and extracted with ethyl acetate three times. The organic phase was washed with saturated brine, and concentrated to obtain a crude product, that was purified by column chromatography to obtain compound **308** (351 mg, 0.6 mmol), with a yield of 60%. MS: *m*/*z* 586 [M+H]⁺. ¹H NMR (400 MHz, CDCl₃): δ 7.66-7.51 (m, 1H), 7.31 (m, 2H), 7.21-7.08 (m, 3H), 6.62 (s, 1H), 5.52 (m, 1H), 4.70 (m, 2H), 4.47 (m, 1H), 4.25 (m, 1H), 2.99 (m, 1H), 2.56-2.47 (m, 1H), 2.39 (m, 3H), 2.27-2.18 (m, 3H), 1.38-1.28 (m, 3H), 1.22-1.10 (m, 3H), 1.10-1.03 (m, 1H).

### Example 309 Synthesis of compound 1-((S)-2',4'-dioxo-3'-(2-oxo-2-((R)-2-phenylpyrrolidin-1-yl)ethyl)-2,3-dihydrospiro[indene-1,5'-oxazolidine]-5-yl)-3-methylurea (309)

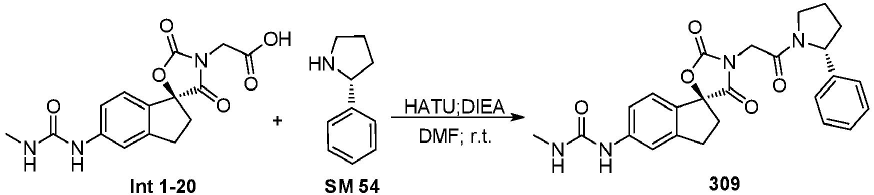

Intermediate **Int 1-15** (33 mg, 0.1 mmol) was dissolved in DMF (1 mL), to which were added DIEA (39 mg, 0.3 mmol) and HATU (38 mg, 0.1 mmol), followed by addition **of SM 54** (20 mg, 0.1 mmol), and the mixture was stirred at room temperature for 2 h. To the reaction solution was added water (10 mL), and extracted with ethyl acetate two times. The organic phase was washed with saturated brine, and concentrated to obtain the crude product, that was purified by column chromatography to obtain product **309** (23 mg) as off-white solid, with a yield of 50%. MS: *m*/*z* 463 [M+H]⁺. ¹H NMR (400 MHz, DMSO): δ 8.71 (m, 1H), 7.54 (m, 1H), 7.45-7.09 (m, 7H), 6.15-6.00 (m, 1H), 5.29-5.13 (m, 1H), 4.53 (m, 1H), 3.93 (m, 1H), 3.71 (m, 1H), 3.65-3.51 (m, 1H), 3.09 (m, 1H), 3.02-2.90 (m, 1H), 2.73 (s, 1H), 2.68-2.55 (m, 3H), 2.48-2.34 (m, 1H), 2.25 (m, 1H), 1.98-1.67 (m, 3H).

### Example 310 Synthesis of compound 1-((S)-2',4'-dioxo-3'-(2-oxo-2-((S)-2-phenylpyrrolidin-1-yl)ethyl)-2,3-dihydrospiro[indene-1, 5'-oxazolidine]-5-yl)-3-methylurea (310)

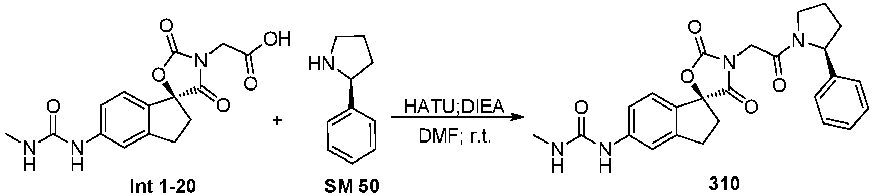

Intermediate **Int 1-15** (33 mg, 0.1 mmol) was dissolved in DMF (1 mL), to which were added DIEA (39 mg, 0.3 mmol) and HATU (38 mg, 0.1 mmol), followed by addition **of SM 50** (20 mg, 0.1 mmol), and the mixture was stirred at room temperature for 2 h. To the reaction solution was added water (10 mL), and extracted with ethyl acetate two times. The organic phase was washed with saturated brine, and concentrated to obtain the crude product, that was purified by column chromatography to obtain product **310** (23 mg) as off-white solid, with a yield of 50%. MS: *m*/*z* 463 [M+H]⁺. ¹H NMR (400 MHz, CDCl₃): *δ* 7.37 (m, 3H), 7.28-6.84 (m, 5H), 5.12 (m, 1H), 4.49 (s, 1H), 4.29 (m, 1H), 3.83 (m, 2H), 2.99 (m, 2H), 2.71 (s, 3H), 2.41 (m, 2H), 2.07-1.82 (m, 4H).

### Reference Example 311 Synthesis of compound (S)-1-(3'-(2-(3,4-dihydroisoquinolin-2(1H)-yl)-2-oxoethyl)-2',4'-dioxo-2,3-dihydrospiro[indene-1,5'-oxazolidine]-5-yl)-3-methylurea (311)

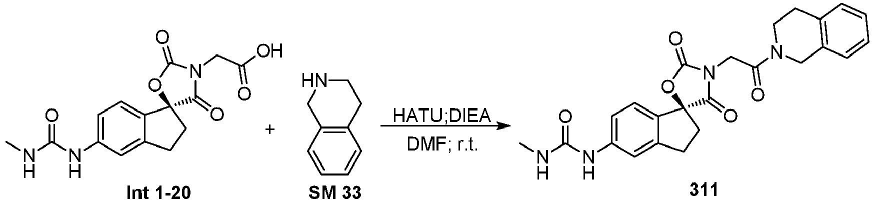

Intermediate **Int 1-15** (33 mg, 0.1 mmol) was dissolved in DMF (1 mL), to which were added DIEA (39 mg, 0.3 mmol) and HATU (38 mg, 0.1 mmol), followed by addition **of SM 33** (15 mg, 0.1 mmol), and the mixture was stirred at room temperature for 2 h. To the reaction solution was added water (10 mL), and extracted with ethyl acetate two times. The organic phase was washed with saturated brine, and concentrated to obtain the crude product, that was purified by column chromatography to obtain product 311 (22 mg) as off-white solid, with a yield of 50%. MS: *m*/*z* 449 [M+H]⁺. ¹H NMR (400 MHz, CDCl₃): δ 7.50 (m, 1H), 7.36 (s, 1H), 7.25-7.09 (m, 4H), 6.94 (s, 1H), 4.70 (m, 2H), 4.51 (s, 2H), 3.78 (m, 2H), 3.22-2.45 (m, 9H).

### Example 314 Sythesis of compound (R)-1-(2',4'-dioxo-3'-(2-oxo-2-(3- phenylpyrrolidin-1-yl)ethyl)-2,3-dihydrospiro[indene-1,5' -oxazolidine]-5 -yl)-3 -methylurea (314)

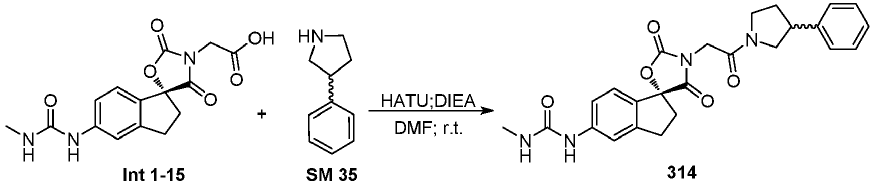

To a 25 mL reaction flask, was added DMF (1 mL), to which were then added **Int 1-15** (33 mg, 0.1 mmol), **SM 35** (15 mg, 0.1 mmol), DIEA (39 mg, 0.3 mmol), followed by addition of HATU (57 mg, 0.15 mmol). The system was reacted 1 h at 20 °C. After completion of the reation, water (5 mL) was added to the reaction solution, and the reasultant solution was extracted with ethyl acetate (5 mL×3). The organic phases were combined, dried over anhydrous Na₂SO₄, and purified by column chromatography to obtain compound **314** (25 mg), with a yield of 55%. MS: *m*/*z* 463 [M+H]⁺. ¹H NMR (400 MHz, DMSO) δ 8.73 (s, 1H), 7.57 (s, 1H), 7.40-7.31 (m, 5H), 7.26 (ddd, J = 17.1, 9.6, 5.2 Hz, 2H), 6.09 (d, J = 4.5 Hz, 1H), 4.45 (dtd, J = 45.6, 17.1, 3.6 Hz, 2H), 3.88 (dt, J = 35.4, 8.0 Hz, 1H), 3.65 (dt, J = 16.6, 10.5 Hz, 1H), 3.51 (t, J = 9.1 Hz, 1H), 3.44-3.37 (m, 1H), 3.32-3.22 (m, 1H), 3.13 (dt, J = 15.6, 7.6 Hz, 1H), 3.06-2.94 (m, 1H), 2.70-2.59 (m, 4H), 2.56-2.51 (m, 1H), 2.40-2.20 (m, 1H), 2.13-1.89 (m, 1H).

### Example 315 Synthesis of compound 1-((S)-3'-(2-((1R,4R)-2-oxa-5-azabicyclo [2.2.1]heptan-5-yl)-2-oxoethyl)-2',4'-dioxo-2,3-dihydrospiro[indene-1,5'-oxazolidine]-5-yl)-3-methylurea (315)

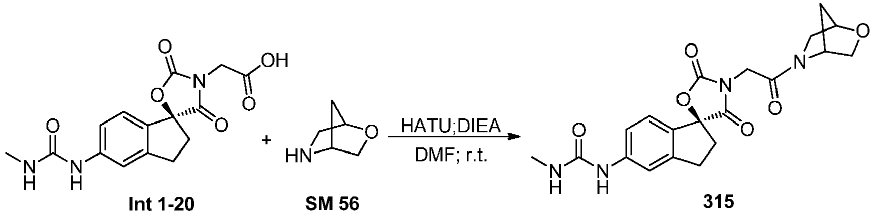

Intermediate **Int 1-15** (33 mg, 0.1 mmol) was dissolved in DMF (1 mL), to which were added DIEA (39 mg, 0.3 mmol) and HATU (38 mg, 0.1 mmol), followed by addition of starting material (1R,4R)-2-oxa- 5-azabicyclo[2.2.1]heptane (16 mg, 0.1 mmol), and the mixture was stirred at room temperature for 2 h. To the reaction solution was added water (10 mL), and extracted with ethyl acetate two times. The organic phase was washed with saturated brine, and concentrated to obtain the crude product, that was purified by column chromatography to obtain product **315** (26 mg) as off-white solid, with a yield of 50%. MS: *m*/*z* 415 [M+H]⁺. ¹H NMR (400 MHz, CDCl₃): δ 7.58-7.25 (m, 2H), 7.10-6.87 (m, 1H), 5.00-4.25 (m, 3H), 4.00-3.75 (m, 2H), 3.60-3.40 (m, 2H), 3.20-2.90 (m, 2H), 2.75 (s, 3H), 2.55-2.45 (m, 1H), 2.00-1.80 (m, 2H), 1.45-1.20 (m, 2H).

### Reference Example 317 Synthesis of compound (S)-1-(2',4'-dioxo-3'-((5-phenyl-1,3,4-oxadiazole-2-yl)methyl)-2,3-dihydrospiro[indene-1,5'-oxazolidine]-5-yl)-3-methylurea (317)

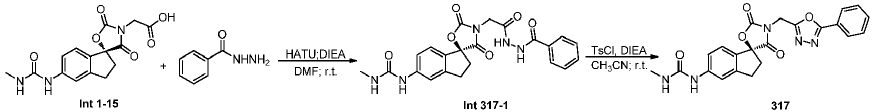

### Synthesis of intermediate (S)-1-(3'-(2-(2-benzoylhydrazino)-2-oxoethyl)-2',4'-dioxo- 2,3-dihydrospiro[indene-1,5'-oxazolidine]-5-yl)-3-methylurea (Int 317-1)

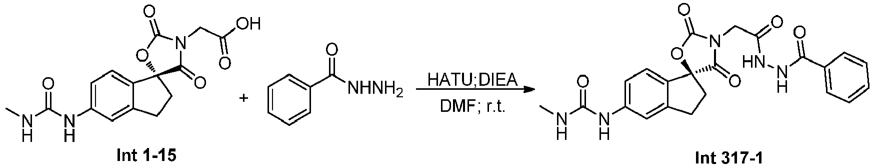

Intermediate **Int 1-15** (66 mg, 0.2 mmol), benzoylhydrazine (27 mg, 0.2 mmol), HATU (100 mg, 0.26 mmol), and DIEA(40 mg, 0.3 mmol) were dissolved in DMF(10 mL), and the mixture was stirred overnight, to which was added water. The solution was extracted with EA, concentrated, and purified by column chromatography, to obtain **Int 317-1** (41 mg) as pale yellow solid, with a yield of 46%. MS: *m*/*z* 452 [M+H]⁺.

### Synthesis of compound (S)-1-(2',4'-dioxo-3'-((5-phenyl-1,3,4-oxadiazol-2-yl)methyl)- 2,3-dihydrospiro[indene-1,5'-oxazolidine]-5-yl)-3-methylurea (317)

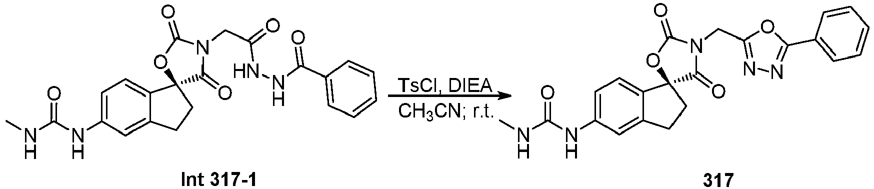

Intermediate **Int 317-1** (41 mg, 0.09 mmol), p-toluenesulfonyl chloride (30 mg, 0.16 mmol), and DIEA (27 mg, 0.2 mmol) were dissolved in acetonitrile (5 mL), and the mixture was stirred at room temperature for 2 h. The solution was concentrated, and the residue was purified by column chromatography to obtain 317 (21 mg) as yellow solid, with a yield of 54%. MS: *m*/*z* 434 [M+H]⁺. ¹H NMR (400 MHz, DMSO) δ 8.76 (s, 1H), 8.07-7.96 (m, 2H), 7.69-7.60 (m, 3H), 7.54 (s, 1H), 7.30-7.21 (m, 2H), 6.10 (d, J = 4.5 Hz, 1H), 5.14 (d, J = 3.0 Hz, 2H), 3.19-3.07 (m, 1H), 3.06-2.97 (m, 1H), 2.75-2.61 (m, 4H), 2.60-2.53 (m, 1H).

### Example 318 Synthesis of compound 1-((1R)-2',4'-dioxo-3'-(2-oxo-2-(2-m-methylphenyl)-pyrrolidin-1-yl)ethyl)-2,3-dihydrospiro[indene-1,5'-oxazolidine]-5-yl)-3-methylurea (318)

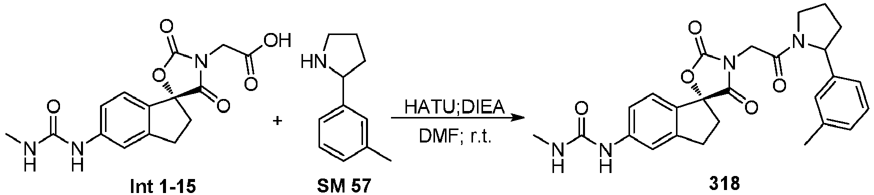

To a 25 mL reaction flask, was added DMF (1 mL), to which were then added **Int 1-15** (33 mg, 0.1 mmol), **SM 304** (16 mg, 0.1 mmol), DIEA (39 mg, 0.3 mmol), followed by addition of HATU (57 mg, 0.15 mmol). The system was reacted 1 h at 20 °C. After completion of the reation, water (5 mL) was added to the reaction solution, and the reasultant solution was extracted with ethyl acetate (5 mL×3). The organic phases were combined, dried over anhydrous Na₂SO₄, and purified by column chromatography to obtain compound **318** (20 mg), with a yield of 42%. MS: *m*/*z* 477 [M+H]⁺. ¹H NMR (400 MHz, DMSO) δ 8.74-8.67 (m, 1H), 7.54 (d, J = 5.3 Hz, 1H), 7.33-6.93 (m, 6H), 6.13-6.02 (m, 1H), 5.15 (dd, J = 64.2, 7.6 Hz, 1H), 4.66-3.87 (m, 2H), 3.76-3.65 (m, 1H), 3.64-3.52 (m, 1H), 3.17-3.03 (m, 1H), 3.01-2.92 (m, 1H), 2.67-2.54 (m, 4H), 2.45-2.36 (m, 1H), 2.34-2.16 (m, 4H), 2.00-1.66 (m, 3H).

### Example 319 Synthesis of compound 1-((1R)-3'-(2-(2-(2-methoxyphenyl) pyrrolidin-1-yl)-2-oxoethyl)-2',4'-dioxo-2,3-dihydrospiro[indene-1,5'-oxazolidine]-5-yl)-3-methylurea (319)

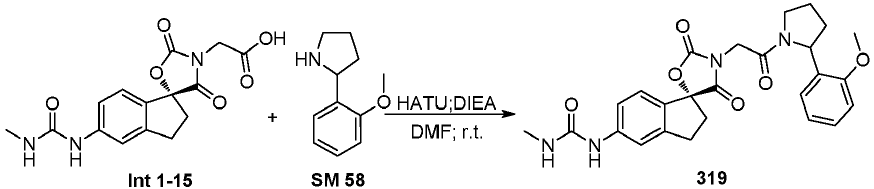

To a 25 mL reaction flask, was added DMF (1 mL), to which were then added **Int 1-15** (33 mg, 0.1 mmol), **SM304** (18 mg, 0.1 mmol), DIEA (39 mg, 0.3 mmol), followed by addition of HATU (57 mg, 0.15 mmol). The system was reacted 1h at 20 °C. After completion of the reation, water (5 mL) was added to the reaction solution, and the reasultant solution was extracted with ethyl acetate (5 mL×3). The organic phases were combined, dried over anhydrous Na₂SO₄, and purified by column chromatography to obtain compound **319** (19 mg), with a yield of 39%. MS: *m*/*z* 493 [M+H]⁺. ¹H NMR (400 MHz, DMSO) δ 8.75-8.67 (m, 1H), 7.54 (d, J = 6.0 Hz, 1H), 7.33-7.09 (m, 5H), 7.06-6.98 (m, 1H), 6.14-6.04 (m, 1H), 5.32 (dd, J = 82.8, 7.9 Hz, 1H), 4.69-3.95 (m, 2H), 3.82-3.68 (m, 1H), 3.65-3.46 (m, 1H), 3.09 (dd, J = 15.8, 7.7 Hz, 1H), 2.98 (dd, J = 12.1, 8.9 Hz, 1H), 2.65-2.52 (m, 4H), 2.45-2.35 (m, 3H), 2.29 (d, J = 21.6 Hz, 2H), 2.04-1.70 (m, 3H).

### Example 322 Synthesis of compound 1-((1R)-2',4'-dioxo-3'-(2-oxo-2-(2- phenylpiperidin-1-yl)ethyl)-2,3-dihydrospiro[indene-1,5'-oxazolidine]-5-yl)-3-methylurea (322)

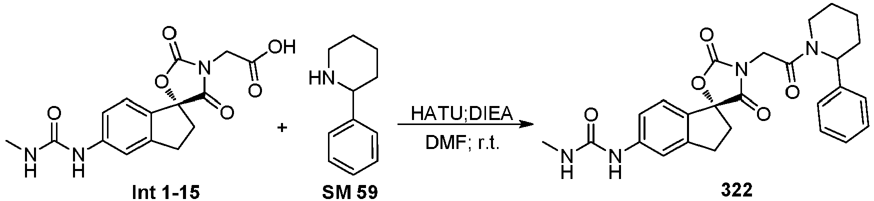

To a 25 mL reaction flask, was added DMF (1 mL), to which were then added **Int 1-15** (33 mg, 0.1 mmol), **SM304** (16 mg, 0.1 mmol), DIEA (39 mg, 0.3 mmol), followed by addition of HATU (57 mg, 0.15 mmol). The system was reacted 1 h at 20 °C. After completion of the reation, water (5 mL) was added to the reaction solution, and the reasultant solution was extracted with ethyl acetate (5 mL×3). The organic phases were combined, dried over anhydrous Na₂SO₄, and purified by column chromatography to obtain compound **322** (18 mg), with a yield of 38%. MS: *m*/*z* 477 [M+H]⁺. ¹H NMR (400 MHz, DMSO) δ 8.71 (s, 1H), 7.56 (s, 1H), 7.47-7.13 (m, 7H), 6.08 (s, 1H), 5.52 (d, J = 165.7 Hz, 1H), 4.85-4.31 (m, 2H), 4.20-3.79 (m, 1H), 3.13 (dt, J = 15.5, 7.6 Hz, 1H), 3.01 (dd, J = 12.5, 9.0 Hz, 1H), 2.64 (d, J = 2.9 Hz, 4H), 2.41 (d, J = 12.9 Hz, 1H), 1.89 (d, J = 82.8 Hz, 1H), 1.61 (s, 3H), 1.49-1.05 (m, 3H).

### Example 323 Synthesis of compound 1-((1R)-3'-(2-(2-(2-fluorophenyl)pyrrolidin- 1-yl)-2-oxoethyl)-2',4'-dioxo-2,3-dihydrospiro[indene-1,5'-oxazolidine]-5-yl)-3-methylurea (323)

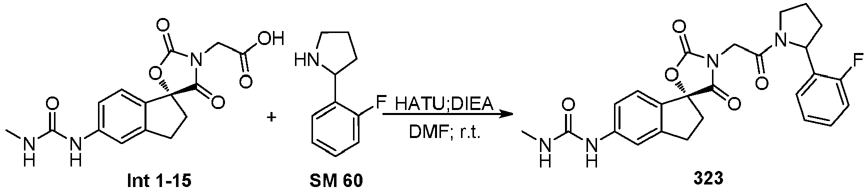

To a 25 mL reaction flask, was added DMF (1 mL), to which were then added **Int 1-15** (33 mg, 0.1 mmol), **SM304** (17 mg, 0.1 mmol), DIEA (39 mg, 0.3 mmol), followed by addition of HATU (57 mg, 0.15 mmol). The system was reacted 1 h at 20 °C. After completion of the reation, water (5 mL) was added to the reaction solution, and the reasultant solution was extracted with ethyl acetate (5 mL×3). The organic phases were combined, dried over anhydrous Na₂SO₄, and purified by column chromatography to obtain compound **323** (20 mg), with a yield of 42%. MS: *m*/*z* 481.2 [M+H]⁺. ¹H NMR (400 MHz, DMSO) δ 8.74-8.65 (m, 1H), 7.55 (d, J = 6.7 Hz, 1H), 7.41-7.08 (m, 6H), 6.12-6.02 (m, 1H), 5.38 (dd, J = 93.9, 7.8 Hz, 1H), 4.66-3.91 (m, 2H), 3.80-3.67 (m, 1H), 3.64-3.52 (m, 1H), 3.10 (dd, J = 15.5, 7.6 Hz, 1H), 3.03-2.93 (m, 1H), 2.66-2.55 (m, 4H), 2.47-2.37 (m, 1H), 2.35-2.23 (m, 1H), 1.99 (dd, J = 9.4, 5.8 Hz, 1H), 1.88 (d, J = 7.2 Hz, 1H), 1.73 (d, J = 7.8 Hz, 1H).

### Example 324 Synthesis of compound 1-((1R)-2',4'-dioxo-3'-(2-oxo-2-(2-o-methylphenyl)pyrrolidin-1-yl)ethyl)-2,3-dihydrospiro[indene-1,5'-oxazolidine]-5-yl)-3-methylurea (324)

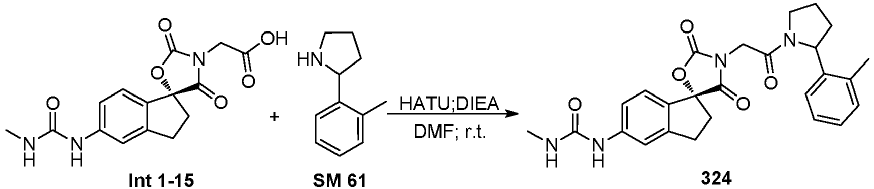

To a 25 mL reaction flask, was added DMF (1 mL), to which were then added **Int 1-15** (33 mg, 0.1 mmol), **SM304** (16 mg, 0.1 mmol), DIEA (39 mg, 0.3 mmol), followed by addition of HATU (57 mg, 0.15 mmol). The system was reacted 1 h at 20 °C. After completion of the reation, water (5 mL) was added to the reaction solution, and the reasultant solution was extracted with ethyl acetate (5 mL×3). The organic phases were combined, dried over anhydrous Na₂SO₄, and purified by column chromatography to obtain compound **324** (22 mg), with a yield of 46%. MS: *m*/*z* 477.2 [M+H]⁺. ¹H NMR (400 MHz, DMSO) δ 8.75-8.61 (m, 1H), 7.54 (d, J = 9.5 Hz, 1H), 7.34-7.25 (m, 1H), 7.26-7.09 (m, 2H), 7.09-6.82 (m, 3H), 6.12-6.02 (m, 1H), 5.41-5.22 (m, 1H), 4.70-3.92 (m, 2H), 3.84 (d, J = 11.5 Hz, 3H), 3.75-3.64 (m, 1H), 3.61-3.49 (m, 1H), 3.09 (td, J = 14.8, 7.0 Hz, 1H), 3.02-2.92 (m, 1H), 2.61 (dt, J = 14.1, 5.5 Hz, 4H), 2.47-2.39 (m, 1H), 2.25 (ddd, J = 24.9, 16.7, 9.3 Hz, 1H), 1.98-1.62 (m, 3H).

### Example 325 Synthsis of compound 1-(2',4'-dioxo-3'-(2-oxo-2-((S)-2- phenylpyrrolidin-1-yl)ethyl)-2,3-dihydrospiro[cyclopropane-indene-1,5'-oxazolidine]-5-yl)-3-methylurea (325)

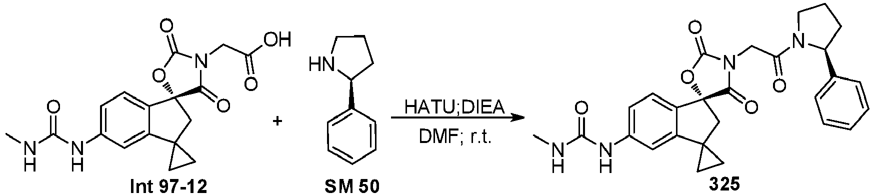

Intermediate **Int 97-12** (35 mg, 0.1 mmol) was dissolved in DMF (1 mL), to which were added DIEA (39 mg, 0.3 mmol) and HATU (38 mg, 0.1 mmol), followed by addition of (S)-2-phenylpyrrolidine (22 mg, 0.15 mmol), and the mixture was stirred at room temperature for 2 h. To the reaction solution was added water (10 mL), and extracted with ethyl acetate two times. The organic phase was washed with saturated brine, and concentrated to obtain the crude product, that was purified by silica gel column chromatography to obtain product **325** (24 mg) as off-white solid, with a yield of 50%. MS: *m*/*z* 489 [M+H]⁺. ¹H NMR (400 MHz, CDCl₃): δ 7.68-6.18 (m, 8H), 5.11 (m, 1H), 4.57-4.19 (m, 1H), 3.79 (m, 2H), 2.75 (m, 4H), 2.54-2.23 (m, 2H), 2.16-1.68 (m, 4H), 1.27 (m, 1H), 1.14-0.91 (m, 3H).

### Example 328 Synthesis of compound 1-(3'-(2-((2S)-2-(2-oxa- 5-azabicyclo[2.2.1] heptan-5-carbonyl)pyrrolidin-1-yl)-2-oxoethyl)-2',4'-dioxo-2,3-dihydrospirofindene-1,5'-oxazolidine]-5-yl)-3-methylurea (328)

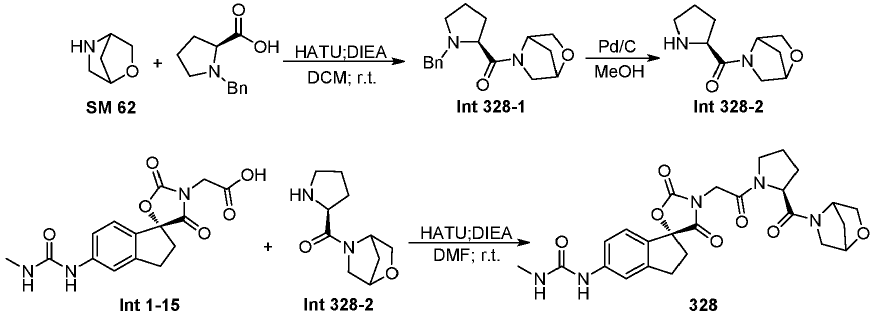

### Synthesis of intermediate 5-(benzyl-L-prolyl)-2-oxa-5-azabicyclo[2.2.1]heptane (Int 328-1)

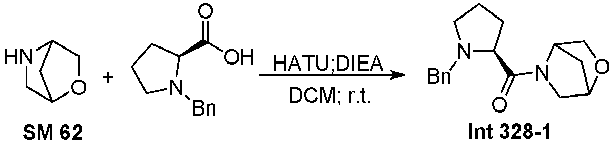

Benzyl-L-proline (205 mg, 1 mmol), 2-oxa-5-azabicyclo[2.2.1]heptane (99 mg, 1 mmol), HATU (570 mg, 1.5 mmol), and DIEA (260 mg, 2 mmol) were dissolved in dichloromethane (50 mL), and the mixture was stirred overnight. The solution was concentrated, and the residue was purified by column chromatography to obtain **Int 328-1** (210 mg) as pale yellow solid, with a yield of 73%. MS: *m*/*z* 287 [M+H]⁺.

### Synthesis of intermediate 5-(-L-prolyl)-2-oxa-5-azabicyclo[2.2.1]heptane (Int 328-2)

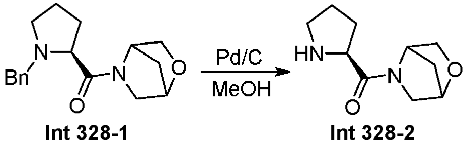

Intermediate **Int 328-1** (30 mg, 0.1 mmol) was dissolved in methanol (10 mL), to which was added Pd/C (2 mg), and the mixture was stirred under hydrogen atmosphere for 2 h. The solution was filtered, and concentrated to obtain colorless liquid **Int 328-2** (28 mg), with a yield of 99%. MS: *m*/*z* 197 [M+H]⁺.

### Synthesis of compound 1-(3'-(2-((2S)-2-(2-oxa-5-azabicyclo[2.2.1]heptan-5-carbonyl) pyrrolidin-1-yl)-2-oxoethyl)-2',4'-dioxo-2,3-dihydrospiro[indene-1, 5'-oxazolidine]-5-yl)-3-methylurea (328)

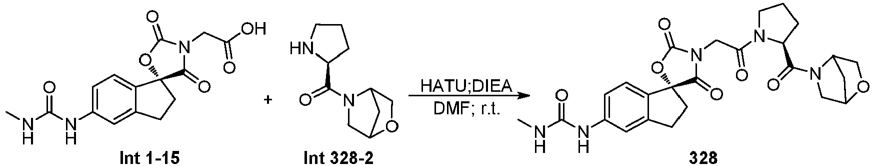

Intermediate **Int 1-15** (33 mg, 0.1 mmol), **Int 328-2** (28 mg, 0.11 mmol), HATU (50 mg, 0.13 mmol), and DIEA (20 mg, 0.15 mmol) were dissolved in dichloromethane (5 mL), and the mixture was stirred overnight. The solution was concentrated, and the residue was subjected to column chromatography, to obtain **328** as pale yellow solid (38 mg), with a yield of 74%. MS: *m*/*z* 512 [M+H]⁺. ¹H NMR (400 MHz, DMSO): δ 8.73 (s, 1H), 7.57-7.48 (m, 1H), 7.35 (t, J = 5.6 Hz, 1H), 7.24 (dd, J = 15.5, 5.3 Hz, 1H), 6.08 (s, 1H), 4.71 (m, 4H), 3.99-3.45 (m, 7H), 3.13-2.94 (m, 2H), 2.65 (m, 5H), 2.03-1.69 (m, 6H).

### Example 330 Synthesis of compound 1-(3'-(2-(2-(1H-imidazol-2-yl)pyrrolidin- 1-yl)-2-oxoethyl)-2',4'-dioxo-2,3-dihydrospiro[indene-1,5'-oxazolidine]-5-yl)-3-methylurea (330)

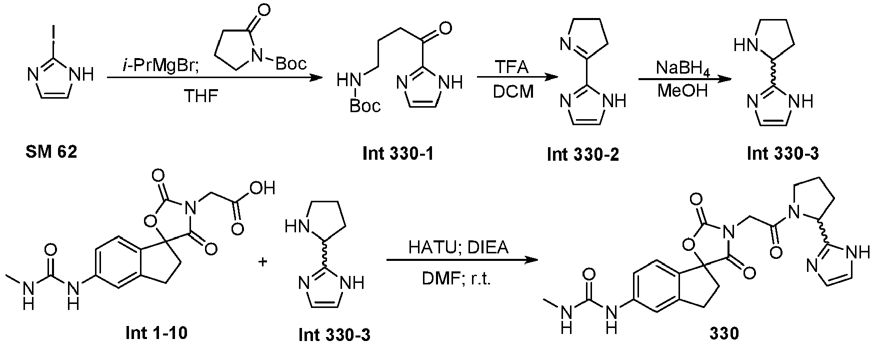

### Synthesis of intermediate (4-(1H-imidazol-2-yl)-4-oxobutyl)carbamic acid t-butyl ester (Int 333-1)

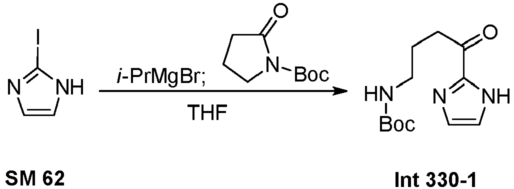

To a reaction flask containing **SM 330** (1.93 g, 10 mmol), was added dry THF (20 mL), and then under N₂ protection, the solution of isopropylmagnesium bromide in tetrahydrofuran (10 mL, 1M in THF) was added in an ice-water bath. After that, the ice-water bath was removed, and the mixture was stirred at room temperature for 7 h, and N-Boc-2-pyrrolidone (1.85 g, 10 mmol) was finally added. The mixture was further allowed to react at room temperature for 12 h. The reaction solution was poured into the saturated aqueous solution of ammonium chloride, and extracted with 30 mL (10 mL×3) of ethyl acetate. The organic phases were combined, dried over anhydrous Na₂SO₄, and purified by column chromatography to obtain **Int 330-1** (1.52 g, 6 mmol), with a yield of 60%. MS: *m*/*z* 254 [M+H]⁺.

### Synthesis of intermediate 2-(3,4-dihydro-2H-pyrrol-5-yl)-1H-imidazole (Int 330-2)

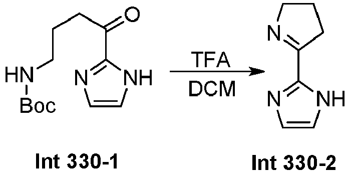

**Int 330-1** (1.52 g, 6 mmol) was dissolved in 15 mL DCM, to which was added 3.7 mL TFA, and the mixture was stirred at room temperature and detected by TLC. After completion of the reaction, the pH of the reaction solution was adjusted to be neutral with saturated NaHCO₃ aqueous solution, and then extracted with DCM (10 mL × 3), followed by drying over anhydrous Na₂SO₄ and rotatory evaporation to dry, to obtain **Int.330-2** (0.72 g; 89%), MS: *m*/*z* 136 [M+H]⁺.

### Synthesis of intermediate 2-(pyrrolidin- 2-yl)-1H-imidazole (Int 330-3)

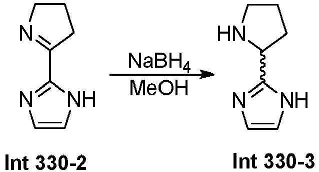

**Int. 330-2** (0.72 g; 5.34 mmol) was introduced into the reaction flask containing 7 mL methanol, to which was added sodium borohydride (0.68 g; 18 mmol) in batches under stirring at room temperature. After that, the reaction was still stirred at room temperature and detected by TLC. After completion of the reaction, the reaction solution was poured to water, and extracted with 30mL (10mL×3) of EA. The organic phase was combined, dried over anhydrous Na₂SO₄, and separated by column chromatography, to obtain **Int.330-3** (0.37 g; 50%), MS: *m*/*z* 138 [M+H]⁺.

### Synthesis of compound 1-(3'-(2-(2-(1H-imidazol-2-yl)pyrrolidin-1-yl)-2-oxoethyl)- 2',4'-dioxo-2,3-dihydrospiro[indene-1,5'-oxazolidine]-5-yl)-3-methylurea (330)

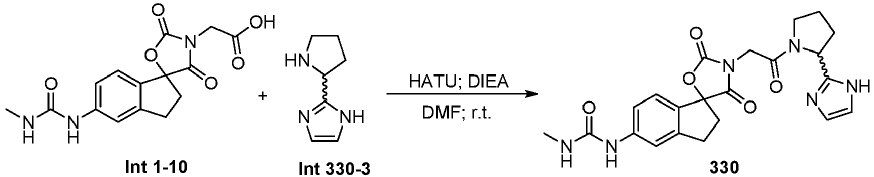

Intermediate **Int 1-10** (30 mg, 0.09 mmol) was dissolved in DMF (1 mL), to which were added DIEA (35 mg, 0.027 mmol) and HATU (41 mg, 0.011 mmol), followed by addition of **Int.330-3** (1.5 eq), and the mixture was stirred at room temperature for 16 h. To the reaction solution was added water (10 mL), and extracted with ethyl acetate two times. The organic phase was washed with saturated brine, and concentrated to obtain the crude product, that was purified by column chromatography to obtain product 330 (25 mg) as off-white solid, with a yield of 61%. MS: *m*/*z* 453 [M+H]⁺.

### Example 331 Synthesis of compound 1-((R)-3'-(2-((S)-2-cyanopyrrolidin- 1-yl)-2-oxoethyl)-2',4'-dioxo-2,3-dihydrospiro[indene-1,5'-oxazolidine]-5-yl)-3-methylurea (331)

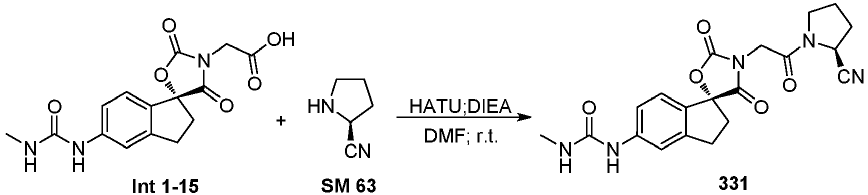

To a 25 mL reaction flask, was added DMF (1 mL), to which were then added **Int 1-15** (33 mg, 0.1 mmol), **SM63** (10 mg, 0.1 mmol), DIEA (39 mg, 0.3 mmol), followed by addition of HATU (57 mg, 0.15 mmol). The system was reacted overnight at room temperature. After completion of the reation, water (5 mL) was added to the reaction solution, and the reasultant solution was extracted with ethyl acetate (5 mL×3). The organic phases were combined, dried over anhydrous Na₂SO₄, and purified by column chromatography to obtain compound **331** (17 mg, yield 41%). MS: *m*/*z* 412.1 [M+H]⁺. ¹H NMR (400 MHz, DMSO) δ 8.74 (s, 1H), 7.56 (s, 1H), 7.31 (d, J = 8.4 Hz, 1H), 7.24 (dd, J = 8.4, 1.7 Hz, 1H), 6.10 (q, J = 4.3 Hz, 1H), 4.87 (dd, J = 7.2, 3.7 Hz, 1H), 4.51 (dd, J = 34.6, 17.1 Hz, 2H), 3.86-3.77 (m, 1H), 3.57 (dd, J = 15.8, 8.7 Hz, 1H), 3.14 (dt, J = 15.7, 7.6 Hz, 1H), 3.00 (ddd, J = 16.2, 8.7, 3.2 Hz, 1H), 2.71-2.62 (m, 4H), 2.59-2.51 (m, 1H), 2.23-2.13 (m, 2H), 2.12-1.99 (m, 2H).

### Example 332 Synthesis of compound 1-((1R)-2',4'-dioxo-3'-(2-oxo-2-(2-(pyridin-2-yl) pyrrolidin-1-yl)ethyl)-2,3-dihydrospirofindene-1,5'-oxazolidine-methylurea (332)

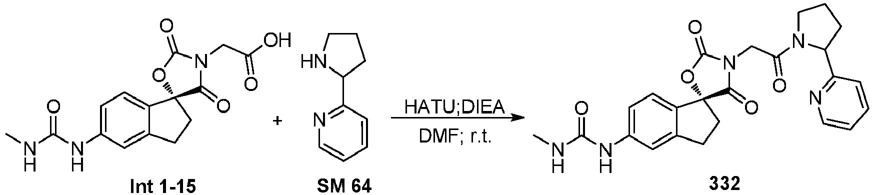

To a 25 mL reaction flask, was added DMF (1 mL), to which were then added **Int 1-15** (33 mg, 0.1 mmol), **SM64** (15 mg, 0.1 mmol), DIEA (39 mg, 0.3 mmol), followed by addition of HATU (57 mg, 0.15 mmol). The system was reacted overnight at room temperature. After completion of the reation, water (5 mL) was added to the reaction solution, and the reasultant solution was extracted with ethyl acetate (5 mL×3). The organic phases were combined, dried over anhydrous Na₂SO₄, and purified by column chromatography to obtain compound **332** (15 mg, yield 32%). MS: *m*/*z* 412.1 [M+H]⁺. ¹H NMR (400 MHz, DMSO) δ 8.75-8.67 (m, 1H), 8.53 (dd, J = 19.4, 15.2 Hz, 1H), 7.89-7.68 (m, 1H), 7.54 (s, 1H), 7.40-7.28 (m, 1H), 7.28-7.10 (m, 3H), 6.10-6.02 (m, 1H), 5.21 (dd, J = 90.2, 8.1 Hz, 1H), 4.62-3.87 (m, 2H), 3.71 (ddd, J = 12.4, 11.5, 5.9 Hz, 1H), 3.63-3.50 (m, 1H), 3.09 (dd, J = 14.3, 6.4 Hz, 1H), 3.05-2.92 (m, 1H), 2.70-2.56 (m, 4H), 2.46-2.36 (m, 1H), 2.34-2.19 (m, 1H), 2.05-1.80 (m, 3H).

### Example 333 Synthesis of compound 1-methyl-3-((R)-3'-(2-((S)-2-(3-methyl-1H- pyrazol-5-yl)pyrrolidin-1-yl)-2-oxoethyl)-2',4'-dioxo-2,3-dihydrospiro[indene-1,5'-oxazolidine]-5-yl)urea (333)

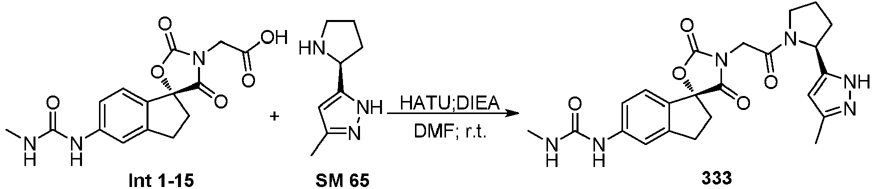

To a 25 mL reaction flask, was added DMF (1 mL), to which were then added **Int 1-15** (33 mg, 0.1 mmol), **SM65** (15 mg, 0.1 mmol), DIEA (39 mg, 0.3 mmol), followed by addition of HATU (57 mg, 0.15 mmol). The system was reacted overnight at room temperature. After completion of the reation, water (5 mL) was added to the reaction solution, and the reasultant solution was extracted with ethyl acetate (5 mL×3). The organic phases were combined, dried over anhydrous Na₂SO₄, and purified by column chromatography to obtain compound **333** (14 mg, yield 30%). MS: *m*/*z* 467.2 [M+H]⁺.

### Example 334 Synthesis of compound 1-((1R)-2',4'-dioxo-3'-(2-oxo-2-(2-(pyridin- 3-yl)pyrrolidin-1-yl)ethyl)-2,3-dihydrospiro[indene-1,5'-oxazolidine]-5-yl)-3-methylurea (334)

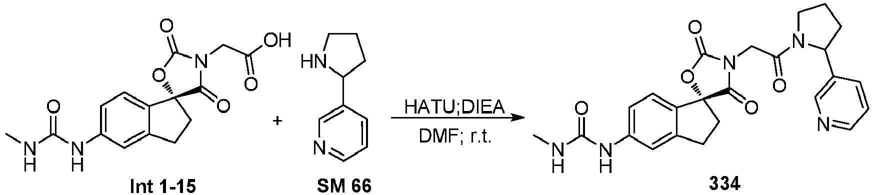

To a 25 mL reaction flask, was added DMF (1 mL), to which were then added **Int 1-15** (33 mg, 0.1 mmol), **SM66** (15 mg, 0.1 mmol), DIEA (39 mg, 0.3 mmol), followed by addition of HATU (57 mg, 0.15 mmol). The system was reacted overnight at room temperature. After completion of the reation, water (5 mL) was added to the reaction solution, and the reasultant solution was extracted with ethyl acetate (5 mL×3). The organic phases were combined, dried over anhydrous Na₂SO₄, and purified by column chromatography to obtain compound **334** (18 mg, yield 39%). MS: *m*/*z* 464.2 [M+H]⁺.

### Example 335 Synthesis of compound 1-((1R)-3'-(2-(2-(3-methoxyphenyl) pyrrolidin-1-yl)-2-oxoethyl)-2',4'-dioxo-2,3-dihydrospiro[indene-1,5'-oxazolidine]-5-yl)-3-methylurea (335)

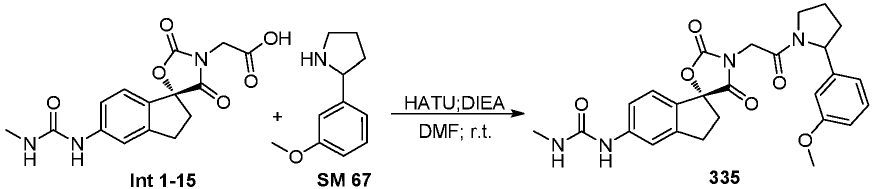

To a 25 mL reaction flask, was added DMF (1 mL), to which were then added **Int 1-15** (33 mg, 0.1 mmol), **SM67** (18 mg, 0.1 mmol), DIEA (39 mg, 0.3 mmol), followed by addition of HATU (57 mg, 0.15 mmol). The system was reacted overnight at room temperature. After completion of the reation, water (5 mL) was added to the reaction solution, and the reasultant solution was extracted with ethyl acetate (5 mL×3). The organic phases were combined, dried over anhydrous Na₂SO₄, and purified by column chromatography to obtain compound **335** (19 mg, yield 39%). MS: *m*/*z* 493.2 [M+H]⁺. ¹H NMR (400 MHz, DMSO) δ 8.76-8.65 (m, 1H), 7.55 (d, J = 6.3 Hz, 1H), 7.32 (dq, J = 10.7, 3.4 Hz, 1H), 7.27-7.11 (m, 2H), 6.90-6.69 (m, 3H), 6.13-6.02 (m, 1H), 5.17 (dd, J = 61.1, 7.1 Hz, 1H), 4.65-3.89 (m, 2H), 3.79-3.66 (m, 4H), 3.64-3.53 (m, 1H), 3.16-3.05 (m, 1H), 3.02-2.91 (m, 1H), 2.68-2.55 (m, 4H), 2.49-2.39 (m, 1H), 2.40-2.17 (m, 1H), 2.01- 1.70 (m, 3H).

### Example 336 Synthesis of compound 1-((1R)-3'-(2-(2-(2-bromophenyl)pyrrolidin- 1-yl)-2-oxoethyl)-2',4'-dioxo-2,3-dihydrospiro[indene-1,5'-oxazolidine]-5-yl)-3-methylurea (336)

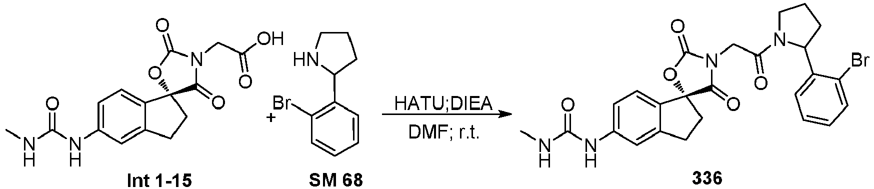

To a 25 mL reaction flask, was added DMF (1 mL), to which were then added **Int 1-15** (33 mg, 0.1 mmol), **SM68** (23 mg, 0.1 mmol), DIEA (39 mg, 0.3 mmol), followed by addition of HATU (57 mg, 0.15 mmol). The system was reacted overnight at room temperature. After completion of the reation, water (5 mL) was added to the reaction solution, and the resultant solution was extracted with ethyl acetate (5 mL×3). The organic phases were combined, dried over anhydrous Na₂SO₄, and purified by column chromatography to obtain compound **336** (16 mg, yield 30%). MS: *m*/*z* 541.2, 543.2 [M+H]⁺. ¹H NMR (400 MHz, DMSO) δ 8.74-8.65 (m, 1H), 7.54 (d, J = 9.9 Hz, 1H), 7.47-7.09 (m, 6H), 6.09 (d, J = 4.6 Hz, 1H), 5.18 (dd, J = 44.2, 8.0 Hz, 1H), 4.70-4.00 (m, 2H), 3.73 (dd, J = 17.6, 8.7 Hz, 1H), 3.64-3.52 (m, 1H), 3.18-3.03 (m, 1H), 2.98 (ddd, J = 13.1, 9.1, 4.4 Hz, 1H), 2.69-2.54 (m, 4H), 2.42 (dd, J = 15.9, 7.2 Hz, 1H), 2.36-2.19 (m, 1H), 2.01-1.67 (m, 3H).

### Example 337 Synthesis of compound 1-((1R)-3'-(2-(2-(4-fluoro-2- methoxyphenyl)-5-methylpyrrolidin-1-yl)-2-oxoethyl)-2',4'-dioxo-2,3-dihydrospiro[indene-1, 5'-oxazolidine]-5-yl)-3-methylurea (337)

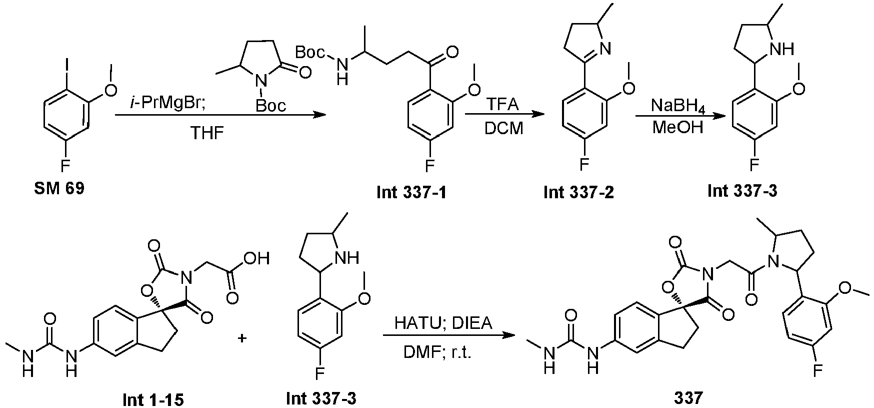

### Synthesis of intermediate (5-(4-fluoro-2-methoxyphenyl)-2-methyl-5-oxobutyl) carbamic acid t-butyl ester (Int 337-1)

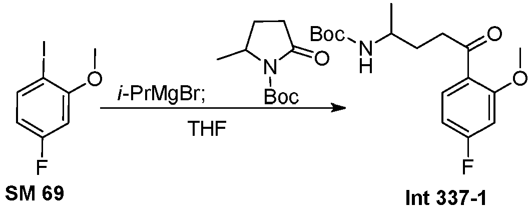

To a reaction flask containing **SM 69** (2.52 g, 10 mmol), was added dry THF (20 mL), and then under N₂ protection, isopropylmagnesium bromide (10 mL, 1M in Hexane) was added to the flask in an ice-water bath. After that, the ice-water bath was removed, and the mixture was reacted at room temperature for 7 h, then 2-methyl-5-oxopyrrolidin-1-carboxylic acid t-butyl ester (1.99 g, 10 mmol) was finally added. The mixture was further allowed to react at room temperature for 12 h. The reaction solution was poured into the saturated aqueous solution of ammonium chloride, and extracted with 30 mL (10 mL×3) of ethyl acetate. The organic phases were combined, dried over anhydrous Na₂SO₄, and purified by column chromatography to obtain **Int 337-1** (2.28 g, 7 mmol), with a yield of 70%. MS: *m*/*z* 326 [M+H]⁺.

### Synthesis of intermediate 5-(4-fluoro-2-methoxyphenyl)-2-methyl-3,4-dihydro-2H- pyrrole (Int 337-2)

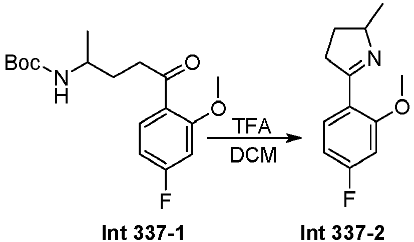

**Int 337-1** (2.28 g, 7 mmol) was dissolved in DCM (22 mL), to which was added TFA (3.7 mL), and the reaction was stirred at room temperature and detected by TLC. After completion of the reaction, the pH of the reaction solution was adjusted to be neutral with saturated NaHCO₃ aqueous solution, and then extracted with DCM (10 mL × 3), followed by drying over anhydrous Na₂SO₄ and rotatory evaporation to dry, to obtain **Int 337-2** (0.87 g; 90%), MS: *m*/*z* 208 [M+H]⁺.

### Synthesis of compound 2-(4-fluoro-2-methoxyphenyl)-5-methylpyrrolidine (Int 337-3)

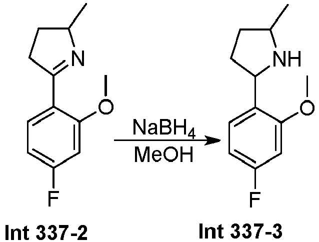

**Int. 337-2** (0.87 g; 4.2 mmol) was introduced into the reaction flask containing 9 mL methanol, to which was added sodium borohydride (0.68 g; 18 mmol) in batches under stirring at room temperature. After that, the reaction was still stirred at room temperature and detected by TLC. After completion of the reaction, the reaction solution was poured to water, and extracted with 30 mL (10 mL×3) of EA. The organic phase was combined, dried over anhydrous Na₂SO₄, and separated by column chromatography, to obtain **Int.337-3** (0.70 g; 80%), MS: *m*/*z* 210 [M+H]⁺.

### Synthesis of compound 1-((1R)-3'-(2-(2-(4-fluoro-2-methoxyphenyl)-5- methylpyrrolidin-1-yl)-2-oxoethyl)-2',4'-dioxo-2,3-dihydrospiro[indene-1,5'-oxazolidine]-5-yl)-3-methylurea (337)

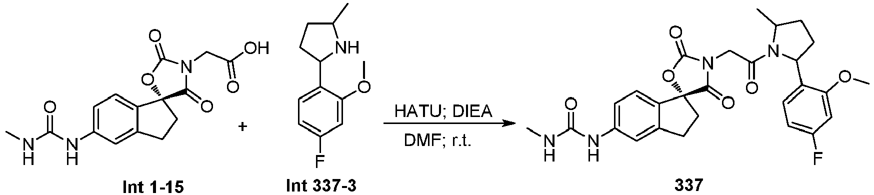

Intermediate **Int 1-15** (30 mg, 0.09 mmol) was dissolved in DMF (1 mL), to which were added DIEA (35 mg, 0.027 mmol) and HATU (41 mg, 0.011 mmol), followed by addition of **Int.337-3** (28.2 mg, 0.135 mmol), and the mixture was stirred at room temperature for 16 h. To the reaction solution was added water (10 mL), and extracted with ethyl acetate two times. The organic phase was washed with saturated brine, and concentrated to obtain the crude product, that was purified by column chromatography to obtain product **337** (36 mg) as off-white solid, with a yield of 76%. MS: *m*/*z* 525 [M+H]⁺.

### Example 338 Synthesis of compound 1-((R)-3'-(2-(2-(2-methoxyphenyl)pyrrolidin- 1-yl)-2-oxoethyl)-2',4'-dioxo-2,3-dihydrospiro[indene-1,5'-oxazolidine]-5-yl)-3-methylurea (338)

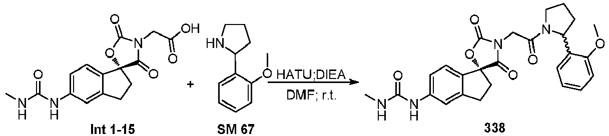

To a 25 mL reaction flask, was added DMF (5 mL), to which were then added **Int 1-15** (333 mg, 1 mmol), **SM 67** (210 mg, 1 mmol), DIEA (390 mg, 3 mmol), followed by addition of HATU (570 mg, 1.5 mmol). The system was reacted overnight at room temperature. After completion of the reation, the reaction solution was poured to water (10 mL), and the reasultant solution was extracted with ethyl acetate (10 mL×3). The organic phases were combined, dried over anhydrous Na₂SO₄, and then obtained crude product was subjected to chiral separation, to provide compound **338** (300 mg). MS: *m*/*z* 493.2 [M+H]⁺. ¹H NMR (400 MHz, DMSO) δ 8.71 (d, J = 7.3 Hz, 1H), 7.55 (d, J = 10.7 Hz, 1H), 7.34-7.26 (m, 1H), 7.24-7.10 (m, 2H), 7.09-6.81 (m, 3H), 6.08 (dd, J = 7.1, 4.6 Hz, 1H), 5.31 (dd, J = 45.5, 7.5 Hz, 1H), 4.69-3.93 (m, 2H), 3.84 (d, J= 12.1 Hz, 3H), 3.75-3.64 (m, 1H), 3.55 (dd, J = 17.9, 11.1 Hz, 1H), 3.09 (dt, J = 12.9, 7.2 Hz, 1H), 2.97 (dd, J = 10.1, 6.5 Hz, 1H), 2.69-2.56 (m, 4H), 2.45-2.38 (m, 1H), 2.37-2.13 (m, 1H), 1.99-1.62 (m, 3H).

### Example 340 Synthesis of compound 1-((1R)-3'-(2-(2-(4-fluoro-2-methoxyphenyl) pyrrolidin-1-yl)-2-oxoethyl)-2',4'-dioxo-2,3-dihydrospiro[indene-1, 5'-oxazolidine]-5-yl)-3-methylurea (340)

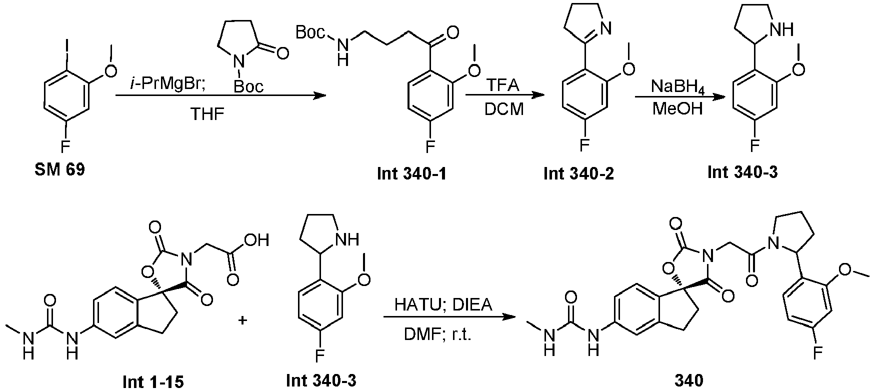

### Synthesis of intermediate (4-(4-fluoro-2-methoxyphenyl)-4-oxobutyl)carbamic acid t-butyl ester (Int 340-1)

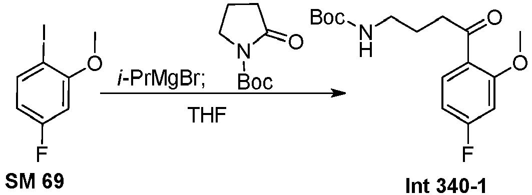

To a reaction flask, was added dry THF (30 mL), followed by addition of **SM 69** (2.5 g, 10 mmol), and then under N₂ protection, 1N isopropylmagnesium bromide (10 mL, 1M in THF) was added in an ice-water bath. After that, the mixture was reacted at room temperature for 7 h, and 5-oxopyrrolidin-1-carboxylic acid t-butyl ester (1.85 g, 10 mmol) was finally added, and the mixture was further allowed to react at room temperature for 12 h. The reaction solution was poured into the saturated aqueous solution of ammonium chloride, and extracted with 30 mL (10 mL×3) of ethyl acetate. The organic phases were combined, dried over anhydrous Na₂SO₄, and purified by column chromatography to obtain **Int 340-1** (1.7 g, 5.6 mmol), with a yield of 56%. MS: *m*/*z* 312 [M+H]⁺.

### Synthesis of intermediate 5-(4-fluoro-2-methoxyphenyl)-3,4-dihydro-2H-pyrrole (Int 340-2)

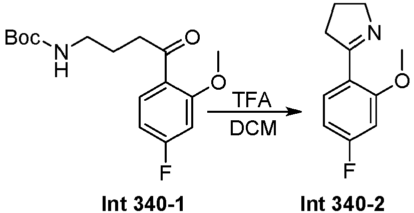

To a reaction solution was added DCM (20 mL), to which was then added **Int 340-1** (1.56 g, 5 mmol), followed by addition of TFA (2 mL). After that, the mixture was reacted at room temperature for 7 h, and the reaction solution was poured into the saturated aqueous solution of sodium bicarbonate. The resultant solution was extracted with 30 mL (10 mL × 3) of DCM. The organic phases were combined, dried over anhydrous Na₂SO₄, and purified by column chromatography, to obtain **Int 340-2** (772 mg, 4 mmol), with a yield of 80%. MS: *m*/*z* 194 [M+H]⁺.

### Synthesis of intermediate 2-(4-fluoro-2-methoxyphenyl)pyrrolidine (Int 340-3)

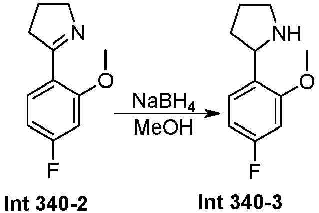

Methanol (15 mL) was added to a reaction flask, to which was then added **Int 340-2** (772 g, 4 mmol), followed by addition of sodium borohydride (0.76 g, 20 mmol) in batches. After that, the mixture was reacted at room temperature for 1 h. The reaction solution was poured into water, and extracted with 30 mL (10 mL×3) of EA. The organic phases were combined, dried over anhydrous Na₂SO₄, and concentrated to obtain **Int 340-3** (721 g, 3.7 mmol), with a yield of 94%. MS: *m*/*z* 196 [M+H]⁺.

### Synthesis of compound 1-((1R)-3'-(2-(2-(4-fluoro-2-methoxyphenyl)pyrrolidin- 1-yl)-2-oxoethyl)-2',4'-dioxo-2,3-dihydrospiro[indene-1,5'-oxazolidine]-5-yl)-3-methylurea (340)

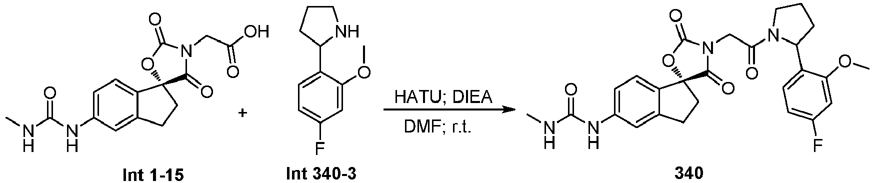

To a 25 mL reaction flask, was added DMF (3 mL), to which were then added **Int 1-15** (33 mg, 0.1 mmol), **SM 340-3** (19.5 mg, 0.1 mmol), DIEA (38.7 mg, 0.3 mmol), followed by addition of HATU (38 mg, 0.1 mmol). The system was reacted 1 h at 20 °C. After completion of the reation, the reaction solution was poured to water (10 mL), and the reasultant solution was extracted with 30 mL (10 mL×3) of ethyl acetate. The organic phases were combined, dried over anhydrous Na₂SO₄, and purified by column chromatography to obtain compound **340** (28 mg), with a yield of 55%. MS: *m*/*z* 511 [M+H]⁺.

### Example 341 Synthesis of compound 1-((1R)-3'-(2-(2-(2,4-dimethoxyphenyl) pyrrolidin-1-yl)-2-oxoethyl)-2',4'-dioxo-2,3-dihydrospiro[indene-1,5'oxazolidin]-5-yl)-3-methylurea (341)

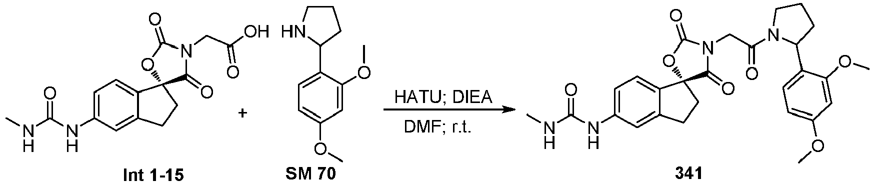

To a 25 mL reaction flask, was added DMF (1 mL), to which were then added **Int 1-15** (33 mg, 0.1 mmol), **SM 70** (21 mg, 0.1 mmol), DIEA (39 mg, 0.3 mmol), followed by addition of HATU (57 mg, 0.15 mmol). The system was reacted overnight at room temperature. After completion of the reation, water (5 mL) was added to the reaction solution, and the reasultant solution was extracted with ethyl acetate (5 mL×3). The organic phases were combined, dried over anhydrous Na₂SO₄, and purified by column chromatography to obtain compound **341** (19 mg, yield 36%). MS: *m*/*z* 523.2 [M+H]⁺. ¹H NMR (400 MHz, DMSO) δ 8.71 (d, J = 6.2 Hz, 1H), 7.55 (d, J = 11.3 Hz, 1H), 7.34-7.09 (m, 2H), 6.88 (dd, J = 35.3, 8.4 Hz, 1H), 6.64-6.36 (m, 2H), 6.13-6.00 (m, 1H), 5.23 (dd, J = 38.6, 7.4 Hz, 1H), 4.69-3.89 (m, 2H), 3.82 (d, J = 12.4 Hz, 3H), 3.77-3.70 (m, 3H), 3.66 (t, J = 8.7 Hz, 1H), 3.61-3.46 (m, 1H), 3.17-3.03 (m, 1H), 2.97 (d, J = 7.3 Hz, 1H), 2.61 (dt, J = 14.2, 5.6 Hz, 4H), 2.46-2.37 (m, 1H), 2.20 (d, J = 66.6 Hz, 1H), 1.95-1.60 (m, 3H).

### Example 343 Synthesis of compound 2-(1-(2-((R)-5-(3-methylureido) -2',4'-dioxo-2,3-dihydrospirofindene-1,5'-oxazole]-3'-yl)acetyl)pyrrolidin-2-yl)pyridin-1-oxide (343)

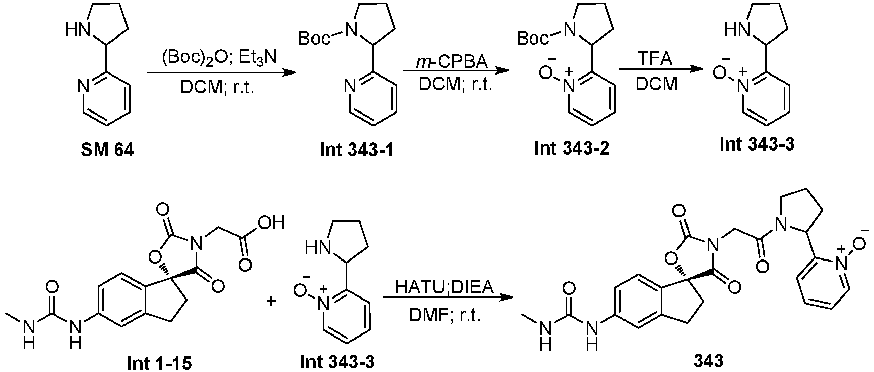

### Synthesis of intermediate 2-pyridin-2-ylpyrrolidin-1-carboxylic acid t-butyl ester (Int 343-1)

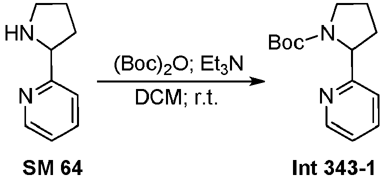

Intermediate **SM 64** (1.48 g, 10 mmol) was dissolved in DCM (20 mL), to which was added triethylamine (2 g, 20 mmol), followed by addition of (Boc)₂O (2.18 g, 10 mmol), and the mixture was stirred at room temperature for 2 h. To the reaction solution was added water (20 mL), and the solution was extracted three times. The organic phase was washed with saturated brine, dried over Na₂SO₄, and concentrated to obtain the crude product, that was purified by column chromatography to provide **Int 343-1** (2.23 g, 9 mmol), with a yield of 90%. MS: *m*/*z* 249 [M+H]⁺.

### Synthesis of intermediate 2-(1-t-butoxycarbonyl)pyrrolidin-2-yl)pyridin-1-oxide (Int 343-2)

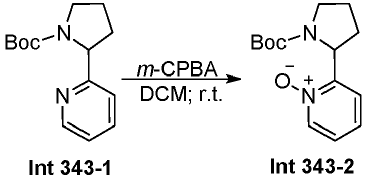

Intermediate **Int 343-1** (2.23 g, 9 mmol) was dissolved in DCM (20 mL), to which was added m-CPBA (1.73 g, 10 mmol), and the mixture was stirred 2 h at room temperature. The reaction solution was concentrated to remove most of DCM, and the crude product was subjected to reversed-phase column chromatography, to obtain the product **Int 343-2** (1.9, 7.2 mmol), with a yield of 80%. MS: *m*/*z* 265 [M+H]⁺.

### Synthesis of intermediate 2-pyrrolidin-2-ylpyridin-1-oxide (Int 343-3)

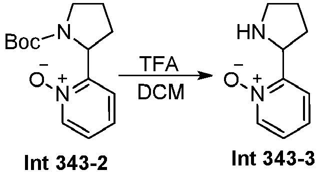

Intermediate **Int 343-2** (1.9 g, 7.2 mmol) was dissolved in DCM (20 mL), to which was added TFA (2 mL), and the mixture was stirred 2 h at room temperature. The reaction solution was concentrated to obtain **Int 343-3** (1.18 g, 7.2 mmol), with a yield of 100%. MS: *m*/*z* 165 [M+H]⁺.

### Synthesis of compound 2-(1-(2-((R)-5-(3-methylureido)-2',4'-dioxo-2,3-dihydrospiro [indene-1,5'-oxazole]-3'-yl)acetyl)pyrrolidin- 2-yl)pyridin-1-oxide (343)

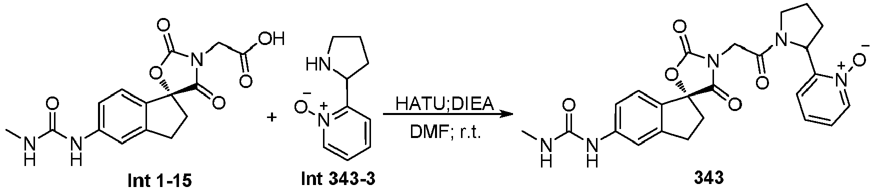

Intermediate **Int 1-15** (33 mg, 0.1 mmol) was dissolved in DMF (1 mL), to which were added DIEA (39 mg, 0.3 mmol) and HATU (38 mg, 0.1 mmol), followed by addition of **Int 343-3** (20 mg, 0.1 mmol), and the mixture was stirred at room temperature for 16 h. To the reaction solution was added water (10 mL), and extracted with ethyl acetate two times. The organic phase was washed with saturated brine, and concentrated to obtain the crude product, that was purified by column chromatography to obtain product **343** (24 mg) as off-white solid, with a yield of 50%. MS: *m*/*z* 480 [M+H]⁺.

### Example 347 Synthesis of compound 1-((1R)-3'-(2-(2-(4-fluoro-2-methylphenyl) pyrrolidin-1-yl)-2-oxoethyl)-2',4'-dioxo-2,3-dihydrospiro[indene-1,5'-oxazolidine]-5-yl)-3-methylurea (347)

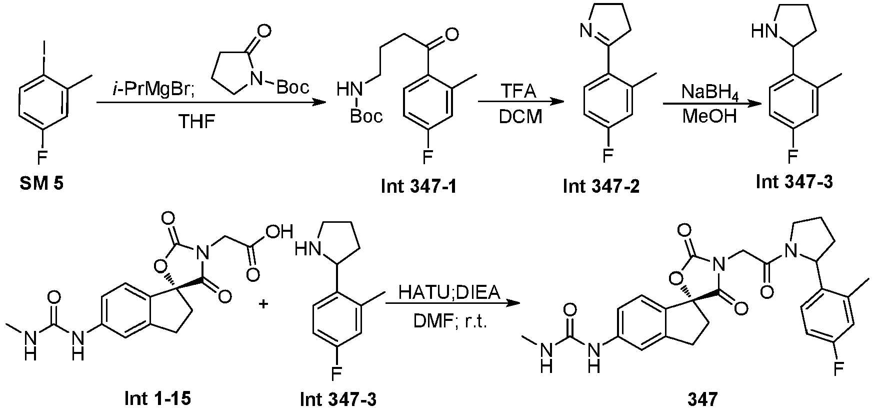

### Synthesis of intermediate (4-(4-fluoro-2-methylphenyl)-4-oxobutyl)carbamic acid t-butyl ester(Int 347-1)

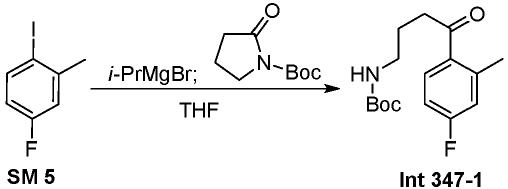

To a reaction flask containing **SM 5** (2.36 g, 10 mmol), was added dry THF (20 mL), and then under N₂ protection, the solution of isopropylmagnesium bromide in tetrahydrofuran (10 mL, 1M in THF) was added in an ice-water bath. After that, the ice-water bath was removed, and the mixture was stirred at room temperature for 7 h, and N-Boc-2-pyrrolidone (1.85 g, 10 mmol) was finally added. The mixture was further allowed to react at room temperature for 12 h. The reaction solution was poured into the saturated aqueous solution of ammonium chloride, and extracted with 30 mL (10 mL×3) of ethyl acetate. The organic phases were combined, dried over anhydrous Na₂SO₄, and purified by column chromatography to obtain **Int 347-1** (2.36 g, 8 mmol), with a yield of 80%. MS: *m*/*z* 296 [M+H]⁺.

### Synthesis of intermediate 5-(4-fluoro-2-methylphenyl)-3,4-dihydro-2H-pyrrole (Int 347-2)

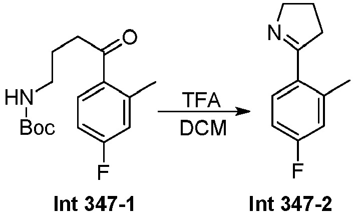

Intermediate **Int 347-1** (2.36 g, 8 mmol) was dissolved in DCM (20 mL), to which was added TFA (2.3 mL), and the reaction was stirred at room temperature and detected by TLC. After completion of the reaction, the pH of the reaction solution was adjusted to be neutral with saturated NaHCO₃ aqueous solution, and then extracted with DCM (10 mL × 3), followed by drying over anhydrous Na₂SO₄ and rotatory evaporation to dry, to obtain **Int 347-2** (1.2 g, 85%), MS: *m*/*z* 178 [M+H]⁺.

### Synthesis of intermediate 2-(4-fluoro-2-methylphenyl)pyrrolidine(Int 347-3)

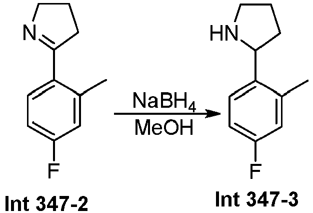

**Int. 347-2** (1.2 g, 6.8 mmol) was introduced into the reaction flask containing methanol (12 mL), to which was added sodium borohydride (0.90 g, 23.8 mmol) in batches under stirring at room temperature. After that, the reaction was still stirred at room temperature and detected by TLC. After completion of the reaction, the reaction solution was poured to water, and extracted with 30 mL (10 mL×3) of EA. The organic phase was combined, dried over anhydrous Na₂SO₄, and separated by column chromatography, to obtain **Int 347-3** (0.97 g, 80%), MS: *m*/*z* 180 [M+H]⁺.

### Synthesis of compound 1-((1R)-3'-(2-(2-(4-fluoro-2-methylphenyl)pyrrolidin- 1-yl)-2-oxoethyl)-2',4'-dioxo-2,3-dihydrospiro[indene-1,5'-oxazolidine]-5-yl)-3-methylurea (347)

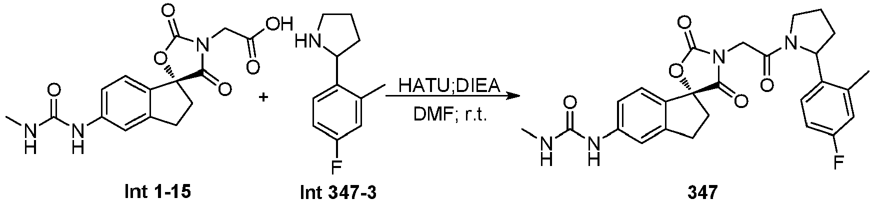

Intermediate **Int 1-15** (30 mg, 0.09 mmol) was dissolved in DMF (1 mL), to which were added DIEA (35 mg, 0.027 mmol) and HATU (41 mg, 0.011 mmol), followed by addition of **Int 347-3** (24 mg, 0.135 mmol), and the mixture was stirred at room temperature for 16 h. To the reaction solution was added water (10 mL), and extracted with ethyl acetate two times. The organic phase was washed with saturated brine, and concentrated to obtain the crude product, that was purified by column chromatography to obtain product **347** (32 mg) as off-white solid, with a yield of 72%. MS: *m*/*z* 495 [M+H]⁺.

### Example 348 Synthesis of compound 1-((1R)-3'-(2-(2-(4-fluorophenyl)-5-methylpyrrolidin-1-yl)-2-oxoethyl)-2',4'-dioxo-2,3-dihydrospiro[indene-1,5'-oxazolidine]-5-yl)-3-methylurea (348)

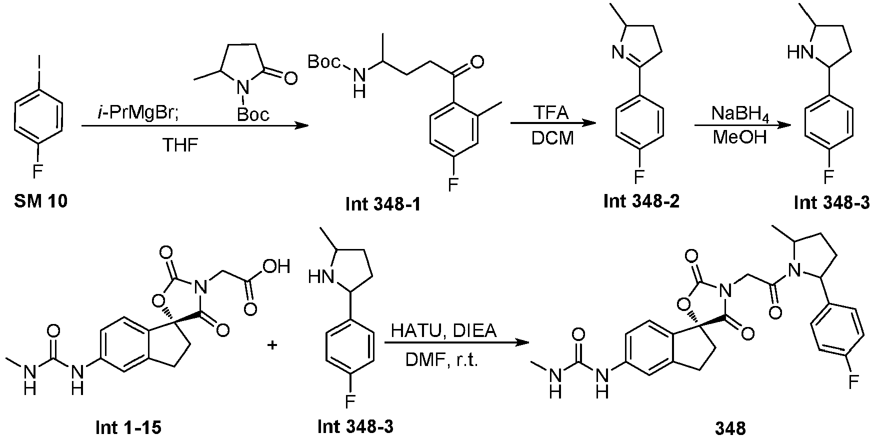

### Synthesis of intermediate (5-(4-fluorophenyl)-5-oxopentan-2-yl)carbamic acid t-butyl ester(348)

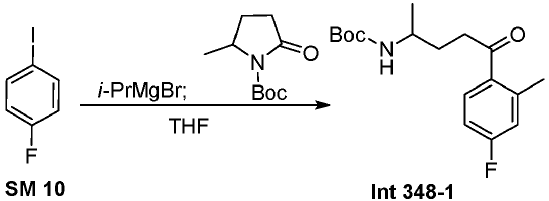

To a reaction flask, was added dry THF (30 mL), followed by addition of **SM 10** (2.22 g, 10 mmol), and then under N₂ protection, 1N isopropylmagnesium bromide (10 mL, 1M in THF) was added in an ice-water bath. After that, the mixture was reacted at room temperature for 7 h, and 2-methyl-5-oxopyrrolidin-1-carboxylic acid t-butyl ester (1.99 g, 10 mmol) was finally added, and the mixture was further allowed to react at room temperature for 12 h. The reaction solution was poured into the saturated aqueous solution of ammonium chloride, and extracted with 30 mL (10 mL×3) ethyl acetate. The organic phases were combined, dried over anhydrous Na₂SO₄, and purified by column chromatography to obtain **Int 348-1** (1.5 g, 5 mmol), with a yield of 50%. MS: *m*/*z* 296 [M+H]⁺.

### Synthesis of intermediate 5-(4-fluorophenyl)-2-methyl-3,4-dihydro-2H-pyrrole (Int 348-2)

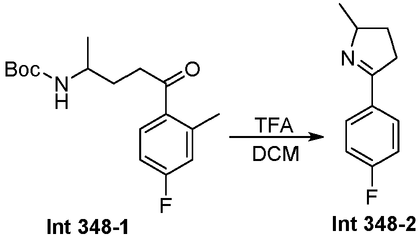

To a reaction solution was added DCM (20 mL), to which was then added **Int 348-1** (1.5 g, 5 mmol), followed by addition of TFA (2 mL). After that, the mixture was reacted at room temperature for 7 h, and the reaction solution was poured into the saturated aqueous solution of sodium bicarbonate. The resultant solution was extracted with 30 mL (10 mL × 3) DCM. The organic phases were combined, dried over anhydrous Na₂SO₄, and purified by column chromatography, to obtain **Int 348-2** (0.8 g, 4.5 mmol), with a yield of 90%. MS: *m*/*z* 178 [M+H]⁺.

### Synthesis of intermediate 2-(4-fluorophenyl)-5-methylpyrrolidine (Int 348-3)

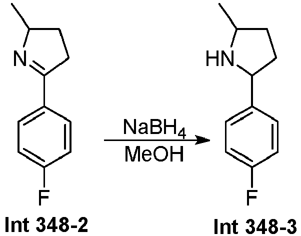

Methanol (15 mL) was added to a reaction flask, to which was then added **Int 348-2** (0.8 g, 4.5 mmol), followed by addition of sodium borohydride (0.76 g, 20 mmol) in batches. After that, the mixture was reacted at room temperature for 1 h. The reaction solution was poured into water, and extracted with 30 mL (10 mL×3) of EA. The organic phases were combined, dried over anhydrous Na₂SO₄, and concentrated to obtain **Int 348-3** (0.8 g, 4.4 mmol), with a yield of 98%. MS: *m*/*z* 180 [M+H]⁺.

### Synthesis of compound 1-((1R)-3'-(2-(2-(4-fluorophenyl)-5-methylpyrrolidin-1-yl)-2-oxoethyl)-2',4'-dioxo-2,3-dihydrospiro[indene-1,5'-oxazolidine]-5-yl)-3-methylurea (348)

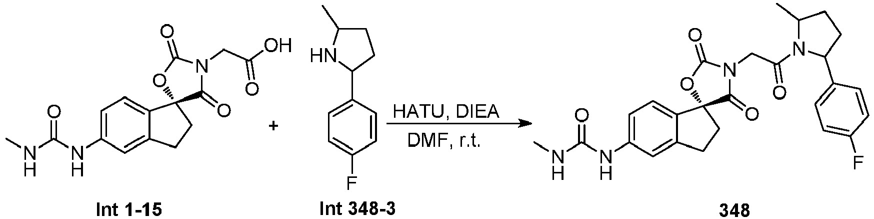

Intermediate **Int 1-15** (33 mg, 0.1 mmol) was dissolved in DMF (1 mL), to which were added DIEA (39 mg, 0.3 mmol) and HATU (38 mg, 0.01 mmol), followed by addition of **Int 348-3** (24 mg, 0.1 mmol), and the mixture was stirred at room temperature for 16 h. To the reaction solution was added water (10 mL), and extracted with ethyl acetate two times. The organic phase was washed with saturated brine, and concentrated to obtain the crude product, that was purified by column chromatography to obtain product **348** (25 mg) as off-white solid, with a yield of 50%. MS: *m*/*z* 495 [M+H]⁺. ¹H NMR (400 MHz, CDCl₃): δ 7.59-7.41 (m, 1H), 7.37-7.27 (m, 2H), 7.21-6.88 (m, 4H), 4.97 (m, 1H), 4.49-4.35 (m, 1H), 4.17-4.08 (m, 1H), 3.73 (m, 1H), 3.08-2.95 (m, 1H), 2.78-2.64 (m, 3H), 2.59-2.36 (m, 2H), 2.08-1.92 (m, 2H), 1.49-1.16 (m, 6H).

### Example 349 Synthesis of compound 1-((1R)-3'-(2-(2,2-bis(4-methoxyphenyl)-5-methylpyrrolidin-1-yl)-2-oxoethyl)-2',4'-dioxo-2,3-dihydrospiro[indene-1, 5'-oxazolidine]-5-yl)-3-methylurea (349)

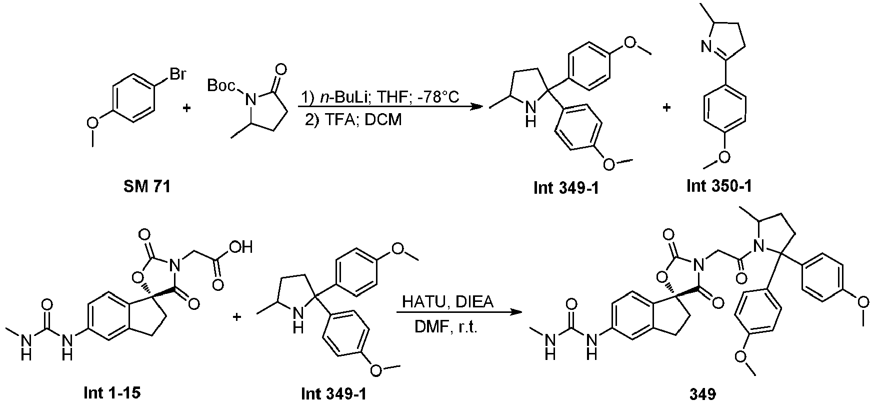

### Synthesis of intermediate 2,2-bis(4-methoxyphenyl)-5-methylpyrrolidine (Int 349-1) and 5-(4-methoxyphenyl)-2-methyl-3,4-dihydro-2H-pyrrole (Int 350-1):

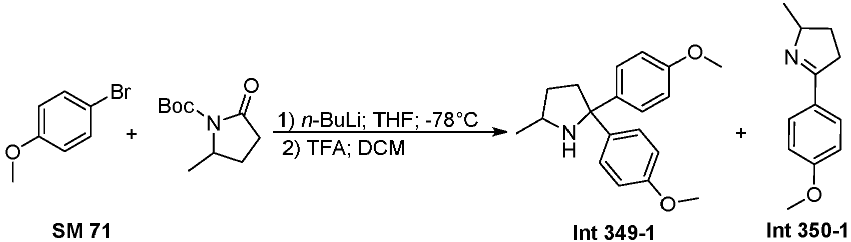

**SM 71** (1.9 g, 10 mmol) was dissolved in dry THF (50 mL), to which was filled with N₂, and then n-butyl lithium (10 mL, 1 M in hexane) was slowly added dropwise at -78°C. The mixture was stirred for 30 min, and then 2-methyl-5-oxopyrrolidin-1-carboxylic acid t-butyl ester (2 g, 10 mmol) was slowly added dropwise. The mixture was further stirred for 30 min. The saturated aqueous solution of ammonium chloride was added. The reaction solution was extracted with ethyl acetate, dried over anhydrous sodium sulfate, and concentrated. The obtained crude product was dissolved in dichloromethane (20 mL), to which was added trifluoroacetic acid (2 mL). The reaction was stirred overnight, concentrated, and separated by column chromatography to obtain intermediate **Int 349-1** (500 mg), MS: *m*/*z* 298 [M+H]⁺; intermediate **Int 350-1** (300 mg), MS: *m*/*z* 190 [M+H]⁺.

### Synthesis of compound 1-((1R)-3'-(2-(2,2-bis(4-methoxyphenyl)-5-methylpyrrolidin- 1-yl)-2-oxoethyl)-2',4'-dioxo-2,3-dihydrospiro[indene-1,5'-oxazolidine]-5-yl)-3-methylurea (349)

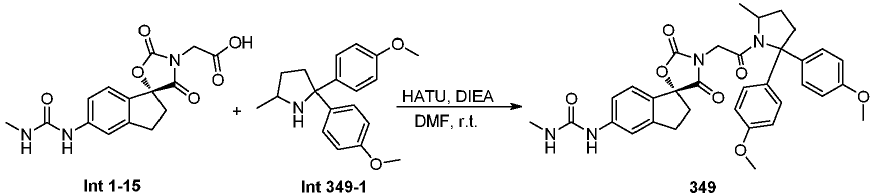

Intermediate **Int 1-15** (33 mg, 0.1 mmol), intermediate **Int 349-1** (30 mg, 0.11 mmol), HATU (50 mg, 0.13 mmol), and DIEA (20 mg, 0.15 mmol) were dissolved in DMF (5 mL), and the mixture was stirred overnight, to which was added water. The reaction solution was extracted with EA, concentrated, and subjected to column chromatography, to obtain **349** as pale yellow solid (38 mg), with a yield of 62%. MS: *m*/*z* 613 [M+H]⁺. ¹H NMR (400 MHz, DMSO): δ 8.79 (s, 1H), 8.25 (s, 1H), 7.57 (d, J = 5.4 Hz, 1H), 7.34 (t, J = 8.9 Hz, 1H), 7.24 (d, J = 8.3 Hz, 1H), 7.12-7.01 (m, 4H), 7.00-6.91 (m, 2H), 6.81 (d, J = 8.8 Hz, 1H), 6.74 (d, J = 8.8 Hz, 1H), 6.13 (d, J = 4.6 Hz, 1H), 5.91 (s, 1H), 4.22-4.06 (m, 2H), 3.93 (td, J = 13.1, 6.2 Hz, 1H), 3.78 (t, J = 5.9 Hz, 3H), 3.72-3.67 (m, 3H), 3.16-2.95 (m, 2H), 2.69-2.44 (m, 5H), 2.21 (t, J = 5.9 Hz, 2H), 1.05 (dd, J = 6.6, 2.4 Hz, 3H).

### Example 350 Synthesis of compound 1-((1R)-3'-(2-(2-(4-methoxyphenyl)-5-methylpyrrolidin-1-yl)-2-oxoethyl)-2',4'-dioxo-2,3-dihydrospiro[indene-1, 5'-oxazolidine]-5-yl)-3-methylurea (350)

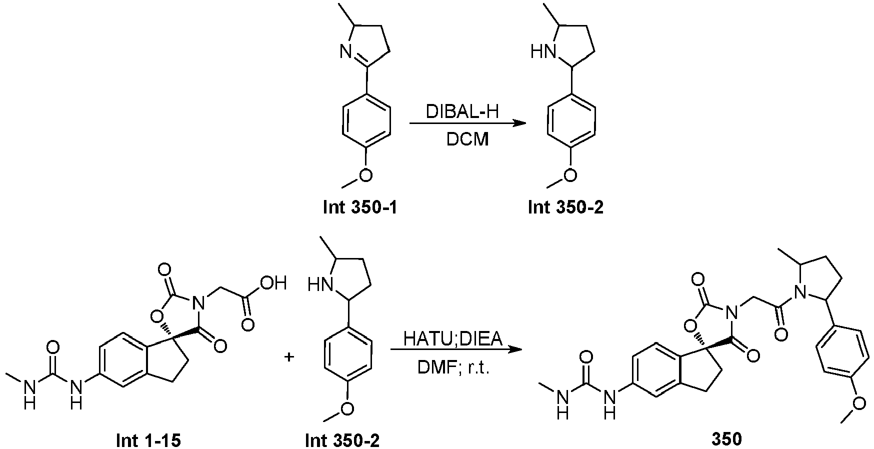

### Synthesis of intermediate 2-(4-methoxyphenyl)-5-methylpyrrolidine (Int 350-2)

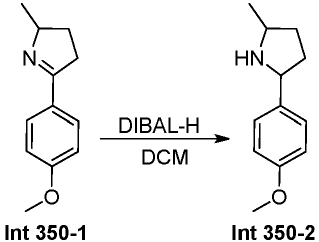

Intermediate **Int 350-1** (190 mg, 1 mmol) was dissolved in dichloromethane (10 mL), to which was added diisobutylaluminum hydride (4 mL, 1M in DCM) in an ice bath, and the mixture was stirred 4 h, followed by addition of celite and water (10 mL). The solution was filtered, extracted with ethyl acetate, dried over anhydrous Na₂SO₄, concentrated, and subjected to column chromatography, to obtain **Int 350-2** (100 mg) as pale yellow liquid, with a yield of 53%.

### Synthesis of compound 1-((1R)-3'-(2-(2-(4-methoxyphenyl)-5-methylpyrrolidin- 1-yl)-2-oxoethyl)-2',4'-dioxo-2,3-dihydrospiro[indene-1,5'-oxazolidine]-5-yl)-3-methylurea (350)

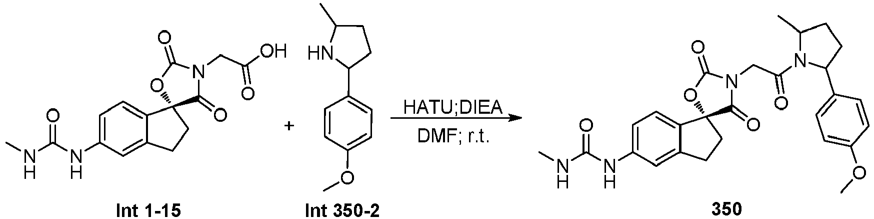

**Int 1-15** (33 mg, 0.1 mmol), intermediate **Int 350-2** (20 mg, 0.11 mmol), HATU (50 mg, 0.13 mmol), and DIEA(20 mg, 0.15 mmol)were dissolved in DMF(5 mL), and the mixture was stirred overnight, to which was added water. The reaction solution was extracted with EA, concentrated, and subjected to column chromatography, to obtain **350** as pale yellow solid (37 mg), with a yield of 73%. MS: *m*/*z* 507 [M+H]⁺. ¹H NMR (400 MHz, DMSO): δ 8.71 (s, 1H), 7.53 (d, J = 4.8 Hz, 1H), 7.28 (d, J = 8.6 Hz, 2H), 7.23-7.08 (m, 2H), 6.98 (dd, J = 8.6, 1.9 Hz, 2H), 6.09 (d, J = 4.5 Hz, 1H), 4.37 (dd, J = 20.2, 12.2 Hz, 1H), 3.78 (d, J = 16.7 Hz, 2H), 3.74-3.71 (m, 1H), 3.34 (s, 3H), 3.14-2.91 (m, 2H), 2.70-2.47 (m, 5H), 2.45-2.28 (m, 2H), 2.06-1.82 (m, 2H), 1.36 (dd, J = 6.7, 3.1 Hz, 3H).

### Example 351 Synthesis of compound 1-((1R)-3'-(2-(2-(4-chloro-2-methylphenyl)-5-methylpyrrolidin-1-yl)-2-oxoethyl)-2',4'-dioxo-2,3-dihydrospiro[indene-1, 5'-oxazolidine]-5-yl)-3-methylurea (351)

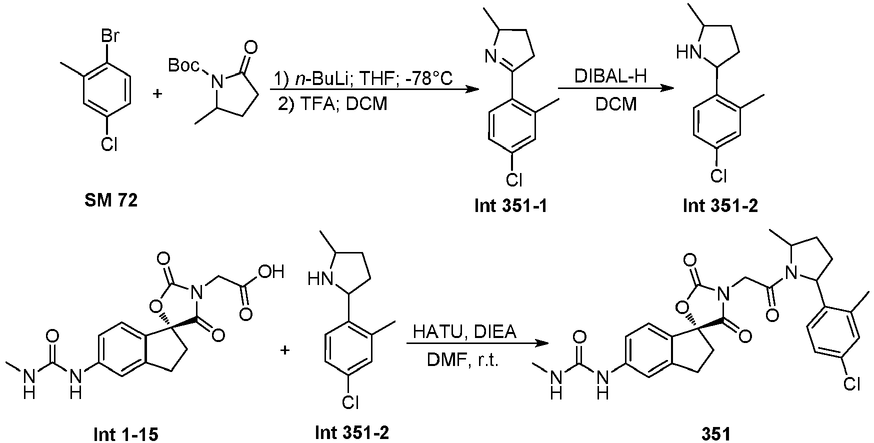

### Synthesis of intermediate 5-(4-chloro-2-methylphenyl)-2-methyl-3,4-dihydro-2H- pyrrole (Int 351-1)

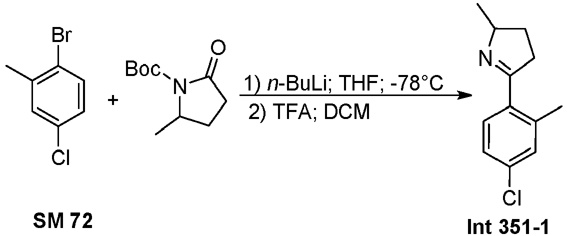

**SM 72** (2.1 g, 10 mmol) was dissolved in dry THF (50 mL), to which was filled with N₂, and then n-butyl lithium (10 mL, 1 M in hexane) was slowly added dropwise at -78°C. The mixture was stirred for 30 min, and then 2-methyl-5-oxopyrrolidin-1-carboxylic acid t-butyl ester (2 g, 10 mmol) was slowly added dropwise. The mixture was further stirred for 30 min. The saturated aqueous solution of ammonium chloride was added. The reaction solution was extracted with ethyl acetate, dried over anhydrous sodium sulfate, and concentrated. The obtained crude product was dissolved in dichloromethane (20 mL), to which was added trifluoroacetic acid (2 mL). The reaction was stirred overnight, concentrated, and separated by column chromatography to obtain intermediate intermediate **Int 351-1** (600 mg), with a two-step yield of 29%. MS: *m*/*z* 208 [M+H]⁺.

### Synthesis of intermediate 2-(4-chloro-2-methylphenyl)-5-methylpyrrolidine (Int 352-2)

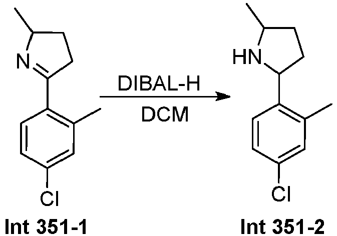

Intermediate **Int 351-1** (207 mg, 1 mmol) was dissolved in dichloromethane (10 mL), to which was added diisobutylaluminum hydride (4 mL, 1M in DCM) in an ice bath, and the mixture was stirred 4 h, followed by addition of celite and water (10 mL). The solution was filtered, extracted with ethyl acetate, dried over anhydrous Na₂SO₄, concentrated, and subjected to column chromatography, to obtain **Int 351-2** (153 mg) as pale yellow liquid, with a yield of 73%. MS: *m*/*z* 210 [M+H]⁺.

### Synthesis of compound 1-((1R)-3'-(2-(2-(4-chloro-2-methylphenyl)-5-methylpyrrolidin-1-yl)-2-oxoethyl)-2',4'-dioxo-2,3-dihydrospiro[indene-1,5'-oxazolidine]-5-yl)-3-methylurea (351)

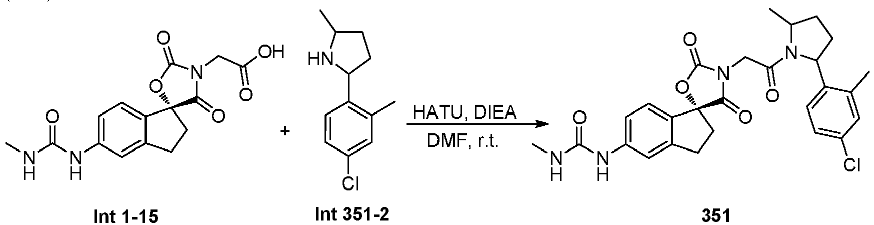

**Int 1-15** (33 mg, 0.1 mmol), intermediate **Int 351-2** (21 mg, 0.11 mmol), HATU (50 mg, 0.13 mmol), and DIEA (20 mg, 0.15 mmol) were dissolved in DMF (5 mL), and the mixture was stirred overnight, to which was added water. The reaction solution was extracted with EA, concentrated, and subjected to column chromatography, to obtain **351** as pale yellow solid (38 mg), with a yield of 72%. MS: *m*/*z* 525 [M+H]⁺. ¹H NMR (400 MHz, DMSO): δ 8.71 (s, 1H), 7.53 (s, 1H), 7.36-7.01 (m, 5H), 6.08 (d, J = 4.5 Hz, 1H), 5.39-5.31 (m, 1H), 4.52-4.34 (m, 2H), 4.22 (dd, J = 12.5, 6.3 Hz, 1H), 3.06 (dd, J = 27.3, 20.0 Hz, 2H), 2.69-2.52 (m, 5H), 2.46-2.29 (m, 5H), 1.79-1.47 (m, 2H), 1.44-1.37 (m, 3H).

### Example 352 Synthesis of compound 1-methyl-3-((1R)-3'-(2-(2-methyl-5-(2- methyl-4-(trifluoromethyl)phenyl)pyrrolidin-1-yl)-2-oxoethyl)-2',4'-dioxo-2,3-dihydrospiro[indene-1,5'-oxazolidine]-5-yl)urea (352)

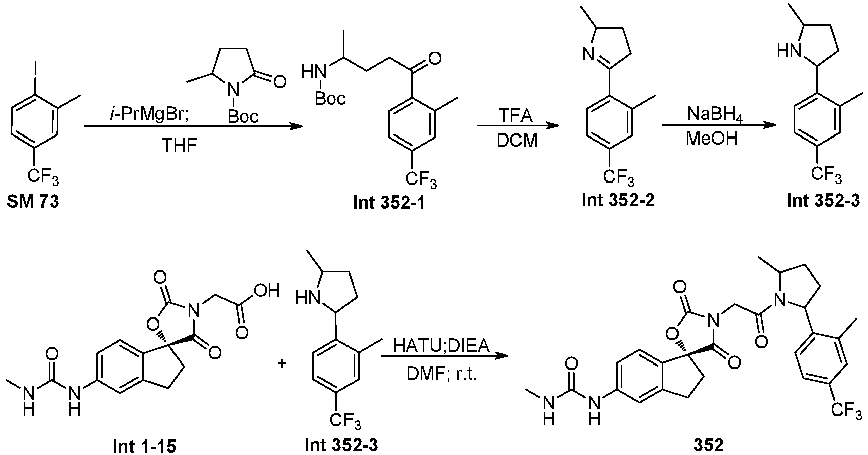

### Synthesis of intermediate (5-(2-methyl-4-(trifluoromethyl)phenyl)-5-oxopentan-2-yl) carbamic acid t-butyl ester(Int 352-1)

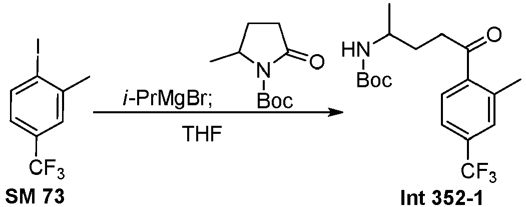

To a reaction flask, was added dry THF (30 mL), followed by addition of **SM 73** (2.86 g, 10 mmol), and then under N₂ protection, 1N isopropylmagnesium bromide (10 mL, 1M in THF) was added in an ice-water bath. After that, the mixture was reacted at room temperature for 7 h, and t-butyl 2-methyl-5-oxopyrrolidin-1-carboxylate (1.99 g, 10 mmol) was finally added, and the mixture was further allowed to react at room temperature for 12 h. The reaction solution was poured into the saturated aqueous solution of ammonium chloride, and extracted with 30 mL (10 mL×3) of ethyl acetate. The organic phases were combined, dried over anhydrous Na₂SO₄, and purified by column chromatography to obtain **Int 352-1** (1.8 g, 5 mmol), with a yield of 50%. MS: *m*/*z* 360 [M+H]⁺.

### Synthesis of intermediate 5-(2-methyl-4-(trifluoromethyl)phenyl)-2-methyl-3,4- dihydro-2H-pyrrole (Int 352-2)

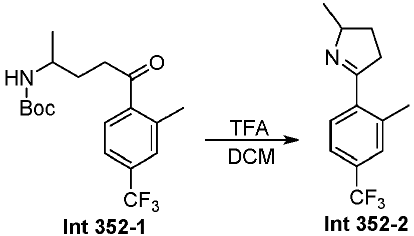

To a reaction solution was added DCM (20 mL), to which was then added **Int 352-1** (1.8 g, 5 mmol), followed by addition of TFA (2 mL). After that, the mixture was reacted at room temperature for 7 h, and the reaction solution was poured into the saturated aqueous solution of sodium bicarbonate. The resultant solution was extracted with 30 mL (10 mL × 3) of DCM. The organic phases were combined, dried over anhydrous Na₂SO₄, and purified by column chromatography, to obtain **Int 352-2** (1.08 g, 4.5 mmol), with a yield of 90%. MS: *m*/*z* 242 [M+H]⁺.

### Synthesis of intermediate 2-(2-methyl-4-(trifluoromethyl)phenyl)-5-methylpyrrolidine (Int 352-3)

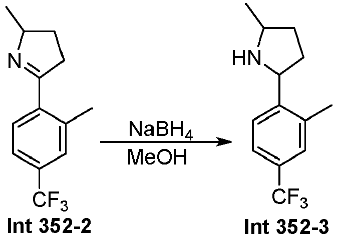

Methanol (15 mL) was added to a reaction flask, to which was then added **Int 352-2** (1.08 g, 4.5 mmol), followed by addition of sodium borohydride (0.76 g, 20 mmol) in batches. After that, the mixture was reacted at room temperature for 1 h. The reaction solution was poured into water, and extracted with 30 mL (10 mL×3) of EA. The organic phases were combined, dried over anhydrous Na₂SO₄, and concentrated to obtain **Int 352-3** (1.07 g, 4.4 mmol), with a yield of 98%. MS: *m*/*z* 244 [M+H]⁺.

### Synthesis of compound 1-methyl-3-((1R)-3'-(2-(2-methyl-5-(2-methyl-4-(trifluoromethyl)phenyl)pyrrolidin-1-yl)-2-oxoethyl)-2',4'-dioxo-2,3-dihydrospiro[indene-1,5' -oxazolidine] -5 -yl)urea (352)

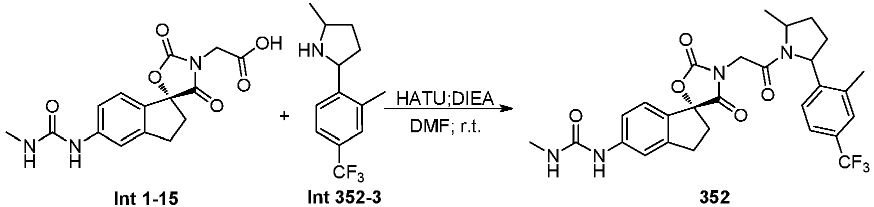

Intermediate **Int 1-15** (33 mg, 0.1 mmol) was dissolved in DMF (1 mL), to which were added DIEA (39 mg, 0.3 mmol) and HATU (38 mg, 0.01 mmol), followed by addition of **Int 352-3** (26 mg, 0.1 mmol), and the mixture was stirred at room temperature for 16 h. To the reaction solution was added water (10 mL), and extracted with ethyl acetate two times. The organic phase was washed with saturated brine, and concentrated to obtain the crude product, that was purified by column chromatography to obtain product **352** (28 mg) as off-white solid, with a yield of 50%. MS: *m*/*z* 559 [M+H]⁺.

### Example 353 Synthesis of compound 1-((1R)-3'-(2-(2-(4-fluoro-2-methylphenyl)-5-methylpyrrolidin-1-yl)-2-oxoethyl)-2',4'-dioxo-2,3-dihydrospiro[indene-1,5'-oxazolidine]-5-yl)-3-methylurea (353)

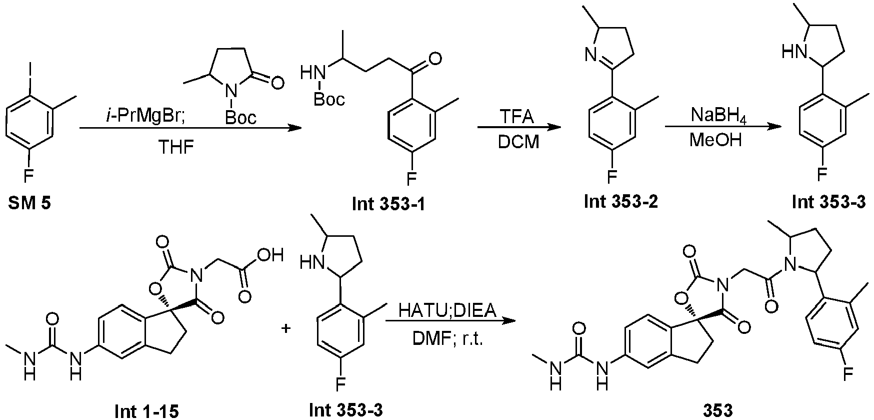

### Synthesis of intermediate (5-(4-fluoro-2-methylphenyl)-2-methyl-5-oxobutyl) carbamic acid t-butyl ester(Int 353-1)

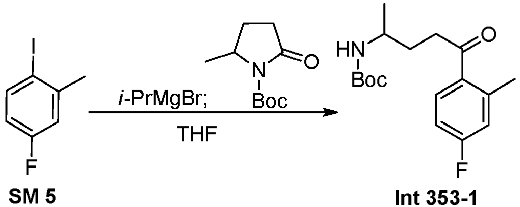

To a reaction flask containing **SM 5** (2.36 g, 10 mmol), was added dry THF (20 mL), and then under N₂ protection, isopropylmagnesium bromide (10 mL, 1M in THF) was added to the reaction flask in an ice-water bath. After that, the ice-water bath was removed, and the mixture was reacted at room temperature for 7 h, and 2-methyl-5-oxopyrrolidin-1-carboxylic acid t-butyl ester (1.99 g, 10 mmol) was finally added, and the mixture was further allowed to react at room temperature for 12 h. The reaction solution was poured into the saturated aqueous solution of ammonium chloride, and extracted with 30 mL (10 mL×3) of ethyl acetate. The organic phases were combined, dried over anhydrous Na₂SO₄, and purified by column chromatography to obtain **Int 353-1** (2.63 g, 8.5 mmol), with a yield of 85%. MS: m/z 310 [M+H]⁺.

### Synthesis of intermediate 5-(4-fluoro-2-methylphenyl)-2-methyl-3,4-dihydro- 2H-pyrrole (Int 353-2)

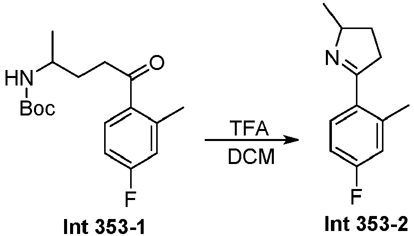

**Int 353-1** (2.63 g, 8.5 mmol) was dissolved in DCM (23 mL), to which was added TFA (2.3 mL), and the reaction was stirred at room temperature and detected by TLC. After completion of the reaction, the pH of the reaction solution was adjusted to be neutral with saturated NaHCO₃ aqueous solution, and then extracted with DCM (10 mL × 3), followed by drying over anhydrous Na₂SO₄ and rotatory evaporation to dry, to obtain **Int 353-2** (1.41 g), with a yield of 87%, MS: *m*/*z* 192 [M+H]⁺.

### Synthesis of intermediate 2-(4-fluoro-2-methylphenyl)-5-methylpyrrole (Int 353-3)

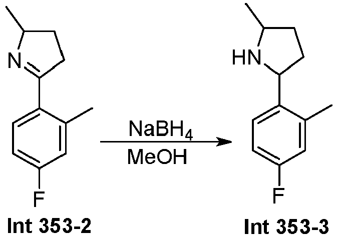

**Int. 353-2** (1.41 g, 7.4 mmol) was introduced into the reaction flask containing methanol (12 mL), to which was added sodium borohydride (1.09 g, 29.58 mmol) in batches under stirring at room temperature. After that, the reaction was still stirred at room temperature and detected by TLC. After completion of the reaction, the reaction solution was poured to water, and extracted with 30 mL (10 mL×3) of EA. The organic phase was combined, dried over anhydrous Na₂SO₄, and separated by column chromatography, to obtain **Int 353-3** (1.17 g), with a yield of 82%. MS: *m*/*z* 194 [M+H]⁺.

### Synthesis of compound 1-((1R)-3'-(2-(2-(4-fluoro-2-methylphenyl)-5-methylpyrrolidin-1-yl)-2-oxoethyl)-2',4'-dioxo-2,3-dihydrospiro[indene-1,5'-oxazolidine]-5-yl)-3-methylurea (353)

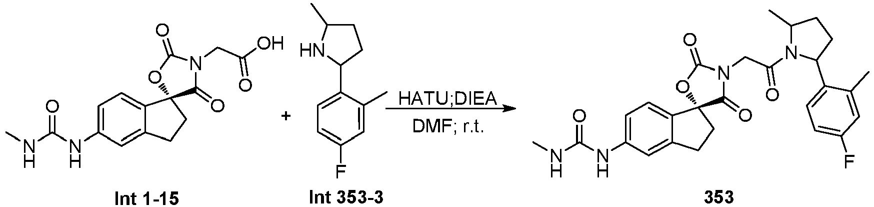

Intermediate **Int 1-15** (30 mg, 0.09 mmol) was dissolved in DMF (1 mL), to which were added DIEA (35 mg, 0.027 mmol) and HATU (41 mg, 0.011 mmol), followed by addition of **Int 353-3** (26 mg, 0.135 mmol), and the mixture was stirred at room temperature for 16 h. To the reaction solution was added water (10 mL), and extracted with ethyl acetate two times. The organic phase was washed with saturated brine, and concentrated to obtain the crude product, that was purified by column chromatography to obtain product **353** (37 mg) as off-white solid, with a yield of 81%. MS: *m*/*z* 509 [M+H]⁺.

### Example 354 Synthesis of compound 1-((1R)-3'-(2-(2-(2-bromo-4-fluorophenyl)- 5-methylpyrrolidin-1-yl)-2-oxoethyl)-2',4'-dioxo-2,3-dihydrospiro[indene-1, 5'-oxazolidine]-5-yl)-3-methylurea (354)

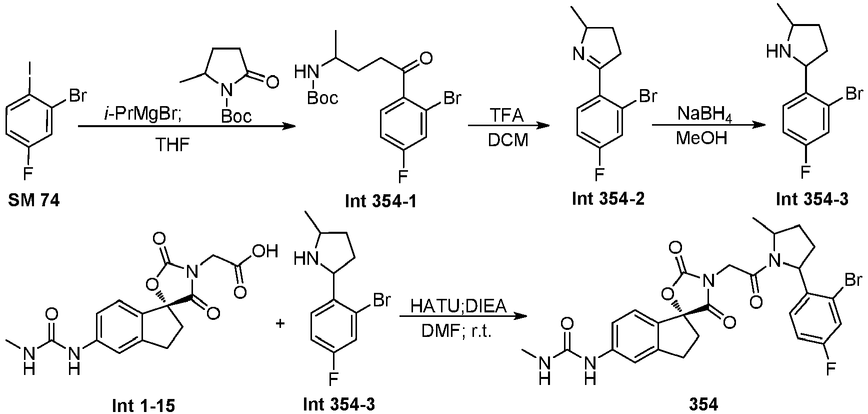

### Synthesis of intermediate (5-(2-bromo-4-fluorophenyl)-5-oxopentan-2-yl)carbamic acid t-butyl ester(Int 351-1)

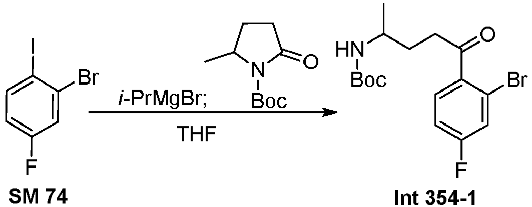

To a reaction flask, was added dry THF (30 mL), followed by addition of **SM 74** (3 g, 10 mmol), and then under N₂ protection, 1N isopropylmagnesium bromide (10 mL, 1M in THF) was added in an ice-water bath. After that, the mixture was reacted at room temperature for 7 h, and t-butyl 2-methyl-5-oxopyrrolidin-1-carboxylate (1.99 g, 10 mmol) was finally added. The mixture was further allowed to react at room temperature for 12 h. The reaction solution was poured into the saturated aqueous solution of ammonium chloride, and extracted with 30 mL (10 mL×3) of ethyl acetate. The organic phases were combined, dried over anhydrous Na₂SO₄, and purified by column chromatography to obtain **Int 354-1** (1.87 g, 5 mmol), with a yield of 50%. MS: *m*/*z* 374, 376 [M+H]⁺.

### Synthesis of intermediate 5-(2-bromo-4-fluorophenyl)-2-methyl-3,4-dihydro-2H- pyrrole (Int 354-2)

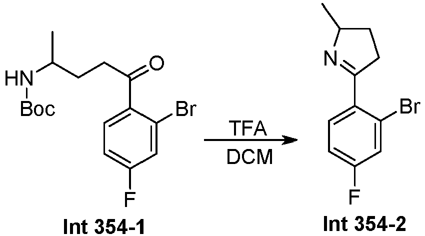

To a reaction solution was added DCM (20 mL), to which was then added **Int 354-1** (1.87 g, 5 mmol), followed by addition of TFA (2 mL). After that, the mixture was reacted at room temperature for 7 h, and the reaction solution was poured into the saturated aqueous solution of sodium bicarbonate. The resultant solution was extracted with 30 mL (10 mL × 3) of DCM. The organic phases were combined, dried over anhydrous Na₂SO₄, and purified by column chromatography, to obtain **Int 354-2** (1.15 g, 4.5 mmol), with a yield of 90%. MS: *m*/*z* 256, 258 [M+H]⁺.

### Synthesis of intermediate 2-(2-bromo-4-fluorophenyl)-5-methylpyrrolidine (Int 354-3)

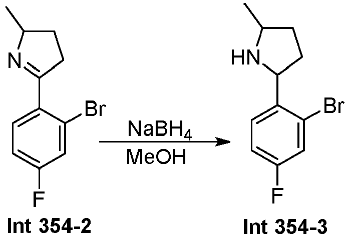

Methanol (15 mL) was added to a reaction flask, to which was then added **Int 354-2** (1.15 g, 4.5 mmol), followed by addition of sodium borohydride (0.76 g, 20 mmol) in batches. After that, the mixture was reacted at room temperature for 1 h. The reaction solution was poured into water, and extracted with 30 mL (10 mL×3) of EA. The organic phases were combined, dried over anhydrous Na₂SO₄, and concentrated to obtain **Int 354-3** (1.14 g, 4.4 mmol), with a yield of 98%. MS: *m*/*z* 258, 260 [M+H]⁺.

### Synthesis of compound 1-((1R)-3'-(2-(2-(2-bromo-4-fluorophenyl)-5- methylpyrrolidin-1-yl)-2-oxoethyl)-2',4'-dioxo-2,3-dihydrospiro[indene-1,5'-oxazolidine]-5-yl)-3-methylurea (354)

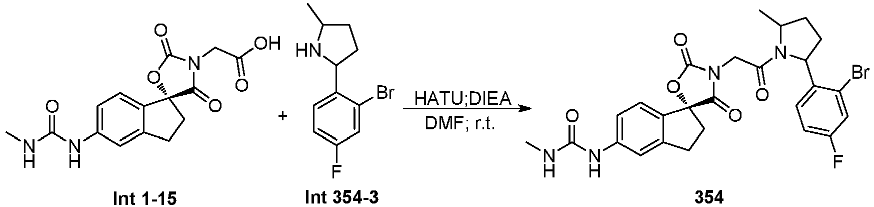

Intermediate **Int 1-15** (33 mg, 0.1 mmol) was dissolved in DMF (1 mL), to which were added DIEA (39 mg, 0.3 mmol) and HATU (38 mg, 0.01 mmol), followed by addition of **Int 354-3** (27 mg, 0.1 mmol), and the mixture was stirred at room temperature for 16 h. To the reaction solution was added water (10 mL), and extracted with ethyl acetate two times. The organic phase was washed with saturated brine, and concentrated to obtain the crude product, that was purified by column chromatography to obtain product **354** (29 mg) as off-white solid, with a yield of 50%. MS: *m*/*z* 573, 575 [M+H]⁺. ¹H NMR (400 MHz, CDCl₃): δ 7.64 - 7.26 (m, 3H), 7.28-6.82 (m, 3H), 5.27 (m, 1H), 4.77-4.05 (m, 2H), 3.82-3.36 (m, 1H), 3.18-2.89 (m, 1H), 2.84-2.40 (m, 4H), 2.30-1.85 (m, 3H), 1.86-1.15 (m, 6H).

**Example 355** Synthesis of compound 1-(3'-(2-(9-acetyl-1,9- 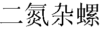 [5.5] 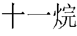 -1-yl)-2-oxoethyl)-2',4'-dioxo-2,3-dihydrospiro[indene-1,5'-oxazolidine]-5-yl)-3-methylurea (**355)**

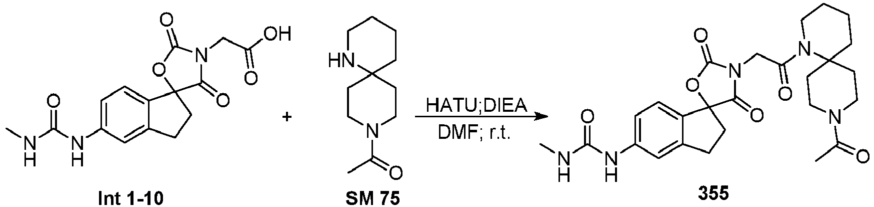

Intermediate **Int 1-15** (33 mg, 0.1 mmol) was dissolved in DMF (1 mL), to which were added DIEA (39 mg, 0.3 mmol) and HATU (38 mg, 0.01 mmol), followed by addition of **SM 75** (24 mg, 0.1 mmol), and the mixture was stirred at room temperature for 16 h. To the reaction solution was added water (10 mL), and extracted with ethyl acetate two times. The organic phase was washed with saturated brine, and concentrated to obtain the crude product, that was purified by column chromatography to obtain product **355** (26 mg) as off-white solid, with a yield of 50%. MS: *m*/*z* 512 [M+H]⁺. ¹H NMR (400 MHz, DMSO): δ 8.77 (s, 1H), 7.55 (m, 1H), 7.36-7.16 (m, 2H), 6.13 (d, J = 4.5 Hz, 1H), 4.43 (m, 2H), 3.70-3.55 (m, 1H), 3.47 (m, 3H), 3.34-3.27 (m, 1H), 3.23-2.96 (m, 3H), 2.82 (m, 1H), 2.73-2.56 (m, 5H), 2.00 (m, 3H), 1.69 (m, 6H), 1.41 (m, 2H), 1.23 (s, 1H).

### Example 356 Synthesis of 1-((1R)-3'-(2-(2-(4-fluoro-2-(trifluoromethyl)phenyl)- 5-methylpyrrolidin-1-yl)-2-oxoethyl)-2',4'-dioxo-2,3-dihydrospiro[indene-1, 5'-oxazolidine]-5-yl)-3-methylurea (356)

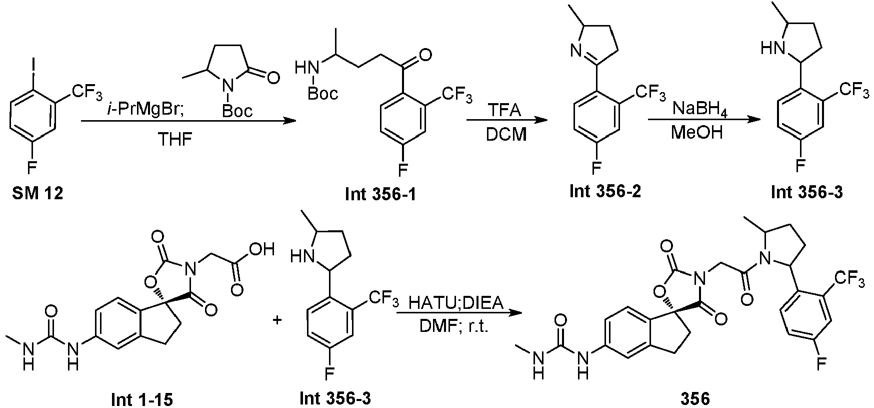

### Synthesis of intermediate (5-(2-trifluoromethyl-4-fluorophenyl)-5-oxopentan-2-yl) carbamic acid t-butyl ester (Int 356-1)

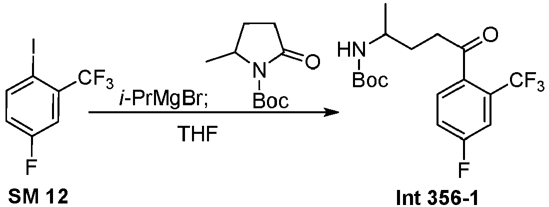

To a reaction flask, was added dry THF (30 mL), followed by addition of **SM 12** (2.9 g, 10 mmol), and then under N₂ protection, 1N isopropylmagnesium bromide (10 mL, 1M in THF) was added in an ice-water bath. After that, the mixture was reacted at room temperature for 7 h, and t-butyl 2-methyl-5-oxopyrrolidin-1-carboxylate (1.99 g, 10 mmol) was finally added. The mixture was further allowed to react at room temperature for 12 h. The reaction solution was poured into the saturated aqueous solution of ammonium chloride, and extracted with 30 mL (10 mL×3) of ethyl acetate. The organic phases were combined, dried over anhydrous Na₂SO₄, and purified by column chromatography to obtain **Int 356-1** (1.82 g, 5 mmol), with a yield of 50%. MS: *m*/*z* 364 [M+H]⁺.

### Synthesis of intermediate 5-(2-trifluoromethyl-4-fluorophenyl)-2-methyl-3,4-dihydro- 2H-pyrrole (Int 356-2)

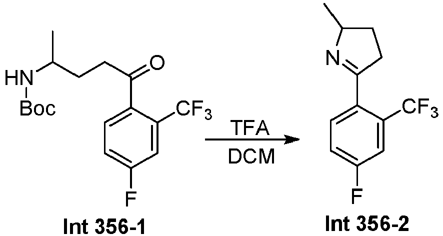

To a reaction solution was added DCM (20 mL), to which was then added Int 356-1 (1.82 g, 5 mmol), followed by addition of TFA (2 mL). After that, the mixture was reacted at room temperature for 7 h, and the reaction solution was poured into the saturated aqueous solution of sodium bicarbonate. The resultant solution was extracted with 30 mL (10 mL × 3) of DCM. The organic phases were combined, dried over anhydrous Na₂SO₄, and purified by column chromatography, to obtain **Int 356-2** (1.1 g, 4.5 mmol), with a yield of 90%. MS: *m*/*z* 246 [M+H]⁺.

### Synthesis of intermediate 2-(2-trifluoromethyl-4-fluorophenyl)-5-methylpyrrolidine (Int 356-3)

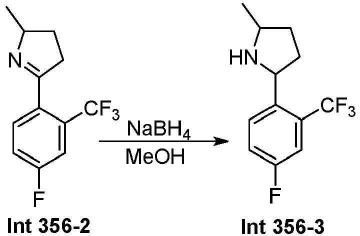

Methanol (15 mL) was added to a reaction flask, to which was then added **Int 356-2** (1.1 g, 4.5 mmol), followed by addition of sodium borohydride (0.76 g, 20 mmol) in batches. After that, the mixture was reacted at room temperature for 1 h. The reaction solution was poured into water, and extracted with 30 mL (10 mL×3) of EA. The organic phases were combined, dried over anhydrous Na₂SO₄, and concentrated to obtain **Int 356-3** (1.1 g, 4.4 mmol), with a yield of 98%. MS: *m*/*z* 248 [M+H]⁺.

### Synthesis of compound 1-((1R)-3'-(2-(2-(4-fluoro-2-(trifluoromethyl)phenyl)-5-methylpyrrolidin-1-yl)-2-oxoethyl)-2',4'-dioxo-2,3-dihydrospiro[indene-1,5'-oxazolidine]-5-yl)-3-methylurea (356)

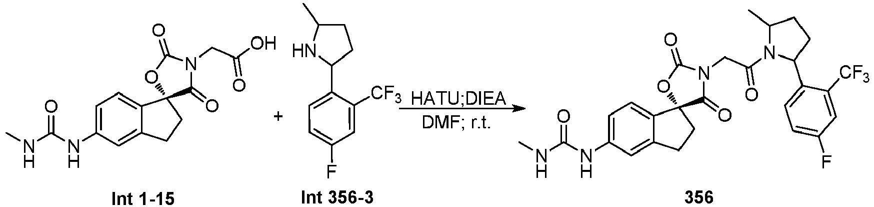

Intermediate **Int 1-15** (33 mg, 0.1 mmol) was dissolved in DMF (1 mL), to which were added DIEA (39 mg, 0.3 mmol) and HATU (38 mg, 0.01 mmol), followed by addition of **Int 356-3** (24 mg, 0.1 mmol), and the mixture was stirred at room temperature for 16 h. To the reaction solution was added water (10 mL), and extracted with ethyl acetate two times. The organic phase was washed with saturated brine, and concentrated to obtain the crude product, that was purified by column chromatography to obtain product **356** (28 mg) as off-white solid, with a yield of 50%. MS: *m*/*z* 563 [M+H]⁺. ¹H NMR (400 MHz, DMSO): δ 8.92-8.56 (s, 1H), 7.78-7.39 (m, 4H), 7.32-7.01 (m, 2H), 6.17-6.01 (m, 1H), 5.47-4.99 (m, 1H), 4.77-4.47 (m, 1H), 4.45-4.17 (m, 2H), 3.30 (m, 1H), 3.18-2.87 (m, 2H), 2.71-2.59 (m, 3H), 2.39 (m, 2H), 2.14 (m, 1H), 1.89-1.55 (m, 2H), 1.45 (m, 3H).

### Example 357 Synthesis of compound 1-((R)-3'-(2-((2S,5S)-2-cyclohexyl-5-methylpyrrolidin-1-yl)-2-oxoethyl)-2',4'-dioxo-2,3-dihydrospiro[indene-1, 5'-oxazolidine]-5-yl)-3-methylurea (357)

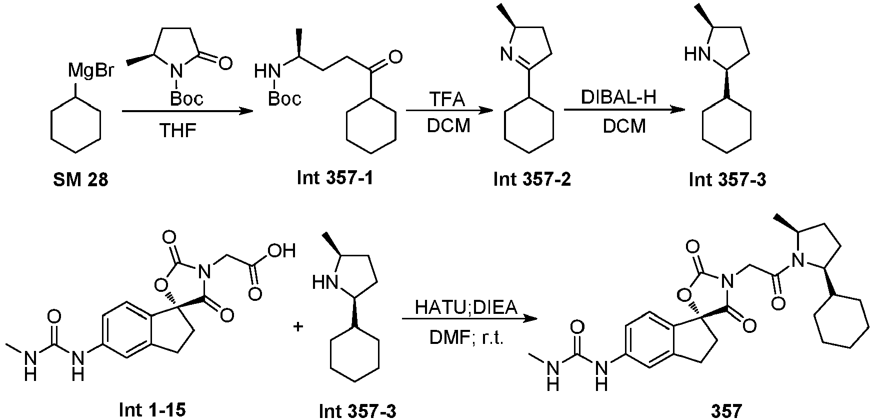

### Synthesis of intermediate (S)-(5-cyclohexyl-5-oxopentan-2-yl)carbamic acid t-butyl ester(Int 357-1)

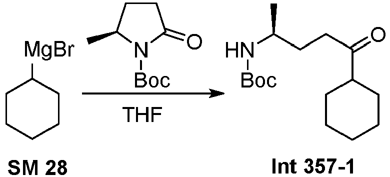

To a reaction solution was added dry THF (30 mL), to which was then added **SM 76** (10 mL, 10 mmol), and under nitrogen protection, (S)-2-methyl-5-oxopyrrolidin- 1-carboxylic acid t-butyl ester (2 g, 10 mmol) was added in an ice-water bath. After that, the mixture was reacted at room temperature for 12 h. The reaction solution was poured into the saturated aqueous solution of ammonium chloride, and extracted with 30 mL (10 mL×3) of ethyl acetate. The organic phases were combined, dried over anhydrous Na₂SO₄, and purified by column chromatography to obtain **Int 357-1** (1.7 g, 6 mmol), with a yield of 60%. MS: *m*/*z* 284 [M+H]⁺.

### Synthesis of intermediate (S)-5-cyclohexyl-2-methyl-3,4-dihydro-2H-pyrrole (Int 357-2)

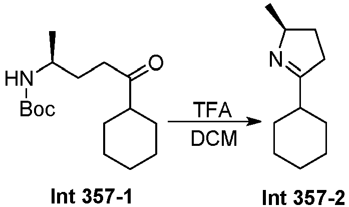

To a reaction solution was added DCM (20 mL), to which was then added **Int 357-1** (1.42 g, 5 mmol), followed by addition of TFA (2 mL). After that, the mixture was reacted at room temperature for 7 h, and the reaction solution was poured into the saturated aqueous solution of sodium bicarbonate. The resultant solution was extracted with 30 mL (10 mL × 3) of DCM. The organic phases were combined, dried over anhydrous Na₂SO₄, and purified by column chromatography, to obtain **Int 357-2** (701 mg, 4.25 mmol), with a yield of 85%. MS: *m*/*z* 166 [M+H]⁺.

### Synthesis of intermediate (2S,5S)-2-cyclohexyl-5-methylpyrrolidine (Int 357-3)

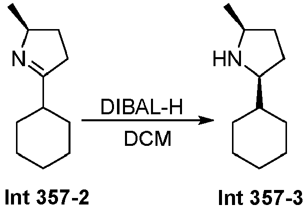

To a reaction flask was added DCM (15 mL), to which was then added **Int 357-2** (660 mg, 4 mmol), followed by addition of DIBAL-H (16 mL, 1M in DCM) in ice-water bath. After that, the mixture was reacted at room temperature for 1 h, and the reaction was quenched by adding water. The solution was filtered and washed with 30 mL (10 mL×3) of DCM. The organic phases were combined, dried over anhydrous Na₂SO₄, and concentrated, to obtain **Int 357-3** (618 mg, 3.7 mmol), with a yield of 94%. MS: *m*/*z* 168 [M+H]⁺.

### Synthesis of compound 1-((R)-3'-(2-((2S,5S)-2-cyclohexyl-5-methylpyrrolidin-1-yl)-2-oxoethyl)-2',4'-dioxo-2,3-dihydrospiro[indene-1,5'-oxazolidine]-5-yl)-3-methylurea (357)

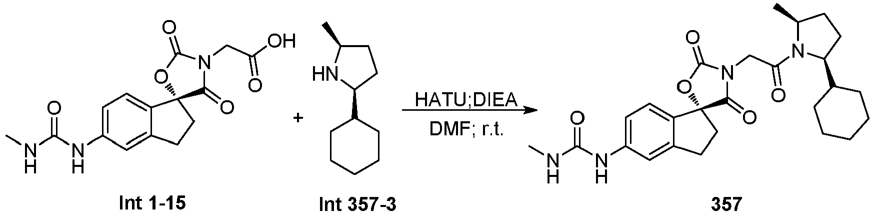

To a 25 mL reaction flask, was added DMF (3 mL), to which were then added **Int 1-15** (33.3 mg, 0.1 mmol), **SM 357-3** (16.7 mg, 0.1 mmol), DIEA (38.7 mg, 0.3 mmol), followed by addition of HATU (38.1 mg, 0.1 mmol). The system was reacted 1 h at 20 °C. After completion of the reation, the reaction solution was poured to water (20 mL), and the reasultant solution was extracted with 30 mL (10 mL×3) of ethyl acetate. The organic phases were combined, dried over anhydrous Na₂SO₄, and purified by column chromatography to obtain compound **357** (26.5 mg), with a yield of 55%. MS: *m*/*z* 483 [M+H]⁺.

### Example 359 Synthesis of compound 1-((1R)-3'-(2-(2-(3-chloro-4-fluorophenyl)- 5-methylpyrrolidin-1-yl)-2-oxoethyl)-2',4'-dioxo-2,3-dihydrospiro[indene-1, 5'-oxazolidine]-5-yl)-3-methylurea (359)

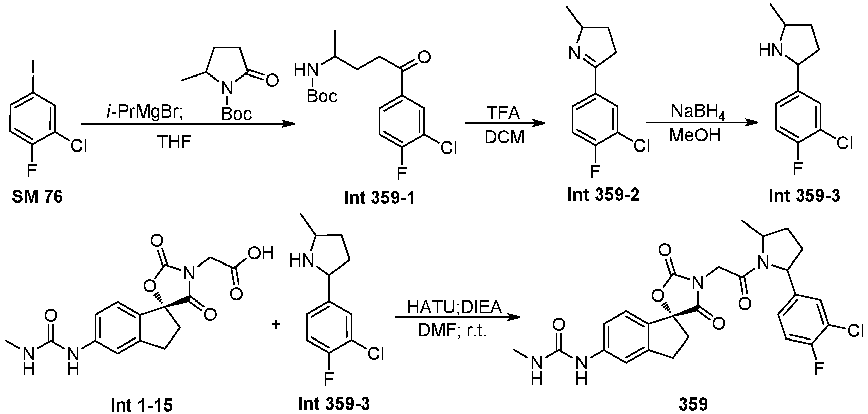

### Synthesis of intermediate (5-(3-chloro-4-fluorophenyl)-5-oxopentan-2-yl)carbamic acid t-butyl ester(Int 359-1)

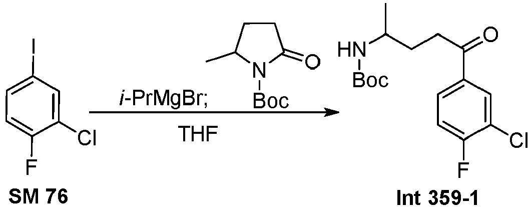

To a reaction flask, was added dry THF (30 mL), followed by addition of **SM 76** (2.58 g, 10 mmol), and then under N₂ protection, 1N isopropylmagnesium bromide (10 mL, 1M in THF) was added in an ice-water bath. After that, the mixture was reacted at room temperature for 7 h, and t-butyl 2-methyl-5-oxopyrrolidin-1-carboxylate (1.99 g, 10 mmol) was finally added. The mixture was further allowed to react at room temperature for 12 h. The reaction solution was poured into the saturated aqueous solution of ammonium chloride, and extracted with 30 mL (10 mL×3) of ethyl acetate. The organic phases were combined, dried over anhydrous Na₂SO₄, and purified by column chromatography to obtain **Int 359-1** (1.66 g, 5 mmol), with a yield of 50%. MS: *m*/*z* 330, 331 [M+H]⁺.

### Synthesis of intermediate 5-(3-chloro-4-fluorophenyl)-2-methyl-3,4-dihydro-2H- pyrrole (Int 359-2)

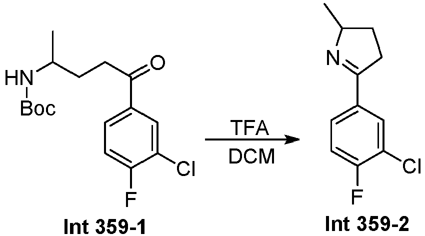

To a reaction solution was added DCM (20 mL), to which was then added **Int 359-1** (1.66 g, 5 mmol), followed by addition of TFA (2 mL). After that, the mixture was reacted at room temperature for 7 h, and the reaction solution was poured into the saturated aqueous solution of sodium bicarbonate. The resultant solution was extracted with 30 mL (10 mL × 3) of DCM. The organic phases were combined, dried over anhydrous Na₂SO₄, and purified by column chromatography, to obtain **Int 359-2** (0.95 g, 4.5 mmol), with a yield of 90%. MS: *m*/*z* 212, 213 [M+H]⁺.

### Synthesis of intermediate 2-(3-chloro-4-fluorophenyl)-5-methylpyrrolidine (Int 359-3)

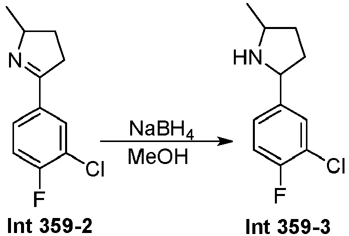

Methanol (15 mL) was added to a reaction flask, to which was then added **Int 359-2** (0.95 g, 4.5 mmol), followed by addition of sodium borohydride (0.76 g, 20 mmol) in batches. After that, the mixture was reacted at room temperature for 1 h. The reaction solution was poured into water, and extracted with 30 mL (10 mL×3) of EA. The organic phases were combined, dried over anhydrous Na₂SO₄, and concentrated to obtain **Int 359-3** (0.95 g, 4.4 mmol), with a yield of 98%. MS: *m*/*z* 214, 215 [M+H]⁺.

### Synthesis of compound 1-((1R)-3'-(2-(2-(3-chloro-4-fluorophenyl)-5- methylpyrrolidin-1-yl)-2-oxoethyl)-2',4'-dioxo-2,3-dihydrospiro[indene-1,5'-oxazolidine]-5-yl)-3-methylurea (359)

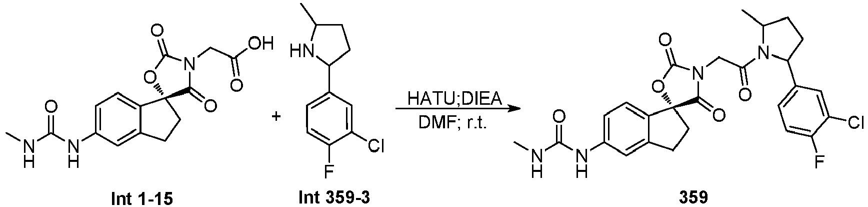

Intermediate **Int 1-15** (33 mg, 0.1 mmol) was dissolved in DMF (1 mL), to which were added DIEA (39 mg, 0.3 mmol) and HATU (38 mg, 0.01 mmol), followed by addition of **Int 359-3** (24 mg, 0.1 mmol), and the mixture was stirred at room temperature for 16 h. To the reaction solution was added water (10 mL), and extracted with ethyl acetate two times. The organic phase was washed with saturated brine, and concentrated to obtain the crude product, that was purified by column chromatography to obtain product **359** (25 mg) as off-white solid, with a yield of 50%. MS: *m*/*z* 529, 530 [M+H]⁺. ¹H NMR (400 MHz, CDCl₃): δ 7.61-7.45 (m, 1H), 7.44-7.25 (m, 2H), 7.16-6.77 (m, 3H), 5.42-5.22 (m, 1H), 4.96-4.78 (m, 1H), 4.54-4.20 (m, 1H), 3.67 (m, 1H), 3.10-2.94 (m, 1H), 2.92-2.84 (m, 1H), 2.62(s, 3H), 2.37 (m, 2H), 2.05-1.74 (m, 2H), 1.62-1.48 (m, 1H), 1.45-1.30 (m, 3H), 1.23-1.13 (m, 1H).

### Example 360 Synthesis of compound 1-((R)-3'-(2-((R)-2-(4-fluorophenyl)-5-(hydroxymethyl)pyrrolidin-1-yl)-2-oxoethyl)-2',4'-dioxo-2,3-dihydrospiro[indene-1,5'-oxazolidine]-5-yl)-3-methylurea (360)

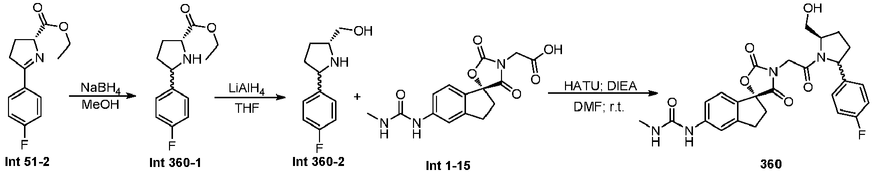

### Synthesis of intermediate (R)-5-(4-fluorophenyl)pyrrolidin-2-carboxylic acid ethyl ester (Int 360-1)

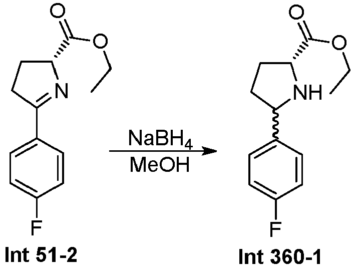

**Int 51-2** (1.06 g, 4.5 mmol) was introduced into the reaction flask containing methanol (10 mL), to which was added sodium borohydride (0.68 g, 18 mmol) in batches under stirring at room temperature. After that, the reaction was still stirred at room temperature and detected by TLC. After completion of the reaction, the reaction solution was poured to water, and extracted with 30 mL (10 mL×3) of EA. The organic phase was combined, dried over anhydrous Na₂SO₄, and separated by column chromatography, to obtain **Int 360-1** (0.91 g), with a yield of 85%. MS: *m*/*z* 238 [M+H]⁺.

### Synthesis of intermediate (R)-(5-(4-fluorophenyl)pyrrolidin-2-yl)methanol (Int 360-2)

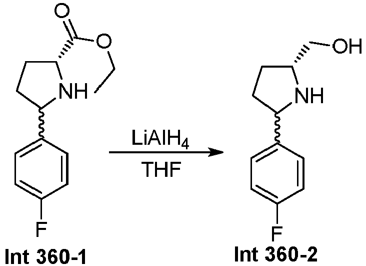

**Int 360-1** (0.91 g, 3.8 mmol) was added to the reaction flask containing THF (10 mL), to which was added lithium aluminum hydride (0.58 g, 15.3 mmol) in batches under stirring at room temperature. After addition, the reaction was still stirred at room temperature and detected by TLC. After completion of the reaction, Na₂SO₄·10H₂O was added to the reaction solution. The resultant solution was stirred, filtered, dried over anhydrous sodium sulfate, and evaporated under reduced pressure, to obtain **Int 360-2** (0.44 g), with a yield of 60%. MS: *m*/*z* 196 [M+H]⁺.

### Synthesis of compound 1-((R)-3'-(2-((R)-2-(4-fluorophenyl)-5-(hydroxymethyl) pyrrolidin-1-yl)-2-oxoethyl)-2',4'-dioxo-2,3-dihydrospiro[indene-1,5'-oxazolidine]-5-yl)-3-methylurea (360)

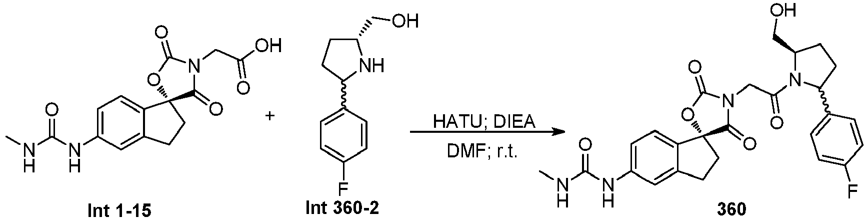

Intermediate **Int 1-15** (30 mg, 0.09 mmol) was dissolved in DMF (1 mL), to which were added DIEA (35 mg, 0.027 mmol) and HATU (41 mg, 0.011 mmol), followed by addition of starting material **Int 347-3** (26 mg, 0.135 mmol), and the mixture was stirred at room temperature for 16 h. To the reaction solution was added water (10 mL), and extracted with ethyl acetate two times. The organic phase was washed with saturated brine, and concentrated to obtain the crude product, that was purified by column chromatography to obtain product **360** (36 mg) as off-white solid, with a yield of 78%. MS: *m*/*z* 511 [M+H]⁺. ¹H NMR (400 MHz, CDCl₃): δ 7.55-7.45 (m, 2H), 7.30 (dd, J = 11.2, 6.8 Hz, 2H), 7.09 (dd, J = 15.5, 7.0 Hz, 2H), 6.96-6.90 (m, 1H), 5.33 (d, J = 4.5 Hz, 1H), 5.03 (d, J = 6.3 Hz, 1H), 4.33 (dd, J = 12.4, 6.8 Hz, 1H), 4.25 (d, J = 16.5 Hz, 1H), 3.86-3.71 (m, 3H), 3.17-3.07 (m, 1H), 2.99 (dd, J = 13.4, 5.4 Hz, 2H), 2.81 (s, 2H), 2.74 (d, J = 4.2 Hz, 3H), 2.43 (d, J = 7.3 Hz, 1H), 2.00 (d, J = 12.9 Hz, 1H), 1.83 (d, J = 6.5 Hz, 1H), 1.76-1.67 (m, 1H), 1.25 (s, 1H).

### Example 361 Synthesis of compound 1-(3'-(2-((2S,5R)-2-(4-fluorophenyl)-5-(hydroxymethyl)pyrrolidin-1-yl)-2-oxoethyl)-2',4'-dioxo-2,3-dihydrospiro[indene-1,5'-oxazolidine]-5-yl)-3-methylurea (361)

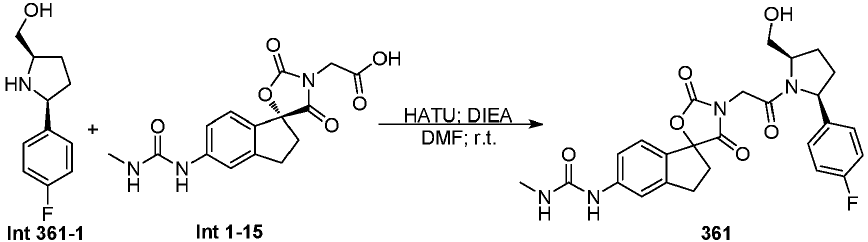

Intermediate **Int 1-15** (30 mg, 0.09 mmol) was dissolved in DMF (1 mL), to which were added DIEA (35 mg, 0.027 mmol) and HATU (41 mg, 0.011 mmol), followed by addition of starting material **Int 361-1** (26 mg, 0.135 mmol), and the mixture was stirred at room temperature for 16 h. To the reaction solution was added water (10 mL), and extracted with ethyl acetate two times. The organic phase was washed with saturated brine, and concentrated to obtain the crude product, that was purified by column chromatography to obtain product **361** (33 mg) as off-white solid, with a yield of 71%. MS: *m*/*z* 511 [M+H]⁺. ¹H NMR (400 MHz, CDCl₃): δ 7.55-7.45 (m, 2H), 7.30 (dd, J = 11.2, 6.8 Hz, 2H), 7.09 (dd, J = 15.5, 7.0 Hz, 2H), 6.96-6.90 (m, 1H), 5.33 (d, J = 4.5 Hz, 1H), 5.03 (d, J = 6.3 Hz, 1H), 4.33 (dd, J = 12.4, 6.8 Hz, 1H), 4.25 (d, J = 16.5 Hz, 1H), 3.86-3.71 (m, 3H), 3.17-3.07 (m, 1H), 2.99 (dd, J = 13.4, 5.4 Hz, 2H), 2.81 (s, 2H), 2.74 (d, J = 4.2 Hz, 3H), 2.43 (d, J = 7.3 Hz, 1H), 2.00 (d, J = 12.9 Hz, 1H), 1.83 (d, J = 6.5 Hz, 1H), 1.76-1.67 (m, 1H), 1.25 (s, 1H).

### Example 362 Synthesis of compound 1-(3'-(2-((2S,5R)-2-(4-fluorophenyl)-5-(methoxymethyl)pyrrolidin-1-yl)-2-oxoethyl)-2',4'-dioxo-2,3-dihydrospiro[indene-1,5'-oxazolidine]-5-yl)-3-methylurea (362)

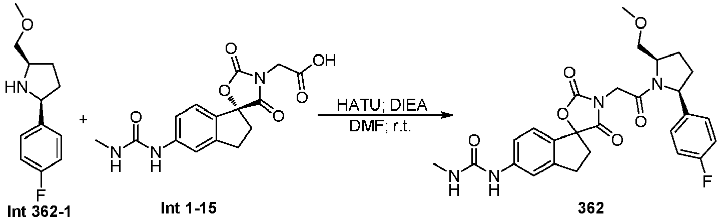

### Synthesis of compound 1-(3'-(2-((2S,5R)-2-(4-fluorophenyl)-5-(methoxymethyl) pyrrolidin-1-yl)-2-oxoethyl)-2',4'-dioxo-2,3-dihydrospiro[indene-1,5'-oxazolidine]-5-yl)-3-methylurea (362)

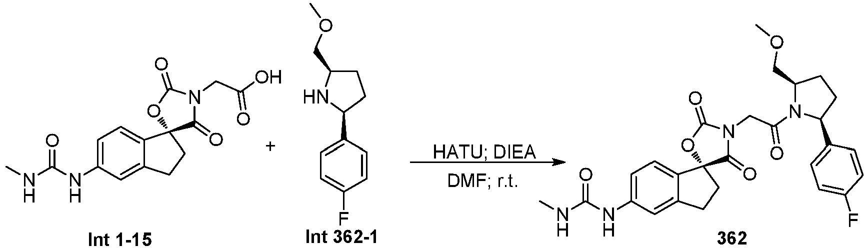

Intermediate **Int 1-15** (30 mg, 0.09 mmol) was dissolved in DMF (1 mL), to which were added DIEA (35 mg, 0.027 mmol) and HATU (41 mg, 0.011 mmol), followed by addition of **Int 362-1** (27 mg, 0.135 mmol), and the mixture was stirred at room temperature for 16 h. To the reaction solution was added water (10 mL), and extracted with ethyl acetate two times. The organic phase was washed with saturated brine, and concentrated to obtain the crude product, that was purified by column chromatography to obtain product **362** (36 mg) as off-white solid, with a yield of 76%. MS: *m*/*z* 525 [M+H]⁺. ¹H NMR (400 MHz, CDCl₃): δ 7.55-7.45 (m, 2H), 7.30 (dd, J = 11.2, 6.8 Hz, 2H), 7.09 (dd, J = 15.5, 7.0 Hz, 2H), 6.96-6.90 (m, 1H), 5.33 (d, J = 4.5 Hz, 1H), 5.03 (d, J = 6.3 Hz, 1H), 4.33 (dd, J = 12.4, 6.8 Hz, 1H), 4.25 (d, J = 16.5 Hz, 1H), 3.86-3.71 (m, 2H), 3.31(s, 3H), 3.17-3.07 (m, 1H), 2.99 (dd, J = 13.4, 5.4 Hz, 2H), 2.81 (s, 2H), 2.74 (d, J = 4.2 Hz, 3H), 2.43 (d, J = 7.3 Hz, 1H), 2.00 (d, J = 12.9 Hz, 1H), 1.83 (d, J = 6.5 Hz, 1H), 1.76-1.67 (m, 1H), 1.25 (s, 1H).

### Example 363 Synthesis of compound 1-((1R)-3'-(2-((5S)-2-(4-fluorophenyl)-5-(hydroxymethyl)pyrrolidin-1-yl)-2-oxoethyl)-2',4'-dioxo-2,3-dihydrospiro[indene-1,5'-oxazolidine]-5-yl)-3-methylurea (363)

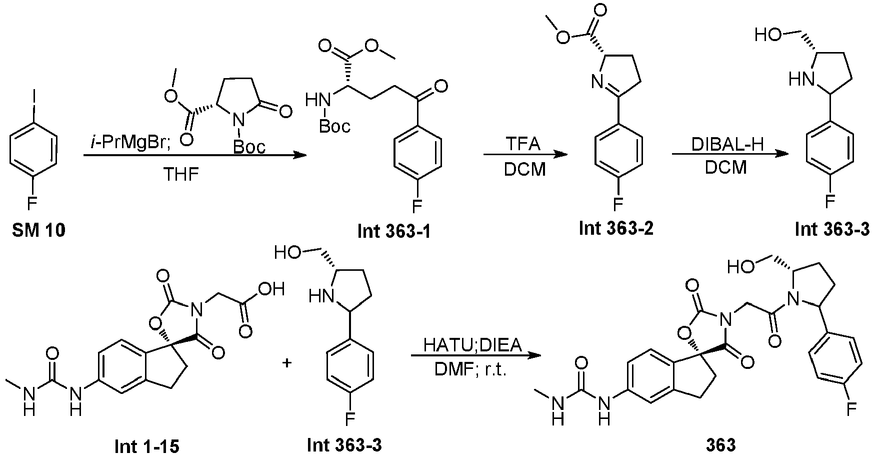

### Synthesis of intermediate (S)-methyl-2-((t-butoxycarbonyl)amino)-5-(4- fluorophenyl)-5-oxovalerate (Int 363-1)

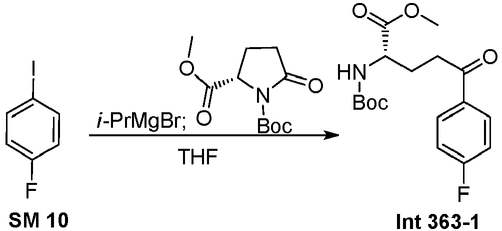

To a reaction flask, was added dry THF (30 mL), followed by addition **of SM 10** (2.22 g, 10 mmol), and then under N₂ protection, 1N isopropylmagnesium bromide (10 mL, 1M in THF) was added in an ice-water bath. After that, the mixture was reacted at room temperature for 7 h, and (S)-1-butyl 2-methyl-5-oxopyrrolidin-1,2-dicarboxylate (2.43 g, 10 mmol) was finally added. The mixture was further allowed to react at room temperature for 12 h. The reaction solution was poured into the saturated aqueous solution of ammonium chloride, and extracted with 30 mL (10 mL×3) of ethyl acetate. The organic phases were combined, dried over anhydrous Na₂SO₄, and purified by column chromatography to obtain **Int 363-1** (1.7 g, 5 mmol), with a yield of 50%. MS: *m*/*z* 340 [M+H]⁺.

### Synthesis of intermediate (S)-methyl-5-(4-fluorophenyl)-3,4-dihydro-2H-pyrrole-2-carboxylate (Int 363-2)

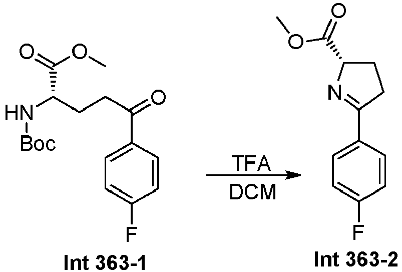

To a reaction solution was added DCM (20 mL), to which was then added **Int 363-1** (1.7 g, 5 mmol), followed by addition of TFA (2 mL). After that, the mixture was reacted at room temperature for 7 h, and the reaction solution was poured into the saturated aqueous solution of sodium bicarbonate. The resultant solution was extracted with 30 mL (10 mL × 3) of DCM. The organic phases were combined, dried over anhydrous Na₂SO₄, and purified by column chromatography, to obtain **Int 363-2** (1 g, 4.5 mmol), with a yield of 90%. MS: *m*/*z* 222 [M+H]⁺.

### Synthesis of intermediate ((2S)-5-(4-fluorophenyl)pyrrolidin-2-yl)methanol

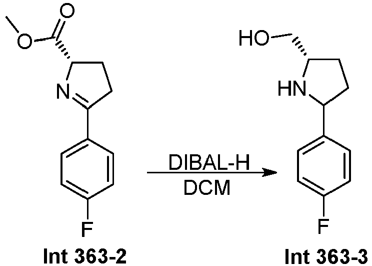

To a reaction flask was added DCM (15 mL), to which was then added **Int 363-2** (1 g, 4.5 mmol). Under nitrogen protection, the temperature was lowered to -78 °C, and DIBAH (36 mL, 1M in DCM) was added to the flask, and then the resultant mixture was gradually warmed to room temperature and reacted for 15 h. The reaction solution was poured into an ice-cold 1N aqueous solution of sodium hydroxide, extracted with DCM. The organic phases were combined, dried over anhydrous sodium sulfate, and concentrated to obtain **Int 363-3** (0.86 g, 4.4 mmol), with a yield of 98%. MS: *m*/*z* 196 [M+H]⁺.

### Synthesis of compound 1-((1R)-3'-(2-((5S)-2-(4-fluorophenyl)-5-(hydroxymethyl) pyrrolidin-1-yl)-2-oxoethyl)-2',4'-dioxo-2,3-dihydrospiro[indene-1,5'-oxazolidine]-5-yl)-3-methylurea (363)

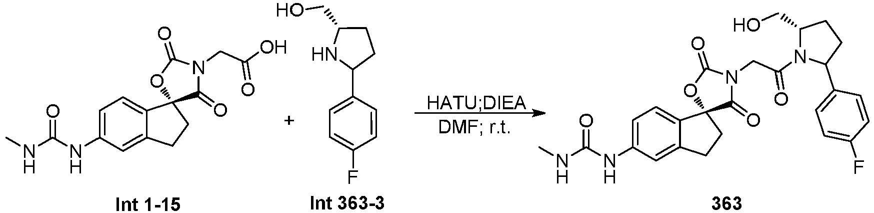

Intermediate **Int 1-15** (33 mg, 0.1 mmol) was dissolved in DMF (1 mL), to which were added DIEA (39 mg, 0.3 mmol) and HATU (38 mg, 0.1 mmol), followed by addition of **Int 363-3** (24 mg, 0.1 mmol), and the mixture was stirred at room temperature for 16 h. To the reaction solution was added water (10 mL), and extracted with ethyl acetate two times. The organic phase was washed with saturated brine, and concentrated to obtain the crude product, that was purified by column chromatography to obtain product **363** (26 mg), with a yield of 50%. MS: *m*/*z* 511 [M+H]⁺. ¹H NMR (400 MHz, CDCl₃): δ 7.54-7.29 (m, 3H), 7.16-7.04 (m, 2H), 6.91 (m, 1H), 5.38 (m 1H), 5.10-4.96 (m, 1H), 4.55-4.27 (m, 2H), 3.86-3.76 (m, 2H), 3.19-2.86 (m, 2H), 2.81 (s, 3H), 2.78-2.65 (m, 3H), 2.52-2.32 (m, 2H), 2.04 (m, 2H), 1.26 (m, 1H).

### Example 365 Synthesis of compound 1-((1R)-3'-(2-(5-(4-fluorophenyl)-2,2-dimethylpyrrolidin-1-yl)-2-oxoethyl)-2',4'-dioxo-2,3-dihydrospiro[indene-1,5'-oxazolidine]-5-yl)-3-methylurea (365)

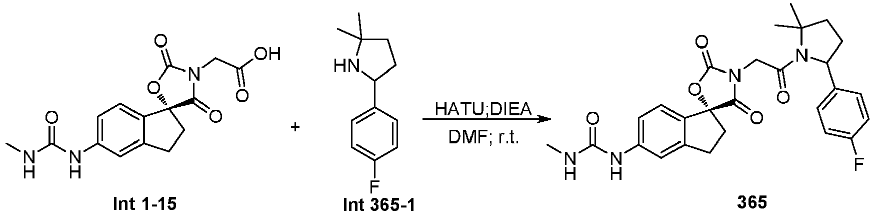

To a 25 mL reaction flask, was added DMF (3 mL), to which were then added **Int 1-15** (33.3 mg, 0.1 mmol), **SM 365-1** (19.3 mg, 0.1 mmol), DIEA (38.7 mg, 0.3 mmol), followed by addition of HATU (38.1 mg, 0.1 mmol). The system was reacted 1 h at 20 °C. After completion of the reation, the reaction solution was poured to water (20 mL), and the reasultant solution was extracted with 30 mL (10 mL×3) of ethyl acetate. The organic phases were combined, dried over anhydrous Na₂SO₄, and purified by column chromatography to obtain compound 365 (28 mg), with a yield of 55%. MS: *m*/*z* 509 [M+H]⁺.

### Example 366 Synthesis of compound 1-((1R)-3'-(2-(5-(3,4-difluorophenyl)-2,2-dimethylpyrrolidin-1-yl)-2-oxoethyl)-2',4'-dioxo-2,3-dihydrospiro[indene-1,5'-oxazolidine]-5-yl)-3-methylurea (366)

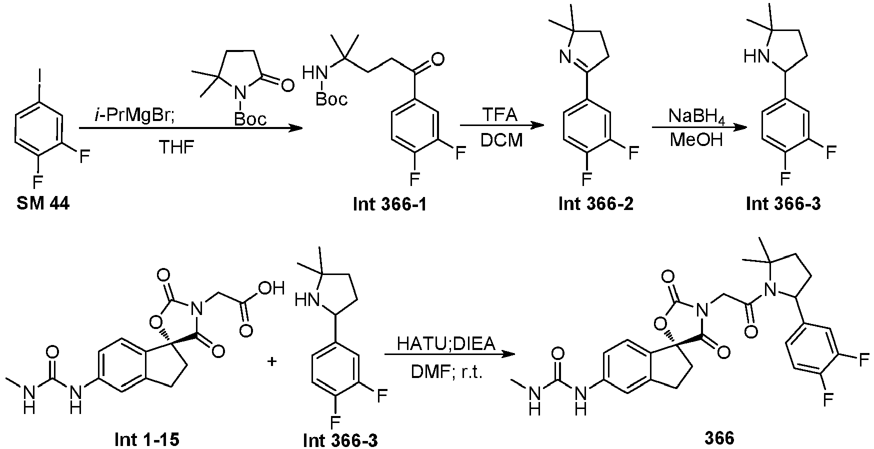

### Synthesis of intermediate (5-(3,4-difluorophenyl)-2-methyl-5-oxopentan-2-yl) carbamic acid t-butyl ester (Int 366-1)

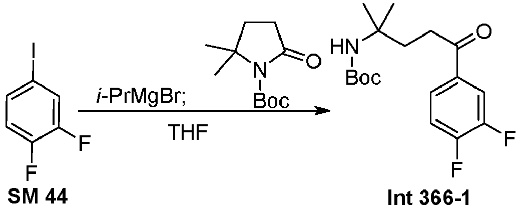

To a reaction flask, was added dry THF (30 mL), followed by addition of **SM 44** (2.4 g, 10 mmol), and then under N₂ protection, 1N isopropylmagnesium bromide (10 mL, 1M in THF) was added in an ice-water bath. After that, the mixture was reacted at room temperature for 7 h, and **Int 39-3** (2.13 g, 10 mmol) was finally added. The mixture was further allowed to react at room temperature for 12 h. The reaction solution was poured into the saturated aqueous solution of ammonium chloride, and extracted with 30 mL (10 mL×3) of ethyl acetate. The organic phases were combined, dried over anhydrous Na₂SO₄, and purified by column chromatography to obtain **Int 366-1** (1.8 g, 5.5 mmol), with a yield of 55%. MS: *m*/*z* 328 [M+H]⁺.

### Synthesis of intermediate 5-(3,4-difluorophenyl)-2,2-dimethyl-3,4-dihydro-2H- pyrrole (Int 366-2)

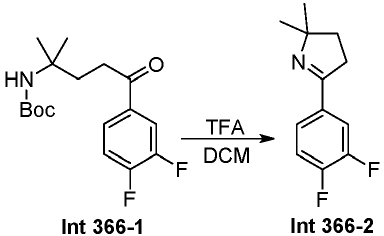

To a reaction solution was added DCM (20 mL), to which was then added **Int 366-1** (1.64 g, 5 mmol), followed by addition of TFA (2 mL). After that, the mixture was reacted at room temperature for 7 h, and the reaction solution was poured into the saturated aqueous solution of sodium bicarbonate. The resultant solution was extracted with 30 mL (10 mL × 3) of DCM. The organic phases were combined, dried over anhydrous Na₂SO₄, and purified by column chromatography, to obtain **Int 366-2** (941 mg, 4.5 mmol), with a yield of 90%. MS: *m*/*z* 210 [M+H]⁺.

### Synthesis of intermediate 5-(3,4-difluorophenyl)-2,2-dimethylpyrrolidine (Int 366-3)

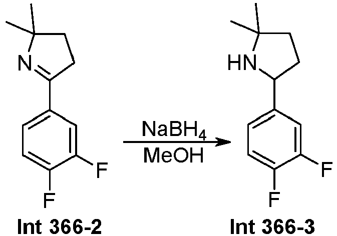

Methanol (15 mL) was added to a reaction flask, to which was then added **Int 366-2** (836 g, 4 mmol), followed by addition of sodium borohydride (0.76 g, 20 mmol) in batches. After that, the mixture was reacted at room temperature for 1 h. The reaction solution was poured into water, and extracted with 30 mL (10 mL×3) of EA. The organic phases were combined, dried over anhydrous Na₂SO₄, and concentrated to obtain **Int 366-3** (802 mg, 3.8 mmol), with a yield of 95%. MS: *m*/*z* 212 [M+H]⁺.

### Synthesis of compound 1-((1R)-3'-(2-(5-(3,4-difluorophenyl)-2,2-dimethylpyrrolidin- 1-yl)-2-oxoethyl)-2',4'-dioxo-2,3-dihydrospiro[indene-1,5'-oxazolidine]-5-yl)-3-methylurea (366)

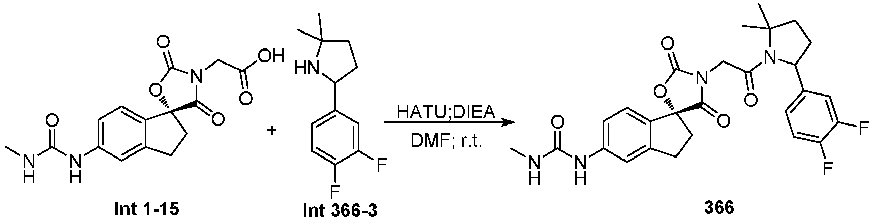

To a 25 mL reaction flask, was added DMF (3 mL), to which were then added **Int 1-15** (33.3 mg, 0.1 mmol), **SM 366-3** (21.1 mg, 0.1 mmol), DIEA (38.7 mg, 0.3 mmol), followed by addition of HATU (38.1 mg, 0.1 mmol). The system was reacted 1 h at 20 °C. After completion of the reation, the reaction solution was poured to water (20 mL), and the reasultant solution was extracted with 30 mL (10 mL×3) of ethyl acetate. The organic phases were combined, dried over anhydrous Na₂SO₄, and purified by column chromatography to obtain compound **366** (26.3 mg), with a yield of 50%. MS: *m*/*z* 527 [M+H]⁺. ¹H NMR (400 MHz, CDCl₃): δ 7.51 (s, 1H), 7.29 (t, J = 8.2 Hz, 1H), 7.19-7.00 (m, 5H), 6.90 (s, 1H), 5.35 (d, J = 8.6 Hz, 1H), 5.13 (s, 1H), 4.35-3.66 (m, 2H), 3.22-2.90 (m, 2H), 2.77 (d, J = 8.9 Hz, 3H), 2.50 (m, 2H), 1.99-1.89 (m, 2H), 1.79 (m, 1H), 1.49 (d, J = 5.8 Hz, 6H).

### Example 367 Synthesis of compound 1-((R)-3'-(2-((2S,5R)-2-(4-fluorophenyl)-5-vinylpyrrolidin-1-yl)-2-oxoethyl)-2',4'-dioxo-2,3-dihydrospiro[indene-1,5'-oxazolidine]-5-yl)-3-methylurea (367)

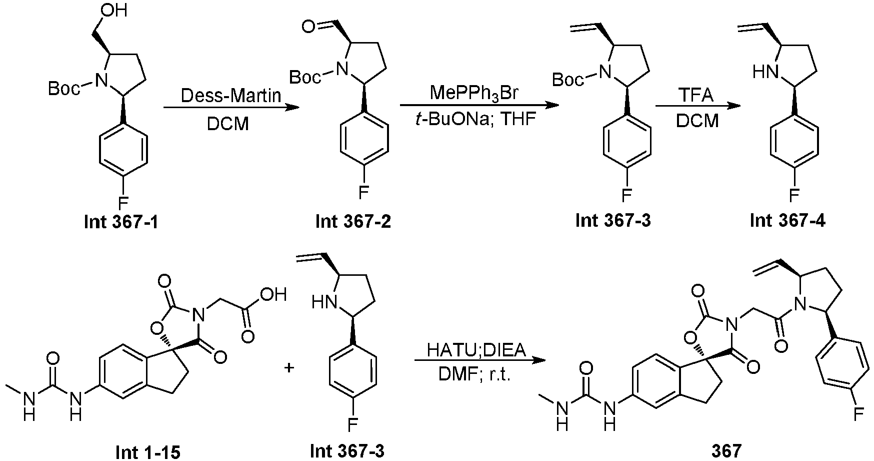

### Synthesis of intermediate (2S,5R)-2-(4-fluorophenyl)-5-formylpyrrolidin- 1-carboxylic acid t-butyl ester (Int 367-1)

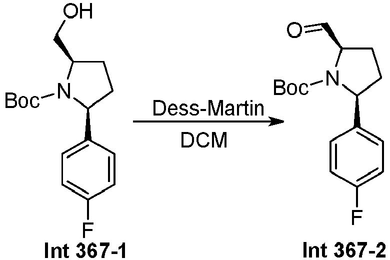

To a 25 mL reaction flask, were added **Int 367-1** (400 mg, 1.4 mmol) and dichloromethane (5 mL), and then Dess-Martin reagent (579 mg, 1.4 mmol) was finally added in batches. The system reacted overnight at room temperature. After completion of the reaction, the solution was filtered, and washed with NaHCO₃ aqueous solution. The organic layer was dried over anhydrous Na₂SO₄, and purified by column chromatography to obtain compound **Int 367-2** (375 mg), with a yield of 94%. MS: *m*/*z* 294.2 [M+H]⁺.

### Synthesis of intermediate (2S,SR)-2-(4-fluorophenyl)-5-vinylpyrrolidin-1-carboxylic acid t-butyl ester (Int 367-2)

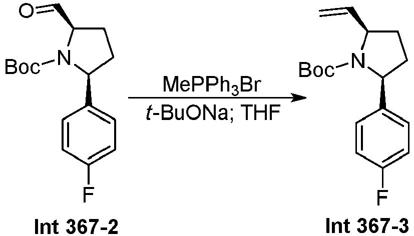

To a 25 mL reaction flask, were added methyltriphenylphosphonium bromide (146 mg, 0.41 mmol) and THF (3 mL), to which was added sodium tert-butoxide (44 mg, 0.45 mmol) in batches in an ice bath. After reacting for about 1 h, **Int 367-2** (100 mg, 0.34 mmol) was added to the reaction solution, and the mixture was reacted at room temperature overnight. Water (5 mL) was added to the reaction flask, and the resultant solution was extracted with ethyl acetate (5 mL×3). The organic phases were combined, dried over anhydrous Na₂SO₄, and purified by column chromatography, to obtain compound **Int 367-3** (33 mg), with a yield of 33%. MS: *m*/*z* 292.2 [M+H]⁺.

### Synthesis of intermediate ((2S,5R)-2-(4-fluorophenyl)-5-vinylpyrrolidine ( Int 367-4)

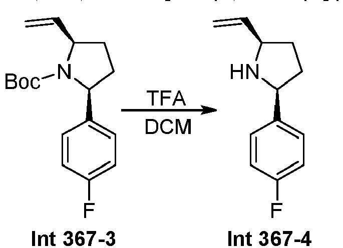

To a 25 mL reaction flask, were added **Int 367-3** (33 mg, 0.12 mmol) and dichloromethane (0.5 mL), and finally TFA (1 mL) was added. The mixture was reacted for about 3 h. After completion of the reaction, the solvent was evaporated *in vacuum.* Dichloromethane (5 mL) was added to the residue, and the resultant solution was washed with aqueous NaHCO₃ solution. The organic layer was dried over anhydrous sodium sulfate, and the solvent was rotatory evaporated under vacuum, to obtain crude **Int 367-4** (13 mg), with a yield of 59%. MS: *m*/*z* 192.2 [M+H]⁺.

### Synthesis of compound 1-((R)-3'-(2-((2S,SR)-2-(4-fluorophenyl)-5-vinylpyrrolidin- 1-yl)-2-oxoethyl)-2',4'-dioxo-2,3-dihydrospiro[indene-1,5'-oxazolidine]-5-yl)-3-methylurea (367)

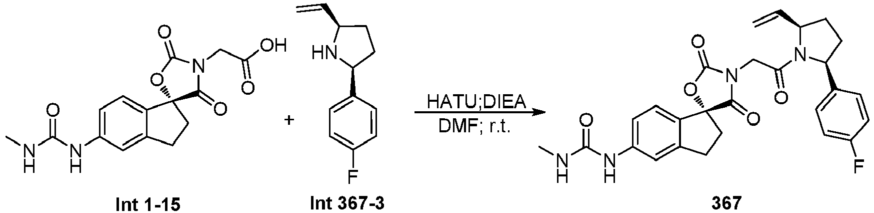

To a 25 mL reaction flask, was added DMF (1 mL), to which were then added **Int 1-15** (23 mg, 0.07 mmol), **Int 367-3** (13 mg, 0.07 mmol), DIEA (27 mg, 0.21 mmol), followed by addition of HATU (42 mg, 0.11 mmol). The system was reacted overnight at room temperature. After completion of the reation, water (5 mL) was added to the reaction solution, and the reasultant solution was extracted with ethyl acetate (5 mL×3). The organic phases were combined, dried over anhydrous Na₂SO₄, and purified by column chromatography to obtain compound **367** (9 mg), with a yield of 25%. MS: *m*/*z* 507.2 [M+H]⁺.

### Example 368 Synthesis of compound (R)-1-(2',4'-dioxo-3'-(2-oxo-2-(2,2,6,6-tetramethylpiperazine-1-yl)ethyl)-2,3 -dihydrospiro [indene-1, 5' -oxazolidine -5 -yl)-3 - methylurea (368)

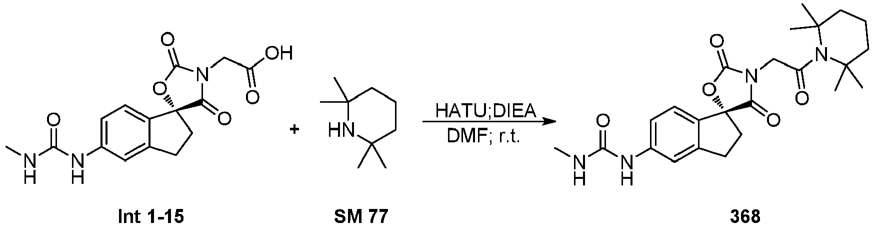

Intermediate **Int 1-15** (33 mg, 0.1 mmol), **SM** 77 (15 mg, 0.11 mmol), HATU (50 mg, 0.13 mmol), and DIEA (20 mg, 0.15 mmol) were dissolved in dichloromethane (5 mL), and the mixture was stirred overnight. The reaction solution was concentrated and subjected to column chromatography, to obtain pale yellow solid **368** (35 mg), with a yield of 77%. MS: *m*/*z* 457 [M+H]⁺.

### Example 369 Synthesis of compound 1-((R)-3'-(2-((2R,5S)-2-(3-chloro-4- fluorophenyl)-5-methylpyrrolidin-1-yl)-2-oxoethyl)-2',4'-dioxo-2,3-dihydrospiro[indene-1,5'-oxazolidine]-5-yl)-3-methylurea (369)

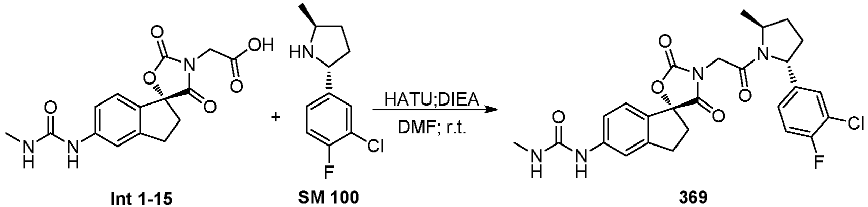

DMF (3 mL) was added to a 25 mL reaction flask, to which were then added **Int 1-15** (333 mg, 1 mmol), **Int 213-4** (213 mg, 1 mmol), and DIEA (387 mg, 3 mmol), and finally HATU (381 mg, 1 mmol) was added. The system was reacted at 20 °C for 1 h. After completion of the reaction, the reaction solution was poured into water (20 mL), and extracted with 30 mL (10 mL×3) ethyl acetate. The organic phases were combined, dried over anhydrous Na₂SO₄, and purified by column chromatography to obtain compound **369** (270 mg), with a yield of 59%. MS: *m*/*z* 529 [M+H]⁺.

### Example 370 Synthesis of compound 1-((R)-3'-(2-((5S)-2-(2,4-difluorophenyl)- 5-methylpyrrolidin-1-yl)-2-oxoethyl)-2',4'-dioxo-2,3-dihydrospiro[indene-1, 5'-oxazolidine]-5-yl)-3-methylurea (370)

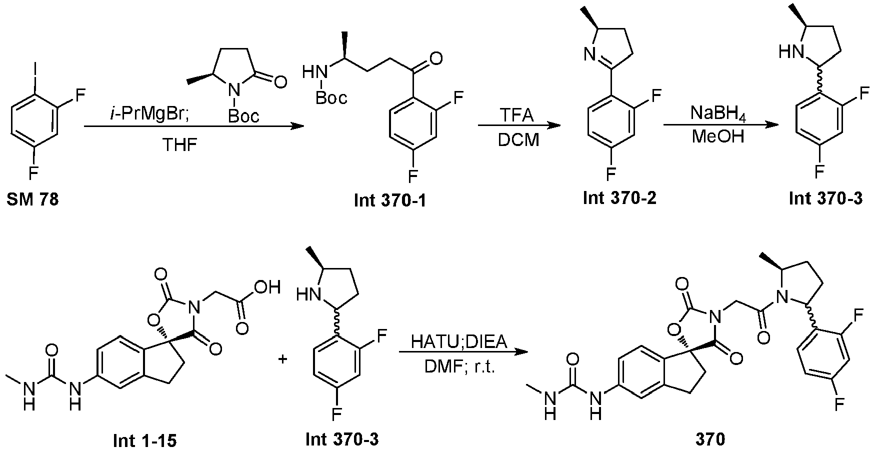

### Synthesis of intermediate (S)-(5-(2,4-difluorophenyl)-5-oxopentan-2-yl)carbamic acid t-butyl ester(Int 370-1)

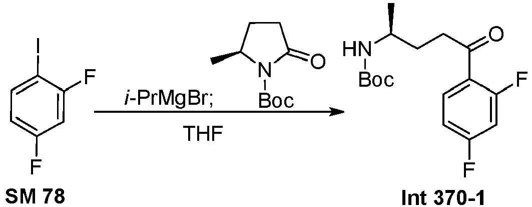

To a reaction flask, was added dry THF (30 mL), followed by addition of **SM 78** (2.4 g, 10 mmol), and then under N₂ protection, 1N isopropylmagnesium bromide (10 mL, 1M in THF) was added in an ice-water bath. After that, the mixture was reacted at room temperature for 7 h, and (S)-2-methyl-5-oxopyrrolidin-1-carboxylic acid t-butyl ester (1.99 g, 10 mmol) was finally added. The mixture was further allowed to react at room temperature for 12 h. The reaction solution was poured into the saturated aqueous solution of ammonium chloride, and extracted with 30 mL (10 mL×3) ethyl acetate. The organic phases were combined, dried over anhydrous Na₂SO₄, and purified by column chromatography to obtain **Int 370-1** (1.5 g, 5 mmol), with a yield of 50%. MS: *m*/*z* 314 [M+H]⁺.

### Synthesis of intermediate (S)-5-(2,4-difluorophenyl)-2-methyl-3,4-dihydro-2H- pyrrole (Int 370-2)

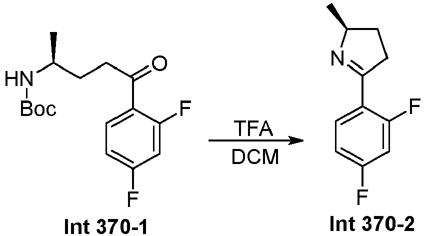

To a reaction solution was added DCM (20 mL), to which was then added **Int 370-1** (1.5 g, 5 mmol), followed by addition of TFA (2 mL). After that, the mixture was reacted at room temperature for 7 h, and the reaction solution was poured into the saturated aqueous solution of sodium bicarbonate. The resultant solution was extracted with 30 mL (10 mL × 3) DCM. The organic phases were combined, dried over anhydrous Na₂SO₄, and purified by column chromatography, to obtain **Int 370-2** (0.8 g, 4.5 mmol), with a yield of 90%. MS: *m*/*z* 196 [M+H]⁺.

### Synthesis of intermediate (5 S)-2-(2,4-difluorophenyl)-5 -methylpyrroli dine (Int 370-3)

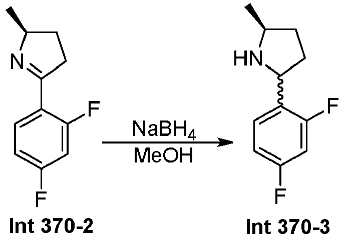

Methanol (15 mL) was added to a reaction flask, to which was then added **Int 370-2** (0.8 g, 4.5 mmol), followed by addition of sodium borohydride (0.76 g, 20 mmol) in batches. After that, the mixture was reacted at room temperature for 1 h. The reaction solution was poured into water, and extracted with 30 mL (10 mL×3) EA. The organic phases were combined, dried over anhydrous Na₂SO₄, and concentrated to obtain the crude product of racemate, which was purified to provide **Int 370-3** (0.8 g, 4.4 mmol), with a yield of 800%. MS: *m*/*z* 198 [M+H]⁺.

### Synthesis of compound 1-((R)-3'-(2-((2R,5S)-2-(2,4-difluorophenyl)-5-methylpyrrolidin-1-yl)-2-oxoethyl)-2',4'-dioxo-2,3-dihydrospiro[indene-1,5'-oxazolidine]-5-yl)-3-methylurea (370)

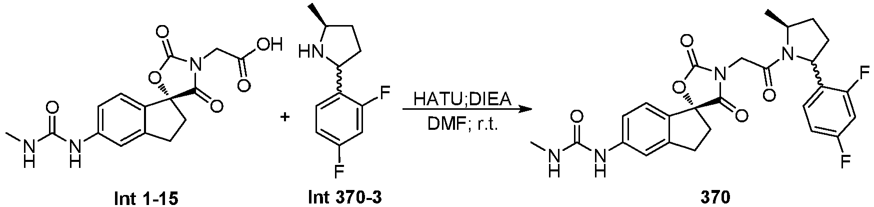

Intermediate **Int 1-15** (33 mg, 0.1 mmol) was dissolved in DMF (1 mL), to which were added DIEA (39 mg, 0.3 mmol) and HATU (38 mg, 0.1 mmol), followed by addition of **Int 370-3** (24 mg, 0.1 mmol), and the mixture was stirred at room temperature for 16 h. To the reaction solution was added water (10 mL), and extracted with ethyl acetate two times. The organic phase was washed with saturated brine, and concentrated to obtain the crude product, that was purified by column chromatography to obtain product **370** (26 mg) as off-white solid, with a yield of 50%. MS: *m*/*z* 513 [M+H]⁺. ¹H NMR (400 MHz, CDCl₃): δ 7.47 (m, 2H), 7.25-7.05 (m, 1H), 6.99-6.51 (m, 3H), 5.47-5.11 (m, 1H), 4.57-4.37 (m, 1H), 4.30 (m, 1H), 3.81 (m, 1H), 3.13-2.90 (m, 2H), 2.76-2.65 (m, 3H), 2.64-2.40 (m, 2H), 2.24-1.51 (m, 4H), 1.34-1.22 (m, 3H).

### Example 371 Synthesis of compound 1-((R)-3'-(2-((2S,5S)-2-(4-fluorophenyl)-5-methylpyrrolidin-1-yl)-2-oxoethyl)-2',4'dioxo-2,3-dihydrospiro[indene-1,5'-oxazolidine]-5-yl)-3-cyclopropylurea (371)

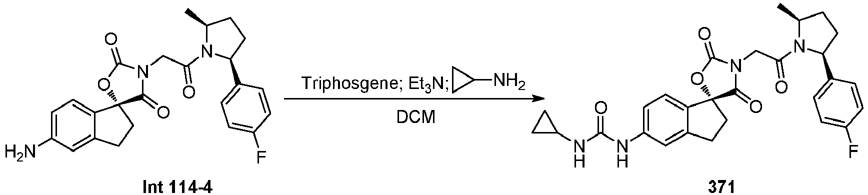

Triphosgene (35 mg, 0.12 mmol) was placed in dichloromethane (5 mL), to which was slowly dropped the mixed solution of compound **Int 114-4** (62 mg, 0.2 mmol), triethylamine (200 mg, 2 mmol), and dichloromethane (5 mL) in an ice bath. After addition, the mixture was stirred for 1 h, to which was added cyclopropylamine (33 mg, 0.5 mmol), and then the mixture was stirred for additional 4 h. Water (10 mL) was added, and the organic layer was separated. The solution was extracted with dichloromethane. The organic phases were combined, dried over anhydrous sodium sulfate, concentrated, and subjected to column chromatography, to obtain a yellow solid **371** (30 mg), with a yield of 45%. MS: *m*/*z* 521 [M+H]⁺.

### Example 373 Synthesis of compound 1-(3'-(2-((2S,5S)-2-(4-fluorophenyl)-5-methylpyrrolidin-1-yl)-2-oxoethyl)-2',4'-dioxo-dihydrospiro[cyclopropane-1,1'-indene-3',5'-oxazolidin]-6'-yl)-3-methylurea (373)

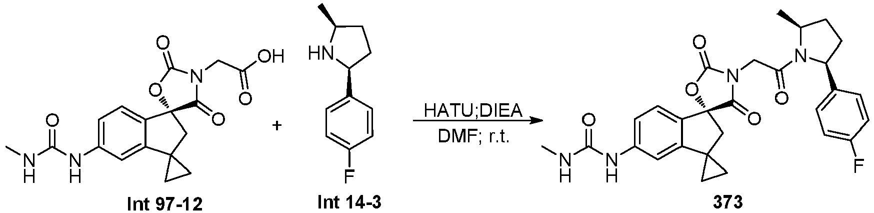

Intermediate **Int 97-12** (36 mg, 0.1 mmol), intermediate **Int 14-3** (18 mg, 0.11 mmol), HATU (50 mg, 0.13 mmol), and DIEA (20 mg, 0.15 mmol) were dissolved in dichloromethane (5 mL), and the mixture was stirred overnight. The reaction solution was concentrated and subjected to column chromatography, to obtain pale yellow solid **373** (37 mg), with a yield of 71%. MS: *m*/*z* 521 [M+H]⁺.

### Example 374 Synthesis of compound 1-((R)-3'-(2-((5S)-2-(3,4-difluorophenyl)-5-methylpyrrolidin-1-yl)-2-oxoethyl)-2',4'-dioxo-2,3-dihydrospiro[indene-1, 5'-oxazolidine]-5-yl)-3-methylurea (374)

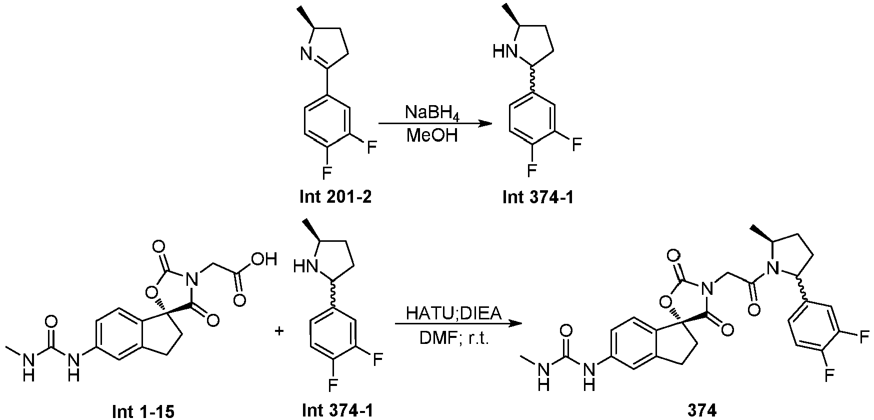

### Synthesis of intermediate (5 S)-2-(3,4-difluorophenyl)-5 -methylpyrroli dine (Int 374-1)

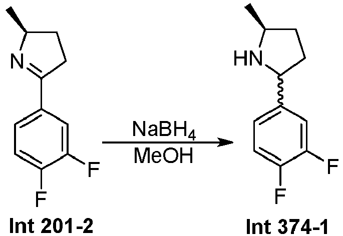

To a reaction flask, was added DCM (15 mL), followed by addition of **Int 201-2** (195 mg, 1 mmol), and then sodium borohydride (152 mg, 4 mmol) was added in batches. After that, the mixture was reacted at room temperature for 1 h. The reaction solution was poured into water, and extracted with 30 mL (10 mL×3) EA. The organic phases were combined, dried over anhydrous sodium sulfate, and concentrated to obtain **Int 374-1** (187 mg, 0.95 mmol), with a yield of 95%. MS: *m*/*z* 198 [M+H]⁺.

### Synthesis of compound 1-((R)-3'-(2-((2R,SS)-2-(3,4-difluorophenyl)-5- methylpyrrolidin-1-yl)-2-oxoethyl)-2',4'-dioxo-2,3-dihydrospiro[indene-1,5'-oxazolidine]-5-yl)-3-methylurea (374)

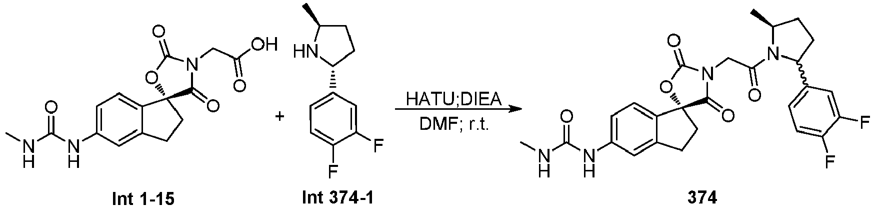

To a 25 mL reaction flask, was added DMF (3 mL), to which were then added **Int 1-15** (33.3 mg, 0.1 mmol), **SM 374-1** (19.7 mg, 0.1 mmol), DIEA (38.7 mg, 0.3 mmol), followed by addition of HATU (38.1 mg, 0.1 mmol). The system was reacted 1 h at 20 °C. After completion of the reation, the reaction solution was poured to water (20 mL), and the reasultant solution was extracted with 30 mL (10 mL×3) ethyl acetate. The organic phases were combined, dried over anhydrous Na₂SO₄, and purified by column chromatography to obtain compound **374** (28.2 mg), with a yield of 55%. MS: *m*/*z* 513 [M+H]⁺.

### Example 375 Synthesis of compound 1-methyl-3-(3'-(2-((2S)-2-methyl-5- (pyridin-4-yl)pyrrolidin-1-yl)-2-oxoethyl)-2',4'-dioxo-2,3-dihydrospiro[indene-1,5'-oxazolidine]-5-yl)urea (375)

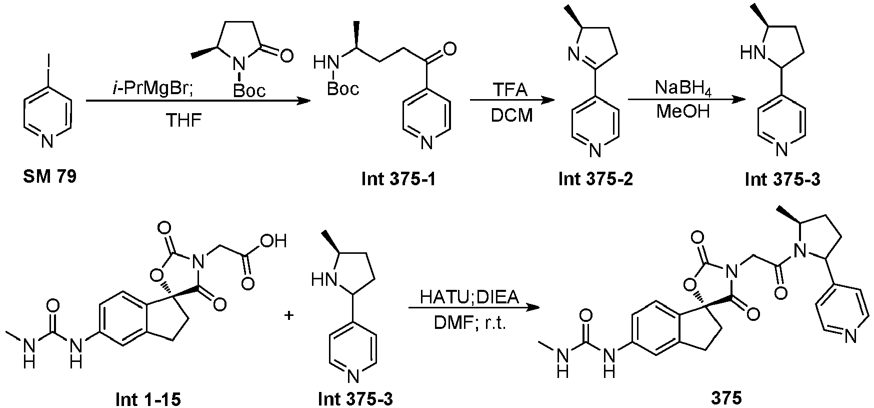

### Synthesis of intermediate (S)-(5-oxo-5-(pyridin-4-yl)pentan-2-yl)carbamic acid t-butyl ester (Int 375-1)

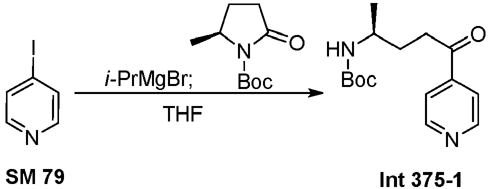

To a reaction flask, was added dry THF (30 mL), followed by addition of **SM 79** (2.05 g, 10 mmol), and then under N₂ protection, 1N isopropylmagnesium bromide (10 mL, 1M in THF) was added in an ice-water bath. After that, the mixture was reacted at room temperature for 7 h, and (S)-2-methyl-5-oxopyrrolidin-1-carboxylic acid t-butyl ester (2.13 g, 10 mmol) was finally added. The mixture was further allowed to react at room temperature for 12 h. The reaction solution was poured into the saturated aqueous solution of ammonium chloride, and extracted with 30 mL (10 mL×3) ethyl acetate. The organic phases were combined, dried over anhydrous Na₂SO₄, and purified by column chromatography to obtain **Int 375-1** (1.53 g, 5.5 mmol), with a yield of 55%. MS: *m*/*z* 279 [M+H]⁺.

### Synthesis of intermediate (S)-4-(2-methyl-3,4-dihydro-2H-pyrrol-5-yl)pyridine (Int 375-2)

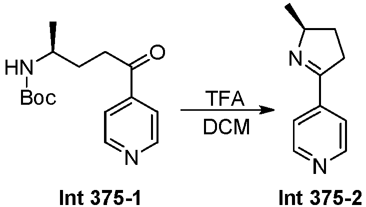

To a reaction solution was added DCM (20 mL), to which was then added **Int 375-1** (1.39 g, 5 mmol), followed by addition of TFA (2 mL). After that, the mixture was reacted at room temperature for 7 h, and the reaction solution was poured into the saturated aqueous solution of sodium bicarbonate. The resultant solution was extracted with 30 mL (10 mL × 3) DCM. The organic phases were combined, dried over anhydrous Na₂SO₄, and purified by column chromatography, to obtain **Int 375-2** (640 mg, 4 mmol), with a yield of 80%. MS: *m*/*z* 161 [M+H]⁺.

### Synthesis of intermediate 4-((5S)-5-methylpyrrolidin-2-yl)pyridine (Int 375-3)

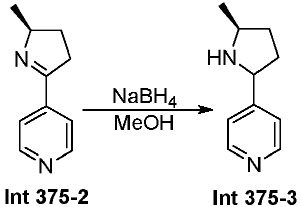

To a reaction flask, was added DCM (15 mL), followed by addition **of Int 375-2** (483 mg, 3 mmol), and then sodium borohydride (0.57 g, 15 mmol) was added in batches. After that, the mixture was reacted at room temperature for 1 h. The reaction solution was poured into water, and extracted with 30 mL (10 mL×3) EA. The organic phases were combined, dried over anhydrous sodium sulfate, and concentrated to obtain **Int 375-3** (462 mg, 2.85 mmol), with a yield of 95%. MS: *m*/*z* 163 [M+H]⁺.

### Synthesis of compound 1-methyl-3-(3'-(2-((2S)-2-methyl-5-(pyridin-4-yl)pyrrolidin- 1-yl)-2-oxoethyl)-2',4'-dioxo-2,3-dihydrospiro[indene-1,5'-oxazolidine]-5-yl)urea (375)

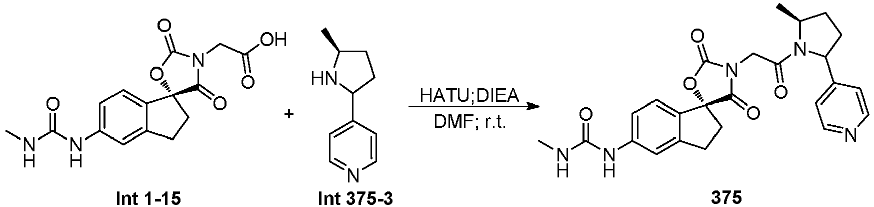

To a 25 mL reaction flask, was added DMF (3 mL), to which were then added **Int 1-15** (33.3 mg, 0.1 mmol), **SM 375-3** (16.2 mg, 0.1 mmol), DIEA (38.7 mg, 0.3 mmol), followed by addition of HATU (38.1 mg, 0.1 mmol). The system was reacted 1 h at 20 °C. After completion of the reation, the reaction solution was poured to water (20 mL), and the reasultant solution was extracted with 30 mL (10 mL×3) ethyl acetate. The organic phases were combined, dried over anhydrous Na₂SO₄, and purified by column chromatography to obtain compound **375** (24.8 mg), with a yield of 52%. MS: *m*/*z* 478 [M+H]⁺. ¹H NMR (400 MHz, CDCl₃): δ 8.63 (dd, J = 10.7, 6.0 Hz, 1H), 8.40 (dd, J = 38.2, 6.0 Hz, 1H), 7.65-7.41 (m, 2H), 7.18 (m, 1H), 7.06 (m, 1H), 6.96-6.55 (m, 1H), 5.51-5.29 (m, 1H), 5.05 m, 1H), 4.61-4.39 (m, 1H), 4.35-4.13 (m, 1H), 3.80-3.44 (m, 1H), 3.07 m, 1H), 2.98-2.91 (m, 1H), 2.68 m, 3H), 2.48-2.42 (m, 1H), 2.27-2.18 (m, 2H), 2.01-1.76 (m, 1H), 1.49 (m, 2H), 1.26-1.22 (m, 4H).

### Example 376 Synthesis of compound 2,2-difluoro-N-((R)-3'-(2-((2S,5S)-2-(4-fluorophenyl)-5-methylpyrrolidin-1-yl)-2-oxoethyl)-2',4'-dioxo-2,3-dihydrospiro[indene-1,5'-oxazolidine]-5-yl)acetamide (376)

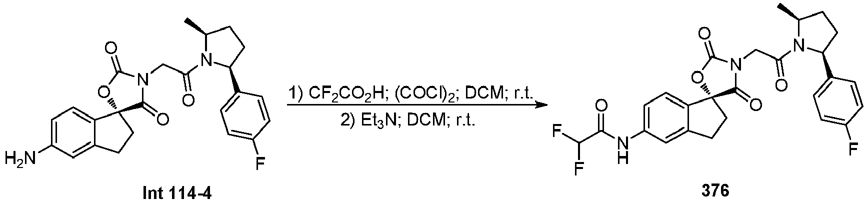

2,2-Difluoroacetic acid (100 mg, 1 mmol) was dissolved in dichloromethane (5 mL), to which was added oxalyl chloride (127 mg, 1 mmol) dropwise, and the mixture was stirred at room temperature for 1 h, and concentrated. The resultant crude product was added to dichloromethane (5 mL), and then triethylamine (101 mg, 1 mmol) and **Int 114-4** (44 mg, 0.1 mmol) were added. The mixture was stirred at room temperature overnight, followed by concentration and column chromatography, to give yellow solid **376** (33 mg), with a yield of 64%. MS: *m*/*z* 516 [M+H]⁺.

### Example 377 Synthesis of compound (S)-N-((R)-3'-(2-((2S,5S)-2-(4-fluorophenyl)-5-methylpyrrolidin-1-yl)-2-oxoethyl)-2',4'-dioxo-2,3-dihydrospiro[indene-1,5'-oxazolidine]-5-yl)-2-methoxypropionamide (377)

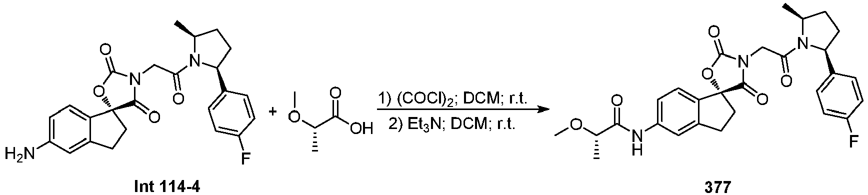

(S)-2-Methoxypropionic acid (104 mg, 1 mmol) was dissolved in dichloromethane (5 mL), to which was added oxalyl chloride (127 mg, 1 mmol) dropwise, and the mixture was stirred at room temperature for 1 h, and concentrated. The resultant crude product was added to dichloromethane (5 mL), and then triethylamine (101 mg, 1 mmol) and **Int 114-4** (44 mg, 0.1 mmol) were added. The mixture was stirred at room temperature overnight, followed by concentration and column chromatography, to give yellow solid **377** (33 mg), with a yield of 67%. MS: *m*/*z* 524 [M+H]⁺.

### Example 378 Synthesis of compound 1-((1R)-3'-(2-((2S)-2-((1,1-difluoro-6-aza-spiro[2.5]octan-6-yl)methyl)-5-methylpyrrolidin-1-yl)-2-oxoethyl)-2',4'-dioxo-2,3-dihydrospiro[indene-1,5'-oxazolidine]-5-yl)-3-methylurea (378)

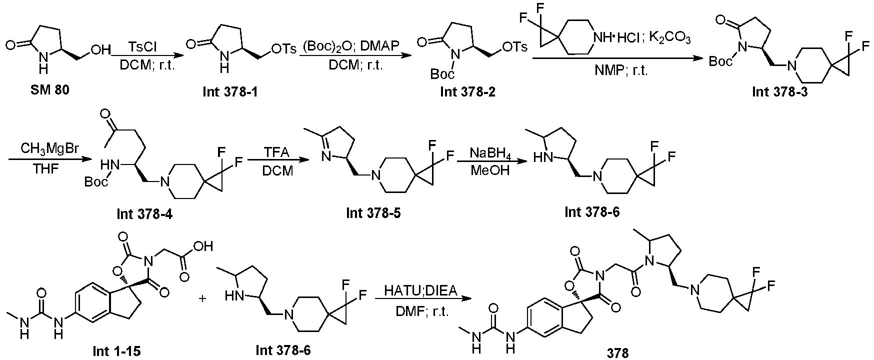

### Synthesis of intermediate (S)-(5-oxopyrrolidin-2-yl)methyl 4-methylbenzenesulfonate (Int 378-1)

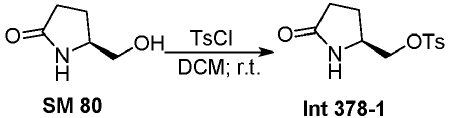

To a reaction flask, was added DCM (30 mL), to which were then added TsCl (1.9 g, 10 mmol), **SM 80** (4.6 g, 40 mmol), triethylamine (2 g, 20 mmol), and the reaction was stirred 5 h at room temperature. The reaction solution was poured into water, and extracted with 30 mL (10 mL×3) DCM. The organic phases were combined, dried over anhydrous Na₂SO₄, and purified by column chromatography to obtain **Int 378-1** (2.55 g, 9.5 mmol), with a yield of 95%. MS: *m*/*z* 270 [M+H]⁺.

### Synthesis of intermediate ((S)-(1-t-butoxycarbonyl-5-oxopyrrolidin-2-yl)-methyl-4-methylbenzenesulfonate (Int 378-2)

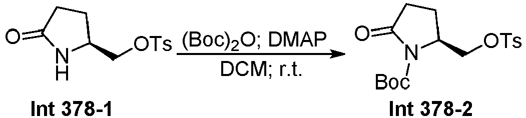

To a reaction flask, was added DCM (30 mL), to which were then added **Int 378-1** (2.55 g, 9.5 mmol), di-t-butyl carbonate (2.18 g, 10 mmol), and DMAP (122 mg, 1 mmol). After that, the mixture was reacted at room temperature for 15 h. The reaction solution was poured into water, and extracted with 30 mL (10 mL×3) DCM. The organic phases were combined, dried over anhydrous sodium sulfate, and purified by column chromatography to obtain **Int 378-2** (1.85 g, 5 mmol), with a yield of 50%. MS: *m*/*z* 370 [M+H]⁺.

### Synthesis of intermediate (S)-2-((1,1-difluoro-6-azaspiro[2.5]octan-6-yl)methyl)- 5-oxopyrrolidin-1-carboxylic acid t-butyl ester (Int 378-3)

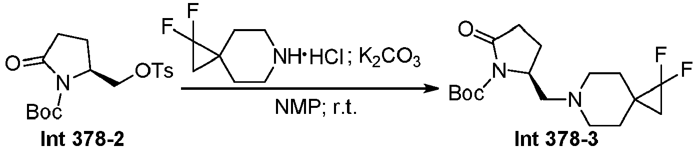

To a reaction flask, was added NMP (20 mL), to which were then added **Int 378-2** (1.85 g, 5 mmol), potassium carbonate (1.38 g, 10 mmol), and 1,1-difluoro-6-azaspiro[2.5]octane hydrochloride (905 mg, 5 mmol), and then the mixture was heated to 80 °C and reacted for 5 h. The reaction solution was poured into water, and extracted with 30 mL (10 mL×3) DCM. The organic phases were combined, dried over anhydrous sodium sulfate, and purified by column chromatography to obtain **Int 378-3** (1.04 g, 3 mmol), with a yield of 60%. MS: *m*/*z* 345 [M+H]⁺.

### Synthesis of intermediate (S)-(1-(1,1-difluoro-6-azaspiro [2.5] octan-6-yl)-5- oxohexan-2-yl)carbamic acid t-butyl ester (Int 378-4)

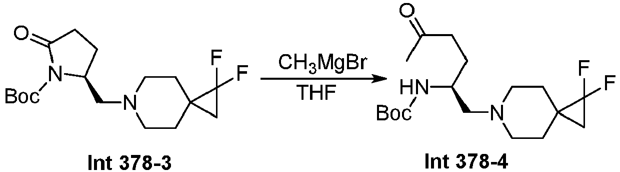

Anhydrous THF (15 ml) was added to the reaction flask, to which was then added **Int 378-3** (1.04 g, 3 mmol), and under nitrogen protection and cooling in an ice-water bath, methylmagnesium bromide solution (3 ml, 1M in hexane) was added. Then, the reaction was carried out at room temperature for 1 h. The reaction solution was poured into the saturated aqueous solution of ammonium chloride, and extracted with 30 ml (10 ml × 3) EA. The organic phases were combined, dried over anhydrous sodium sulfate, and purified by column chromatography to obtain **Int 378-4** (0.72 g, 2 mmol), with a yield of 67%. MS: *m*/*z* 361 [M+H]⁺.

### Synthesis of intermediate (S)-1,1-difluoro-6-((5-methyl-3,4-dihydro-2H-pyrrol-2-yl) methyl)-6-aza-spiro[2.5]octane (Int 378-5)

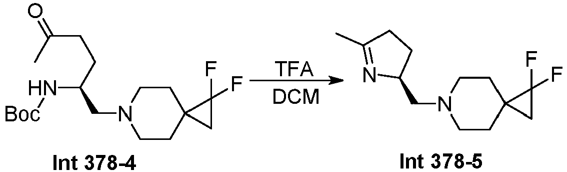

DCM (10 ml) was added to the reaction flask, to which were then added **Int 378-4** (0.72 g, 2 mmol) and TFA (1 ml), and the mixture was reacted at room temperature for 5 h. The reaction solution was rotatory evaporated, neutralized with saturated NaHCO₃ solution, and extracted with 30 ml (10 ml × 3) DCM. The organic phase was combined, dried over anhydrous sodium sulfate, and purified by column chromatography to give **Int 378-5** (0.44 g, 1.8 mmol), with a yield of 90%. MS: *m*/*z* 243 [M+H]⁺.

### Synthesis of intermediate 1,1-difluoro-6-(((2S)-5-methylpyrrolidin-2-yl)methyl)- 6-aza-spiro[2.5]octane (Int 378-6)

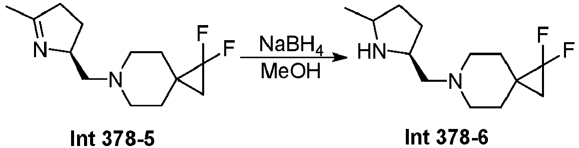

To a reaction flask, was added MeOH (10 mL), to which was then added **Int 378-5** (0.44 g, 1.8 mmol), followed by addition of sodium borohydride (0.38 g, 10 mmol) in batches. After that, the mixture was reacted at room temperature for 1 h. The reaction solution was poured into water, and extracted with 30 mL (10 mL×3) DCM. The organic phases were combined, dried over anhydrous sodium sulfate, and concentrated to obtain **Int 378-6** (0.44 g, 1.75 mmol), with a yield of 98%. MS: *m*/*z* 245 [M+H]⁺.

### Synthesis of compound 1-((1R)-3'-(2-((2S)-2-((1,1-difluoro-6-aza-spiro[2.5]octan- 6-yl)methyl)-5-methylpyrrolidin-1-yl)-2-oxoethyl)-2',4'-dioxo-2,3-dihydrospiro[indene-1,5'-oxazolidine]-5-yl)-3-methylurea (378)

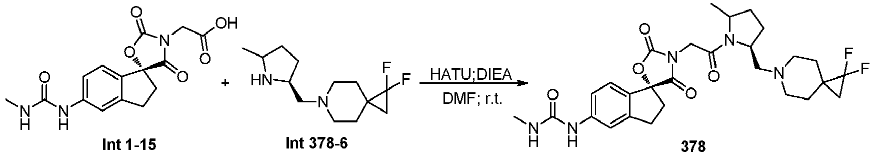

Intermediate **Int 1-15** (33 mg, 0.1 mmol) was dissolved in DMF (1 mL), to which were added DIEA (39 mg, 0.3 mmol) and HATU (38 mg, 0.1 mmol), followed by addition of **Int 378-6** (24 mg, 0.1 mmol), and the mixture was stirred at room temperature for 16 h. To the reaction solution was added water (10 mL), and extracted with ethyl acetate two times. The organic phase was washed with saturated brine, and concentrated to obtain the crude product, that was purified by column chromatography to obtain product **378** (286 mg) as off-white solid, with a yield of 50%. MS: *m*/*z* 560 [M+H]⁺.

### Example 379 Synthesis of compound 1-((R)-3'-(2-((2S,5S)-2-((1,1-difluoro-6-aza-spiro[2.5]octan-6-yl)methyl)-5-methylpyrrolidin-1-yl)-2-oxoethyl)-2',4'-dioxo-2,3-dihydrospiro[indene-1,5'-oxazolidine]-5-yl)-3-methylurea (379)

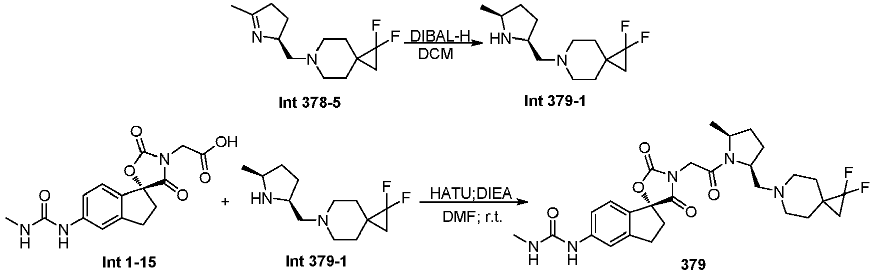

### Synthesis of intermediate 1,1-difluoro-6-(((2S,SS)-5-methylpyrrolidin-2-yl)methyl)- 6-aza-spiro[2.5]octane (Int 379-1)

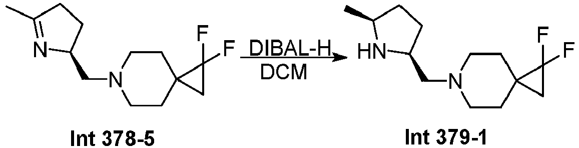

To a reaction flask was added DCM (10 mL), to which was then added **Int 378-5** (0.44 g, 1.8 mmol), and under nitrogen protection, DIBAL-H (10 mL, 1M in DCM) was added in batches in ice-water bath. After that, the mixture was reacted at room temperature for 16 h. The reaction solution was poured into cold aqueous solution of 0.5 N NaOH, and extracted with 30 mL (10 mL×3) DCM. The organic phases were combined, dried with anhydrous Na₂SO₄, and concentrated to obtain **Int 379-1** (0.44 g, 1.75 mmol), with a yield of 98%. MS: *m*/*z* 245 [M+H]⁺.

### Synthesis of compound 1-((R)-3'-(2-((2S,5S)-2-((1,1-difluoro-6-aza-spiro[2.5]octan- 6-yl)methyl)-5-methylpyrrolidin-1-yl)-2-oxoethyl)-2',4'-dioxo-2,3-dihydrospiro[indene-1,5'-oxazolidine]-5-yl)-3-methylurea (379)

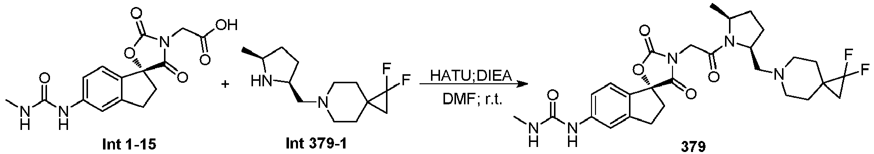

Intermediate **Int 1-15** (33 mg, 0.1 mmol) was dissolved in DMF (1 mL), to which were added DIEA (39 mg, 0.3 mmol) and HATU (38 mg, 0.1 mmol), followed by addition of **Int 379-1** (24 mg, 0.1 mmol), and the mixture was stirred at room temperature for 16 h. To the reaction solution was added water (10 mL), and extracted with ethyl acetate two times. The organic phase was washed with saturated brine, and concentrated to obtain the crude product, that was purified by column chromatography to obtain product 379 (28 mg) as off-white solid, with a yield of 50%. MS: *m*/*z* 560 [M+H]⁺.

### Example 380 Synthesis of compound 1-((R)-3'-(2-((2S,SS)-2-cyclopropyl-5- (4-fluorophenyl)pyrrolidin-1-yl)-2-oxoethyl)-2',4'-dioxo-2,3-dihydrospiro[indene-1, 5'-oxazolidine]-5-yl)-3-methylurea (380)

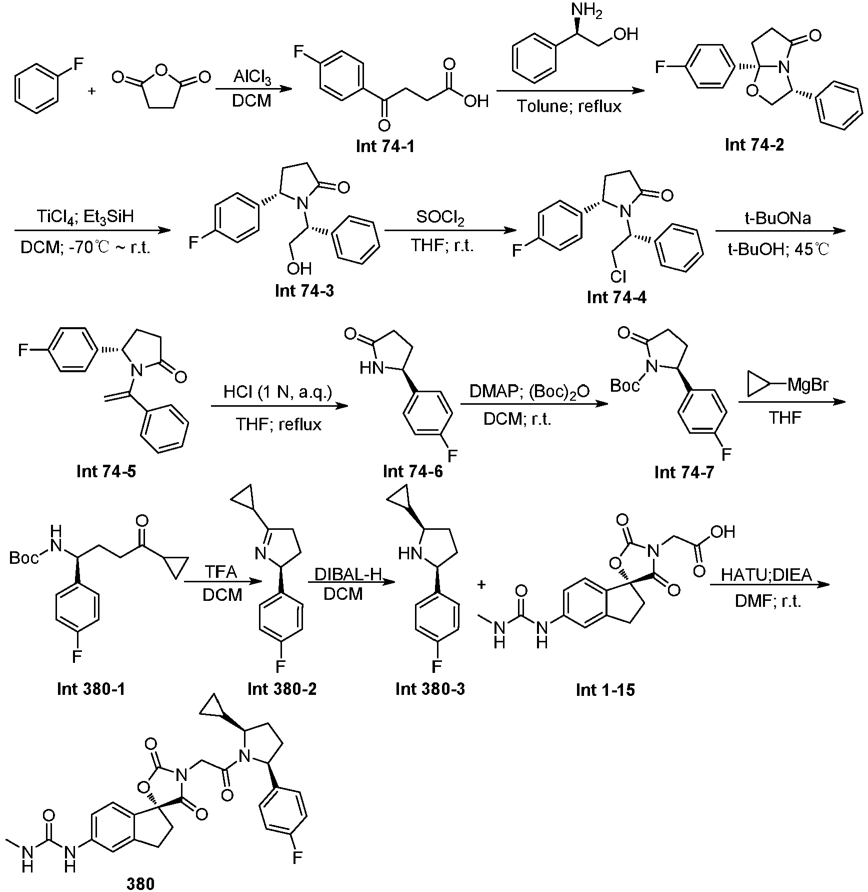

### Synthesis of intermediate (4-(4-fluorophenyl)-4-oxobutyric acid (Int 74-1)

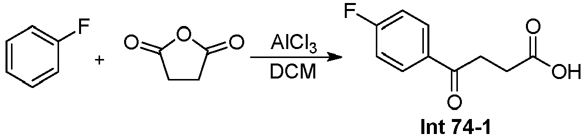

To a 250 ml reaction flask, were added fluorobenzene (10 g, 0.1 mol), succinic anhydride (9.4 g, 0.09 mmol), and dichloromethane (100 ml), and then aluminum trichloride (13.9 g, 0.1 mol) was added to the reaction flask in batches in an ice bath. The mixture was reacted overnight, and the reaction solution was poured into 2N HCl aqueous solution. The solution was separated, and the organic layer was dried with anhydrous sodium sulfate. The solvent was rotatory evaporated *in vacuum* to obtain crude **Int 74-1** (10 g), with a yield of 57%. MS: *m*/*z* 197.1 [M+H]⁺.

### Synthesis of intermediate (3R,7aR)-7a-(4-fluorophenyl)-3-phenyltetrahydropyrrolidin [2,1-b]oxazole-5(6H)-one (Int 74-2)

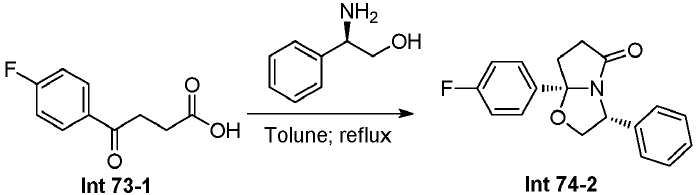

Intermediate **Int 73-1**(6.1 g, 31 mmol) and (R)-2-amino-1-phenylethan-1-ol (4.3 g, 31 mmol) were added in 50 mL toluene, and water was separted using a water separator. The mixture was reacted at 148 °C for 20 h, followed by concentration and column chromatography, to give 6 g Int 74-2 as white solid, with a yield of 66%. MS: *m*/*z* 298 [M+H]⁺.

### Synthesis of intermediate (S)-5-(4-fluorophenyl)-1-((R)-2-hydroxyl-1-phenylethyl) pyrrolidin-2-one (Int 74-3)

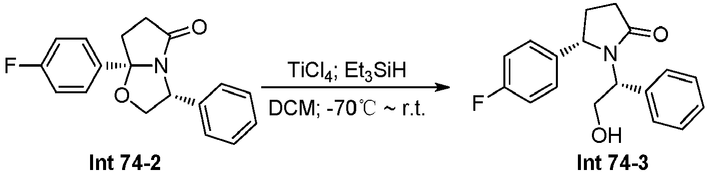

Intermediate **Int 74-2** (6 g, 20 mmol) and triethylsilicon hydride (7.5 g, 65 mmol) were added to 100 mL dichloromethane, to which was drop added titanium tetrachloride solution (20 mL, 1M in hexane) at -78°C. After addition, the reaction was slowly returned to room temperature and stirred for 4 h. The saturated aqueous solution of ammonium chloride was added, and the resultant solution was extracted with ethyl acetate, dried over anhydrous Na₂SO₄, and concentrated, to obtain the crude product, that was directly used in the next step.

### Synthesis of (S)-5-(4-fluorophenyl)-1-((R)-2-chloro-1-phenylethyl)pyrrolidin-2-one (Int 74-4)

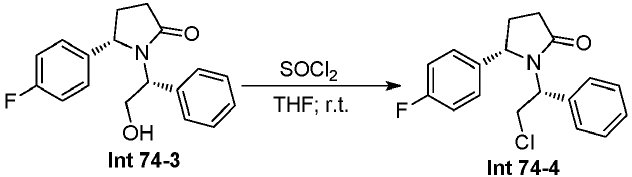

**Int 74-3** (10 g, 33 mmol) and tetrahydrofuran (100 mL) were added to a 250 mL reaction flask, to which was slowly added thionyl chloride (8 g, 67 mmol) in an ice bath. The reaction was naturally warmed to room temperature. The reaction was carried out at room temperature for another 2 h. After the reaction was completed, the solvent was rotatory evaporated *in vacuum* to obtain the crude compound **Int 74-4** (13 g, yield 122%). MS: *m*/*z* 318.1 [M+H]⁺.

### Synthesis of intermediate ((S)-5-(4-fluorophenyl)-1-(1-phenylvinyl)pyrrolidin-2-one (Int 74-5)

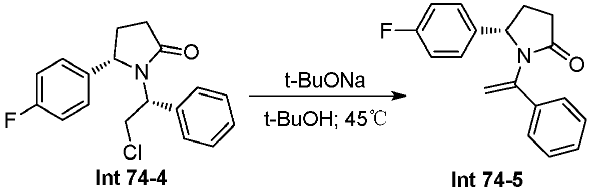

Crude **Int 74-4** (13 g, 41 mmol) and t-butanol (130 mL) were added to a 250 mL reaction flask, to which was then added sodium t-butoxide (7.9 g, 82 mmol), and the reaction was heated to 40 °C and kept for 2~3 h. After the reaction was completed, the solvent was rotatory evaporated, and then water (50 mL) was added. The solution was extracted with ethyl acetate (5 mL×3). The organic phases were combined, dried with anhydrous Na₂SO₄, and the solvent was rotatory evaporated to obtain the crude compound **Int 74-5** (11 g, yield 96%). MS: *m*/*z* 282.1 [M+H]⁺.

### Synthesis of intermediate (R)-5-(4-fluorophenyl)pyrrolidin-2-one (Int 74-6)

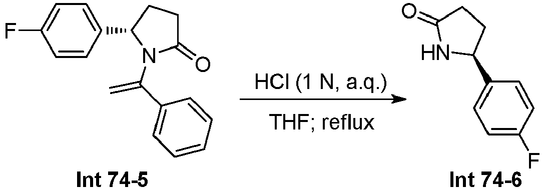

Crude **Int 74-5** (11 g, 39 mmol), 2N aqueous hydrochloric acid (50 mL), and tetrahydrofuran (60 mL) were added to a 250 mL reaction flask, and the reaction was heated to 80 °C and kept for 2 to 3 h. After the reaction was completed, the pH was adjusted to about 7 with saturated aqueous NaHCO₃ solution, and the solution was extracted with ethyl acetate (50 mL×3). The organic phases were combined, dried with anhydrous Na₂SO₄, and the solvent was rotatory evaporated *in vacuo.* The obtained solid was whirled in ethyl acetate (20 mL) for 1 h, followed by filtration, to obtain compound **Int 74-6** (5 g, yield 74%). MS: *m*/*z* 180.1 [M+H]⁺.

### Synthesis of intermediate (S)-2-(4-fluorophenyl)-5-oxopyrrolidin-1-carboxylic acid t-butyl ester (Int 74-7)

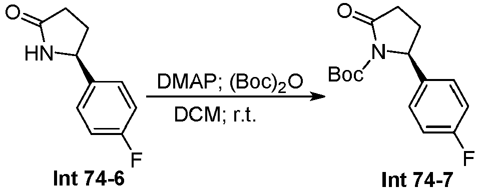

To a 100 mL reaction flask, were added crude **Int 74-6** (5 g, 28 mmol), di-t-butyl dicarbonate (7.4 g, 34 mmol) and dichloromethane (50 mL), to which was then added 4-dimethylaminopyridine (3.4 g, 28 mmol) in batches. The reaction was stirred overnight at room temperature. After the reaction was completed, 0.5 N aqueous hydrochloric acid (20 mL) was added, and the layers were separated. The organic phase was dried with anhydrous sodium sulfate, and the solvent was rotatory evaporated in vacuum. The residue was purified by column chromatography to provide compound **Int 74-7** (7.5 g, yield 96%). MS: *m*/*z* 280.1 [M+H]⁺.

### Synthesis of intermediate ((S)-4-cyclopropyl-1-(4-fluorophenyl)-4-oxobutylcarbamic acid t-butyl ester(Int 380-1)

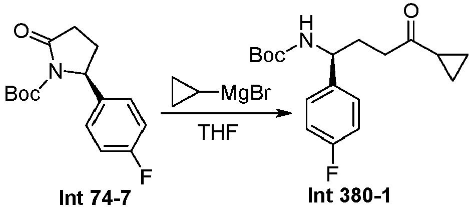

To a 50 mL reaction flask, was addeded crude **Int 74-7** (500 mg, 1.8 mmol) and tetrahydrofuran (5 mL), to which was added cyclopropylmagnesium bromide (1.8 mL, 1M in THF) in an ice bath, and the mixture was reacted for about 2 to 3 h. After the reaction was completed, the saturated aqueous solution of ammonium chloride (5 mL) was added, and the resultant solution was extracted with ethyl acetate (50 mL×3). The organic phases were combined, dried with anhydrous sodium sulfate, and purified by column chromatography to obtain **Int 380-1** (310 mg), with a yield of 54%. MS: *m*/*z* 322.1 [M+H]⁺.

### Synthesis of intermediate (S)-5-cyclopropyl-2-(4-fluorophenyl)-3,4-dihydro-2H- pyrrole (Int 380-2)

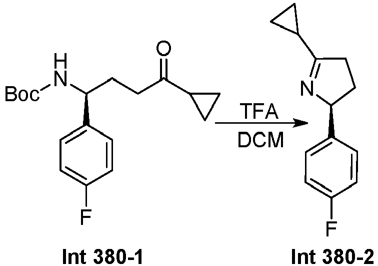

Crude **Int 380-1** (310 mg, 1 mmol) and dichloromethane (3 mL) were added to a 25 mL reaction flask, to which was then added trifluoroacetic acid (0.3 mL), and the mixture was reacted at room temperature overnight. After the reaction was completed, the solvent was rotatory evaporated *in vacuo.* The aqueous solution of sodium bicarbonate was added to adjust pH to about 7, and then extracted with ethyl acetate (5 mL×3). The organic phases were combined, dried over anhydrous sodium sulfate, and the solvent was removed by rotatory evaporation *in vacuo*, to obtain the crude compound **Int 380-2** (125 mg), with a yield of 70%. MS: *m*/*z* 204.1 [M+H]⁺.

### Synthesis of intermediate (2R,5S)-2-cyclopropyl-5-(4-fluorophenyl)pyrrolidine(Int 380-3)

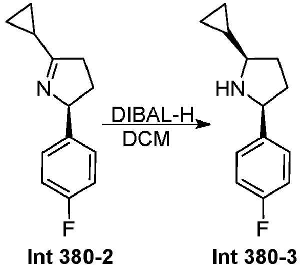

To a 25 mL reaction flask, were added **Int 380-2** (125 mg, 0.68 mmol) and dichloromethane (3 mL), and diisobutylaluminum hydride (2.7 mL, 1 M in hexane) was added dropwise in an ice bath. The mixture was reacted overnight at room temperature. After the reaction was completed, 15% aqueous NaOH solution (0.2 mL) was added, followed by addition of water (0.2 mL). The solution was filtered through celite, and the filtrate was dried with anhydrous sodium sulfate. The solvent was rotatory evaporated *in vacuo* to obtain the crude compound **Int 380-3** (103 mg), with a yield of 82 %. MS: *m*/*z* 206.1 [M+H]⁺.

### Synthesis of compound 1-((R)-3'-(2-((2S,SS)-2-cyclopropyl-5-(4-fluorophenyl) pyrrolidin-1-yl)-2-oxoethyl)-2',4'-dioxo-2,3-dihydrospiro[indene-1,5'-oxazolidine]-5-yl)-3-methylurea (380)

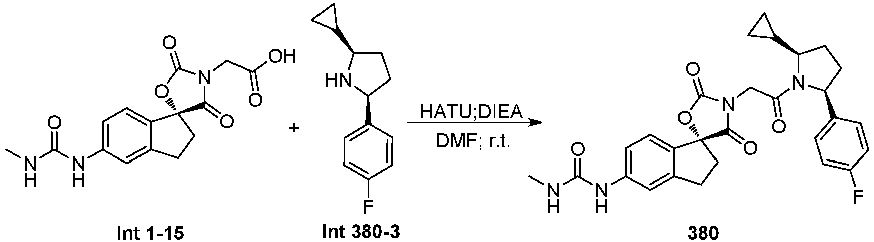

To a 25 mL reaction flask, were added compound **Int 1-15** (33 mg, 0.1 mmol), **Int 380-3** (31 mg, 0.15 mmol), DIEA(39 mg, 0.3 mmol), and DMF (1 mL), to which was added HATU (57 mg, 0.15 mmol) under stirring. The mixture was stirred overnight at room temperature. After the reaction was completed, water (5 mL) was added to the reaction solution, and then extracted with ethyl acetate (5 mL×3). The organic phases were combined, dried with anhydrous Na₂SO₄, and purified by column chromatography to obtain compound **380** (26 mg), with a yield of 50%. MS: *m*/*z* 521.2 [M+H]⁺. ¹H NMR (400 MHz, DMSO) δ 8.71 (s, 1H), 7.58-7.11 (m, 7H), 6.14-6.03 (m, 1H), 5.11 (dt, J = 53.2, 7.3 Hz, 1H), 4.59 (dd, J = 99.5, 16.9 Hz, 1H), 4.39-3.75 (m, 2H), 3.51 (d, J = 16.8 Hz, 1H), 3.07 (dd, J = 15.8, 7.8 Hz, 1H), 3.03-2.89 (m, 1H), 2.70-2.55 (m, 4H), 2.47-2.03 (m, 2H), 1.85 (ddd, J = 53.6, 33.0, 9.3 Hz, 3H), 1.19-1.03 (m, 1H), 0.80-0.44 (m, 3H).

### Example 381 Synthesis of compound 1-((S)-3'-(2-((2S,5S)-2-(3,4- difluorophenyl)-5-methylpyrrolidin-1-yl)-2-oxoethyl)-4-fluoro-2',4'-dioxo-2,3-dihydrospiro[indene-1,5'-oxazolidine]-5-yl)-3-methylurea (381)

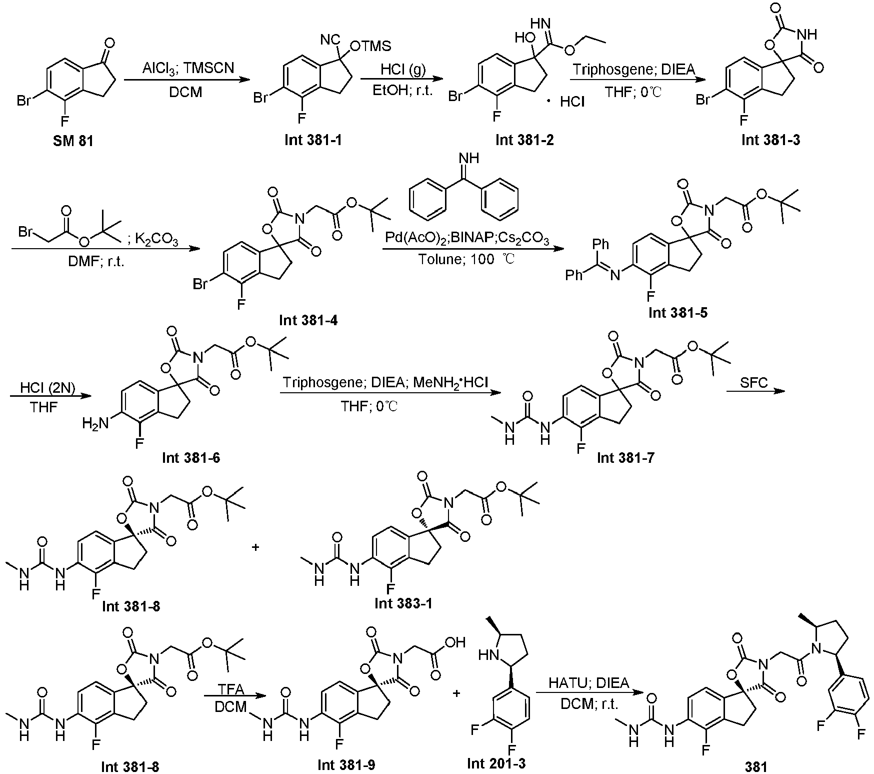

### Synthesis of intermediate 5-bromo-4-fluoro-1-((trimethylsilyl)oxy)-2,3-dihydro- 1H-indene-1-carbonitrile (Int 381-1)

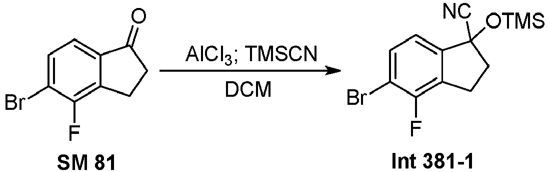

Aluminum trichloride (1.26 g, 9.4 mmol), 5-bromo-4-fluoroindanone (10.7 g, 47 mmol), and dry dichloromethane (100 mL) were added to a 100 mL reaction flask, to which was slowly added trimethylsilyl cyanide (9.4 g, 94 mmol) dropwise in an ice bath. After addition, the mixture was stirred at room temperature for 5 h. The reaction solution was poured into saturated KHCO₃ aqueous solution (200 mL), and extracted with dichloromethane three times. The organic phases were combined, dried over anhydrous sodium sulfate, and concentrated. After column chromatography, the white intermediate **Int 381-1** (10 g) was obtained, with a yield of 65%. MS: *m*/*z* 328, 330 [M+H]⁺.

### Synthesis of intermediate 5-bromo-4-fluoro-1-hydroxyl-2,3-dihydro-1H-indene-1-carboximidic acid ethyl ester (Int 381-2)

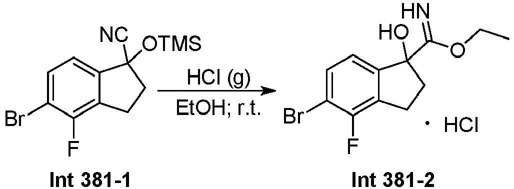

Intermediate **Int 381-1** (10 g, 32 mmol) was dissolved in dry ethanol (60 mL), and dry HCl gas was bubbled in, then the reaction was stirred overnight and detected by TLC. The reaction solution was concentrated, to obtain crude product **Int 381-2** as pale yellow solid (9.5 g), that was directly used in the next step.

### Synthesis of intermediate 5-bromo-4-fluoro-2,3-dihydrospiro[indene-1,5'- oxazolidine]-2',4'-dione (Int 381-3)

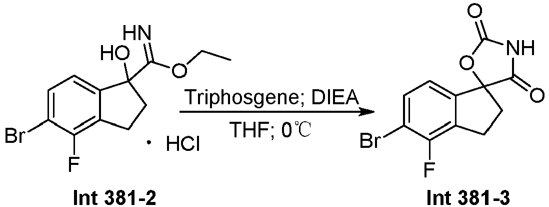

Intermediate **Int 381-2** (9.5 g) was suspended in dry THF (120 mL), to which was added triethylamine (20 mL, 5 e.q.) in an ice bath, followed by adding triphosgene (3.8 g, 0.4 e.q.) in batches. After that, the mixture was stirred for 2 h, and then HCl (2N) was slowly added in an ice bath until pH<5. The mixture was stirred 1h, extracted with EA, dried over anhydrous Na₂SO₄, and concentrated. The residue was recrystallized in EA/PE, followed by stirring and filtering, to obtain white intermediate **Int 381-3** (5 g), with a two-step yield of 53%. MS: *m*/*z* 300, 302 [M+H]⁺.

### Synthesis of intermediate 2-(5-bromo-2',4'-dioxo-2,3-dihydrospiro[indene-1,5'-oxazolidine]-3'-yl)acetic acid t-butyl ester (Int 381-4)

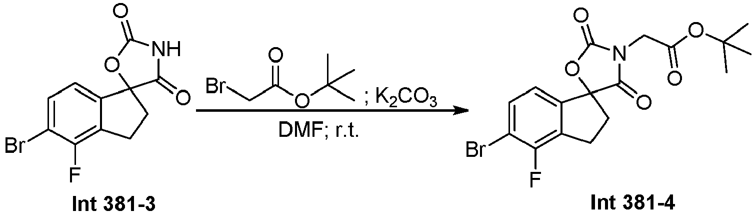

Intermediate **Int 381-3** (10 g, 34 mmol), tert-butyl bromoacetate (7.5 g, 38.4 mmol), and potassium carbonate (7.3 g, 52.9 mmol) were added in 100 mL DMF, and then stirred at room temperature for 4 h, to which was added 200 mL water, followed by extracting with ethyl acetate three times. The organic phase was combined and dried over anhydrous Na₂SO₄, and concentrated. The residue was swirled in petroleum ether and filtered, to obtain white solid **Int 381-4** (12 g), with a yield of 84%. MS: *m*/*z* 414, 416 [M+H]⁺.

### Synthesis of intermediate 2-(5-((diphenylmethylene )amino)-4-fluoro-2',4'-dioxo- 2,3-dihydrospiro[indene-1,5'-oxazolidine]-3'-yl)acetic acid t-butyl ester (Int 381-5)

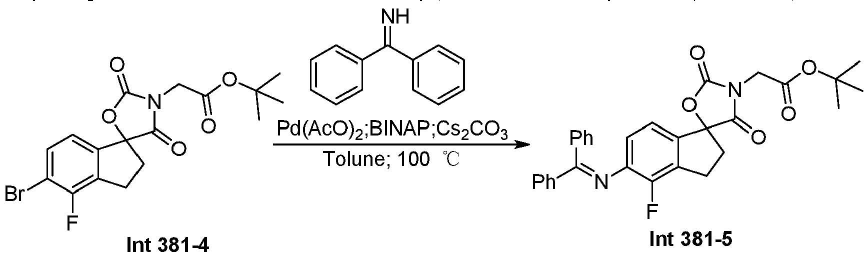

Intermediate **Int 381-4** (12 g, 28 mmol), benzophenonimine (7 g, 38 mmol), palladium acetate (260 mg, 1.16 mmol), 2,2'-bis(diphenylphosphino)-1,1'-binaphthyl (1.61 g, 2.58 mmol), and cesium carbonate (13.2 g, 40 mmol) were dissolved in toluene (50 mL), and the system was filled with N₂. The reaction was heated to 100 °C and stirred for 4h. The solution was concentrated and subjected to column chromatography to obtain **Int 381-5** (12 g) as grey solid, with a yield of 82.1%. MS: *m*/*z* 515 [M+H]⁺.

### Synthesis of intermediate 2-(5-amino-4-fluoro-2',4'-dioxo-2,3-dihydrospiro[indene- 1,5'-oxazolidine]-3'-yl)acetic acid t-butyl ester (Int 381-6)

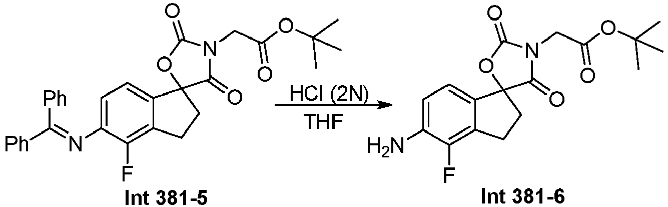

**Int 381-5** (12 g, 24 mmol) was dissolved in THF (20 mL), to which was added hydrochloric acid (2N, 10 mL) at room temperature, and the mixture was stirred 30 min, followed by extracting with ethyl acetate three times. The organic phase was combined, and dried over anhydrous Na₂SO₄, concentrated, and purified by column chromatography to obtain **Int 381-6**(6.8 g) as yellow solid, with a yield of 85%. MS: *m*/*z* 307 [M-43].

### Synthesis of intermediate 2-(5-(3-Methylureido) -4-fluoro-2',4'-dioxo-2,3-dihydrospiro[indene-1,5'-oxazolidine]-3'-yl)acetic acid t-butyl ester (Int 381-7)

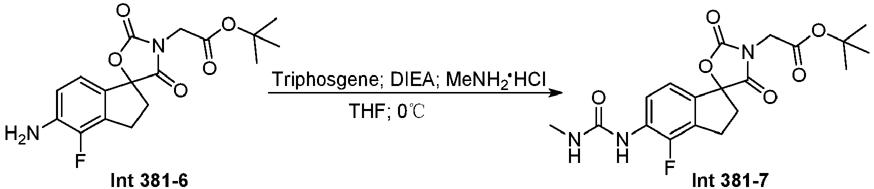

Triphosgene (3.5 g, 12 mmol) was placed in dichloromethane (25 mL). The mixed solution of **Int 381-6** (6.8 g, 20 mmol) and triethylamine (20 g, 200 mmol) and dichloromethane (25 mL) was slowly dropped into the reaction flask in an ice bath. Then, the mixture was stirred for 1 h, to which was added methylamine hydrochloride (7 g, 104 mmol), and the reaction was further stirred for 4 h. Water (100 mL) was added, the organic layer was separated, and then extracted with dichloromethane. The organic phases were combined, dried over anhydrous sodium sulfate, concentrated, and purified by column chromatography, to obtain yellow solid **Int 381-7** (5.1 g), with a yield of 61%. MS: *m*/*z* 425 [M+18].

### Synthesis of intermediate (S)-2-(4-fluoro-5-(3-methylureido)-2',4'-dioxo-2,3-dihydrospiro[indene-1,5'-oxazolidine]-3'-yl)acetic acid t-butyl ester (Int 381-8) and (R)-2-(4-fluoro-5-(3-methylureido)-2',4'-dioxo-2,3-dihydrospiro[indene-1,5'-oxazolidine]-3'-yl)acetic acid t-butyl ester (Int 383-1)

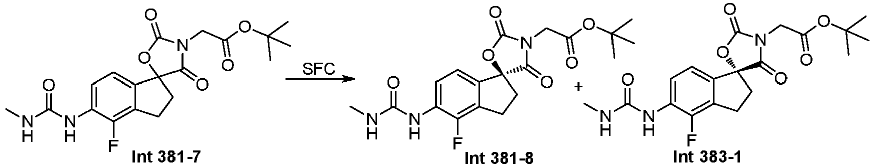

Chiral separation conditions: apparatus: SFC-80 (Thar, Waters); Chiral separation column: CHIRALCEL OD (4.6x100mm 3µm); column temperature: 35°C; mobile phase: A=CO₂, B= MEOH; peak time: t₁=1.61 min, t₂=1.98 min.

Chiral separation of **Int 381-7** (5 g) provided **Int 381-8** (t₁, 2.3 g), e.e =99%; **Int 383-1** (t₂, 2.3 g), e.e = 99%.

### Synthesis of intermediate (S)-2-(4-fluoro-5-(3-methylureido)-2',4'-dioxo-2,3-dihydrospiro[indene-1,5'-oxazolidine]-3'-yl)acetic acid (Int 381-9)

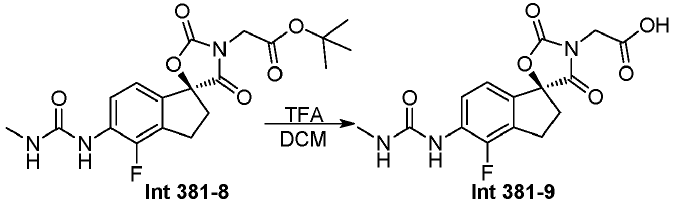

Intermediate **Int 381-8** (2.3 g, 5 mmol) was dissolved in DCM (20 mL), to which was added TFA (2 mL), and the reaction was stirred overnight at room temperature, to obtain **Int 381-9** as pale yellow solid (1.6 g), with a yield of 91%. MS: *m*/*z* 350 [M-H]⁻. [α]₂₀^{D} = - 54.5° (c 1.0, MeOH)

### Synthesis of compound 1-((S)-3'-(2-((2S,5S)-2-(3,4-difluorophenyl)-5- methylpyrrolidin-1-yl)-2-oxoethyl)-4-fluoro-2',4'-dioxo-2,3-dihydrospiro[indene-1,5'-oxazolidine]-5-yl)-3-methylurea (381)

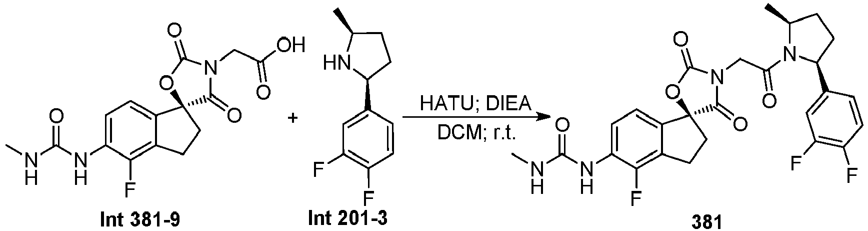

**Int 381-9** (33 mg, 0.1 mmol), **Int 201-3** (20 mg, 0.11 mmol), HATU (50 mg, 0.13 mmol), and DIEA (20 mg, 0.15 mmol) were dissolved in DCM (5 mL). The reaction was stirred overnight, concentrated, and purified by column chromatography to obtain **381** (35 mg) as pale yellow solid, with a yield of 69%. MS: *m*/*z* 531 [M+H]⁺.

### Example 382 Synthesis of compound 1-((S)-4-fluoro-3'-(2-((2S,5S)-2-methyl-5- (3,4,5-trifluorophenyl)pyrrolidin-1-yl)-2-oxoethyl)-2',4'-dioxo-2,3-dihydrospiro[indene-1,5'-oxazolidine]-5-yl)-3-methylurea (382)

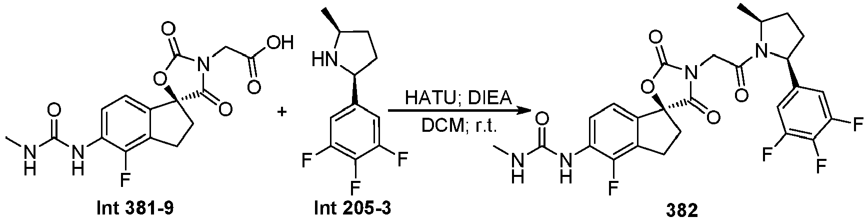

**Int 381-9** (33 mg, 0.1 mmol), **Int 205-3** (20 mg, 0.11 mmol), HATU (50 mg, 0.13 mmol), and DIEA (20 mg, 0.15 mmol) were dissolved in DCM (5 mL). The reaction was stirred overnight, concentrated, and purified by column chromatography to obtain **382** (35 mg) as pale yellow solid, with a yield of 69%. MS: *m*/*z* 549 [M+H]⁺. ¹H NMR (400 MHz, CDCl₃) δ 8.02-7.91 (m, 1H), 7.21 (dd, J = 33.7, 8.6 Hz, 1H), 7.04-6.96 (m, 1H), 6.85 (d, J = 7.1 Hz, 1H), 4.97 (d, J = 35.7 Hz, 1H), 4.44 (dt, J = 69.8, 34.9 Hz, 2H), 3.99 (dd, J = 148.8, 16.4 Hz, 2H), 3.27-3.11 (m, 2H), 2.93-2.75 (m, 4H), 2.61 (s, 1H), 2.52-2.39 (m, 1H), 2.32-1.89 (m, 3H), 1.73 (d, J = 65.5 Hz, 1H), 1.28 (s, 1H).

### Example 383 Synthesis of compound 1-((R)-3'-(2-((2S,5S)-2-(3,4-difluorophenyl)- 5-methylpyrrolidin-1-yl)-2-oxoethyl)-4-fluoro-2',4'-dioxo-2,3-dihydrospiro[indene-1,5'-oxazolidine]-5-yl)-3-methylurea (383)

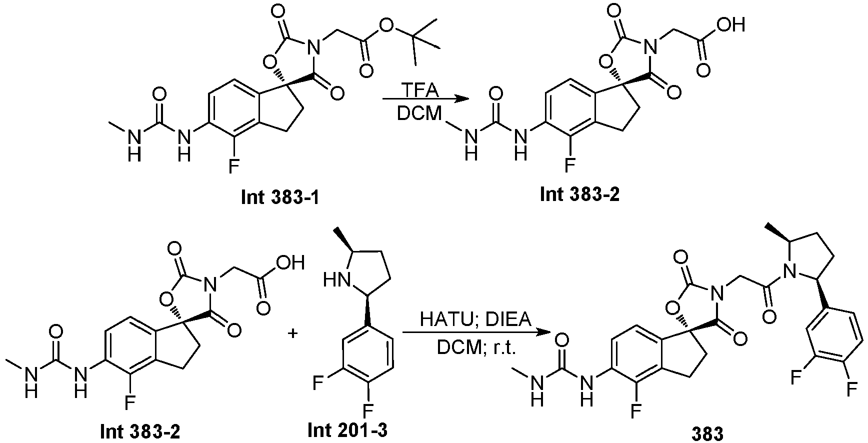

### Synthesis of intermediate (R)-2-(4-fluoro-5-(3-methylureido)-2',4'-dioxo-2,3-dihydrospiro[indene-1,5'-oxazolidine]-3'-yl)acetic acid (Int 383-2)

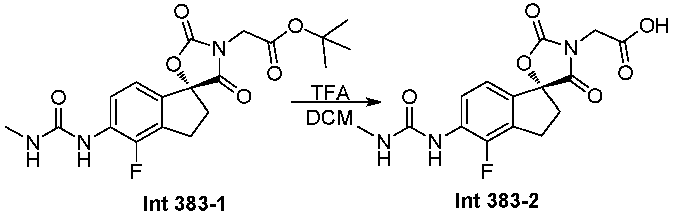

Intermediate **Int 383-1** (2.3 g, 5 mmol) was dissolved in DCM (20 mL), to which was added TFA (2 mL), and the reaction was stirred overnight at room temperature, to obtain **Int 383-2** as pale yellow solid (1.6 g), with a yield of 91%. MS: *m*/*z* 350 [M-H]⁻. [α]₂₀^{D} = + 53.8° (c 1.0, MeOH)

### Synthesis of compound 1-((R)-3'-(2-((2S,SS)-2-(3,4-difluorophenyl)-5- methylpyrrolidin-1-yl)-2-oxoethyl)-4-fluoro-2',4'-dioxo-2,3-dihydrospiro[indene-1,5'-oxazolidine]-5-yl)-3-methylurea (383)

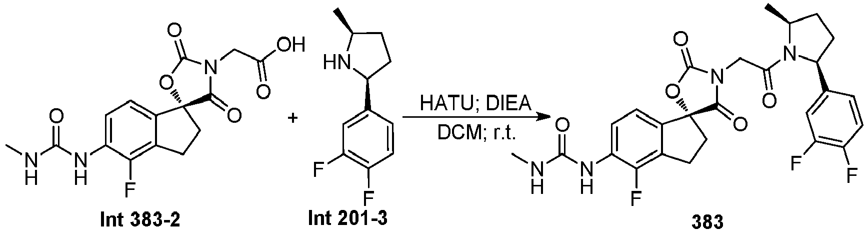

**Int 383-1** (33 mg, 0.1 mmol), **Int 201-3** (20 mg, 0.11 mmol), HATU (50 mg, 0.13 mmol), and DIEA (20 mg, 0.15 mmol) were dissolved in DCM (5 mL). The reaction was stirred overnight, concentrated, and purified by column chromatography to obtain **381** (35 mg) as pale yellow solid, with a yield of 69%. MS: *m*/*z* 531 [M+H]⁺. ¹H NMR (400 MHz, DMSO) δ 8.71 (s, 1H), 7.53 (d, J = 6.4 Hz, 1H), 7.36-7.31 (m, 1H), 7.23 (ddd, J = 29.6, 16.1, 9.6 Hz, 3H), 6.08 (d, J = 4.5 Hz, 1H), 5.38-4.97 (m, 1H), 4.54-4.30 (m, 2H), 4.27-4.17 (m, 1H), 3.44 (t, J = 17.0 Hz, 1H), 3.14-3.03 (m, 1H), 2.98 (s, 1H), 2.69-2.56 (m, 4H), 2.46-2.41 (m, 1H), 2.04 (dd, J = 13.1, 6.1 Hz, 1H), 1.73 (dd, J = 12.6, 6.0 Hz, 1H), 1.65-1.46 (m, 2H), 1.29 (dd, J = 40.9, 5.9 Hz, 2H).

### Example 384 Synthesis of compound 1-((R)-4-fluoro-3'-(2-((2S,5S)-2-methyl- 5-(3,4,5-trifluorophenyl)pyrrolidin-1 -yl)-2-oxoethyl)-2',4'-dioxo-2,3 -dihydrospirofindene-1,5'-oxazolidine]-5-yl)-3-methylurea (384)

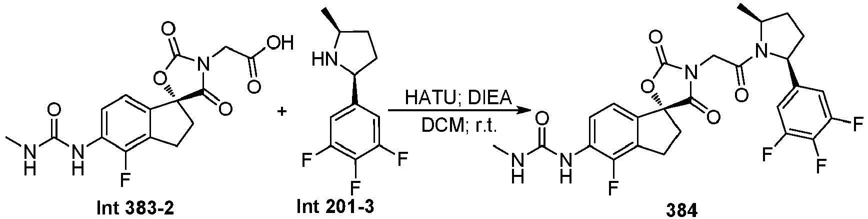

**Int 383-2** (33 mg, 0.1 mmol), **Int 205-3** (20 mg, 0.11 mmol), HATU (50 mg, 0.13 mmol), and DIEA (20 mg, 0.15 mmol) were dissolved in DCM (5 mL). The reaction was stirred overnight, concentrated, and purified by column chromatography to obtain **382** (35 mg) as pale yellow solid, with a yield of 69%. MS: *m*/*z* 549 [M+H]⁺. ¹H NMR (400 MHz, CDCl₃) δ 8.02-7.86 (m, 1H), 7.14 (dd, J = 47.6, 7.9 Hz, 1H), 7.04-6.96 (m, 1H), 6.87-6.78 (m, 1H), 4.96 (d, J = 31.1 Hz, 1H), 4.59-3.73 (m, 4H), 3.18 (d, J = 10.1 Hz, 2H), 2.84 (s, 4H), 2.59 (d, J = 8.2 Hz, 1H), 2.44 (s, 1H), 2.23 (s, 1H), 2.07 (dd, J = 21.2, 13.1 Hz, 2H), 1.75 (d, J = 80.1 Hz, 1H), 1.30 (d, J = 13.0 Hz, 1H).

### Example 385 Synthesis of compound 1-((S)-3'-(2-((2S,5S)-2-(3,4-difluorophenyl)- 5-methylpyrrolidin-1-yl)-2-oxoethyl)-7-fluoro-2',4'-dioxo-2,3-dihydrospiro[indene-1,5'-oxazolidine]-5-yl)-3-methylurea (385)

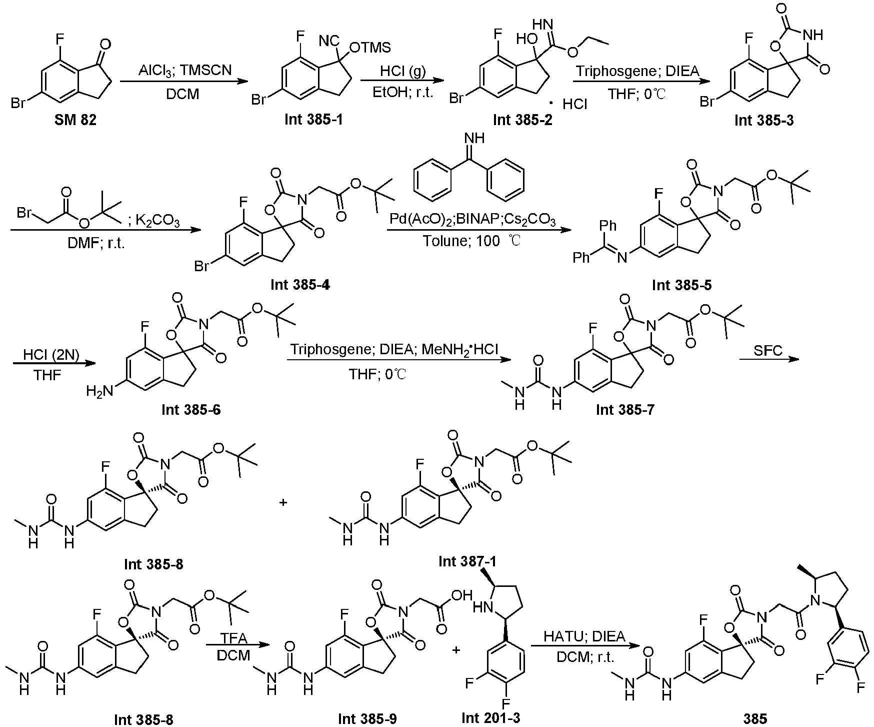

### Synthesis of intermediate 5-bromo-7-fluoro-1-((trimethylsilyl)oxy)-2,3-dihydro-1H- indene-1-carbonitrile (Int 385-1):

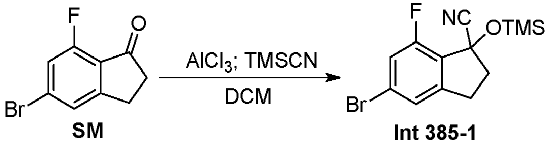

Aluminum trichloride (1.17 g, 8.8 mmol), 5-bromo-7-fluoroindanone (10 g, 44 mmol), and dry dichloromethane (100 mL) were added to a 100 mL reaction flask, to which was slowly added trimethylsilyl cyanide (8.7 g, 88 mmol) dropwise in an ice bath. After addition, the mixture was stirred at room temperature for 5 h. The reaction solution was poured into a saturated KHCO₃ aqueous solution (200 mL), and extracted with dichloromethane three times. The organic phases were combined, dried over anhydrous sodium sulfate, and concentrated. After column chromatography, the white intermediate **Int 385-1** (9 g) was obtained, with a yield of 63%. MS: *m*/*z* 328, 330 [M+H]⁺.

### Synthesis of intermediate 5-bromo-7-fluoro-1-hydroxyl-2,3-dihydro-1H-indene-1-carboximidic acid ethyl ester hydrochloride (Int 385-2):

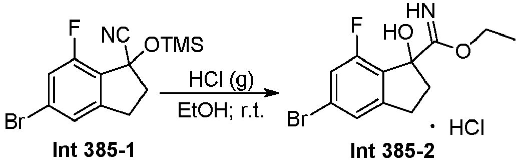

Intermediate **Int 385-1** (9 g, 27 mmol) was dissolved in dry ethanol (60 mL), and dry HCl gas was bubbled in, then the reaction was stirred overnight and detected by TLC. The reaction solution was concentrated, to obtain crude product **Int 385-1** as pale yellow solid (8.5 g), that was directly used in the next step.

### Synthesis of intermediate 5-bromo-7-fluoro-2,3-dihydrospiro[indene-1,5'- oxazolidine]-2',4'-dione (Int 385-3):

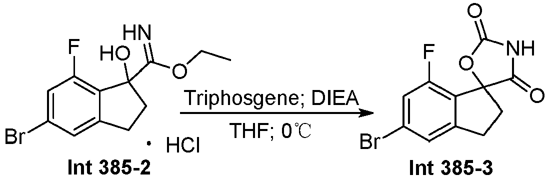

Intermediate **Int 385-2** (8.5 g, 25 mmol) was suspended in dry THF (120 mL), to which was added triethylamine (17.7 g, 175 mmol) in an ice bath, followed by adding triphosgene (3 g, 10 mmol) in batches. After that, the mixture was stirred for 2 h, and then HCl (2N) was slowly added in an ice bath until pH<5. The mixture was stirred 1 h, extracted with EA, dried over anhydrous Na₂SO₄, and concentrated. The residue was recrystallized in EA/PE, followed by stirring and filtering, to obtain white intermediate **Int 385-3** (4 g), with a two-step yield of 50%. MS: *m*/*z* 300, 302 [M+H]⁺.

### Synthesis of intermediate 2-(5-bromo-7-fluoro-2',4'-dioxo-2,3-dihydrospiro[indene- 1,5'-oxazolidine]-3'-yl)acetic acid t-butyl ester (Int 385-4)

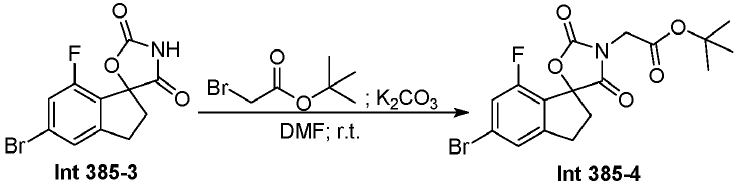

Intermediate **Int. 385-3** (4 g, 13 mmol), tert-butyl bromoacetate (2.6 g, 13 mmol), and potassium carbonate (2.8 g, 20 mmol) were added in DMF (100 mL), and then stirred at room temperature for 4 h, to which was added water (200 mL), followed by extracting with ethyl acetate three times. The organic phase was combined and dried over anhydrous Na₂SO₄, and concentrated. The residue was swirled in petroleum ether and filtered, to obtain white solid **Int 385-4** (4.7 g), with a yield of 85%. MS: *m*/*z* 414, 416 [M+H]⁺.

### Synthesis of intermediate 2-(5-((diphenylmethylene)amino)-7-fluoro-2',4'-dioxo- 2,3-dihydrospiro[indene-1,5'-oxazolidine]-3'-yl)acetic acid t-butyl ester (Int 385-5)

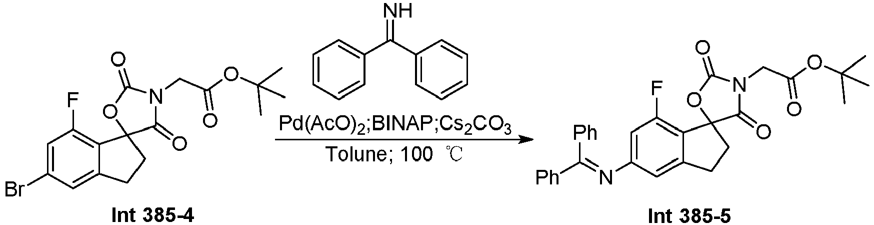

Intermediate **Int 385-4** (4.7 g, 11 mmol), benzophenonimine (2.67 g, 15 mmol), palladium acetate (250 mg, 1.10 mmol), 2,2'-bis(diphenylphosphino)-1,1'-binaphthyl (685 mg, 1.1 mmol), and cesium carbonate (5.54 g, 17 mmol) were dissolved in toluene (50 mL), and the system was filled with N₂. The reaction was heated to 100 °C and stirred for 4h. The solution was concentrated and subjected to column chromatography to obtain **Int 385-5** (4.3 g) as grey solid, with a yield of 76%. MS: *m*/*z* 515 [M+H]⁺.

### Synthesis of intermediate 2-(5-amino-7-fluoro-2',4'-dioxo-2,3-dihydrospiro[indene- 1,5'-oxazolidine]-3'-yl)acetic acid t-butyl ester (Int 385-6)

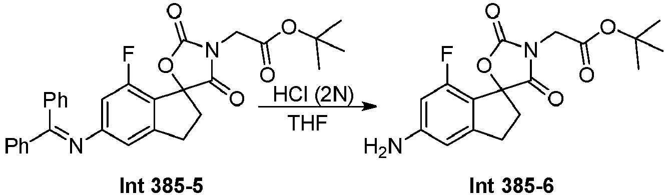

Intermediate **Int 385-5** (4.3 g, 8.4 mmol) was dissolved in THF (20 mL), to which was added hydrochloric acid (10 mL, 2 N) at room temperature, and the mixture was stirred 30 min, followed by extracting with ethyl acetate three times. The organic phase was combined, and dried over anhydrous Na₂SO₄, concentrated, and purified by column chromatography to obtain **Int 385-6** (2.8 g) as yellow solid, with a yield of 96%. MS: *m*/*z* 307 [M-43].

### Synthesis of intermediate 2-(5-(3-methylureido)-7-fluoro-2',4'-dioxo-2,3- dihydrospiro [indene-1,5'-oxazolidine]-3'-yl)acetic acid t-butyl ester (Int 385-7)

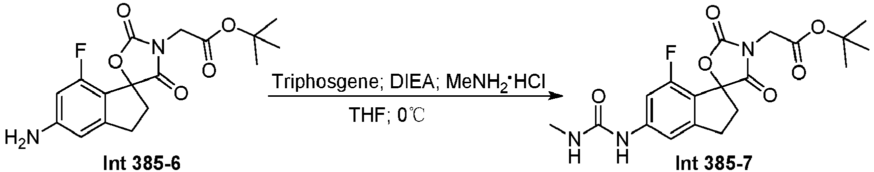

Triphosgene (1.2 g, 4 mmol) was placed in dichloromethane (25 mL). The mixed solution of intermediate **Int 385-6** (2.8 g, 8 mmol) and triethylamine (6.5 g, 64 mmol) and dichloromethane (25 mL) was slowly dropped into the reaction flask in an ice bath. Then, the mixture was stirred for 1 h, to which was added methylamine hydrochloride (1.6 g, 24 mmol), and the reaction was further stirred for 4 h. Water (100 mL) was added, the organic layer was separated, and then extracted with dichloromethane. The organic phases were combined, dried over anhydrous sodium sulfate, concentrated, and purified by column chromatography, to obtain yellow solid **Int 385-7** (5 g), with a yield of 77%. MS: *m*/*z* 408 [M+H]⁺.

### Synthesis of intermediate (S)-2-(7-fluoro-5-(3-methylureido) -2',4'-dioxo-2,3-dihydrospiro[indene-1,5'-oxazolidine]-3'-yl)acetic acid t-butyl ester (Int 385-8) and (R)-2-(7-fluoro-5-(3-methylureido)-2',4'-dioxo-2,3-dihydrospiro[indene-1,5'-oxazolidine]-3'-yl)acetic acid t-butyl ester (Int 387-1):

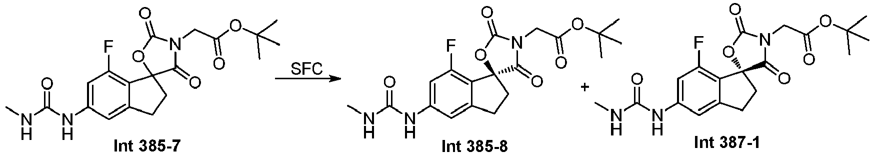

Chiral separation conditions: apparatus: SFC-80 (Thar, Waters); chiral separation column: CHIRALCEL OD (4.6x100mm 3µm); column temperature: 35°C; mobile phase: A=CO₂, B= MEOH; peak time: t₁ = 1.61 min, t₂ =1.98 min.

Chiral separation of **Int 385-7**(5 g) provided **Int 385-8**(t₁, 1.2 g), e.e =99%; **Int 387-1**(t₂, 1.2 g), e.e =99%

### Synthesis of intermediate (S)-2-(7-fluoro-5-(3-Methylureido) -2',4'-dioxo-2,3-dihydrospiro[indene-1,5'-oxazolidine]-3'-yl)acetic acid (Int 385-9):

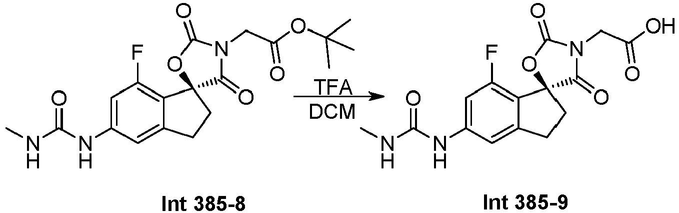

Intermediate **Int 385-8** (2.3 g, 5 mmol) was dissolved in DCM (20 mL), to which was added TFA (2 mL), and the reaction was stirred overnight at room temperature, to obtain **Int 385-9** as pale yellow solid (0.9 g), with a yield of 86%. MS: *m*/*z* 350 [M-H]⁻. [α]₂₀^{D} = - 68° (c 1.0, MeOH)

### Synthesis of compound 1-((S)-3'-(2-((2S,5S)-2-(3,4-difluorophenyl)-5- methylpyrrolidin-1-yl)-2-oxoethyl)-7-fluoro-2',4'-dioxo-2,3-dihydrospiro[indene-1,5'-oxazolidine]-5-yl)-3-methylurea (385)

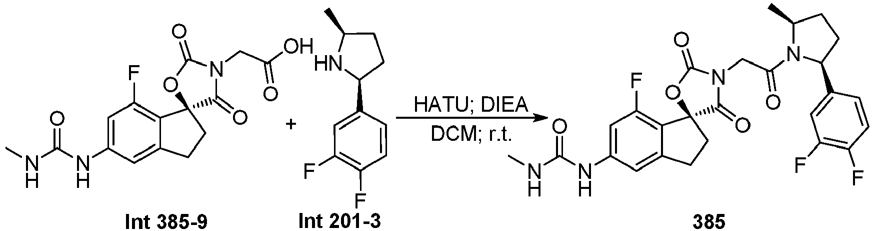

**Int 385-9** (33 mg, 0.1 mmol), **Int 201-3** (20 mg, 0.11 mmol), HATU (50 mg, 0.13 mmol), and DIEA (20 mg, 0.15 mmol) were dissolved in DCM (5 mL). The reaction was stirred overnight, concentrated, and purified by column chromatography to obtain **385** (35 mg) as pale yellow solid, with a yield of 69%. MS: *m*/*z* 531 [M+H]⁺. ¹H NMR (400 MHz, DMSO): δ 8.96 (d, J = 4.4 Hz, 1H), 7.51 (d, J = 10.5 Hz, 1H), 7.42-7.12 (m,4H), 6.22-6.16 (m, 1H), 4.97 (m, 1H), 4.53-4.36 (m, 2H), 3.85 (m, 1H), 3.09 (m, 2H), 2.62-2.51 (m, 5H), 2.45-2.32 (m, 1H), 2.00 (m, 2H), 1.70 (m, 1H), 1.39-1.33 (m, 3H).

### Example 386 Synthesis of compound 1-((S)-7-fluoro-3'-(2-((2S,5S)-2-methyl-5- (3,4,5-trifluorophenyl)pyrrolidin-1-yl)-2-oxoethyl)-2',4'-dioxo-2,3-dihydrospiro[indene-1,5'-oxazolidine]-5-yl)-3-methylurea (386)

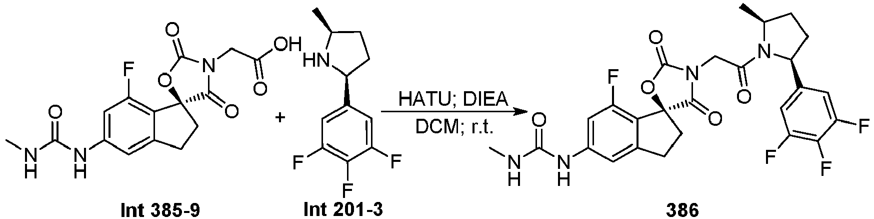

**Int 385-9** (33 mg, 0.1 mmol), **Int 205-3** (20 mg, 0.11 mmol), HATU (50 mg, 0.13 mmol), and DIEA (20 mg, 0.15 mmol) were dissolved in DCM (5 mL). The reaction was stirred overnight, concentrated, and purified by column chromatography to obtain **386** (35 mg) as pale yellow solid, with a yield of 69%. MS: *m*/*z* 549 [M+H]⁺. ¹H NMR (400 MHz, DMSO): δ 8.97 (s, 1H), 7.36-7.24 (m, 3H), 7.15 (s, 1H), 7.11-7.05 (m, 1H), 6.20 (s, 1H), 4.88-4.76 (m, 1H), 4.47 (m, 2H), 4.18-3.54 (m,1H), 3.14 (m, 1H), 3.05 (m, 1H), 2.71-2.57 (m, 4H), 2.41-2.32 (m, 1H), 2.14-2.05 (m, 1H), 2.02-1.91 (m, 1H), 1.81 (m, 1H), 1.47 (m, 1H), 1.36 (t, J = 5.9 Hz, 3H).

### Example 387 Synthesis of compound 1-((R)-3'-(2-((2S,5S)-2-(3,4- difluorophenyl)-5-methylpyrrolidin-1-yl)-2-oxoethyl)-7-fluoro-2',4'-dioxo-2,3-dihydrospiro[indene-1,5'-oxazolidine]-5-yl)-3-methylurea (387)

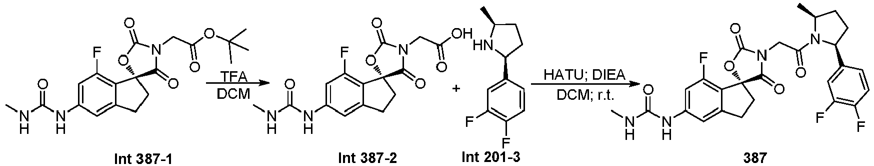

### Synthesis of intermediate (R)-2-(7-fluoro-5-(3-methylureido)-2',4'-dioxo-2,3-dihydrospiro[indene-1,5'-oxazolidine]-3'-yl)acetic acid (Int 387-2)

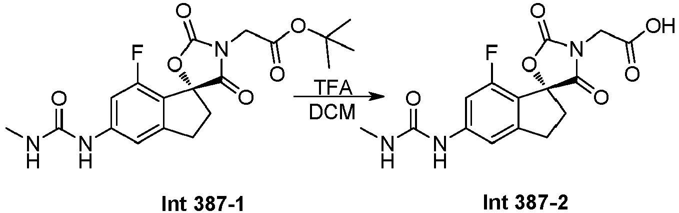

Intermediate **Int 387-1** (1.2 g, 3 mmol) was dissolved in DCM (20 mL), to which was added TFA (2 mL), and the reaction was stirred overnight at room temperature, to obtain **Int 387-2** as pale yellow solid (0.9 g), with a yield of 86%. [α]₂₀^{D} = + 67° (c 1.0, MeOH)

### Synthesis of compound 1-((R)-3'-(2-((2S,SS)-2-(3,4-difluorophenyl)-5- methylpyrrolidin-1-yl)-2-oxoethyl)-7-fluoro-2',4'-dioxo-2,3-dihydrospiro[indene-1,5'-oxazolidine]-5-yl)-3-methylurea (387)

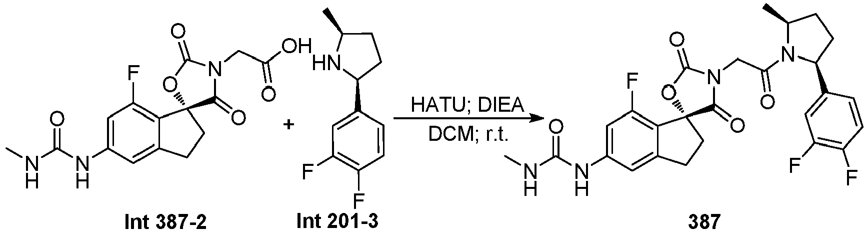

**Int 387-2** (33 mg, 0.1 mmol), **Int 201-3** (20 mg, 0.11 mmol), HATU (50 mg, 0.13 mmol), and DIEA (20 mg, 0.15 mmol) were dissolved in DCM (5 mL). The reaction was stirred overnight, concentrated, and purified by column chromatography to obtain **387** (35 mg) as pale yellow solid, with a yield of 69%. MS: *m*/*z* 531 [M+H]⁺. ¹H NMR (400 MHz, DMSO): δ 8.97 (s, 1H), 7.52 (dd, J = 18.9, 8.4 Hz, 1H), 7.42-7.29 (m, 2H), 7.23-7.12 (m, 2H), 6.20 (d, J = 4.5 Hz, 1H), 5.06 (m,1H), 4.74-4.34 (m, 2H), 3.98 (m, 1H), 3.15 (m, 1H), 3.03 (m, 1H), 2.71-2.57 (m, 4H), 2.42-2.33 (m, 1H), 2.14-1.98 (m, 1H), 1.92 (m, 1H), 1.87-1.64 (m, 1H), 1.49 (m, 1H), 1.40-1.31 (m, 3H).

### Example 388 Synthesis of compound 1-(R)-7-fluoro-3'-(2-((2S,5S)-2-methyl-5- (3,4,5-trifluorophenyl)pyrrolidin-1-yl)-2-oxoethyl)-2',4'-dioxo-2,3-dihydrospiro[indene-1,5'-oxazolidine]-5-yl)-3-methylurea (388):

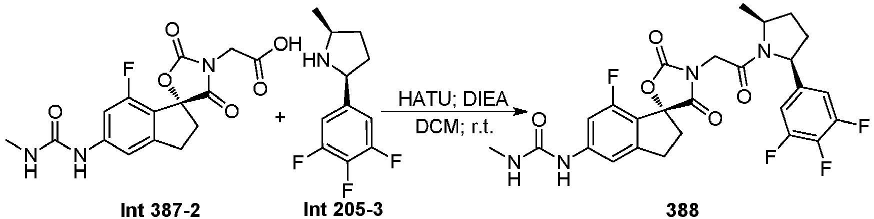

**Int 387-2** (33 mg, 0.1 mmol), **Int 205-3** (20 mg, 0.11 mmol), HATU (50 mg, 0.13 mmol), and DIEA (20 mg, 0.15 mmol) were dissolved in DCM (5 mL). The reaction was stirred overnight, concentrated, and purified by column chromatography to obtain **387** (35 mg) as pale yellow solid, with a yield of 69%. MS: *m*/*z* 549 [M+H]⁺. ¹H NMR (400 MHz, DMSO): δ 8.97 (s, 1H), 7.35-7.29 (m, 1H), 7.26 (dd, J = 13.4, 4.9 Hz, 1H), 7.15 (s, 1H), 7.10 (dd, J = 9.0, 6.9 Hz, 1H), 6.20 (d, J = 4.8 Hz, 1H), 5.27-4.85 (m, 1H), 4.76-4.37 (m, 2H), 4.20-3.57 (m, 1H), 3.15 (m, 1H), 3.08-3.00 (m, 1H), 2.67-2.55 (m, 4H), 2.42-2.31 (m, 1H), 2.17-2.05 (m, 1H), 2.02-1.91 (m, 1H), 1.88-1.74 (m, 1H), 1.48 (d, J = 6.8 Hz, 1H), 1.39-1.31 (m, 3H).

### Example 389 Synthesis of compound 1-((R)-3'-(2-((2S,5S)-2-(3,4- difluorophenyl)-5-methylpyrrolidin-1-yl)-2-oxoethyl)-6-fluoro-2',4'-dioxo-2,3-dihydrospiro[indene-1,5'-oxazolidine]-5-yl)-3-methylurea (389)

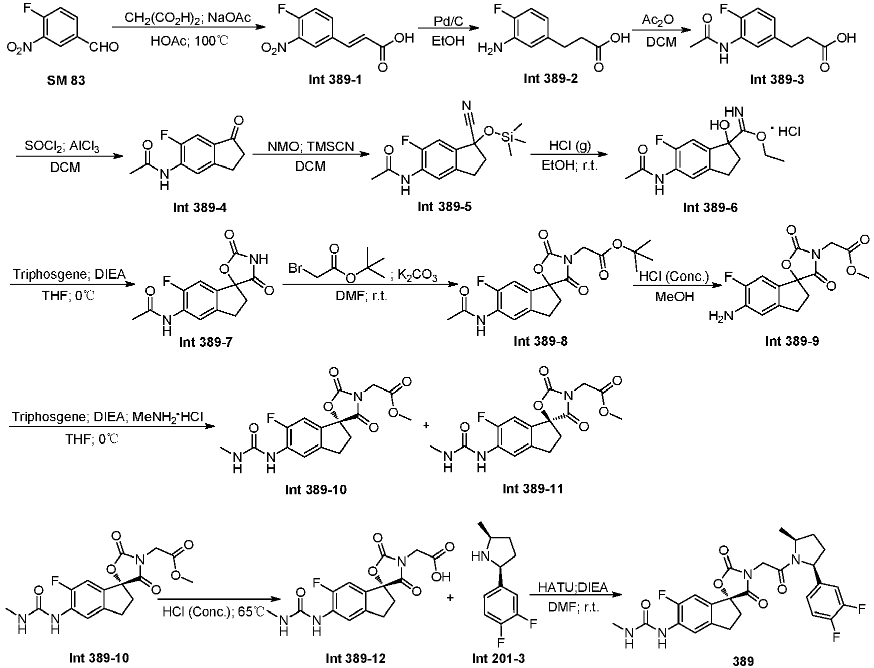

### Synthesis of 3-(4-fluoro-3-nitrophenyl)acrylic acid (Int 389-1):

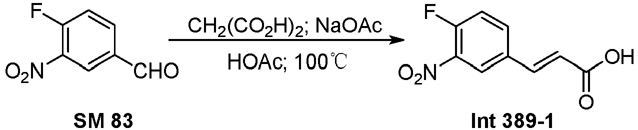

4-Fluoro-3-nitrobenzaldehyde (50 g, 295.7 mmol), malonic acid (30.75 g, 295.7 mmol), glacial acetic acid (200 mL) were added to the reaction flask, to which was added sodium acetate (7.27 g, 88.7 mmol). The mixture was heated to 100 °C and reacted for 16 h. The reaction solution was poured into hydrochloric acid solution (800 mL, 0.1 N) and filtered. The filter cake was washed with water (100 mL), then with MTBE (50 mL), and finally with ethanol (20 mL), and drained. The filter cake was dried to obtain intermediate **Int 389-1** (40 g) with a yield of 63%. MS: *m*/*z* 210 [M-H]⁻.

### Synthesis of 3-(3-amino-4-fluorophenyl)propionic acid (Int 389-2)

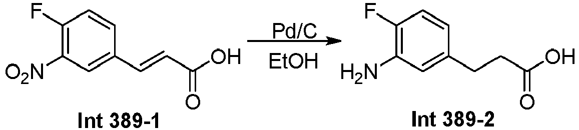

Intermediate **Int 389-1** (40 mg, 189.4 mmol) was dissolved in ethanol (600 mL), to which was added Pd/C (2 g), and the system was purged with hydrogen three times. The mixture was reacted 18 h. The solution was filtered, and the filtrate was concentrated to obtain intermediate **Int 389-2** (33 mg), with a yield of 95%. MS: *m*/*z* 184 [M+H]⁺.

### Synthesis of 3-(3-acetylamino-4-fluorophenyl) propionic acid(Int 389-3)

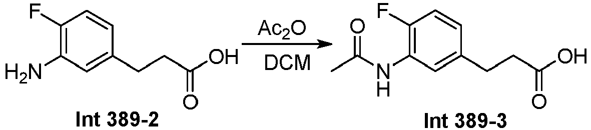

Intermediate **Int 389-2** (18.3 g, 100 mmol) was dissolved in DCM (200 mL), to which was added acetic anhydride (10.2 g, 100 mmol), and the reaction was carried out at room temperature for 2 h. Water (50 mL) was added, and the mixture was stirred for 5 min. The solution was concentrated to remove dichloromethane, and then water (100 mL) was added. The resultant solution was stirred and filtered. The filter cake was washed with water, drained, and rotatory evaporated to obtain intermediate **Int 389-3** (20.2 g), with a yield of 90%. MS: *m*/*z* 226 [M+H]⁺.

### Synthesis of N-(6-fluoro-1-oxo-2,3-dihydro-1H-inden-5-yl)acetamide (Int 389-4):

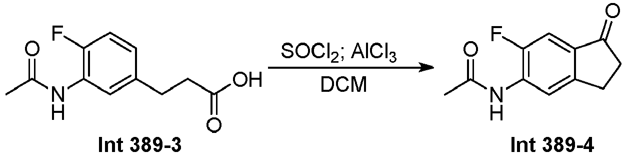

Intermediate **Int 389-3** (22.5 g, 100 mmol) was dissolved in DCM (200 mL), to which was added thionyl chloride (24 g, 200 mmol), and the mixture was reacted at room temperature for 1 h. The reaction solution was rotatory evaporated to dry, and the residue was dissolved in DCM (200 mL), to which was added anhydrous aluminum chloride (26.6 g, 200 mmol). The mixture was heated to 40° C and reacted for 16 h. The reaction solution was poured into ice water (400 mL), and extracted. The organic phase was washed with saturated brine, dried over anhydrous sodium sulfate, concentrated, and recrystallized to obtain intermediate **Int 389-4** (14.5 g), with a yield of 70%. MS: *m*/*z* 208 [M+H]⁺.

### Synthesis of N-(-cyano-6-fluoro-1-((trimethylsilyl)oxy)-2,3-dihydro-1H-inden-5-yl) acetamide (Int 389-5)

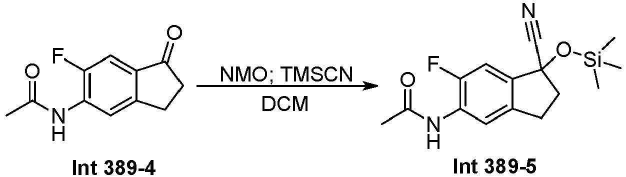

Intermediate **Int 389-4** (2.07 g, 10 mmol) was dissolved in DCM (20 mL), to which was added NMO (468 mg, 4 mmol), followed by addition of TMSCN (2 g, 20 mmol), and the mixture was reacted 18 h at room temperature. DCM was rotatory evaporated, and the residue was subjected to column chromatography to obtain intermediate **Int 389-5** (2.14 g), with a yield of 70%. MS: *m*/*z* 324 [M+18].

### Synthesis of 5-acetylamino-6-fluoro-1-hydroxyl-2,3-dihydro-1H-indene-1- carboximidic acid ethyl ester (Int 389-6):

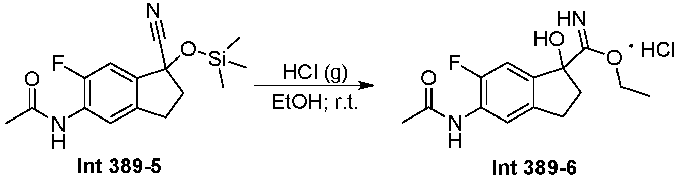

Intermediate **Int 389-5** (3.06 g, 10 mmol) was dissolved in ethanol (40 mL), and dry HCl gas was bubbled in, then the reaction was stirred overnight and detected by TLC. The reaction solution was concentrated, to obtain crude product intermediate **Int 389-6** (3 g), that was directly used in the next step.

### Synthesis of N-(6-fluoro-2',4'-dioxo-2,3-dihydrospiro[indene-1,5'-oxazolidine]-5-yl) acetamide (Int 389-7)

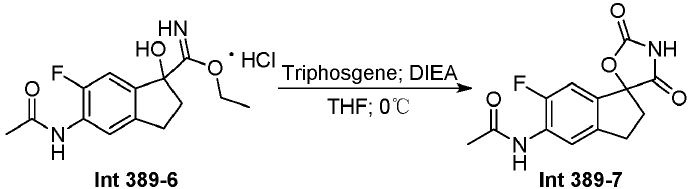

Intermediate **Int 389-6** (3.16 g, 10 mmol) was suspended in dry THF (30 mL), to which was added DIEA (20 mL; 50 mmol) in an ice bath, followed by adding triphosgene (1.5 g, 5 mmol) in batches. After that, the mixture was stirred for 2 h, and then HCl (2N) was slowly added in an ice bath until pH<5. The mixture was stirred 1h, extracted with EA, dried over anhydrous Na₂SO₄, and concentrated. The residue was recrystallized in ethyl acetate/petroleum ether, to obtain white intermediate **Int 389-7** (1.75 g), with a yield of 60%. MS: *m*/*z* 279 [M+H]⁺.

### Synthesis of 2-(5-acetylamino-6-fluoro-2',4'-dioxo-2,3-dihydrospiro[indene-1,5'-oxazolidine]-3'-yl)acetic acid t-butyl ester (Int 389-8)

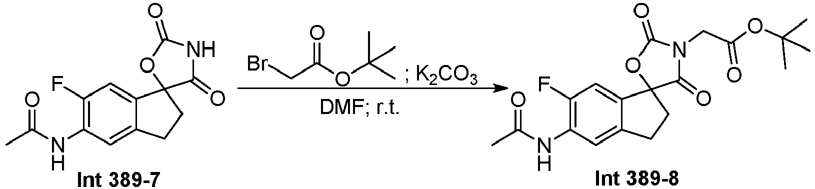

**Int 389-7** (2.78 g, 10 mmol), tert-butyl bromoacetate (1.95 g, 10 mmol), and potassium carbonate (2.76 g, 20 mmol) were added in DMF (15 mL), and then stirred at room temperature for 2 h, to which was added water (30 mL), followed by extracting with ethyl acetate three times. The organic phase was combined and dried over anhydrous Na₂SO₄, and concentrated. The residue was swirled in petroleum ether and filtered, to obtain intermediate **Int 389-8** (3.2 g), with a yield of 80%. MS: *m*/*z* 410 [M+18].

### Synthesis of 2-(5-amino-6-fluoro-2',4'-dioxo-2,3-dihydrospiro[indene-1,5'- oxazolidine]-3'-yl)acetic acid methyl ester (Int 389-9)

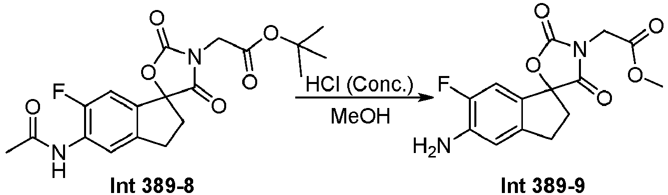

**Int 389-8** (3.92 g, 10 mmol) was dissolved in methanol (20 mL), to which was added concentrated hydrochloric acid (20 mL), and the mixture was heated to 65° C and reacted for 18 h. The solvent was rotatory evaporated, and the intermediate **Int 389-9** (2.46 g) was obtained by column chromatography, with a yield of 80%. MS: *m*/*z* 265 [M-43].

### Synthesis of (R)-2-(6-fluoro-5-(3-methylureido)-2',4'-dioxo-2,3-dihydrospiro[indene- 1,5'-oxazolidine]-3'-yl)acetic acid methyl ester (Int 389-10) and (S)-2-(6-fluoro-5- (3-methylureido)-2',4'-dioxo-2,3-dihydrospiro[indene-1,5'-oxazolidine]-3'-yl)acetic acid methyl ester (Int 389-11)

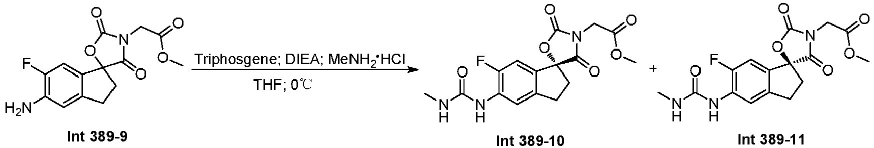

Intermediate **Int 389-6** (3.16 g, 10 mmol) was dissolved in DCM (150 mL), to which was added triphosgene (7.1 g, 24 mmol) under the condition of cooling in an ice-water bath, and the reaction was carried out 10 min. DIEA (36.1 g, 280 mmol) was added, followed by addition of methylamine hydrochloride (4.1 g, 60 mmol), and the mixture was reacted for half an hour. Water (100 mL) was added, and the organic layer was separated. The solution was extracted with dichloromethane, and the organic phases were combined, dried over anhydrous Na₂SO₄, concentrated, and subjected to column chromatography to obtain yellow solid (10.2 g), with a yield of 70%. MS: *m*/*z* 366 [M+H]⁺. **Int 389-11**(t₁, 2.3 g), e.e =99%, MS: *m*/*z* 366 [M+H]⁺.

Chiral separation conditions: apparatus: SFC-80 (Thar, Waters); chiral separation column: CHIRALCEL OD (4.6×100mm 3µm); column temperature: 35°C; mobile phase: A=CO₂, B= MEOH; peak time: t₁ = 1.848 min, t₂ = 2.228 min.

### Synthesis of (R)-2-(6-fluoro-5-(3-methylureido) -2',4'-dioxo-2,3-dihydrospiro[indene- 1,5'-oxazolidine]3'-yl)acetic acid (Int 389-12):

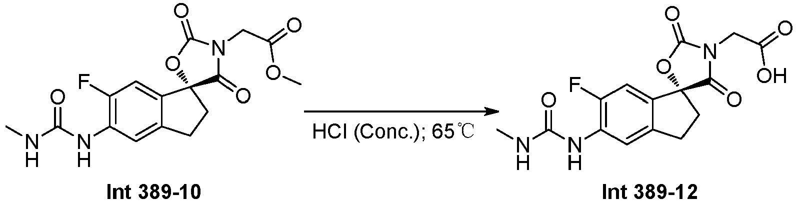

**Int 389-10** (3.65 g, 10 mmol) was dissolved in concentrated hydrochloric acid (30 mL), and the mixture was heated to 65° C and reacted for 3 h. The reaction solution was concentrated to dryness to obtain solid **Int 389-12** (3.4 g), with a yield of 96%. MS: *m*/*z* 352 [M+H]⁺. [α]₂₀^{D}= + 39.3° (c 1.0, MeOH)

### Synthesis of compound 1-((R)-3'-(2-((2S,SS)-2-(3,4-difluorophenyl)-5- methylpyrrolidin-1-yl)-2-oxoethyl)-6-fluoro-2',4'-dioxo-2,3-dihydrospiro[indene-1,5'-oxazolidine]-5-yl)-3-methylurea (389)

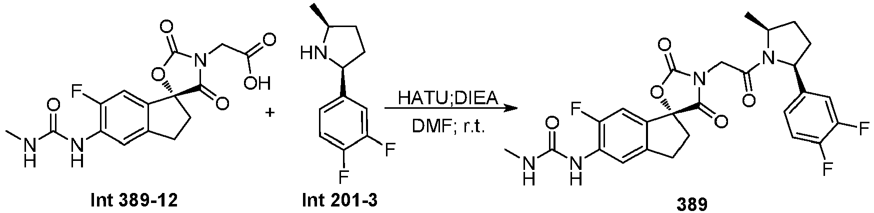

**Int 389-12** (35 mg, 0.1 mmol), **Int 201-3** (20 mg, 0.11 mmol), HATU (50 mg, 0.13 mmol), and DIEA (20 mg, 0.15 mmol) were dissolved in DMF (3 mL), and the mixture was stirred 2 h. The reaction solution was poured to water, and extracted. The organic phase was washed with saturated brine, dried with anhydrous sodium sulfate, and subjected to column chromatography to obtain white solid **389** (40 mg), with a yield of 80%. MS: *m*/*z* 531 [M+H]⁺. ¹H NMR (400 MHz, CDCl₃): δ 8.10 (m, 1H), 7.28-6.82 (m, 5H), 4.98 (m, 1H), 4.49-3.67 (m, 3H), 3.08 (m, 2H), 2.76 (m, 3H), 2.56-2.39 (m, 2H), 2.03 (m, 3H), 1.71-1.58 (m, 1H), 1.48 (m, 3H).

### Example 390 Synthesis of compound 1-((R)-6-fluoro-3'-(2-((2S,5S)-2-methyl-5- (3,4,5-trifluorophenyl)pyrrolidin-1 -yl)-2-oxoethyl)-2',4'-dioxo-2,3 -dihydrospirofindene-1,5'-oxazolidine]-5-yl)-3-methylurea (390)

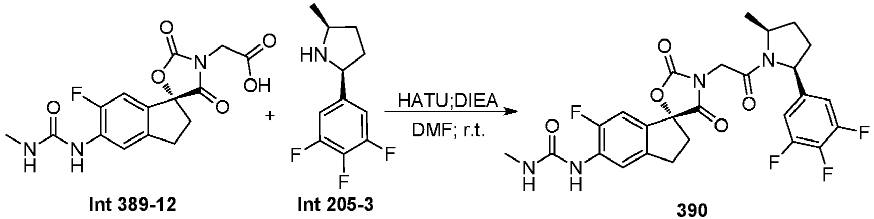

**Int 389-12** (35 mg, 0.1 mmol), **Int 205-3** (20 mg, 0.11 mmol), HATU (50 mg, 0.13 mmol), and DIEA (20 mg, 0.15 mmol) were dissolved in DMF (3 mL), and the mixture was stirred 2 h. The reaction solution was poured to water, and extracted. The organic phase was washed with saturated brine, dried over anhydrous sodium sulfate, and subjected to column chromatography, to obtain white solid **390** (40 mg), with a yield of 80%. MS: *m*/*z* 549 [M+H]⁺. ¹H NMR (400 MHz, CDCl₃): δ 8.12 (m, 1H), 7.07 (m, 3H), 6.86-6.74 (m, 1H), 5.02-4.85 (m, 1H), 4.50-3.74 (m, 3H), 3.21-2.95 (m, 2H), 2.79 (s, 3H), 2.59-2.32 (m, 2H), 2.28-1.65 (m, 4H), 1.49 (m, 3H).

### Example 391 Compound

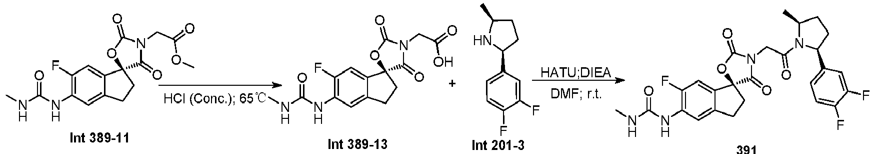

### Synthesis of intermediate (S)-2-(6-fluoro-5-(3-methylureido) -2',4'-dioxo-2,3-dihydrospiro[indene-1,5'-oxazolidine]-3'-yl)acetic acid (Int 389-13)

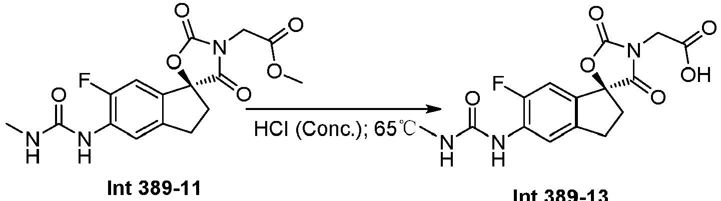

**Int 389-11** (3.65 g, 10 mmol) was dissolved in concentrated hydrochloric acid (30 mL), and the mixture was heated to 65° C and reacted for 3 h. The reaction solution was concentrated to dryness to obtain solid **Int 389-12** (3.4 g), with a yield of 96%. MS: *m*/*z* 352 [M+H]⁺. [α]₂₀^{D} = - 34.9° (c 1.0, MeOH)

### Synthesis of compound 1-((S)-3'-(2-((2S,5S)-2-(3,4-difluorophenyl)-5- methylpyrrolidin-1-yl)-2-oxoethyl)-6-fluoro-2',4'-dioxo-2,3-dihydrospiro[indene-1,5'-oxazolidine] -5-yl)-3-methylurea (391)

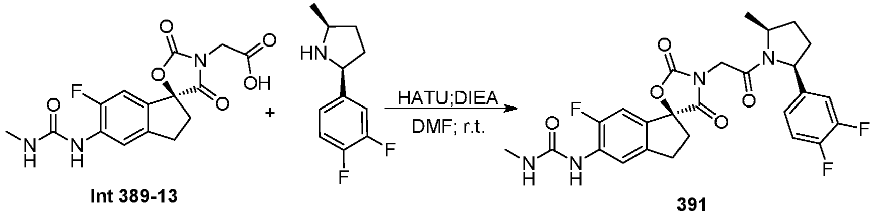

**Int 389-13** (35 mg, 0.1 mmol), **Int 201-3** (20 mg, 0.11 mmol), HATU (50 mg, 0.13 mmol), and DIEA (20 mg, 0.15 mmol) were dissolved in DMF (3 mL), and the mixture was stirred 2 h. The reaction solution was poured to water, and extracted. The organic phase was washed with saturated brine, dried over anhydrous sodium sulfate, and subjected to column chromatography, to obtain white solid **391** (40 mg), with a yield of 80%. MS: *m*/*z* 531 [M+H]⁺. ¹H NMR (400 MHz, CDCl₃): δ 8.10 (m, 1H), 7.28-6.82 (m, 5H), 4.98 (m, 1H), 4.49-3.67 (m, 3H), 3.08 (m, 2H), 2.76 (m, 3H), 2.56-2.39 (m, 2H), 2.03 (m, 3H), 1.71-1.58 (m, 1H), 1.48 (m, 3H).

### Example 392 Synthesis of 1-((S)-6-fluoro-3'-(2-((2S,5S)-2-methyl-5- (3,4,5-trifluorophenyl)pyrrolidin-1 -yl)-2-oxoethyl)-2',4'-dioxo-2,3 -dihydrospirofindene-1,5'-oxazolidine]-5-yl)-3-methylurea (392):

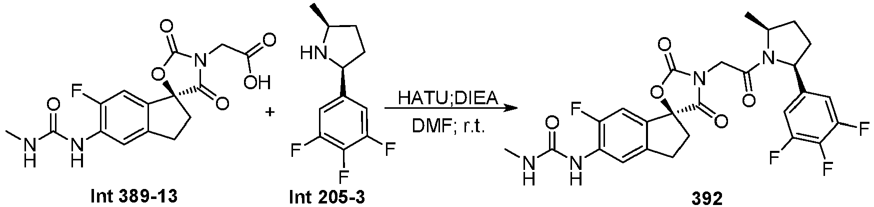

**Int 389-13** (35 mg, 0.1 mmol), **Int 205-3** (20 mg, 0.11 mmol), HATU (50 mg, 0.13 mmol), and DIEA (20 mg, 0.15 mmol) were dissolved in DMF (3 mL), and the mixture was stirred 2 h. The reaction solution was poured to water, and extracted. The organic phase was washed with saturated brine, dried over anhydrous sodium sulfate, and subjected to column chromatography, to obtain white solid **392** (40 mg), with a yield of 80%. MS: *m*/*z* 549 [M+H]⁺. ¹H NMR (400 MHz, CDCl₃): δ 8.12 (m, 1H), 7.07 (m, 3H), 6.86-6.74 (m, 1H), 5.02-4.85 (m, 1H), 4.50-3.74 (m, 3H), 3.21-2.95 (m, 2H), 2.79 (s, 3H), 2.59-2.32 (m, 2H), 2.28-1.65 (m, 4H), 1.49 (m, 3H).

The beneficial effects of the present invention was demonstrated by following experimental examples.

### Experimental example 1 Detecting the inhibitory activity of compounds on histone acetylase p300

### 1. Experimental method

Using the radioisotope FlashPlate technology, the half inhibitory concentration (IC₅₀) of the compound according to the present invention was detected against histone acetylase p300 (purchased from BPS). The test compounds were gradiently diluted to provide 10 concentrations for detection (single well), and the known histone acetylase p300 inhibitor compound C646 (CAS: 328968-36-1, purchased from Calbiochem) was used as a positive control. Under the catalysis of histone acetylase p300, [3H]-Ac group in the isotope-labeled [3H]-Ac-CoA was transferred to the biotinylated histone polypeptide substrate (purchased from GL Biochem Ltd.), and then the biotinylated histone peptide substrate bound to the streptavidin on the FlashPlate board, so that the isotope was close enough to the FlashPlate board. Thus, the radiation energy of isotope could convert the scintillation fluid coated on the FlashPlate board into photons, that was able to be detected. Detailed procedures were as follows:

### (1) Preparation of the solutions

### 1) Preparation of a reaction buffer and a reaction stop solution

1x reaction buffer: 50 mM Tris-HCl, pH 7.5; 0.01% Tween-20
Reaction stop solution: 750 µM Ac-CoA solution

### (2) Preparation of a compound solution

### 1) Preparation of compound solution

Each test compound was dissolved into 10 mM with 100% DMSO, and then each compound was diluted to the desired concentration in Echo 384-well plate. Echo550 instrument was used to transfer 200 nl compound diluted gradiently from Echo384-well plate to 384-well reaction plate, with compound C646 as a positive control. 200 nl of 100% DMSO was transferred into the well as the negative control.

### 2) Preparation of 2× enzyme solution

p300 was added to 1× reaction buffer to form a 2× enzyme solution (the final concentration of enzyme being 0.2 nM).

### 3) Preparation of 2× substrate solution

The peptide substrate and [3H]-Ac-CoA were added to the 1-fold reaction buffer, to form a 2-fold substrate solution (the final concentration of the substrate being 600 nM and 250 nM, respectively).

### (2) Experimental procedures

### 1) Adding enzyme solution to 384-well plate

10 µl of 2-fold enzyme solution was added to the wells of 384-well reaction plate. For control wells without enzyme activity, the enzyme solution was replaced with 10 µl of 1-fold reaction buffer. The plate was centrifuged at 1000 rpm for 1 min and incubated at room temperature for 15 min.

### 2) Adding the substrate solution to 384-well plate to start the enzymatic reaction

10 µl of 2-fold substrate solution was added to each well of 384-well reaction plate. The plate was centrifuged at 1000 rpm for 1 min, and then reacted at 25°C for 60 min.

### 3) Termination of the enzymatic reaction

10 µl of reaction stop solution was added to each well of 384-well reaction plate to stop the reaction. 25 µL solution was collected from each well of the test plate and transferred to Flashplate, that was placed at room temperature for 1 h. Then, Flashpate plate was washed three times with 0.1% Tween-20 solution.

### 4) Reading data with MicroBeta 2

### 5) Calculating the inhibition rate

The data were copied from Microbeta 2. According to the formula: the inhibition rate (%) = (the maximum value- the sample value)/(the maximum value - the minimum value) × 100%), the inhibition rate was calculated. The maximum value is the conversion rate of the control DMSO, and the minimum value is the conversion rate of the control without enzyme activity. The data were imported into GraphPad Prism5, and the formula "log(inhibitor) vs. response - Variable slope" was used to perform curve fitting, to obtain the half inhibitory concentration (IC₅₀).

### 2. Experimental results

**Table 1. The IC₅₀ value of each compound against P300**

| Compound No. | IC50 (µM) | Compound No. | IC₅₀ (µM) | Compound No. | IC50 (µM) | Compound No. | IC₅₀ (µM) |
|---|---|---|---|---|---|---|---|
| **14** | 0.0057 | **17** | 3.6 | **21** | 0.017 | **43** | 0.0031 |
| **48** | 0.0045 | **96** | 0.0075 | **98** | 0.026 | **114** | 0.026 |
| **115** | 0.064 | **201** | 0.0016 | **205** | 0.0016 | **209** | 0.011 |
| **212** | 0.0093 | **213** | 0.013 | **217** | 0.0055 | **225** | 0.067 |
| **319** | 0.084 | **350** | 0.033 | **362** | 0.015 | **370** | 0.103 |
| **371** | 0.0031 | **380** | 0.0051 | **389** | 0.0019 | C646 (Positive control) | 0.96 |

It could be seen that the compound prepared in the present invention could effectively inhibit histone acetylase p300, and the IC₅₀ values of the compound according to the present invention against p300 was basically below 0.01 µM, which was much lower than the positive control compound C646 (0.96 uM). Therefore, the compound of the present invention could be used to prepare an inhibitor of histone acetylase p300, and its inhibitory effect was significantly better than the known histone acetylase p300 inhibitor-compound C646.

### Experimental example 2 Biological determination of the inhibitory effect of the compound according to the present invention on the proliferation of CWR22RV1 cells

### 1. Experimental procedures:

1) CWR22RV1 cells were subcultured in cell culture medium, and the cells in good growth condition were seeded in a 96-well plate, 80 µL for each well, and the cell number in each well was 1500. The plate was incubated overnight in a 37 °C, 5% CO₂ cell incubator.
2) The drug was prepared as 30 mM stock solution with dimethyl sulfoxide (DMSO). Prior to use, the stock solution was diluted 3 times with DMSO, and then further diluted as 3-fold gradient to obtain 9 concentration gradients. Then, each concentration of compound was diluted 200 times with the culture solution (ensuring that the DMSO concentration in the culture system was 0.1%), and two replicate wells were set for each concentration. 20 µL of diluted compound solution was added to the cell culture well (with final concentrations of 10 µM, 3.3 µM, 1.1 µM...), and the plate was gently shaken to mix. In addition, 3 negative control wells containing only cells and 3 blank control wells containing only culture medium were included (6 wells were each added 20µL DMSO diluted 200 times with culture medium).

### 2. Result detection:

(1) After culturing for 6 days, 10 µL CCK-8 was added to each well, and the plate was continually cultured in a 37 °C, 5% CO₂ cell incubator for 2.5 h.
(2) The absorbance (OD value) was detected at 450 nm using a multifunctional microplate reader.
(3) The data were analyzed by Dose-response-inhibition equation in the software GraphPad Prism6, and the IC₅₀ value was obtained.

Table 2 showed the IC₅₀ (nM) of the compound according to the present invention for inhibiting the activity of CWR22RV1 cells.

A means IC₅₀ is less than or equal to 500 nM; B means IC₅₀ is greater than 500 nM and less than or equal to 2000 nM; C means IC₅₀ is greater than 2000 nM.

**Table 2 The IC₅₀ value of each compound against CWR22RV1 cells**

| Compound No. | IC₅₀ | Compound No. | IC₅₀ | Compound No. | IC₅₀ | Compound No. | IC₅₀ |
|---|---|---|---|---|---|---|---|
| **1** | C | **2** | C | **3** | C | **4** | C |
| **5** | C | **7** | C | **8** | B | **10** | C |
| **11** | B | **12** | C | **13** | C | **14** | A |
| **15** | A | **16** | C | **17** | C | **18** | A |
| **21** | A | **25** | C | **39** | B | **43** | A |
| **96** | A | **97** | C | **98** | B | **99** | C |
| **100** | C | **101** | C | **102** | C | **103** | C |
| **105** | C | **106** | C | **107** | C | **108** | C |
| **109** | C | **110** | C | **111** | C | **112** | C |
| **114** | B | **115** | C | **201** | A | **205** | A |
| **209** | A | **212** | A | **213** | A | **217** | A |
| **221** | C | **225** | B | **300** | C | **302** | C |
| **303** | B | **304** | C | **305** | C | **306** | C |
| **308** | A | **309** | B | **310** | C | **311** | C |
| **314** | C | **315** | C | **317** | C | **318** | C |
| **319** | C | **322** | C | **323** | C | **324** | C |
| **325** | C | **328** | C | **330** | C | **331** | C |
| **332** | C | **333** | C | **334** | C | **335** | C |
| **336** | C | **337** | B | **338** | C | **339** | C |
| **340** | C | **341** | C | **343** | C | **347** | B |
| **348** | A | **349** | C | **350** | B | **351** | A |
| **352** | C | **353** | A | **354** | B | **355** | C |
| **356** | C | **357** | A | **358** | B | **359** | A |
| **360** | C | **361** | B | **362** | A | **363** | C |
| **365** | B | **366** | B | **367** | B | **368** | C |
| **369** | C | **370** | C | **371** | B | **372** | C |
| **373** | C | **374** | C | **375** | C | **376** | B |
| **377** | B | **378** | C | **379** | C | **380** | A |
| **381** | C | **382** | C | **383** | C | **384** | C |
| **385** | C | **386** | C | **387** | B | **388** | C |
| **389** | A | **390** | A | **391** | C | **392** | B |

As shown, the compound prepared in the present invention had obvious inhibitory effect on human prostate cancer cell CWR22RV1, especially compounds **14**, **15**, **18**, **21, 43, 201, 205, 209, 212, 213, 217, 308, 348, 351, 353, 357, 359, 362, 380, 389, 390,** with an IC₅₀ of less than or equal to 500 nM against CWR22RV1 cells.

### Experimental example 3 Biological determination of the inhibitory effect of the compound according to the present invention on the proliferation of other tumor cells

### 1. Experimental method

Using the same method as Experimental example 2, CWR22RV1 cells were replaced with tumor cells in Table 3, and the IC₅₀ (nM) of the compound according to the present invention against the activity of these tumor cells was tested and calculated. The results were shown in Table 3.

### 2. Experimental results

A means IC₅₀ is less than or equal to 500 nM; B means IC₅₀ is greater than 500 nM and less than or equal to 2000 nM

**Table 3 The IC₅₀ of each compound against each tumor cell**

| | Cell lines | **201** | **205** | **389** | **390** |
|---|---|---|---|---|---|
| Prostate cancer | VCAP | A | A | A | A |
| | LNCAP-AR | A | A | A | A |
| | LNCAP | A | A | A | A |
| Leukemia | MOLT-4 | A | A | A | A |
| | TALL-1 | A | A | A | A |
| | HEL | B | B | A | A |
| | ATN-1 | A | A | A | A |
| | MOLM-16 | A | A | A | A |
| | OCI-AML3 | A | A | A | A |
| | MT-2 | B | B | A | B |
| Lymphoma | Pfeiffer | A | A | A | A |
| | KARPAS-422 | A | A | A | A |
| | Z-138 | A | A | A | A |
| | RL | A | A | A | A |
| | MM.1R | A | A | A | B |
| | MLMA | A | A | A | A |
| | MAVER-1 | A | A | A | A |
| | KMS-12-PE | B | B | B | B |
| Breast cancer | MDA-MB-453 | A | A | A | A |
| | MCF-7 | A | A | A | A |
| | BT-474 | A | A | A | A |
| | DU4475 | B | B | B | B |
| | HCC1428 | A | A | A | A |
| | EVSA-T | B | B | B | B |
| Multiple myeloma | KMS-20 | A | A | A | A |
| | KMS-11 | A | A | A | A |
| | MM.1S | B | B | B | B |
| | NCI-H929 | A | A | A | A |
| | OPM-2 | A | A | A | A |

As shown, the compound prepared in the present invention had an potent inhibitory effect on the proliferation of other prostate cancer cells, leukemia cells, lymphoma cells, breast cancer cells, and multiple myeloma cells. It showed that the compound of the present invention had an inhibitory effect on multiple tumors at the same time.

### Experimental example 4 Biological determination of the inhibitory effect of the compound according to the present invention in combination with CDK4/6 inhibitor palbociclib on the proliferation of CWR22RV1 cells

### 1. Experimental procedures:

The IC₅₀ values (nM) of the compound according to the present invention, palbociclib, and the combination of the compound according to the present invention and palbociclib against CWR22RV1 cell proliferation were tested and calculated, and the detailed procedures were as follows:
1) CWR22RV1 cells were subcultured in cell culture medium, and the cells in good growth condition were seeded in a 96-well plate, 60 µL for each well, and the cell number in each well was 2000. The plate was incubated overnight in a 37 °C, 5% CO₂ cell incubator.
2) The drug was prepared as 10 mM stock solution with dimethyl sulfoxide (DMSO). Prior to use, the stock solution was diluted 3 times with DMSO, and then further diluted as 3-fold gradient to obtain 9 concentration gradients. Then, each concentration of compound was diluted 200 times with the culture solution (ensuring that the DMSO concentration in the culture system was 0.1%), and two replicate wells were set for each concentration. 20 µL of diluted compound solution was added to the cell culture well (with final concentrations of 10 µM, 3.3 µM, 1.1 µM...). Palbociclib was diluted with culture medium to 500 nM, 150 nM, 50 nM, 5 nM, and 20 µL diluted compound solution was added to the corresponding cell culture wells (with a final concentration of 100 nM, 30 nM, 10 nM, 1 nM). The plate was gently shaken to mix. In addition, 3 negative control wells containing only cells and 3 blank control wells containing only culture medium were included (6 wells were each added 20 µL DMSO diluted 200 times with culture medium).

### 2. Result detection:

(1) After culturing for 6 days, 10 µL CCK-8 was added to each well, and the plate was continually cultured in a 37 °C, 5% CO₂ cell incubator for 2.5 h.
(2) The absorbance (OD value) was detected at 450 nm using a multifunctional microplate reader.
(3) The data were analyzed by Dose-response-inhibition equation in the software GraphPad Prism6, and the IC₅₀ value was obtained.

Table 4 showed the IC₅₀ (nM) of the compound according to the present invention in combination with palbociclib against the activity of CWR22RV1 cells.

**Table 4 The IC₅₀ value (nM) of the compound in combination with palbociclib against the activity of CWR22RV1 cells**

| Compound No. | IC₅₀ (nM) |
|---|---|
| **205** | 120 |
| palbociclib | 66 |
| 100 nM palbociclib + **205** | < 1 |
| 30 nM palbociclib + **205** | 40 |
| 10 nM palbociclib + **205** | 110 |
| 1 nM palbociclib + **205** | 130 |

As shown, when combined with CDK4/6 inhibitor palbociclib, the inhibitory activity of compound **205** according to the present invention on CWR22RV1 cells was much higher than that of compound **205** alone or palbociclib alone. It showed that the combination of the compound according to the present invention and palbociclib has a synergistic effect in inhibiting prostate cancer.

### Experimental example 5 Biological determination of the compound according to the present invention in combination with CDK4/6 inhibitor palbociclib against the proliferation of MCF-7 cells

### 1. Experimental method

Using the same method as Experimental example 4, CWR22RV1 cells were replaced with MCF-7 cells, and the IC₅₀ values (nM) of the compound according to the present invention, palbociclib, as well as the combination of the compound according to the present invention and palbociclib against the proliferation of MCF-7 cells were tested and calculated. The results were shown in Table 5.

### 2. Experimental results

**Table 5 The IC₅₀ values of compound in combination with palbociclib against the activity of MCF-7 cells (nM)**

| Compound No. | IC₅₀ (nM) |
|---|---|
| palbociclib | 34 |
| **205** | 81 |
| 100nM palbociclib + **205** | 1.5 |
| 30nM palbociclib + **205** | 1.6 |
| 10nM palbociclib + **205** | 19 |

As shown, when combined with CDK4/6 inhibitor palbociclib, the inhibitory activity of compound **205** according to the present invention on MCF-7 cells was much higher than that of compound **205** alone or palbociclib alone. It showed that the combination of the compound according to the present invention and palbociclib has a synergistic effect in inhibiting breast cancer.

In summary, the present invention provided compound of formula II, and this compound could effectively inhibit histone acetylase p300, and thus could effectively inhibit the proliferation of various tumor cells including prostate cancer cells, leukemia cells, lymphoma cells, breast cancer cells, multiple myeloma cells, etc.. Meanwhile, the compound of the present invention was used in combination with CDK4/6 inhibitors to play a synergistic effect in inhibiting the proliferation of tumor cells including prostate cancer cells and breast cancer cells. Therefore, the compound of the present invention had very good application prospects in the preparation of histone acetylase p300 inhibitors and drugs for prevention and/or treatment of cancer, metabolic disease, neurological disease or inflammation, as well as combination drugs.

## Claims

1. Compound of formula (**II**) or a stereoisomer, a solvate or a pharmaceutically acceptable salt, or an isotope-substituted form thereof: 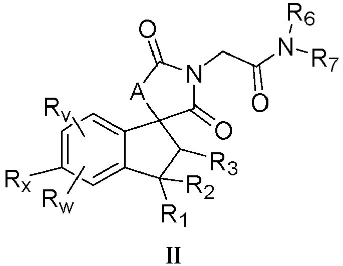
wherein, Rₓ is selected from 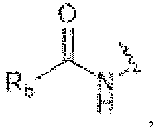 amino, 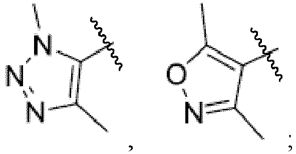 R_{b} is selected from methyl, halomethyl, -YRₐ; Rₐ is selected from methyl and cyclopropyl; Y is selected from NH or O;
Rv and Rw are independently selected from the group consisting of H, halogen, and methyl;
R₁ is selected from H, F, and CH₃; R₂ is selected from H, F, and CH₃; R₃ is H; or, R₁ and R₂ are both CH₂ and linked to form three-membered ring; or, R₂ is CH₂, R₃ is H, and linked to form three-membered ring;
A is O or S;
R₆ and R₇ are linked to form heterocyclic ring or substituted heterocyclic ring; said heterocyclic ring and substituted heterocyclic ring are a 4 to 6 membered ring, the heterocyclic ring or substituted heterocyclic ring formed by R₆ and R₇ is selected from 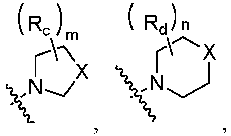 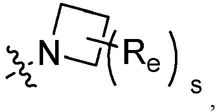 substituted or unsubstituted bridged ring, substituted or unsubstituted fused ring, and substituted or unsubstituted parallel ring, wherein X is CH₂, NH, O, S, or SO₂; each of m, n, and s is independently selected from an integer of 1 to 5; each of R_{c}, R_{d}, and Rₑ is independently selected from the group consisting of H, halogen, cyano, carboxyl, nitro, alkyl, substituted alkyl, alkoxyl, alkenyl, alkynyl, 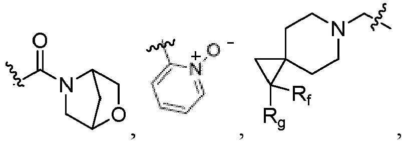 cycloalkyl, substituted cycloalkyl, heterocyclyl, substituted heterocyclyl, aryl, substituted aryl, heteroaryl, substituted heteroaryl, bridged ring, fused ring and parallel ring; R_{f} and R_{g} are halogen;
each of the substituents in said bridged ring, fused ring or parallel ring is independently selected from Boc group, fluorinated C₁₋₆ alkyl, substituted or unsubstituted heterocyclyl, and alkanoyl, preferably selected from Boc group, fluoromethyl, 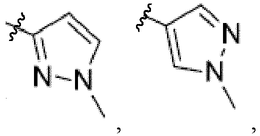 and - COCH₃;
each of the substituents in said R_{c}, R_{d}, and Rₑ is independently selected from the group consisting of halogen, C₁₋₆ alkyl, halogenated C₁₋₆ alkyl, C₁₋₆ alkoxyl, halogenated C₁₋₆ alkoxyl and hydroxyl.

2. The compound according to claim 1 or a stereoisomer, a solvate or a pharmaceutically acceptable salt, or an isotope-substituted form thereof, **characterized in that**:
the heterocyclic ring or substituted heterocyclic ring formed by linkage of said R₆ and R₇ is 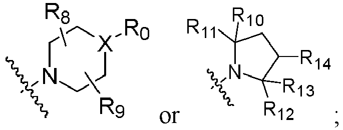
wherein, X is C, N, O, S, or SO₂; R₈ and R₉ are independently selected from the group consisting of H, alkyl, substituted alkyl, alkoxyl, alkenyl, alkynyl, cycloalkyl, substituted cycloalkyl, heterocyclyl, substituted heterocyclyl, aryl, substituted aryl, aromatic heterocyclyl, and substituted aromatic heterocyclyl;
R₀ is none, H or alkoxyl; or, R₈ and R₉ are linked to form a fused ring or bridged ring; R₁₀, R₁₁, R₁₂, R₁₃, and R₁₄ are independently selected from the group consisting of H, halogen, cyano, carboxyl, nitro, alkyl, substituted alkyl, cycloalkyl, substituted cycloalkyl, heterocyclyl, substituted heterocyclyl, aryl, substituted aryl, aromatic heterocyclyl, and substituted aromatic heterocyclyl; or, R₁₀ and R₁₁ are linked to form a ring;
each of the substituents in said R₈, R₉, R₁₀, R₁₁, R₁₂, R₁₃, and R₁₄ is independently selected from the group consisting of halogen, C₁₋₆ alkyl, halogenated C₁₋₆ alkyl, C₁₋₆ alkoxyl, halogenated C₁₋₆ alkoxyl, and hydroxyl.

3. The compound according to claim 2 or a stereoisomer, a solvate or a pharmaceutically acceptable salt, or an isotope-substituted form thereof, **characterized in that**:
X is C or O; R₈ and R₉ are independently selected from the group consisting of H, alkyl, substituted alkyl, aryl, substituted aryl, aromatic heterocyclyl, and substituted aromatic heterocyclyl;
R₁₀, R₁₁, R₁₂, R₁₃, and R₁₄ are independently selected from the group consisting of H, cyano, carboxyl, alkyl, alkenyl, substituted alkyl, cycloalkyl, substituted cycloalkyl, heterocyclyl, substituted heterocyclyl, aryl, substituted aryl, aromatic heterocyclyl, and substituted aromatic heterocyclyl; or, R₁₀ and R₁₁ are linked to form a ring when both of them are alkyl;
the substituents in said R₈, R₉, R₁₀, R₁₁, R₁₂, R₁₃, and R₁₄ are as described in claim 2.

4. The compound according to claim 3 or a stereoisomer, a solvate or a pharmaceutically acceptable salt, or an isotope-substituted form thereof, **characterized in that**:
R₈ and N are linked to the same carbon atom and is selected from phenyl and substituted phenyl; R₉ is selected from H, alkyl, and substituted alkyl;
R₁₀ and R₁₁ are independently selected from the group consisting of H, C₁₋₆ alkyl, cyano, carboxyl, substituted alkyl, C₃₋₆ cycloalkyl, and C₂₋₆ alkenyl; or, R₁₀ and R₁₁ are both CH₂, and linked to form three-membered ring;
R₁₂ and R₁₃ are independently selected from the group consisting of H, methyl, phenyl, substituted phenyl, heteroaryl, substituted heteroaryl, cycloalkyl and substituted cycloalkyl; R₁₄ is selected from H and phenyl;
the substituents in said R₈, R₉, R₁₀, R₁₁, R₁₂, R₁₃, and R₁₄ are as described in claim 7.

5. The compound according to claim 4 or a stereoisomer, a solvate or a pharmaceutically acceptable salt, or an isotope-substituted form thereof, **characterized in that**:
A is O.

6. The compound according to any one of claims 1 to 5 or a stereoisomer, a solvate or a pharmaceutically acceptable salt, or an isotope-substituted form thereof, **characterized in that** said compound has a structure of formula III: 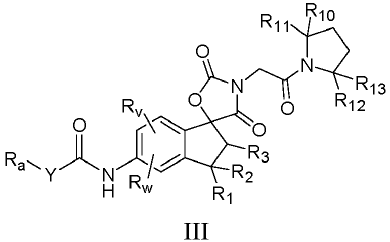
wherein Ra is methyl or cyclopropyl;
Y is NH or O;
Rᵥ and R_{w} are independently selected from the group consisting of H, halogen and methyl;
R₁ is selected from H, F, and CH₃; R₂ is selected from H, F, and CH₃; R₃ is H; or, R₁ and R₂ are both CH₂ and linked to form three-membered ring; or, R₂ is CH₂, R₃ is H, and linked to form three-membered ring;
R₁₀ and R₁₁ are independently selected from the group consisting of H, C₁₋₆ alkyl, cyano, carboxyl, substituted C₁₋₆ alkyl, C₃₋₆ cycloalkyl and C₂₋₆ alkenyl; the substituent in said C₁₋₆ alkyl is selected from halogen, hydroxyl and C₁₋₆ alkyl; or, R₁₀ and R₁₁ are both CH₂, and linked to form three-membered ring; preferably, R₁₀ and R₁₁ are independently selected from the group consisting of H, methyl, ethyl, isopropyl, cyano, carboxyl, halogenated methyl, cyclopropyl, vinyl, methoxy-substituted methyl and hydroxy-substituted methyl; or, R₁₀ and R₁₁ are both CH₂, and linked to form three-membered ring;
R₁₂ and R₁₃ are independently selected from the group consisting of H, methyl, phenyl, substituted phenyl, heteroaryl, substituted heteroaryl, cycloalkyl and substituted cycloalkyl; said cycloalkyl being a 5 to 6 membered cycloalkyl;
each of the substituents in said substituted phenyl, substituted heteroaryl, substituted cycloalkyl is independently selected from halogen, C₁₋₃ alkyl, halogenated C₁₋₃ alkyl, C₁₋₃ alkoxyl, halogenated C₁₋₃ alkoxyl and hydroxy;
the isotopic substitution form is deuterated.

7. The compound according to any one of claims 1 to 6 or a stereoisomer, a solvate or a pharmaceutically acceptable salt, or an isotope-substituted form thereof, **characterized in that** the structure of said compound is one of the following:

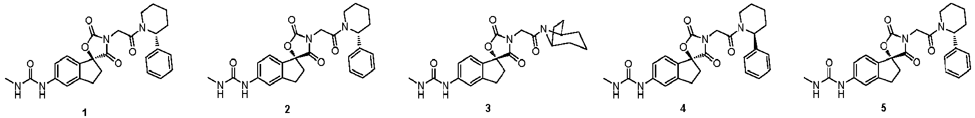

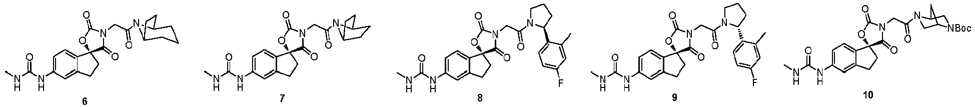

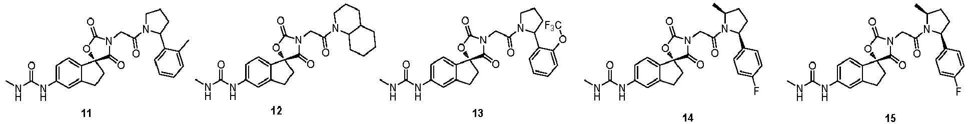

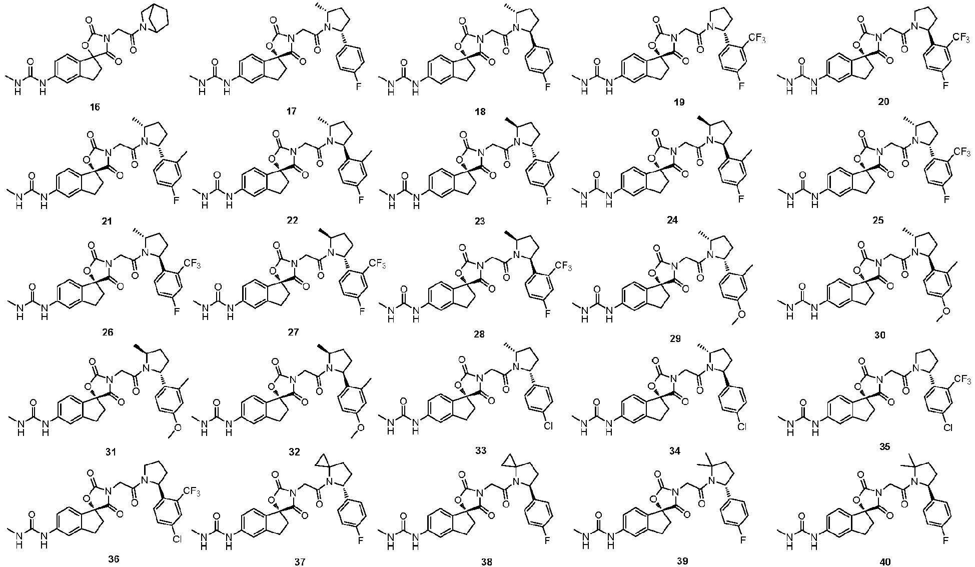

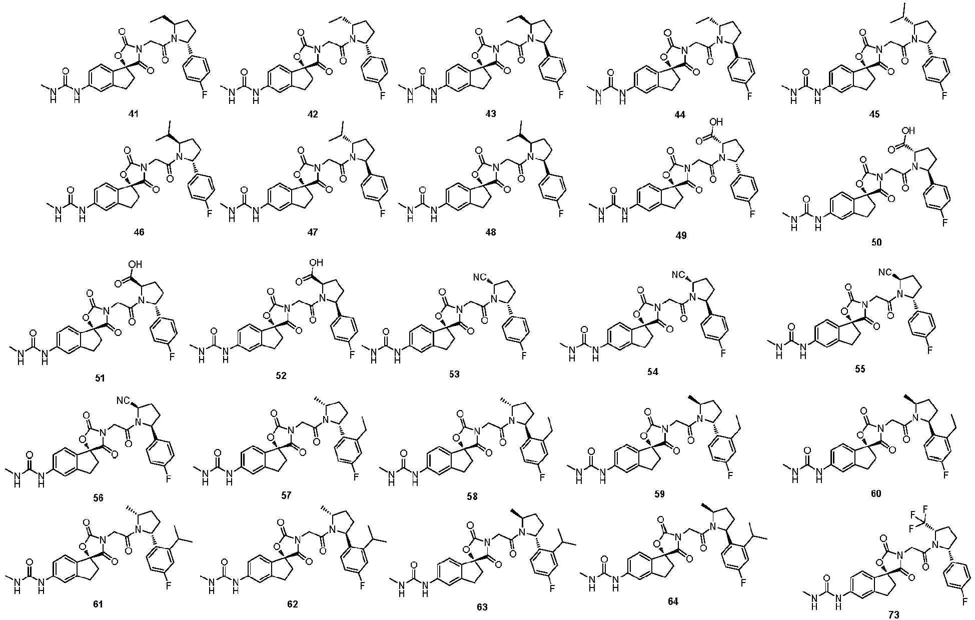

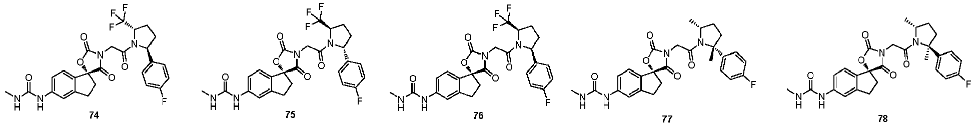

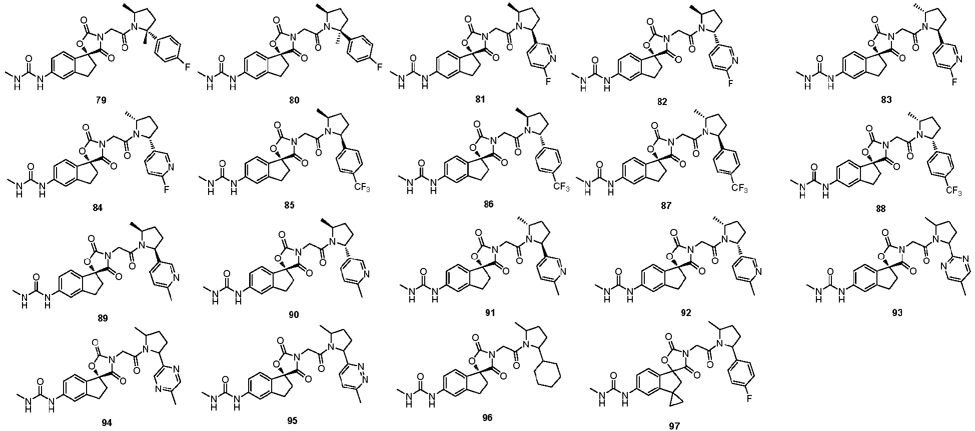

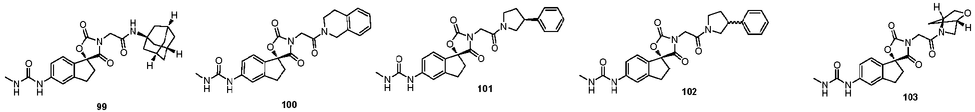

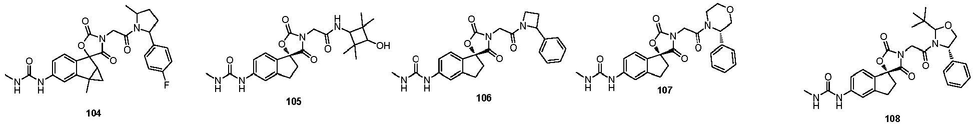

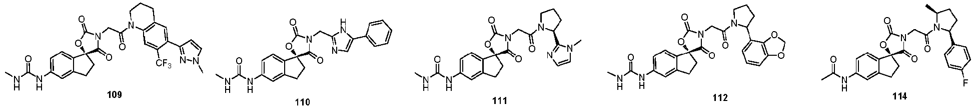

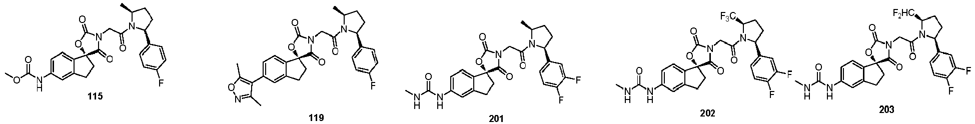

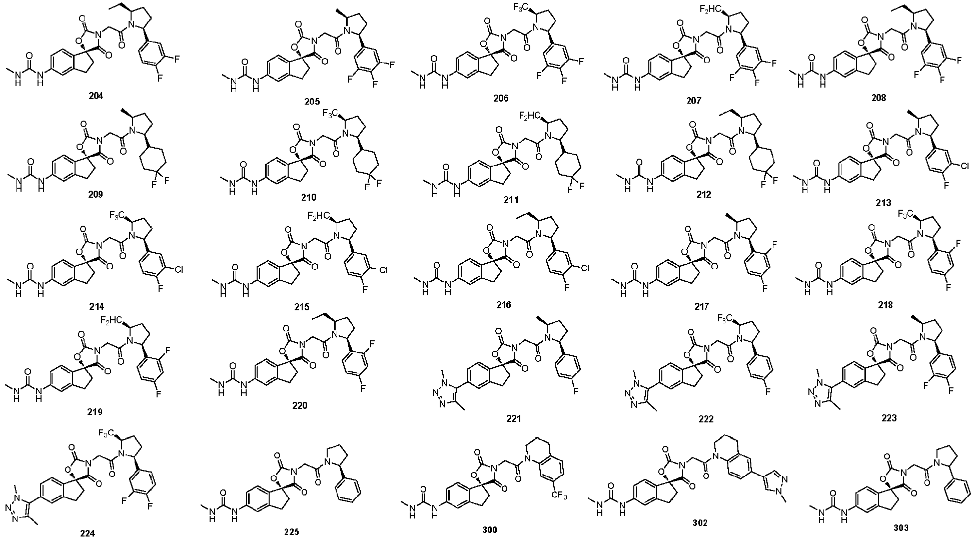

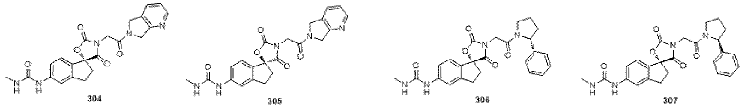

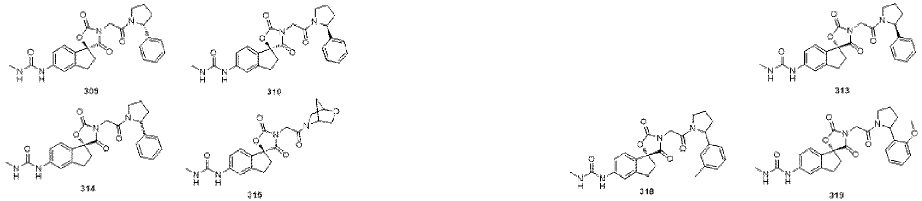

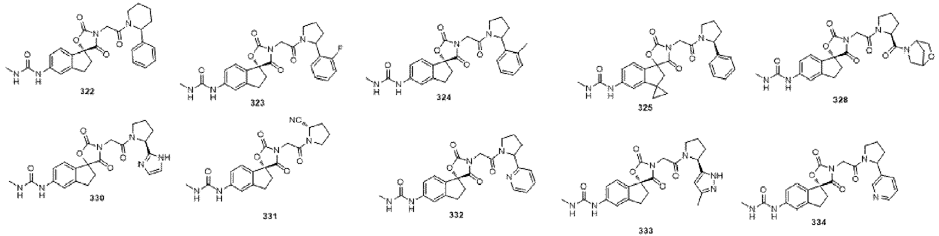

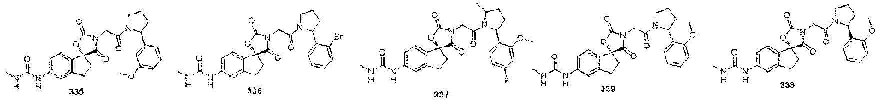

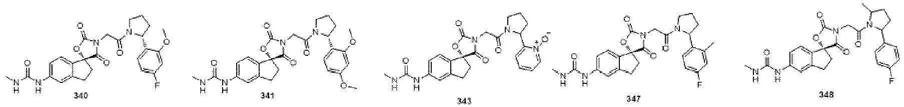

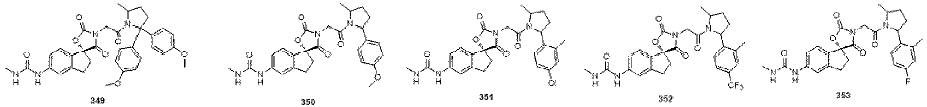

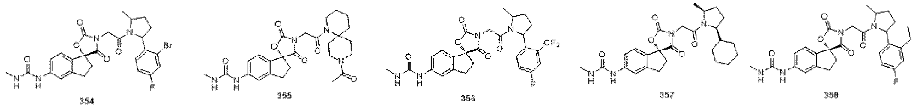

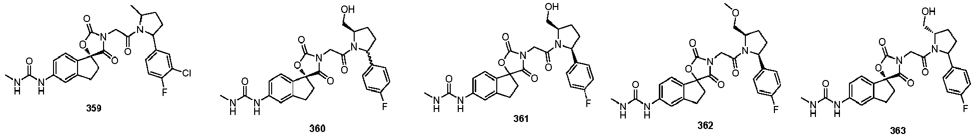

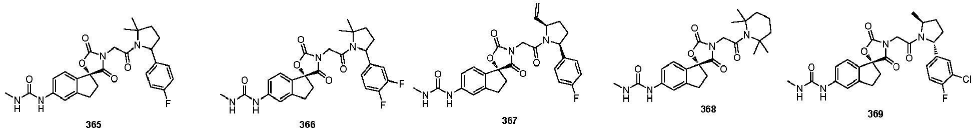

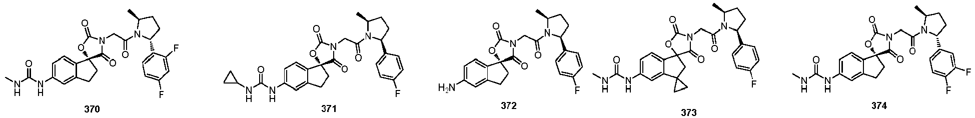

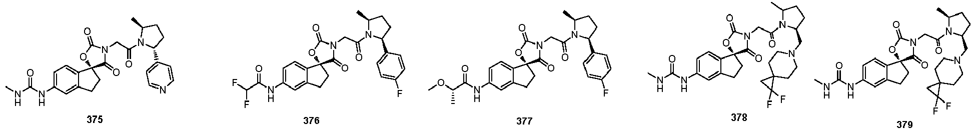

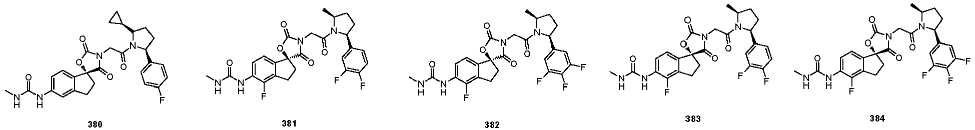

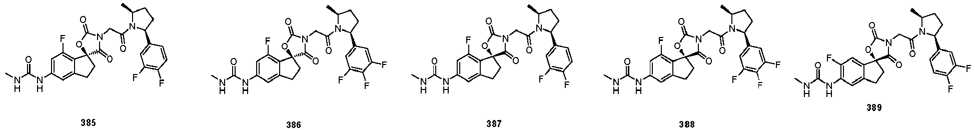

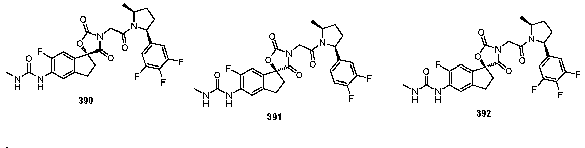

8. A method for preparing the compound according to any one of claims 1 to 7, **characterized in that** said method is: 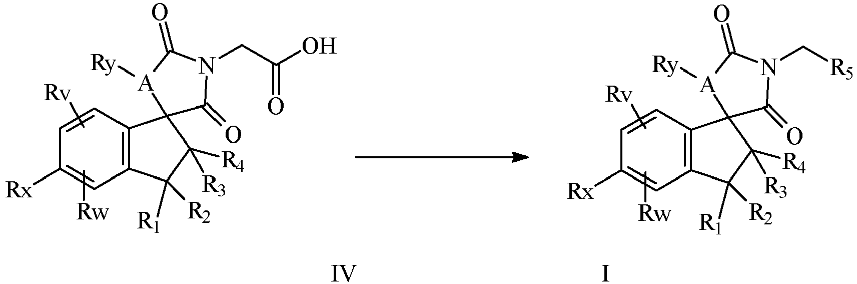
using compound of formula IV and 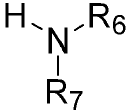 as starting materials to react, to obtain the product;
wherein, A, Ry, Rv, Rw, Rx, R₁, R₂, R₃, R₄, R₅ are as described in any one of claims 1 to 7, while R₆ and R₇ are as described in any one of claims 1 to 7;
wherein preferably
the reaction temperature is in the range of 15 to 30 °C, and the reaction time is in the range of 0.5 to 2 hours; preferably, the reaction temperature is 20 °C, and the reaction time is 1 h;
the reaction is carried out under the action of DIEA and HATU, and the molar ratio of compound of formula IV, 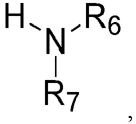 DIEA, and HATU is 1:1:3:1.

9. Use of the compound according to any one of claims 1 to 7 or a stereoisomer, a solvate or a pharmaceutically acceptable salt, or an isotope-substituted form thereof in the preparation of a medicament for the treatment of cancer, metabolic diseases, neurological diseases, and/or inflammation, wherein preferably the cancer is prostrate cancer, leukemia, lymphoma, breast cancer or multiple myeloma, wherein the medicament is a histone acetylase inhibitor, preferably being p300.

10. A compound according to any one of claims 1 to 7 or a stereoisomer, a solvate or a pharmaceutically acceptable salt, or an isotope-substituted form thereof for use as a drug for the treatment of cancer, metabolic diseases, neurological diseases and/or inflammation; wherein preferably the cancer is prostate cancer, leukemia, lymphoma, breast cancer or multiple myeloma, wherein the drug is a histone acetylase inhibitor, preferably being p300.

11. A pharmaceutical composition, **characterized in that** it is obtained by using the compound according to any one of claims 1 to 7 or a stereoisomer, a solvate or a pharmaceutically acceptable salt, or an isotope-substituted form thereof as active ingredient, with the addition of pharmaceutically acceptable excipients.

12. A composition for use as a combination drug, **characterized in that** it contains the same or different specification of unit preparations of the compound according to any one of claims 1 to 7 and an anticancer drug for simultaneous or separated administration, as well as pharmaceutically acceptable carriers;
wherein the anticancer drug preferably is a CDK4/6 inhibitor, wherein the CDK4/6 inhibitor more preferably is palbociclib.

## Patentansprüche

1. Verbindung der Formel (**II**) oder ein Stereoisomer, ein Solvat oder ein pharmazeutisch unbedenkliches Salz oder eine isotopensubstituierte Form davon: 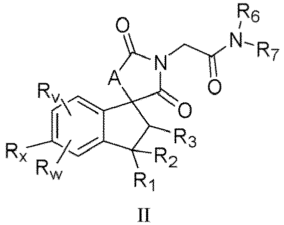
wobei Rₓ aus 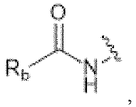 Amino, 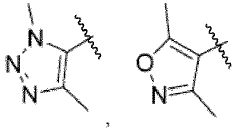 ausgewählt ist; R_{b} aus Methyl, Halogenmethyl, -YRₐ ausgewählt ist; Rₐ aus Methyl und Cyclopropyl ausgewählt ist; Y aus NH oder O ausgewählt;
Rv und Rw unabhängig aus der Gruppe bestehend aus H, Halogen und Methyl ausgewählt sind;
R₁ aus H, F und CH₃ ausgewählt ist; R₂ aus H, F und CH₃ ausgewählt ist; R₃ für H steht; oder R₁ und R₂ beide für CH₂ stehen und zu einem dreigliedrigen Ring verknüpft sind oder R₂ für CH₂ steht und R₃ für H steht und zu einem dreigliedrigen Ring verknüpft sind;
A für O oder S steht;
R₆ und R₇ zu einem heterocyclischen Ring oder substituierten heterocyclischen Ring verknüpft sind; wobei der heterocyclische Ring und der substituierte heterocyclischen ein vier- bis sechsgliedriger Ring ist, wobei der durch R₆ und R₇ gebildete heterocyclische Ring und der substituierte heterocyclische Ring aus 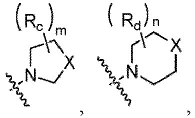 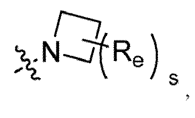 einem substituierten oder unsubstituierten verbrückten Ring, einem substituierten oder unsubstituierten anellierten Ring und einem substituierten oder unsubstituierten parallelen Ring ausgewählt ist, wobei X für CH₂, NH, O, S oder SO₂ steht; m, n und s unabhängig aus einer ganzen Zahl von 1 bis 5 ausgewählt sind; R_{c}, R_{d} und Rₑ jeweils unabhängig aus der Gruppe bestehend aus H, Halogen, Cyano, Carboxyl, Nitro, Alkyl, substituiertem Alkyl, Alkoxyl, Alkenyl, Alkinyl, 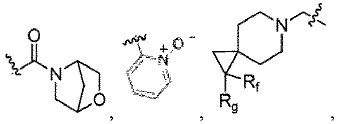 Cycloalkyl, substituiertem Cycloalkyl, Heterocyclyl, substituiertem Heterocyclyl, Aryl, substituiertem Aryl, Heteroaryl, substituiertem Heteroaryl, einem verbrückten Ring, einem anellierten Ring und einem parallelen Ring ausgewählt sind; R_{f} und R_{g} für Halogen stehen;
jeder der Substituenten in dem verbrückten Ring, anellierten Ring oder parallelen Ring unabhängig aus einer Boc-Gruppe, einem fluorierten C₁₋₆-Alkyl, einem substituierten oder unsubstituierten Heterocyclyl und Alkanoyl, vorzugsweise aus einer Boc-Gruppe, Fluormethyl, 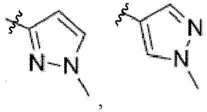 und -COCH₃ ausgewählt ist;
jeder der Substituenten in dem Rₑ, R_{d} und Rₑ unabhängig aus der Gruppe bestehend aus Halogen, C₁₋₆-Alkyl, halogeniertem C₁₋₆-Alkyl, C₁₋₆-Alkoxyl, halogeniertem C₁₋₆-Alkoxyl und Hydroxyl ausgewählt ist.

2. Verbindung nach Anspruch 1 oder ein Stereoisomer, ein Solvat oder ein pharmazeutisch unbedenkliches Salz oder eine isotopensubstituierte Form davon, **dadurch gekennzeichnet, dass**:
es sich bei dem durch Verknüpfung von R₆ und R₇ gebildeten heterocyclischen Ring oder substituierten heterocyclischen Ring um 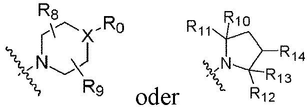 handelt;
wobei X für C, N, O, S oder SO₂ steht; R₈ und R₉ unabhängig aus der Gruppe bestehend aus H, Alkyl, substituiertem Alkyl, Alkoxyl, Alkenyl, Alkinyl, Cycloalkyl, substituiertem Cycloalkyl, Heterocyclyl, substituiertem Heterocyclyl, Aryl, substituiertem Aryl, aromatischem Heterocyclyl und substituiertem aromatischem Heterocyclyl ausgewählt sind;
Ra für nichts, H oder Alkoxyl steht; oder R₈ und R₉ zu einem anellierten Ring oder verbrückten Ring verknüpft sind; R₁₀, R₁₁, R₁₂, R₁₃ und R₁₄ unabhängig aus der Gruppe bestehend aus H, Halogen, Cyano, Carboxyl, Nitro, Alkyl, substituiertem Alkyl, Cycloalkyl, substituiertem Cycloalkyl, Heterocyclyl, substituiertem Heterocyclyl, Aryl, substituiertem Aryl, aromatischem Heterocyclyl und substituiertem aromatischem Heterocyclyl ausgewählt sind; oder R₁₀ und R₁₁ zu einem Ring verknüpft sind;
jeder der Substituenten in R₈, R₉, R₁₀, R₁₁, R₁₂, R₁₃ und R₁₄ unabhängig aus der Gruppe bestehend aus Halogen, C₁₋₆-Alkyl, halogeniertem C₁₋₆-Alkyl, C₁₋₆-Alkoxyl, halogeniertem C₁₋₆-Alkoxyl und Hydroxyl ausgewählt ist.

3. Verbindung nach Anspruch 2 oder ein Stereoisomer, ein Solvat oder ein pharmazeutisch unbedenkliches Salz oder eine isotopensubstituierte Form davon, **dadurch gekennzeichnet, dass**:
X für C oder O steht; R₈ und R₉ unabhängig aus der Gruppe bestehend aus H, Alkyl, substituiertem Alkyl, Aryl, substituiertem Aryl, aromatischem Heterocyclyl und substituiertem aromatischem Heterocyclyl ausgewählt sind;
R₁₀, R₁₁, R₁₂, R₁₃ und R₁₄ unabhängig aus der Gruppe bestehend aus H, Cyano, Carboxyl, Alkyl, Alkenyl, substituiertem Alkyl, Cycloalkyl, substituiertem Cycloalkyl, Heterocyclyl, substituiertem Heterocyclyl, Aryl, substituiertem Aryl, aromatischem Heterocyclyl und substituiertem aromatischem Heterocyclyl ausgewählt sind; oder R₁₀ und R₁₁ zu einem Ring verknüpft sind, wenn sie beide für Alkyl stehen;
die Substituenten in R₈, R₉, R₁₀, R₁₁, R₁₂, R₁₃ und R₁₄ wie in Anspruch 2 beschrieben sind.

4. Verbindung nach Anspruch 3 oder ein Stereoisomer, ein Solvat oder ein pharmazeutisch unbedenkliches Salz oder eine isotopensubstituierte Form davon, **dadurch gekennzeichnet, dass**:
R₈ und N mit demselben Kohlenstoffatom verknüpft sind und aus Phenyl und substituiertem Phenyl ausgewählt ist; R₉ aus H, Alkyl und substituiertem Alkyl ausgewählt ist;
R₁₀ und R₁₁ unabhängig aus der Gruppe bestehend aus H, C₁₋₆-Alkyl, Cyano, Carboxyl, substituiertem Alkyl, C₃₋₆-Cycloalkyl und C₂₋₆-Alkenyl ausgewählt sind; oder R₁₀ und R₁₁ beide für CH₂ stehen und zu einem dreigliedrigen Ring verknüpft sind;
R₁₂ und R₁₃ unabhängig aus der Gruppe bestehend aus H, Methyl, Phenyl, substituiertem Phenyl, Heteroaryl, substituiertem Heteroaryl, Cycloalkyl und substituiertem Cycloalkyl ausgewählt sind; R₁₄ aus H und Phenyl ausgewählt ist;
die Substituenten in R₈, R₉, R₁₀, R₁₁, R₁₂, R₁₃ und R₁₄ wie in Anspruch 7 beschrieben sind.

5. Verbindung nach Anspruch 4 oder ein Stereoisomer, ein Solvat oder ein pharmazeutisch unbedenkliches Salz oder eine isotopensubstituierte Form davon, **dadurch gekennzeichnet, dass**:
A für O steht.

6. Verbindung nach einem der Ansprüche 1 bis 5 oder ein Stereoisomer, ein Solvat oder ein pharmazeutisch unbedenkliches Salz oder eine isotopensubstituierte Form davon, **dadurch gekennzeichnet, dass** die Verbindung die Struktur der Formel III aufweist: 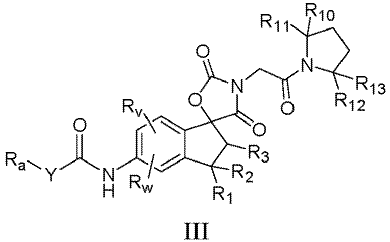
wobei Ra für Methyl oder Cyclopropyl steht;
Y für NH oder O steht;
Rᵥ und R_{w} unabhängig aus der Gruppe bestehend aus H, Halogen und Methyl ausgewählt sind;
R₁ aus H, F und CH₃ ausgewählt ist; R₂ aus H, F und CH₃ ausgewählt ist; R₃ für H steht; oder R₁ und R₂ beide für CH₂ stehen und zu einem dreigliedrigen Ring verknüpft sind; oder, R₂ für CH₂ steht und R₃ für H steht und zu einem dreigliedrigen Ring verknüpft sind;
R₁₀ und R₁₁ unabhängig aus der Gruppe bestehend aus H, C₁₋₆-Alkyl, Cyano, Carboxyl, substituiertem C₁₋₆-Alkyl, C₃₋₆-Cycloalkyl, C₂₋₆-Alkenyl ausgewählt sind; der Substituent in dem C₁₋₆-Alkyl aus Halogen, Hydroxyl, C₁₋₆-Alkyl ausgewählt ist; oder R₁₀ und R₁₁ beide für CH₂ stehen und zu einem dreigliedrigen Ring verknüpft sind;
vorzugsweise R₁₀ und R₁₁ unabhängig aus der Gruppe bestehend aus H, Methyl, Ethyl, Isopropyl, Cyano, Carboxyl, halogeniertem Methyl, Cyclopropyl, Vinyl, methoxysubstituiertem Methyl und Hydroxy substituiertem Methyl ausgewählt sind; oder R₁₀ und R₁₁ beide für CH₂ stehen und zu einem dreigliedrigen Ring verknüpft sind; R₁₂ und R₁₃ unabhängig aus der Gruppe bestehend aus H, Methyl, Phenyl, substituiertem Phenyl, Heteroaryl, substituiertem Heteroaryl, Cycloalkyl oder substituiertem Cycloalkyl ausgewählt sind; es sich bei dem Cycloalkyl um ein 5- bis 6-gliedriges Cycloalkyl handelt,
jeder der Substituenten in dem substituierten Phenyl, substituierten Heteroaryl, substituierten Cycloalkyl unabhängig aus Halogen, C₁₋₃-Alkyl, halogeniertem C₁₋₃-Alkyl, C₁₋₃-Alkoxyl, halogeniertem C₁₋₃-Alkoxyl und Hydroxy ausgewählt ist;
die isotopensubstituierte Form deuteriert ist.

7. Verbindung nach einem der Ansprüche 1 bis 6 oder ein Stereoisomer, ein Solvat oder ein pharmazeutisch unbedenkliches Salz oder eine isotopensubstituierte Form davon, **dadurch gekennzeichnet, dass** die Struktur der Verbindung eine der folgenden ist:

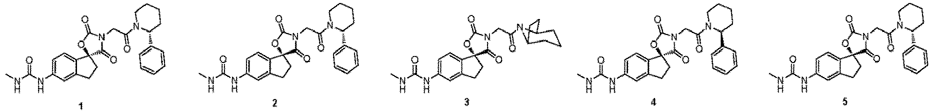

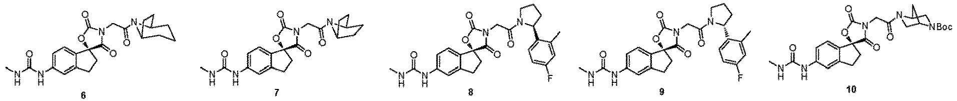

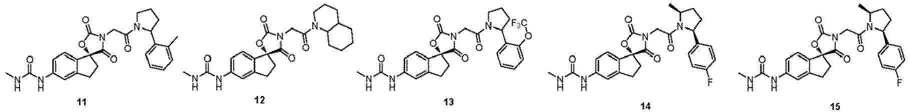

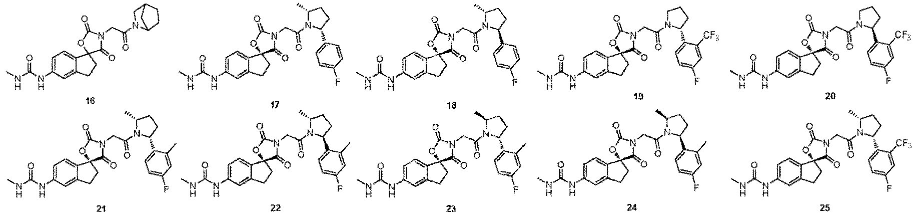

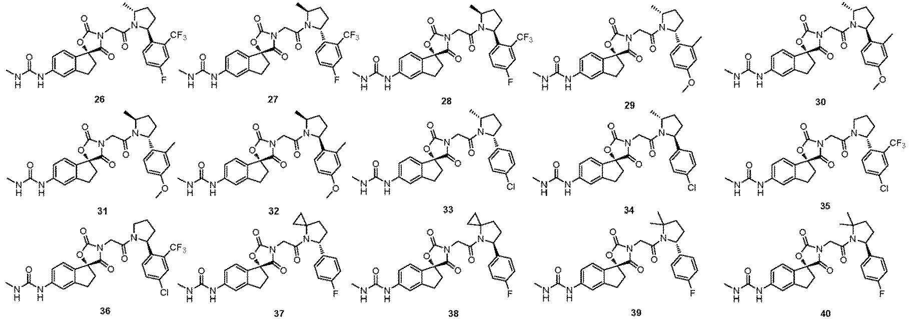

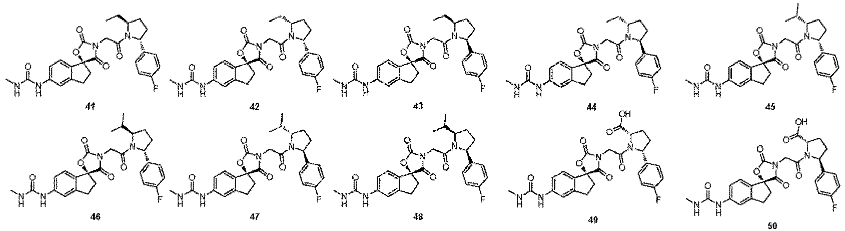

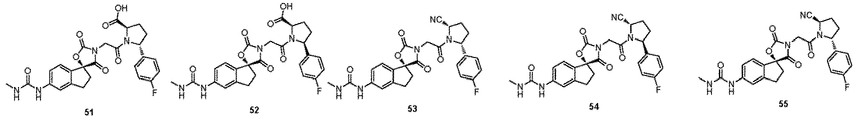

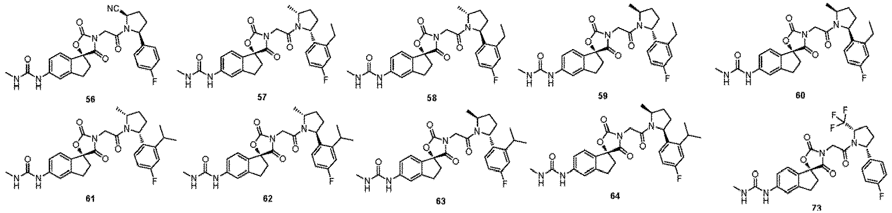

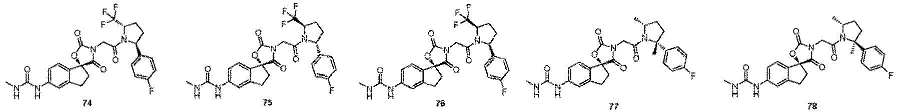

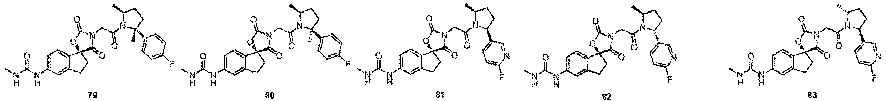

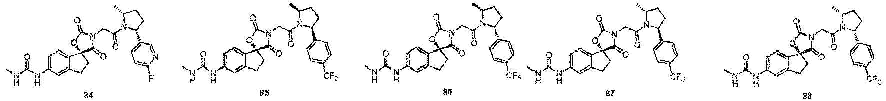

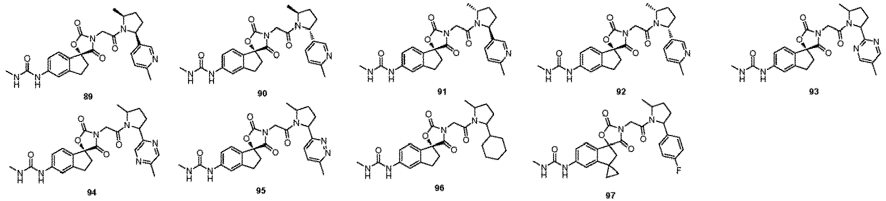

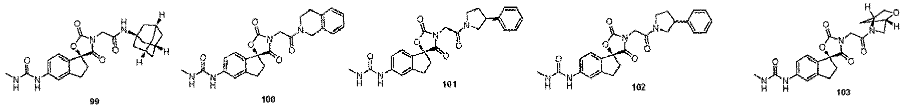

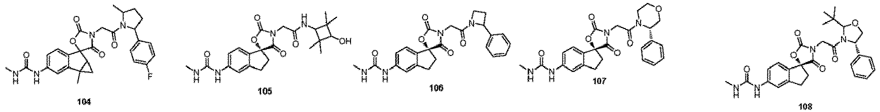

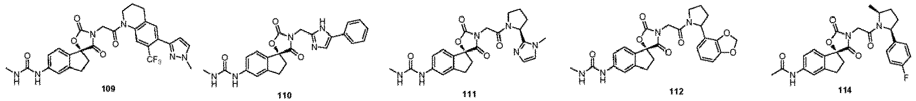

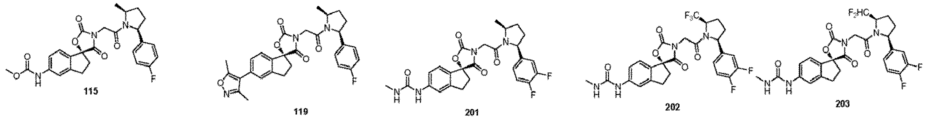

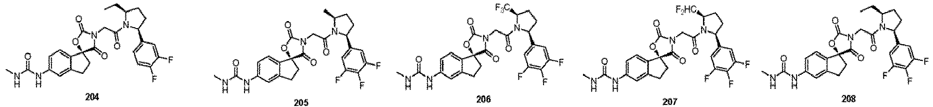

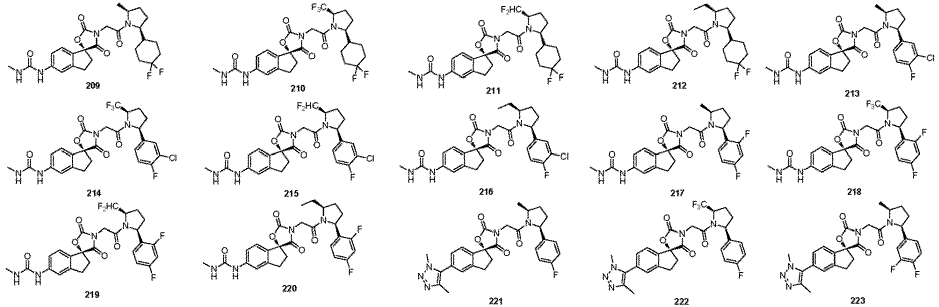

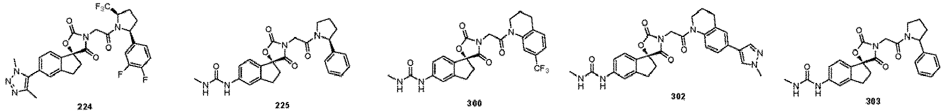

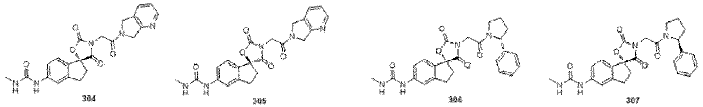

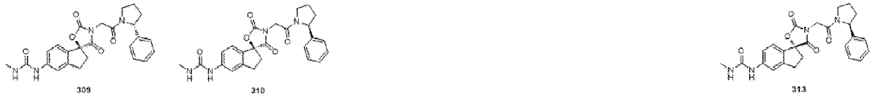

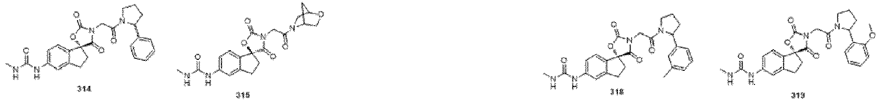

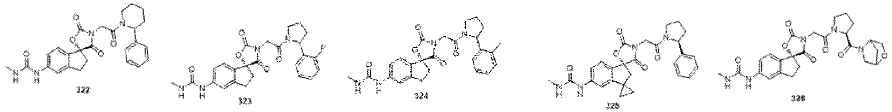

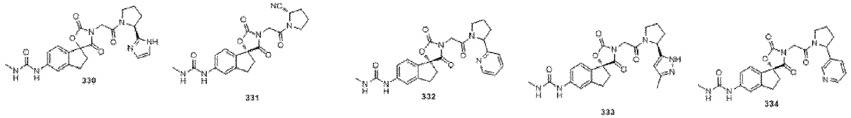

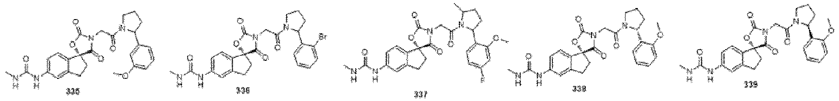

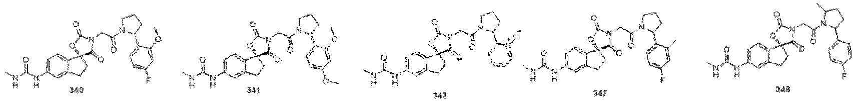

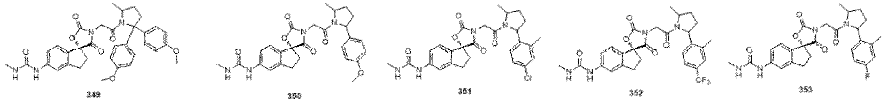

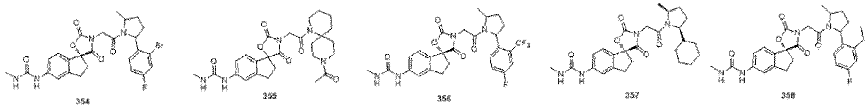

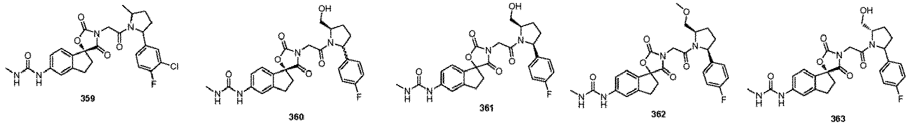

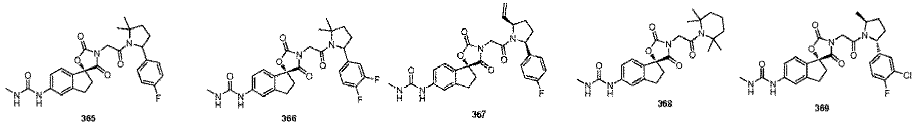

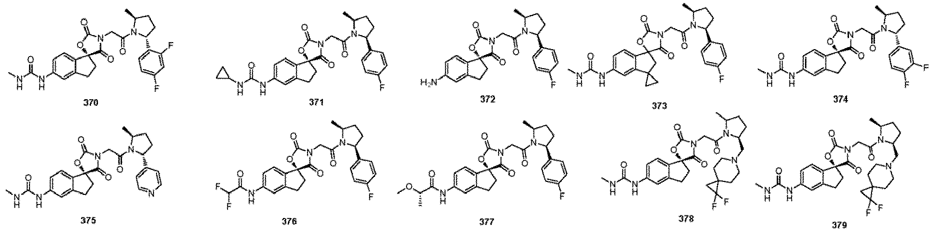

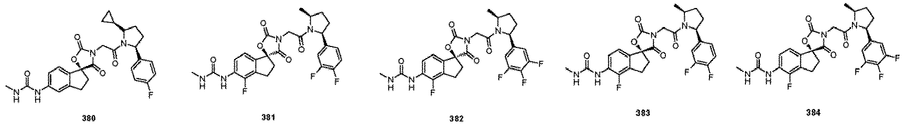

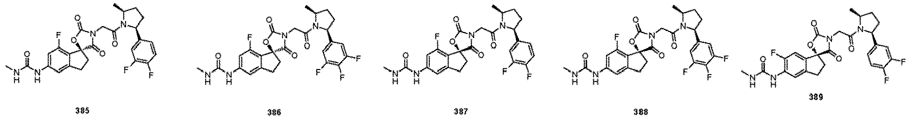

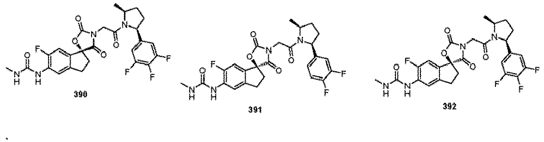

8. Verfahren zur Herstellung der Verbindung nach einem der Ansprüche 1 bis 7, **dadurch gekennzeichnet, dass** es sich bei dem Verfahren um Folgendes handelt: 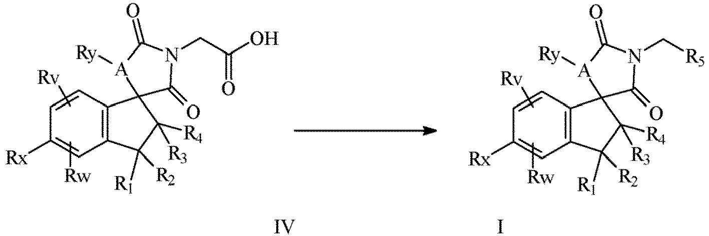 unter Verwendung der Verbindung der Formel IV und 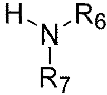 als Ausgangsstoffe für die Umsetzung zum Erhalt des Produkts;
wobei A, Ry, Rv, Rw, Rx, R₁, R₂, R₃, R₄, R₅ wie in einem der Ansprüche 1 bis 7 beschrieben sind, während R₆ und R₇ wie in einem der Ansprüche 1 bis 7 beschrieben sind;
wobei vorzugsweise
die Reaktionstemperatur im Bereich von 15 bis 30 °C liegt und die Reaktionszeit im Bereich von 0,5 bis 2 Stunden liegt; vorzugsweise die Reaktionstemperatur 20 °C beträgt und die Reaktionszeit 1 h beträgt;
die Umsetzung unter Einwirkung von DIEA und HATU durchgeführt wird und das Molverhältnis von Verbindung der Formel IV, 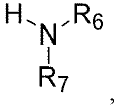 DIEA und HATU 1:1:3:1 beträgt.

9. Verwendung der Verbindung nach einem der Ansprüche 1 bis 7 oder eines Stereoisomers, eines Solvats oder eines pharmazeutisch unbedenklichen Salzes oder einer isotopensubstituierten Form davon bei der Herstellung eines Medikaments für die Behandlung von Krebs, Stoffwechselerkrankungen, neurologischen Erkrankungen und/oder Entzündung, wobei es sich bei dem Krebs vorzugsweise um Prostatakrebs, Leukämie, Lymphom, Brustkrebs oder multiples Myelom handelt, wobei es sich bei dem Medikament um einen Histonacetylase-Inhibitor handelt, bei dem es sich vorzugsweise um p300 handelt.

10. Verbindung nach einem der Ansprüche 1 bis 7 oder ein Stereoisomer, ein Solvat oder ein pharmazeutisch unbedenkliches Salz oder eine isotopensubstituierte Form davon zur Verwendung als Medikament für die Behandlung von Krebs, Stoffwechselerkrankungen, neurologischen Erkrankungen und/oder Entzündung, wobei es sich bei dem Krebs vorzugsweise um Prostatakrebs, Leukämie, Lymphom, Brustkrebs oder multiples Myelom handelt, wobei es sich bei dem Medikament um einen Histonacetylase-Inhibitor handelt, bei dem es sich vorzugsweise um p300 handelt.

11. Pharmazeutische Zusammensetzung, **dadurch gekennzeichnet, dass** sie durch Verwendung der Verbindung nach einem der Ansprüche 1 bis 7 oder eines Stereoisomers, eines Solvats oder eines pharmazeutisch unbedenklichen Salzes oder einer isotopensubstituierten Form davon als Wirkstoff unter Zugabe von pharmazeutisch unbedenklichen Hilfsstoffen erhalten wird.

12. Zusammensetzung zur Verwendung als Kombinationsmedikament, **dadurch gekennzeichnet, dass** es die gleiche Spezifikation oder verschiedene Spezifikationen von Einheitspräparaten der Verbindung nach einem der Ansprüche 1 bis 7 und eines Medikaments gegen Krebs für die gleichzeitige oder getrennte Verabreichung sowie pharmazeutisch unbedenkliche Träger enthält;
wobei es sich bei dem Mittel gegen Krebs vorzugsweise um einen CDK4/6-Inhibitor handelt, wobei es sich bei dem CDK4/6-Inhibitor weiter bevorzugt um Palbociclib handelt.

## Revendications

1. Composé de formule (II) ou sel pharmaceutiquement acceptable, stéréoisomère, solvate ou forme substituée par un isotope correspondant(e): 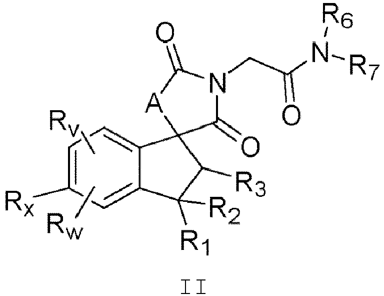
Rₓ étant choisi parmi 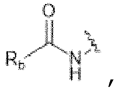 amino, 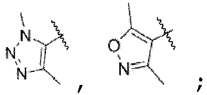 R_{b} étant choisi parmi méthyle, halogénométhyle, -YRₐ ; Rₐ étant choisi parmi méthyle et cyclopropyle ; Y étant choisi parmi NH ou O ;
Rv et Rw étant indépendamment choisis dans le groupe constitué par H, halogène, et méthyle ;
R₁ étant choisi parmi H, F, et CH₃ ; R₂ étant choisi parmi H, F, et CH₃ ; R₃ étant H ; ou, R₁ et R₂ étant tous deux CH₂ et liés pour former un cycle à trois chaînons ; ou, R₂ étant CH₂, R₃ étant H, et liés pour former un cycle à trois chaînons ;
A étant O ou S ;
R₆ et R₇ étant liés pour former un cycle hétérocyclique ou un cycle hétérocyclique substitué ; lesdits cycle hétérocyclique et cycle hétérocyclique substitué étant un cycle à 4 à 6 chaînons, le cycle hétérocyclique ou le cycle hétérocyclique substitué formé par R₆ et R₇ étant choisi parmi 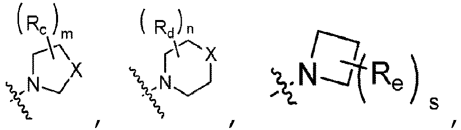 un cycle ponté substitué ou non substitué, un cycle condensé substitué ou non substitué, et un cycle parallèle substitué ou non substitué, X étant CH₂, NH, O, S, ou SO₂ ; chacun parmi m, n, et s étant indépendamment choisi parmi un entier de 1 à 5 ; chacun parmi R_{c}, R_{d}, et Rₑ étant indépendamment choisi dans le groupe constitué par H, halogène, cyano, carboxyle, nitro, alkyle, alkyle substitué, alcoxyle, alcényle, alcynyle, 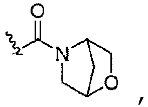 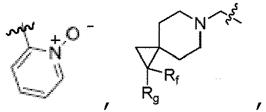 cycloalkyle, cycloalkyle substitué, hétérocyclyle, hétérocyclyle substitué, aryle, aryle substitué, hétéroaryle, hétéroaryle substitué, cycle ponté, cycle condensé et cycle parallèle ; R_{f} et R_{g} étant halogène ;
chacun des substituants dans ledit cycle ponté, cycle condensé ou cycle parallèle étant indépendamment choisi parmi un groupe Boc, C₁₋₆ alkyle fluoré, hétérocyclyle substitué ou non substitué, et alcanoyle, préférablement choisi parmi un groupe Boc, fluorométhyle, 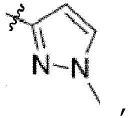 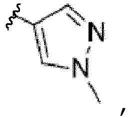 et -COCH₃ ;
chacun des substituants dans lesdits R_{c}, R_{d}, et Rₑ étant indépendamment choisi dans le groupe constitué par halogène, C₁₋₆ alkyle, C₁₋₆ alkyle halogéné, C₁₋₆ alcoxyle, C₁₋₆ alcoxyle halogéné et hydroxyle.

2. Composé selon la revendication 1 ou sel pharmaceutiquement acceptable, stéréoisomère, solvate ou forme substituée par un isotope correspondant(e), caractérisé(e) en ce que :
le cycle hétérocyclique ou le cycle hétérocyclique substitué formé par liaisons desdits R₆ et R₇ est 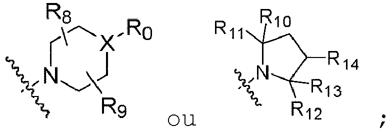
X étant C, N, O, S, ou SO₂ ; R₈ et R₉ étant indépendamment choisis dans le groupe constitué par H, alkyle, alkyle substitué, alcoxyle, alcényle, alcynyle, cycloalkyle, cycloalkyle substitué, hétérocyclyle, hétérocyclyle substitué, aryle, aryle substitué, hétérocyclyle aromatique, et hétérocyclyle aromatique substitué ;
R₀ étant rien, H ou alcoxyle ; ou, R₈ et R₉ étant liés pour former un cycle condensé ou un cycle ponté ; R₁₀, R₁₁, R₁₂, R₁₃, et R₁₄ étant indépendamment choisis dans le groupe constitué par H, halogène, cyano, carboxyle, nitro, alkyle, alkyle substitué, cycloalkyle, cycloalkyle substitué, hétérocyclyle, hétérocyclyle substitué, aryle, aryle substitué, hétérocyclyle aromatique, et hétérocyclyle aromatique substitué ; ou, R₁₀ et R₁₁ étant liés pour former un cycle ;
chacun des substituants dans lesdits R₈, R₉, R₁₀, R₁₁, R₁₂, R₁₃, et R₁₄ étant indépendamment choisi dans le groupe constitué par halogène, C₁₋₆ alkyle, C₁₋₆ alkyle halogéné, C₁₋₆ alcoxyle, C₁₋₆ alcoxyle halogéné, et hydroxyle.

3. Composé selon la revendication 2 ou sel pharmaceutiquement acceptable, stéréoisomère, solvate ou forme substituée par un isotope correspondant(e), caractérisé(e) en ce que :
X est C ou O ; R₈ et R₉ sont indépendamment choisis dans le groupe constitué par H, alkyle, alkyle substitué, aryle, aryle substitué, hétérocyclyle aromatique, et hétérocyclyle aromatique substitué ;
R₁₀, R₁₁, R₁₂, R₁₃, et R₁₄ sont indépendamment choisis dans le groupe constitué par H, cyano, carboxyle, alkyle, alcényle, alkyle substitué, cycloalkyle, cycloalkyle substitué, hétérocyclyle, hétérocyclyle substitué, aryle, aryle substitué, hétérocyclyle aromatique, et hétérocyclyle aromatique substitué ; ou, R₁₀ et R₁₁ sont liés pour former un cycle lorsque tous deux sont alkyle ;
les substituants dans lesdits R₈, R₉, R₁₀, R₁₁, R₁₂, R₁₃, et R₁₄ sont tels que décrits dans la revendication 2.

4. Composé selon la revendication 3 ou sel pharmaceutiquement acceptable, stéréoisomère, solvate ou forme substituée par un isotope correspondant(e), caractérisé(e) en ce que :
R₈ et N sont liés au même atome de carbone et sont choisis parmi phényle et phényle substitué ; R₉ est choisi parmi H, alkyle, et alkyle substitué ;
R₁₀ et R₁₁ sont indépendamment choisis dans le groupe constitué par H, C₁₋₆ alkyle, cyano, carboxyle, alkyle substitué, C₃₋₆ cycloalkyle, et C₂₋₆ alcényle ; ou, R₁₀ et R₁₁ sont tous deux CH₂, et liés pour former un cycle à trois chaînons ;
R₁₂ et R₁₃ sont indépendamment choisis dans le groupe constitué par H, méthyle, phényle, phényle substitué, hétéroaryle, hétéroaryle substitué, cycloalkyle et cycloalkyle substitué ; R₁₄ est choisi parmi H et phényle ;
les substituants dans lesdits R₈, R₉, R₁₀, R₁₁, R₁₂, R₁₃, et R₁₄ sont tels que décrits dans la revendication 7.

5. Composé selon la revendication 4 ou sel pharmaceutiquement acceptable, stéréoisomère, solvate ou forme substituée par un isotope correspondant(e), caractérisé(e) en ce que :
A est O.

6. Composé selon l'une quelconque des revendications 1 à 5 ou sel pharmaceutiquement acceptable, stéréoisomère, solvate ou forme substituée par un isotope correspondant(e), caractérisé(e) en ce que ledit composé possède une structure de formule III : 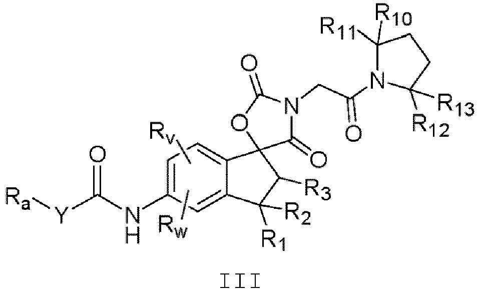
Ra étant méthyle ou cyclopropyle ;
Y étant NH ou O ;
Rᵥ et R_{w} étant indépendamment choisis dans le groupe constitué par H, halogène et méthyle ;
R₁ étant choisi parmi H, F, et CH₃ ; R₂ étant choisi parmi H, F, et CH₃ ; R₃ étant H ; ou, R₁ et R₂ étant tous deux CH₂ et liés pour former un cycle à trois chaînons ; ou, R₂ étant CH₂, R₃ étant H, et liés pour former un cycle à trois chaînons ;
R₁₀ et R₁₁ étant indépendamment choisis dans le groupe constitué par H, C₁₋₆ alkyle, cyano, carboxyle, C₁₋₆ alkyle substitué, C₃₋₆ cycloalkyle et C₂₋₆ alcényle ; le substituant dans ledit C₁₋₆ alkyle étant choisi parmi halogène, hydroxyle et C₁₋₆ alkyle ; ou, R₁₀ et R₁₁ étant tous deux CH₂, et liés pour former un cycle à trois chaînons ; préférablement, R₁₀ et R₁₁ étant indépendamment choisis dans le groupe constitué par H, méthyle, éthyle, isopropyle, cyano, carboxyle, méthyle halogéné, cyclopropyle, vinyle, méthyle substitué par méthoxy et méthyle substitué par hydroxy ; ou, R₁₀ et R₁₁ étant tous deux CH₂, et liés pour former un cycle à trois chaînons ; R₁₂ et R₁₃ étant indépendamment choisis dans le groupe constitué par H, méthyle, phényle, phényle substitué, hétéroaryle, hétéroaryle substitué, cycloalkyle et cycloalkyle substitué ; ledit cycloalkyle étant un cycloalkyle à 5 à 6 chaînons ;
chacun des substituants dans lesdits phényle substitué, hétéroaryle substitué, cycloalkyle substitué étant indépendamment choisi parmi halogène, C₁₋₃ alkyle, C₁₋₃ alkyle halogéné, C₁₋₃ alcoxyle, C₁₋₃ alcoxyle halogéné et hydroxy ;
la forme de substitution isotopique étant deutérée.

7. Composé selon l'une quelconque des revendications 1 à 6 ou sel pharmaceutiquement acceptable, stéréoisomère, solvate ou forme substituée par un isotope correspondant(e), caractérisé(e) en ce que la structure dudit composé est l'une des suivantes :

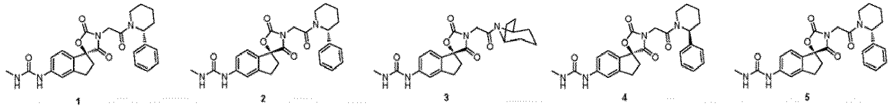

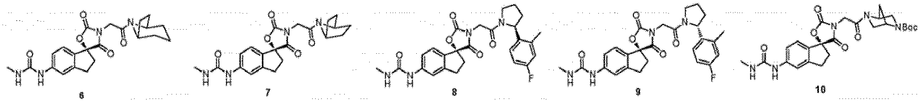

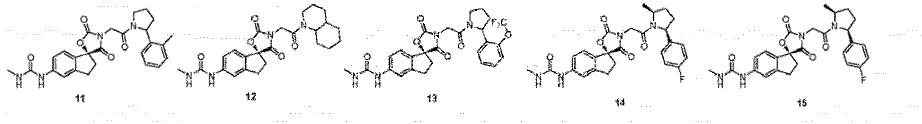

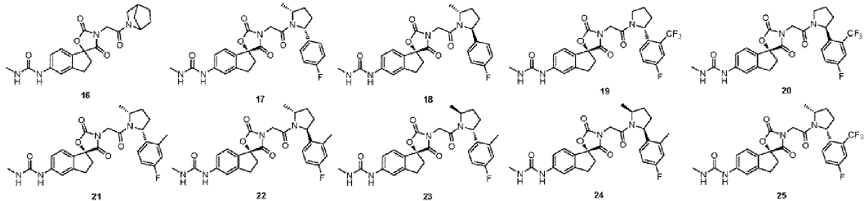

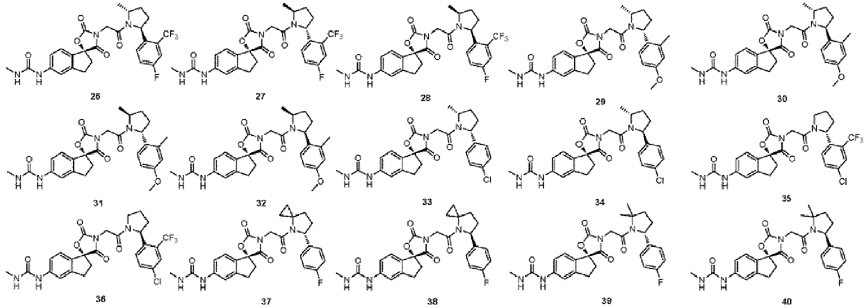

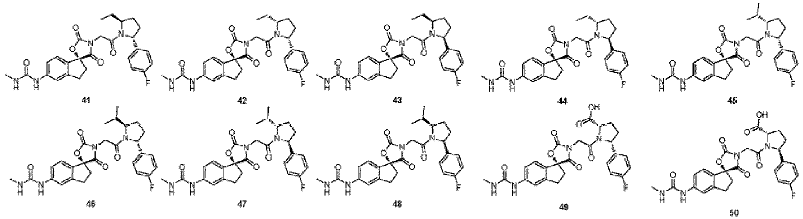

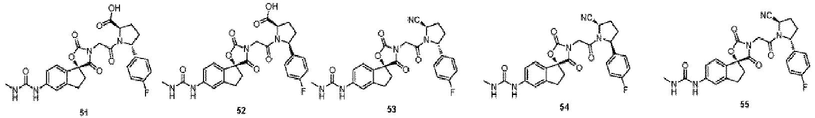

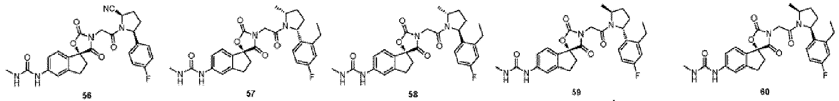

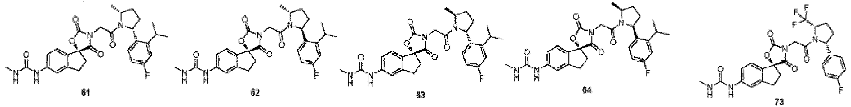

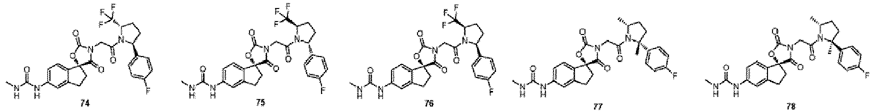

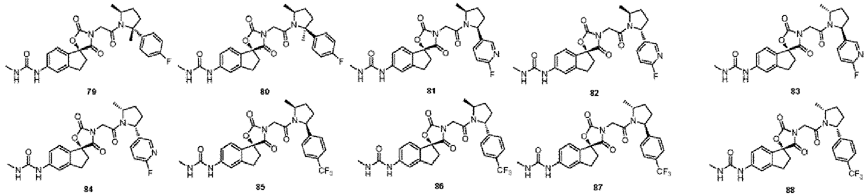

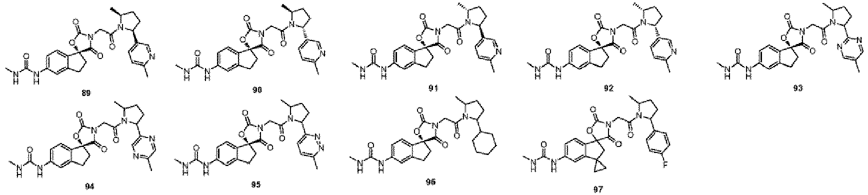

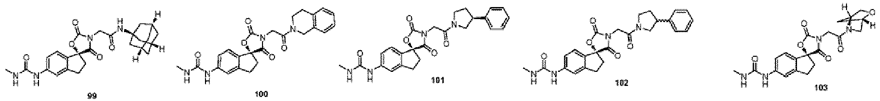

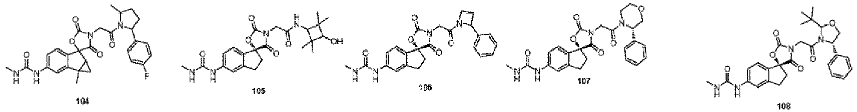

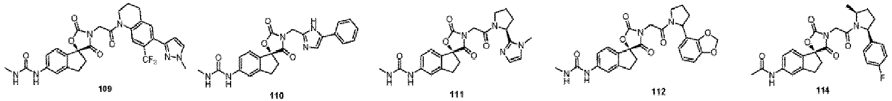

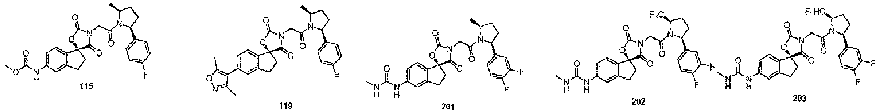

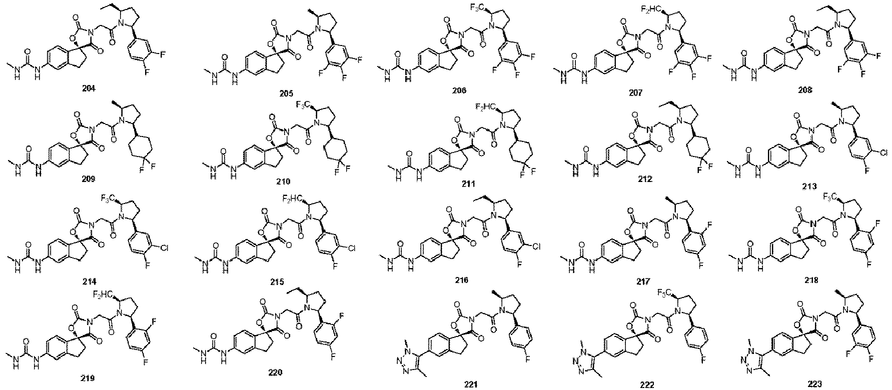

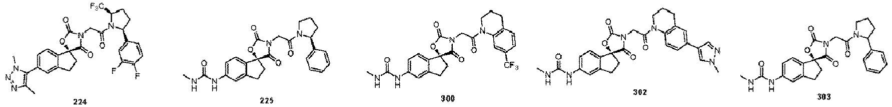

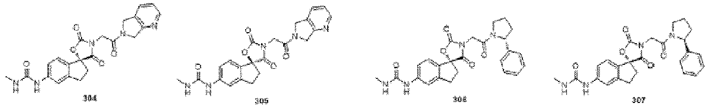

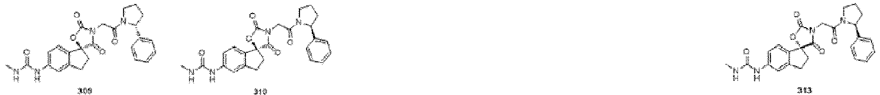

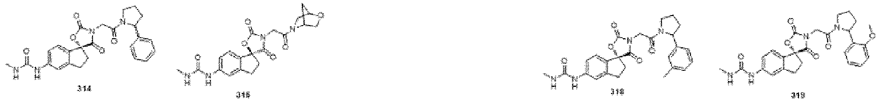

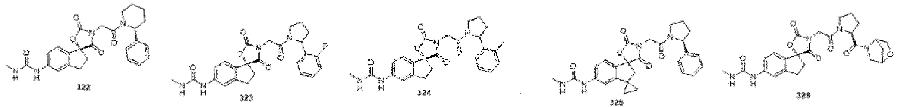

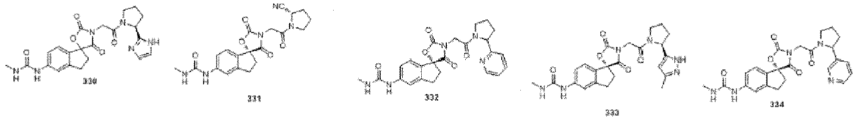

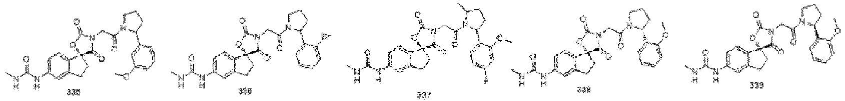

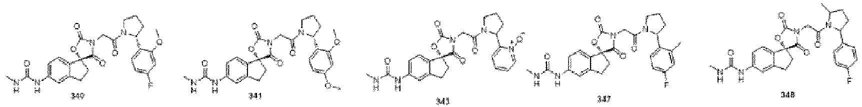

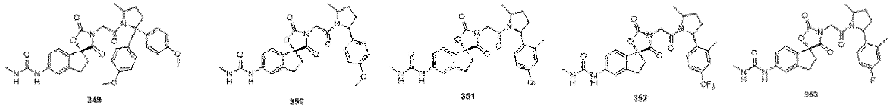

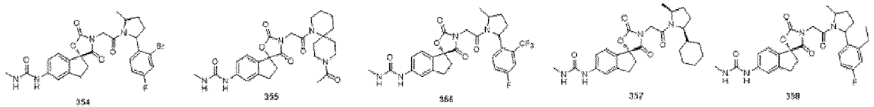

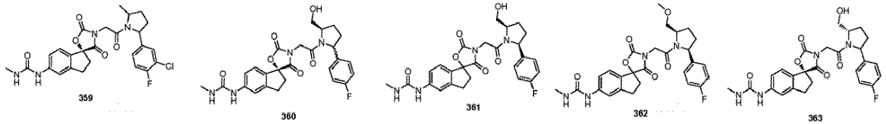

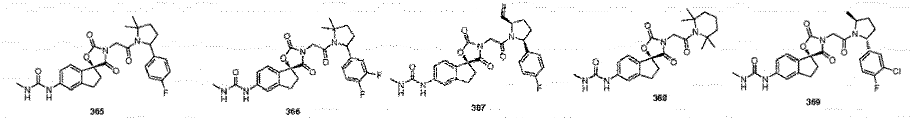

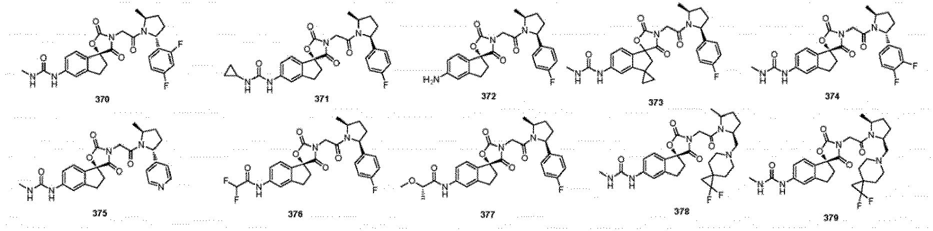

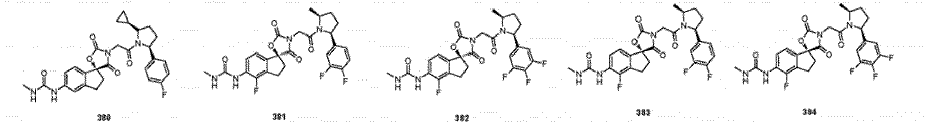

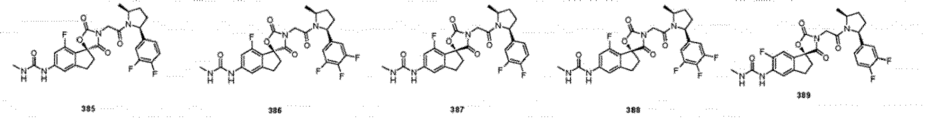

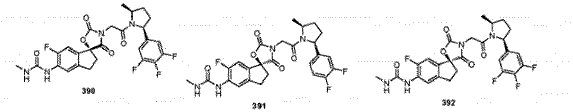

8. Procédé pour la préparation d'un composé selon l'une quelconque des revendications 1 à 7, **caractérisé en ce que** ledit procédé est : 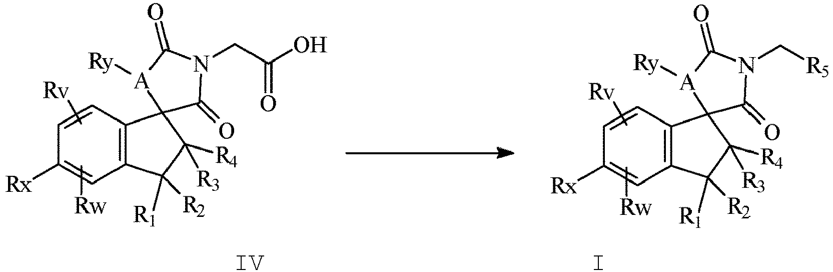 utilisant un composé de formule IV et 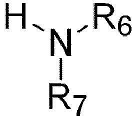 comme matières de départ pour réagir, pour obtenir le produit ;
A, Ry, Rv, Rw, Rx, R₁, R₂, R₃, R₄, R₅ étant tels que décrits dans l'une quelconque des revendications 1 à 7, tandis que R₆ et R₇ sont tels que décrits dans l'une quelconque des revendications 1 à 7 ;
préférablement
la température de réaction étant dans la plage de 15 à 30°C, et la durée de réaction étant dans la plage de 0,5 à 2 heures ; préférablement, la température de réaction étant 20°C, et la durée de réaction étant 1 h ;
la réaction étant réalisée sous l'action de DIEA et HATU, et le rapport molaire du composé de formule IV, 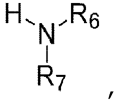 DIEA, et HATU étant 1 : 1 : 3 : 1.

9. Utilisation du composé selon l'une quelconque des revendications 1 à 7 ou d'un sel pharmaceutiquement acceptable, d'un stéréoisomère, d'un solvate ou d'une forme substituée par un isotope correspondant(e) dans la préparation d'un médicament pour le traitement d'un cancer, de maladies métaboliques, de maladies neurologiques et/ou d'une inflammation, préférablement le cancer étant le cancer de la prostate, la leucémie, le lymphome, le cancer du sein ou le myélome multiple, le médicament étant un inhibiteur de l'histone acétylase, préférablement étant p300.

10. Composé selon l'une quelconque des revendications 1 à 7 ou sel pharmaceutiquement acceptable, stéréoisomère, solvate ou forme substituée par un isotope correspondant(e) pour une utilisation comme médicament pour le traitement d'un cancer, de maladies métaboliques, de maladies neurologiques et/ou d'une inflammation ; préférablement le cancer étant le cancer de la prostate, la leucémie, le lymphome, le cancer du sein ou le myélome multiple, le médicament étant un inhibiteur de l'histone acétylase, préférablement étant p300.

11. Composition pharmaceutique, **caractérisée en ce qu'**elle est obtenue en utilisant le composé selon l'une quelconque des revendications 1 à 7 ou un sel pharmaceutiquement acceptable, un stéréoisomère, un solvate ou une forme substituée par un isotope correspondant(e) comme ingrédient actif, avec l'ajout d'excipients pharmaceutiquement acceptables.

12. Composition pour une utilisation en tant que médicament combiné, **caractérisée en ce qu'**elle contient la même spécification de préparations unitaires du composé selon l'une quelconque des revendications 1 à 7 ou une spécification différente de préparations unitaires du composé selon l'une quelconque des revendications 1 à 7 et un médicament anticancéreux pour une administration simultanée ou séparée, ainsi que des supports pharmaceutiquement acceptables ;
le médicament anticancéreux étant préférablement un inhibiteur de CDK4/6, l'inhibiteur de CDK4/6 étant plus préférablement le palbociclib.
